(19) Europäisches Patentamt
European Patent Office
Office européen des brevets

(11) EP 1 996 596 B1

(12) EUROPÄISCHE PATENTSCHRIFT

(45) Veröffentlichungstag und Bekanntmachung des Hinweises auf die Patenterteilung:
**08.07.2015 Patentblatt 2015/28**

(21) Anmeldenummer: **07711873.5**

(22) Anmeldetag: **09.03.2007**

(51) Int Cl.:
***C07D 513/04*** (2006.01) ***A61K 31/429*** (2006.01)
***A61P 25/00*** (2006.01)

(86) Internationale Anmeldenummer:
**PCT/EP2007/002067**

(87) Internationale Veröffentlichungsnummer:
**WO 2007/104485 (20.09.2007 Gazette 2007/38)**

(54) **SUBSTITUIERTE IMIDAZO(2,1-B)THIAZOL-VERBINDUNGEN UND IHRE VERWENDUNG ZUR HERSTELLUNG VON ARZNEIMITTELN**

SUBSTITUTED IMIDAZO(2,1-B)THIAZOLE COMPOUNDS AND THEIR USE FOR PRODUCING DRUGS

COMPOSÉS IMIDAZO(2,1-B)THIAZOLE SUBSTITUÉS ET UTILISATION DANS LA FABRICATION DE MÉDICAMENTS

(84) Benannte Vertragsstaaten:
**AT BE BG CH CY CZ DE DK EE ES FI FR GB GR HU IE IS IT LI LT LU LV MC MT NL PL PT RO SE SI SK TR**
Benannte Erstreckungsstaaten:
**HR**

(30) Priorität: **10.03.2006 DE 102006011574**

(43) Veröffentlichungstag der Anmeldung:
**03.12.2008 Patentblatt 2008/49**

(73) Patentinhaber: **Grünenthal GmbH**
**52078 Aachen (DE)**

(72) Erfinder:
- **KÜHNERT, Sven**
  **52355 Düren (DE)**
- **ZEMOLKA, Saskia**
  **52066 Aachen (DE)**
- **HAURAND, Michael**
  **52078 Aachen (DE)**
- **SCHIENE, Dr. Klaus**
  **41363 Jüchen (DE)**

(74) Vertreter: **Brosch, Oliver et al**
**Kutzenberger Wolff & Partner**
**Theodor-Heuss-Ring 23**
**50668 Köln (DE)**

(56) Entgegenhaltungen:
**WO-A-01/27118 WO-A-2006/029980**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die vorliegende Erfindung betrifft substituierte Imidazo[2,1-b]thiazol-Verbindungen, Verfahren zu ihrer Herstellung, Arzneimittel enthaltend diese Verbindungen sowie deren Verwendung zur Herstellung von Arzneimitteln.

Schmerz gehört zu den Basissymptomen in der Klinik. Es besteht ein weltweiter Bedarf an wirksamen Schmerztherapien. Der dringende Handlungsbedarf für eine patientengerechte und zielorientierte Behandlung chronischer und nichtchronischer Schmerzzustände, wobei hierunter die erfolgreiche und zufriedenstellende Schmerzbehandlung für den Patienten zu verstehen ist, dokumentiert sich auch in der großen Anzahl von wissenschaftlichen Arbeiten, die auf dem Gebiet der angewandten Analgetik bzw. der Grundlagenforschung zur Nozizeption in letzter Zeit erschienen sind.

Klassische Opioide, wie beispielsweise Morphin, sind bei der Therapie starker bis sehr starker Schmerzen wirksam, führen jedoch oftmals zu unerwünschten Begleiterscheinungen wie beispielsweise Atemdepression, Erbrechen, Sedierung, Obstipation oder Toleranzentwicklung. Des Weiteren sind sie bei neuropathischen Schmerzen, unter denen insbesondere Tumorpatienten leiden, häufig nicht ausreichend wirksam.

Die WO 01/27118A beschreibt bizyklische Imidazo-5-yl-amine sowie Arzneimittel enthaltend diese Verbindungen.

Eine Aufgabe der vorliegenden Erfindung bestand daher darin, neue Verbindungen zur Verfügung zu stellen, die sich insbesondere als pharmazeutische Wirkstoffe in Arzneimitteln eignen, bevorzugt in Arzneimitteln zur Behandlung von Schmerzen.

Es wurde nun überraschenderweise gefunden, dass sich die substituierten Imidazo[2,1-b]thiazol-Verbindungen der nachstehend angegebenen allgemeinen Formel I zur mGluR5-Rezeptor-Regulation (mGluR5 = metabotroper Glutamatrezeptor 5) eignen und daher insbesondere als pharmazeutische Wirkstoffe in Arzneimitteln zur Prophylaxe und/oder Behandlung von mit diesen Rezeptoren bzw. Prozessen in Verbindung stehenden Störungen oder Erkrankungen eingesetzt werden können.

Ein Gegenstand der vorliegenden Erfindung sind daher substituierte Imidazo[2,1-b]thiazol-Verbindungen der allgemeinen Formel I,

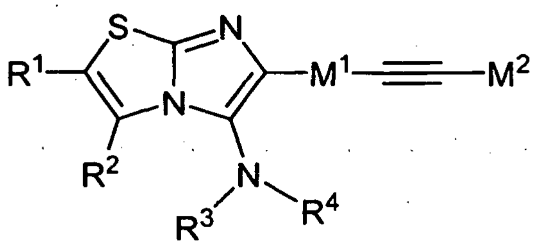

**I,**

worin

$R^1$ und $R^2$, unabhängig voneinander, jeweils für einen Wasserstoff-Rest; für einen Halogen-Rest; $-NO_2$; -CN; $-NH_2$; $-NHR^5$; $-NR^6R^7$; $-NH-C(=O)-R^8$; $-C(=O)-R^9$, $-C(=O)-NH_2$; $-C(=O)-NHR^{10}$; $-C(=O)-NR^{11}R^{12}$; $-C(=O)-OR^{13}$; $-(CH_2)_m-C(=O)-OR^{14}$ mit m = 1, 2, 3, 4 oder 5; $-O-C(=O)-R^{15}$; $-(CH_2)_n-O-C(=O)-R^{16}$ mit n = 1, 2, 3, 4 oder 5; $-OR^{17}$;$-(CH_2)_o-O-R^{18}$ mit o = 1, 2, 3; 4 oder 5; $-SR^{19}$; $-(CH_2)_p-S(=O)_t-R^{20}$ mit p = 1, 2, 3, 4 oder 5 und t = 0, 1 oder 2; $-NH-S(=O)_2-NR^{27}R^{28}$; $-S(=O)_2-NR^{29}R^{30}$; $-SF_5$; $-(CH_2)_u-O-S(=O)_2-R^{31}$ mit u = 1, 2, 3, 4 oder 5; $-(CH_2)_v-O-S(=O)_2-O-R^{32}$ mit v = 1, 2, 3, 4 oder 5; $-(CH_2)_w-O-P(=O)(OR^{33})(OR^{34})$ mit w = 1, 2, 3, 4 oder 5; für einen linearen oder verzweigten, gesättigten oder ungesättigten, unsubstituierten oder wenigstens einfach substituierten aliphatischen Rest; für einen gesättigten oder ungesättigten, unsubstituierten oder wenigstens einfach substituierten, ggf. wenigstens ein Heteroatom als Ringglied aufweisenden cycloaliphatischen Rest, der über eine lineare oder verzweigte, unsubstituierte oder wenigstens einfach substituierte Alkylen-Gruppe gebunden und/oder mit einem unsubstituierten oder wenigstens einfach substituierten mono- oder polyzyklischen Ringsystem kondensiert sein kann; oder für einen unsubstituierten oder wenigstens einfach substituierten Aryl- oder Heteroaryl-Rest, der über eine lineare oder verzweigte, unsubstituierte oder wenigstens einfach substituierte Alkylen-Gruppe gebunden und/oder mit einem unsubstituierten oder wenigstens einfach substituierten mono- oder polyzyklischen Ringsystem kondensiert sein kann, stehen;

$R^3$ und $R^4$, unabhängig voneinander, jeweils für einen Wasserstoff-Rest;- $C(=O)-R^{21}$; $-(CH_2)_q-C(=O)-R^{22}$ mit q = 1, 2, 3, 4 oder 5; $-C(=O)-O-R^{23}$; $-(CH_2)_r-C(=O)-O-R^{24}$ mit r = 1, 2, 3, 4 oder 5; $-C(=O)-NHR^{25}$; $-(CH_2)_s-C(=O)-NHR^{26}$

mit s = 1, 2, 3, 4 oder 5; für einen linearen oder verzweigten, gesättigten oder ungesättigten, unsubstituierten oder wenigstens einfach substituierten aliphatischen Rest; für einen gesättigten oder ungesättigten, unsubstituierten oder wenigstens einfach substituierten, ggf. wenigstens ein Heteroatom als Ringglied aufweisenden cycloaliphatischen Rest, der über eine lineare oder verzweigte, unsubstituierte oder wenigstens einfach substituierte Alkylen-Gruppe gebunden und/oder mit einem unsubstituierten oder wenigstens einfach substituierten mono- oder polyzyklischen Ringsystem kondensiert sein kann; oder für einen unsubstituierten oder wenigstens einfach substituierten Aryl- oder Heteroaryl-Rest, der über eine lineare oder verzweigte, unsubstituierte oder wenigstens einfach substituierte Alkylen-Gruppe gebunden und/oder mit einem unsubstituierten oder wenigstens einfach substituierten mono- oder polyzyklischen Ringsystem kondensiert sein kann, stehen,

oder $R^3$ und $R^4$ zusammen mit dem sie verbindenden Stickstoffatom als Ringglied einen gesättigten oder ungesättigten, unsubstituierten oder wenigstens einfach substituierten ggf. wenigstens ein weiteres Heteroatom als Ringglied aufweisenden heterocycloaliphatischen Rest bilden, der mit einem unsubstituierten oder wenigstens einfach substituierten mono- oder polyzyklischen Ringsystem kondensiert sein kann;

$R^5$, $R^6$, $R^7$, $R^8$, $R^{10}$, $R^{11}$, $R^{12}$, $R^{15}$ und $R^{16}$, jeweils unabhängig voneinander, für einen linearen oder verzweigten, gesättigten oder ungesättigten, unsubstituierten oder wenigstens einfach substituierten aliphatischen Rest oder für einen unsubstituierten oder wenigstens einfach substituierten Aryl- oder Heteroaryl-Rest, der über eine lineare oder verzweigte, unsubstituierte oder wenigstens einfach substituierte Alkylen-Gruppe gebunden und/oder mit einem unsubstituierten oder wenigstens einfach substituierten mono- oder polyzyklischen Ringsystem kondensiert sein kann, stehen;

$R^9$, $R^{13}$, $R^{14}$, $R^{17}$, $R^{18}$, $R^{19}$, $R^{20}$, $R^{21}$, $R^{22}$, $R^{23}$, $R^{24}$, $R^{25}$, $R^{26}$, $R^{27}$, $R^{28}$, $R^{29}$, $R^{30}$, $R^{31}$, $R^{32}$, $R^{33}$ und $R^{34}$, jeweils unabhängig voneinander, für einen Wasserstoff-Rest; für einen linearen oder verzweigten, gesättigten oder ungesättigten, unsubstituierten oder wenigstens einfach substituierten aliphatischen Rest oder für einen unsubstituierten oder wenigstens einfach substituierten Aryl- oder Heteroaryl-Rest, der über eine lineare oder verzweigte, unsubstituierte oder wenigstens einfach substituierte Alkylen-Gruppe gebunden und/oder mit einem unsubstituierten oder wenigstens einfach substituierten mono- oder polyzyklischen Ringsystem kondensiert sein kann, stehen;

$M^1$ für einen Aryl- oder Heteroaryl-Rest steht, der mit wenigstens einem weiteren Substituenten substituiert und/oder mit einem unsubstituierten oder wenigstens einfach substituierten mono- oder polyzyklischen Ringsystem kondensiert sein kann;

und $M^2$ für einen Aryl- oder Heteroaryl-Rest steht, der unsubstituiert oder wenigstens einfach substituiert und mit einem unsubstituierten oder wenigstens einfach substituierten mono- oder polyzyklischen Ringsystem kondensiert sein kann;

jeweils in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Sofern einer oder mehrere der vorstehend genannten Substituenten für einen gesättigten oder ungesättigten aliphatischen Rest, d.h. für einen Alkyl-, Alkenyl- oder Alkinyl-Rest, stehen, der einfach oder mehrfach substituiert ist, kann dieser bevorzugt mit ggf. 1, 2, 3, 4 oder 5, besonders bevorzugt mit 1, 2 oder 3, Substituenten unabhängig voneinander ausgewählt aus der Gruppe bestehend aus F, Cl, Br, I, $-NO_2$, -CN, -OH, -C(=O)-OH, -C(=O)-O-($C_{1-5}$-Alkyl), -SH, $-NH_2$, -N($C_{1-5}$-Alkyl)$_2$, -N($C_{1-5}$-Alkyl)(Phenyl), -N($C_{1-5}$-Alkyl)($CH_2$-Phenyl), -N($C_{1-5}$-Alkyl)($CH_2$-$CH_2$-Phenyl), -C(=S)-$C_{1-5}$-Alkyl, -C(=S)-Phenyl und $-SO_3H$ substituiert sein, wobei die vorstehend genannten $C_{1-5}$-Alkyl-Reste jeweils linear oder verzweigt sein können und die vorstehend genannten Phenyl-Reste bevorzugt mit 1, 2, 3, 4 oder 5 Substituenten unabhängig voneinander ausgewählt aus der Gruppe bestehend aus F, Cl, Br, I, -CN, $-CF_3$, -OH, $-NH_2$, $-O-CF_3$, -SH, $-O-CH_3$, $-O-C_2H_5$, $-O-C_3H_7$, -C(=O)-OH, $-C(=O)-O-CH_3$, $-C(=O)-O-C_2H_5$, Methyl, Ethyl, n-Propyl, Isopropyl, n-Butyl, 2-Butyl, Isobutyl und tert-Butyl substituiert sein können.

Besonders bevorzugt kann ein aliphatischer Rest, d.h. ein Alkyl-, Alkenyl- oder Alkinyl-Rest, mit 1, 2 oder 3 Substituenten unabhängig voneinander ausgewählt werden aus der Gruppe bestehend aus F, Cl, Br, I, $-NO_2$, -CN, -OH, -C(=O)-OH,$-C(=O)-O-CH_3$, -SH, $-NH_2$, $-N(CH_3)_2$. $-N(C_2H_5)_2$ und $-N(CH_3)(C_2H_5)$ substituiert sein.

Alkenyl-Reste weisen wenigstens eine, vorzugsweise 1, 2, 3 oder 4 C-C-Doppelbindungen und Alkinyl-Reste wenigstens eine, vorzugsweise 1, 2, 3 oder 4 C-C-Dreifachbindungen auf.

Als geeignete $C_{1-10}$-Alkyl-Reste, die unsubstituiert oder einfach oder mehrfach substituiert sein können, seien

beispielsweise Methyl, Ethyl, n-Propyl, Isopropyl, n-Butyl, Isobutyl, 2-Butyl, tert-Butyl, n-Pentyl, 2-Pentyl, 3-Pentyl, iso-Pentyl, neo-Pentyl, n-Hexyl, 2-Hexyl, 3-Hexyl, n-Heptyl, n-Octyl, (2,4,4)-Trimethylpent-2-yl, $-C(H)(C_2H_5)_2$, $-C(H)(n-C_3H_7)_2$ und $-CH_2-CH_2-C(H)(CH_3)-(CH_2)_3-CH_3$ genannt.

Unter mehrfach substituierten Alkyl-Resten sind solche Alkyl-Reste zu verstehen, die entweder an verschiedenen oder an gleichen C-Atomen mehrfach, bevorzugt zwei- oder dreifach, substituiert sind, beispielsweise dreifach am gleichen C-Atom wie im Fall von $-CF_3$ oder an verschiedenen Stellen wie im Fall von $-(CHCl)-(CH_2F)$. Die Mehrfachsubstitution kann mit dem gleichen oder mit verschiedenen Substituenten erfolgen. Als geeignete substituierte Alkyl-Reste seien beispielsweise $-CF_3$, $-CF_2H$,$-CFH_2$, $-(CH_2)-OH$, $-(CH_2)-NH_2$, $-(CH_2)-CN$, $-(CH_2)-(CF_3)$, $-(CH_2)-(CHF_2)$, $-(CH_2)-(CH_2F)$, $-(CH_2)-(CH_2)-OH$, $-(CH_2)-(CH_2)-NH_2$, $-(CH_2)-(CH_2)-CN$, $-(CF_2)-(CF_3)$, $-(CH_2)-(CH_2)-(CF_3)$ und $-(CH_2)-(CH_2)-(CH_2)-OH$ genannt.

Als geeignete $C_{2-6}$-Alkenyl-Reste seien beispielsweise Ethenyl, 1-Propenyl, 2-Propenyl, 1-Butenyl, 2-Butenyl, 3-Butenyl, 1-Pentenyl, 2-Pentenyl, 3-Pentenyl, 4-Pentenyl, Hexenyl, $-CH=CH-CH=CH-CH_3$ und $-CH_2-CH_2-CH=CH_2$ genannt.

Unter mehrfach substituierten Alkenyl-Resten sind solche Alkenyl-Reste zu verstehen, die entweder an verschiedenen oder an gleichen C-Atomen mehrfach, bevorzugt zweifach, substituiert sind, beispielsweise zweifach am gleichen C-Atom wie im Fall von $-CH=CCl_2$ oder an verschiedenen Stellen wie im Fall von $-CCl=CH-(CH_2)-NH_2$. Die Mehrfachsubstitution kann mit dem gleichen oder mit verschiedenen Substituenten erfolgen. Als geeignete substituierte Alkenyl-Reste seien beispielsweise $-CH=CH-(CH_2)-OH$, $-CH=CH-(CH_2)-NH_2$ und $-CH=CH-CN$ genannt.

Als geeignete $C_{2-6}$-Alkinyl-Reste seien beispielsweise Ethinyl, 1-Propinyl, 2-Propinyl, 1-Butinyl, 2-Butinyl, 3-Butinyl, 1-Pentinyl, 2-Pentinyl, 3-Pentinyl, 4-Pentinyl und Hexinyl genannt.

Unter mehrfach substituierten Alkinyl-Resten sind solche Alkinyl-Reste zu verstehen, die entweder an verschiedenen C-Atomen mehrfach substituiert sind, beispielsweise zweifach an verschiedenen C-Atomen wie im Fall von $-CHCl-C\equiv CCl$. Als geeignete substituierte Alkinyl-Reste seien beispielsweise $-C\equiv C-F$, $-C\equiv C-Cl$ und $-C\equiv C-I$ genannt.

Sofern einer oder mehrere der vorstehend genannten Substituenten für einen cycloaliphatischen Rest stehen oder einen cycloaliphatischen Rest aufweisen, der einfach oder mehrfach substituiert ist, kann dieser bevorzugt mit ggf. 1, 2, 3, 4 oder 5, besonders bevorzugt mit ggf. 1, 2 oder 3, Substituenten substituiert sein, die unabhängig voneinander ausgewählt werden können aus der Gruppe bestehend aus F, Cl, Br, I, -CN, $-NO_2$, -OH, -SH, $-SF_5$, $-NH_2$, Oxo (=O), Thioxo (=S), -C(=O)-OH, $C_{1-5}$-Alkyl, $-C_{2-5}$-Alkenyl, $-C_{2-5}$-Alkinyl, $-C\equiv C-Si(CH_3)_3$, $-C\equiv C-Si(C_2H_5)_3$, $-(CH_2)-O-C_{1-5}$-Alkyl, $-S-C_{1-5}$-Alkyl, -S-Phenyl, $-S-CH_2$-Phenyl, $-O-C_{1-5}$-Alkyl, -O-Phenyl, $-O-CH_2$-Phenyl, $-CF_3$, $-CHF_2$, $-CH_2F$, $-O-CF_3$, $-O-CHF_2$, $-O-CH_2F$, $-C(=O)-CF_3$, $-S-CF_3$, $-S-CHF_2$, $-S-CH_2F$, $-S(=O)_2$-Phenyl, $-S(=O)_2-C_{1-5}$-Alkyl, $-S(=O)-C_{1-5}$-Alkyl, $-NH-C_{1-5}$-Alkyl, $N(C_{1-5}Alkyl)(C_{1-5}$-Alkyl), $-C(=O)-O-C_{1-5}$-Alkyl, -C(=O)-H, $-C(=O)-C_{1-5}$-Alkyl, $-CH_2-O-C(=O)$-Phenyl, -O-C(=O)-Phenyl, $-NH-S(=O)_2-C_{1-5}$-Alkyl, $-NH-C(=O)-C_{1-5}$-Alkyl, - $C(=O)-NH_2$, $-C(=O)-NH-C_{1-5}$-Alkyl, $-C(=O)-N(C_{1-5}$-Alkyl$)_2$, Pyrazolyl, Phenyl, Furyl (Furanyl), Thiadiazolyl, Thiophenyl (Thienyl) und Benzyl, wobei die vorstehend genannten $C_{1-5}$-Alkyl-Reste jeweils linear oder verzweigt sein können und die zyklischen Substituenten bzw. die zyklischen Reste dieser Substituenten selbst jeweils mit ggf. 1, 2, 3, 4 oder 5, bevorzugt mit ggf. 1, 2, 3 oder 4, Substituenten unabhängig voneinander ausgewählt aus der Gruppe bestehend aus F, Cl, Br, I,-CN, $-CF_3$, -OH, $-NH_2$, $-O-CF_3$, -SH, $-O-C_{1-5}$-Alkyl, -O-Phenyl, $-O-CH_2$-Phenyl, $-(CH_2)-O-C_{1-5}$-Alkyl, $-S-C_{1-5}$-Alkyl, -S-Phenyl, $-S-CH_2$-Phenyl, $-C_{1-5}$-Alkyl, $-C_{2-5}$-Alkenyl, $-C_{2-5}$-Alkinyl,- $C\equiv C-Si(CH_3)_3$, $-C\equiv C-Si(C_2H_5)_3$, $-C(=O)-O-C_{1-5}$-Alkyl und $-C(=O)-CF_3$ substituiert sein können.

Besonders bevorzugt können die Substituenten, jeweils unabhängig voneinander, ausgewählt werden aus der Gruppe bestehend aus F, Cl, Br, I, -CN, $-NO_2$, Methyl, Ethyl, n-Propyl, Isopropyl, n-Butyl, Isobutyl, 2-Butyl, tert-Butyl, n-Pentyl, neo-Pentyl, Ethenyl, Allyl, Ethinyl, Propinyl, $-C\equiv C-Si(CH_3)_3$, $-C\equiv C-Si(C_2H_5)_3$, $-C\equiv C-Si(CH_3)_3$, - $C\equiv C-Si(C_2H_5)_3$, $-CH_2-O-CH_3$, $-CH_2-O-C_2H_5$, -OH, -SH, $-SF_5$, $-NH_2$, Oxo (=0), Thioxo (=S), -C(=O)-OH, $-S-CH_3$, $-S-C_2H_5$, $-S(=O)-CH_3$, $-S(=O)_2-CH_3$, $-S(=O)-C_2H_5$, $-S(=O)_2-C_2H_5$, $-O-CH_3$, $-O-C_2H_5$, $-O-C_3H_7$, $-O-C(CH_3)_3$, $-CF_3$, $-CHF_2$, $-CH_2F$, $-O-CF_3$, $-O-CHF_2$, $-O-CH_2F$, $-C(=O)-CF_3$, $-S-CF_3$, $-S-CHF_2$, $-S-CH_2F$, $-S(=O)_2$-Phenyl, Pyrazolyl,$-N(CH_3)_2$, $-N(C_2H_5)_2$, $-NH-CH_3$, $-NH-C_2H_5$, $-CH_2-O-C(=O)$-Phenyl, $-NH-S(=O)_2-CH_3$,$-C(=O)-O-CH_3$, $-C(=O)-O-C_2H_5$, $-C(=O)-O-C(CH_3)_3$, -C(=O)-H, $-C(=O)-CH_3$, $-C(=O)-C_2H_5$, $-NH-C(=O)-CH_3$; $-NH-C(=O)-C_2H_5$, -O-C(=O)-Phenyl, $-C(=O)-NH_2$, $-C(=O)-NH-CH_3$, $-C(=O)-N(CH_3)_2$, Phenyl, Furyl (Furanyl), Thiadiazolyl, Thiophenyl (Thienyl) und Benzyl, wobei die zyklischen Substituenten bzw. die zyklischen Reste dieser Substituenten selbst mit ggf. 1, 2, 3, 4 oder 5, bevorzugt mit ggf. 1, 2, 3 oder 4, Substituenten unabhängig voneinander ausgewählt aus der Gruppe bestehend aus F, Cl, Br, I, -CN, $-CF_3$, -OH, $-NH_2$, $-O-CF_3$, -SH, $-O-CH_3$, $-O-C_2H_5$, $-O-C_3H_7$, Methyl, Ethyl, n-Propyl, Isopropyl, n-Butyl, 2-Butyl, Isobutyl, tert-Butyl, Ethenyl, Allyl, Ethinyl, Propinyl, $-C\equiv C-Si(CH_3)_3$, $-C\equiv C-Si(C_2H_5)_3$, $-C(=O)-O-C_{1-5}$-Alkyl und $-C(=O)-CF_3$ substituiert sein können.

Sofern die cycloaliphatischen Reste eines oder mehrere Heteroatome als Ringglieder aufweisen, können diese vorzugsweise ggf. 1, 2, 3, 4 oder 5, besonders bevorzugt 1, 2 oder 3, Heteroatom(e) als Ringglied(er) aufweisen, die jeweils unabhängig voneinander, ausgewählt werden können aus der Gruppe bestehend aus Stickstoff, Sauerstoff und Schwefel.

Beispielhaft für cycloaliphatische Rest, die einfach oder mehrfach substituiert sein können, seien Cyclopropyl, Cyclobutyl, Cyclopentyl, Cyclohexyl, Cycloheptyl, Cyclooctyl, Cyclononyl, Cyclodecyl, Cycloundecyl, Cyclododecyl, Cycloheptyl, Cyclopentenyl, Cyclohexenyl, Cycloheptenyl, Cyclooctenyl, Oxiranyl, Aziridinyl, Tetrahydrofuranyl, Tetrahydrothienyl, Pyrrolidinyl, Isoxazolidinyl, Isothioazolidinyl, Pyrazolidinyl, Oxazolidinyl, Thiazolidinyl, Imidazolidinyl, (1,2,4)-Oxadiazolidinyl, (1,2,4)-Thiadiazolidinyl, (1,2,4)-Triazolidin-3-yl, (1,3,4)-Thiadiazolidinyl, (1,3,4)-Triazolidin-1-yl, (1,3,4)-Triazolidin-2-yl, (2,3)-Dihydrofuryl, (2,5)-Dihydrofuryl, (2,3)-Dihydrothienyl, (2,5)-Dihydrothienyl, (2,3)-Dihydropyrrolyl, (2,5)-Dihydropyrrolyl, (2,3)-Dihydroisoxazolyl, (4,5)-Dihydroisoxazolyl, (2,5)-Dihydroisothiazolyl, (2,3)-Dihydropyrazolyl, (4,5)-Dihydropyrazolyl, (2,5)-Dihydropyrazolyl, (2,3)-Dihydrooxazolyl, (4,5)-Dihydrooxazolyl, (2,5)-Dihydrooxazolyl, (2,3)-Dihydrothiazolyl, (4,5)-Dihydrothiazolyl, (2,5)-Dihydrothiazolyl, (2,3)-Dihydroimidazolyl, (4,5)-Dihydroimidazolyl, (2,5)-Dihydroimidazolyl, Morpholinyl, Piperidinyl, Piperazinyl, Azocanyl, Tetrahydropyridazinyl, Tetrahydropyrimidinyl, Tetrahydropyrazinyl, (1,3,5)-Tetrahydrotriazinyl, (1,2,4)-Tetrahydrotriazin-1-yl, (1,2,4)-Tetrahydrotriazin-3-yl, (1,3)-Dihydrooxazinyl, (1,3)-Dithian-2-yl, Tetrahydropyranyl, (1,3)-Dioxolan-2-yl, (3,4,5,6)-Tetrahydropyridin-2-yl, (1,2,5,6)-Tetrahydropyridin-1-yl, (1,2,3,4)-Tetrahydropyridin-1-yl, (1,2)-Dihydropyridin-1-yl, (1,4)-Dihydropyridin-1-yl, 4H-1,3-Thiazinyl, (1,3)-Dihydrooxazin-2-yl, Azepanyl, (1,4)-Diazepanyl, Thiomorpholinyl und Dithiolanyl genannt.

Besonders bevorzugt sein als cycloaliphatische Reste, die einfach oder mehrfach substituiert sein können, Cyclopropyl, Cyclobutyl, Cyclopentyl, Cyclohexyl, Cycloheptyl, Cyclooctyl, Cyclopentenyl, Cyclohexenyl, Cycloheptenyl, Cyclooctenyl, Imidazolidinyl, Tetrahydrofuranyl (Tetrahydrofuryl), Piperidinyl, Piperazinyl, Morpholinyl, Pyrrolidinyl, Tetrahydrothiophenyl, Tetrahydropyranyl, Thiomorpholinyl, Dioxolanyl, Azepanyl, Diazepanyl, Azocanyl und Dithiolanyl genannt.

Sofern der cycloaliphatische Rest mit einem unsubstituierten oder wenigstens einfach substituierten, gesättigten, ungesättigten oder aromatischen mono- oder polyzyklischen Ringsystem kondensiert ist, können geeignete unsubstituierte oder wenigstens einfach substituierte Reste ausgewählt werden aus der Gruppe bestehend aus 2,3-Dihydrobenzo[1,4]dioxinyl; 3,4-Dihydro-2H-benzo[1,4]oxazinyl; Benzo[1,3]dioxolyl; (1,2,3,4)-Tetrahydrochinazolinyl; Indanyl; (1,2,3,4)-Tetrahydronaphthyl; 1H-Indenyl; (1,2,3,4)-Tetrahydrochinolinyl; (1,2,3,4)-Tetrahydroisochinolinyl; (2,3)-Dihydro-1H-indolyl, (2,3)-Dihydro-1 H-isoindolyl und Decahydroisochinolinyl.

Sofern zwei der vorstehend genannten Substituenten zusammen mit dem sie verbindenden Stickstoffatom als Ringglied einen gesättigten oder ungesättigten heterocycloaliphatischen Rest bilden, der einfach oder mehrfach substituiert ist, kann dieser bevorzugt mit ggf. 1, 2, 3, 4 oder 5, besonders bevorzugt mit ggf. 1, 2 oder 3, Substituenten unabhängig voneinander ausgewählt aus der Gruppe bestehend aus F, Cl, Br, I, -CN, $-CF_3$, -OH, $-NH_2$, $-O-CF_3$, -SH, $-SF_5$, $-O-C_{1-5}$-Alkyl, -O-Phenyl, $-O-CH_2$-Phenyl, $-(CH_2)-O-C_{1-5}$-Alkyl, $-S-C_{1-5}$-Alkyl, -S-Phenyl, $-S-CH_2$-Phenyl, $-C_{1-5}$-Alkyl, $-C_{2-5}$-Alkenyl, $-C_{2-5}$-Alkinyl, $-C{\equiv}C-Si(CH_3)_3$, $-C{\equiv}C-Si(C_2H_5)_3$, $-C(=O)-O-C_{1-5}$-Alkyl, - $C(=O)-CF_3$, $-S(=O)_2-C_{1-5}$-Alkyl, $-S(=O)-C_{1-5}$-Alkyl, $-S(=O)_2$-Phenyl, Oxo (=O), Thioxo (=S), $-N(C_{1-5}$-Alkyl$)_2$, $-N(H)(C_{1-5}$-Alkyl), $-NO_2$, $-S-CF_3$, -C(=O)-OH, $-NH-S(=O)_2-C_{1-5}$-Alkyl, $-NH-C(=O)-C_{1-5}$-Alkyl, -C(=O)-H, $-C(=O)-C_{1-5}$-Alkyl, $-C(=O)-NH_2$, $-C(=O)-N(C_{1-5}$-Alkyl$)_2$, $-C(=O)-N(H)(C_{1-5}$-Alkyl) und Phenyl substituiert sein, wobei die vorstehend genannten $C_{1-5}$-Alkyl-Reste jeweils linear oder verzweigt sein können und die Phenyl-Reste jeweils unsubstituiert oder mit 1, 2, 3, 4 oder 5, bevorzugt mit 1, 2, 3 oder 4, Substituenten unabhängig voneinander ausgewählt aus der Gruppe bestehend aus F, Cl, Br, I, -CN, $-CF_3$, -OH, $-NH_2$, $-O-CF_3$, -SH, $-O-C_{1-5}$-Alkyl, -O-Phenyl, $-O-CH_2$-Phenyl, $-(CH_2)-O-C_{1-5}$-Alkyl, $-S-C_{1-5}$-Alkyl, -S-Phenyl, $-S-CH_2$-Phenyl, $-C_{1-5}$-Alkyl, $-C_{2-5}$-Alkenyl, $-C_{2-5}$-Alkinyl, $-C{\equiv}C-Si(CH_3)_3$, $-C{\equiv}C-Si(C_2H_5)_3$, $-C(=O)-O-C_{1-5}$-Alkyl und $-C(=O)-CF_3$ substituiert sein können.

Besonders bevorzugt können die Substituenten, jeweils unabhängig voneinander, ausgewählt werden aus der Gruppe bestehend aus F, Cl, Br, I, -CN, Methyl, Ethyl, n-Propyl, Isopropyl, n-Butyl, 2-Butyl, Isobutyl, tert-Butyl, Ethenyl, Allyl, Ethinyl, Propinyl, $-C{\equiv}C-Si(CH_3)_3$, $-C{\equiv}C-Si(C_2H_5)_3$, -OH, Oxo, Thioxo, $-O-CH_3$, $-O-C_2H_5$, $-O-C_3H_7$,$-(CH_2)-O-CH_3$, $-(CH_2)-O-C_2H_5$, $-NH_2$, $-N(CH_3)_2$, $-N(C_2H_5)_2$, $-NH-CH_3$, $-NH-C_2H_5$, $-NO_2$,$-CF_3$, $-O-CF_3$, $-S-CF_3$, -SH, $-SF_5$, $-S-CH_3$, $-S-C_2H_5$, $-S(=O)-CH_3$, $-S(=O)_2-CH_3$, $-S(=O)-C_2H_5$, $-S(=O)_2-C_2H_5$, $-NH-S(=O)_2-CH_3$, -C(=O)-OH, -C(=O)-H; $-C(=O)-CH_3$, $-C(=O)-C_2H_5$, $-C(=O)-N(CH_3)_2$, $-C(=O)-NH-CH_3$, $-C(=O)-NH_2$, $-NH-C(=O)-CH_3$, $-NH-C(=O)-C_2H_5$, $-C(=O)-O-CH_3$, $-C(=O)-O-C_2H_5$, $-C(=O)-O-C(CH_3)_3$ und Phenyl, wobei der Phenyl-Rest mit 1, 2, 3, 4 oder 5, bevorzugt 1, 2 oder 3, Substituenten unabhängig voneinander ausgewählt aus der Gruppe bestehend aus F, Cl, Br, I, -CN, $-CF_3$, -OH, $-NH_2$, $-O-CF_3$, -SH, $-O-CH_3$, $-O-C_2H_5$, $-O-C_3H_7$, Methyl, Ethyl, n-Propyl, Isopropyl, n-Butyl, 2-Butyl, Isobutyl, tert-Butyl, Ethenyl, Allyl, Ethinyl, Propinyl, $-C{\equiv}C-Si(CH_3)_3$, - $C{\equiv}C-Si(C_2H_5)_3$, $-C(=O)-O-C_{1-5}$-Alkyl und $-C(=O)-CF_3$ substituiert sein kann. Sofern die heterocycloaliphatischen Reste eines oder mehrere weitere Heteroatome als Ringglieder aufweisen, können diese vorzugsweise ggf. 1, 2, 3, 4 oder 5, besonders bevorzugt ggf. 1, 2 oder 3, weitere Heteroatom(e) als Ringglied(er) aufweisen, die jeweils unabhängig voneinander, ausgewählt werden können aus der Gruppe bestehend aus Stickstoff, Sauerstoff und Schwefel.

Als geeignete heterocycloaliphatische Reste, die einfach oder mehrfach substituiert sein können, seien beispielsweise Imidazolidinyl, Piperidinyl, Piperazinyl, Morpholinyl, Pyrrolidinyl, Thiomorpholinyl, Azepanyl, Diazepanyl und Azocanyl genannt.

Sofern der heterocycloaliphatische Rest mit einem unsubstituierten oder wenigstens einfach substituierten, gesättigten, ungesättigten oder aromatischen mono- oder polyzyklischen Ringsystem kondensiert ist, können geeignete unsubstituierte oder wenigstens einfach substituierte Reste ausgewählt werden aus der Gruppe bestehend aus (3,4)-Dihydro-2H-benzo[1,4]oxazinyl; (1,2,3,4)-Tetrahydrochinazolinyl; (1,2,3,4)-Tetrahydrochinolinyl; (1,2,3,4)-Tetrahydroisochinolinyl, (2,3)-Dihydro-1H-indolyl, (2,3)-Dihydro-1 H-isoindolyl und Decahydroisochinolinyl.

Sofern einer oder mehrere der vorstehend genannten Substituenten für einen Aryl- oder Heteroaryl-Rest stehen oder einen Aryl- oder Heteroaryl-Rest aufweisen, der einfach oder mehrfach substituiert ist, kann dieser bevorzugt mit ggf. 1, 2, 3, 4 oder 5, besonders bevorzugt mit ggf. 1, 2 oder 3, Substituenten unabhängig voneinander ausgewählt aus der Gruppe bestehend aus F, Cl, Br, I, -CN, $-CH_2-CN$, $-NO_2$, -OH,-SH, $-SF_5$, $-NH_2$, $-CH_2-NH_2$, -C(=O)-OH, $-C_{1-5}$-Alkyl, $-(CH_2)-O-C_{1-5}$-Alkyl, $-CH_2-OH$, $-C_2-_5$-Alkenyl, $-C_{2-5}$-Alkinyl, $-C{\equiv}C-Si(CH_3)_3$, $-C{\equiv}C-Si(C_2H_5)_3$, $-S-C_{1-5}$-Alkyl, -S-Phenyl, $-S-CH_2$-Phenyl, $-O-C_{1-5}$-Alkyl, -O-Phenyl, $-O-CH_2$-Phenyl, $-CF_3$, $-CHF_2$, $-CH_2F$, $-O-CF_3$,$-O-CHF_2$, $-O-CH_2F$, $-C(=O)-CF_3$, $-S-CF_3$, $-S-CHF_2$, $-S-CH_2F$, $-S(=O)_2$-Phenyl, $-S(=O)_2-C_{1-5}$-Alkyl, $-S(=O)-C_{1-5}$-Alkyl, $-NH-C_{1-5}$-Alkyl, $N(C_{1-5}Alkyl)_2$, $-C(=O)-O-C_{1-5}$-Alkyl, - C(=0)-H; $-C(=O)-C_{1-5}$-Alkyl, $-CH_2-O-C(=O)$-Phenyl, -O-C(=O)-Phenyl, $-NH-S(=O)_2-C_{1-5}$-Alkyl, $-NH-C(=O)-C_{1-5}$-Alkyl, $-NH-C(=NH)-NH_2$, $-NH-S(=O)_2-OH$, $-C(=O)-NH_2$, - $C(=O)-NH-C_{1-5}$-Alkyl, $-C(=O)-N(C_{1-5}\text{-Alkyl})_2$, $-Si(Phenyl)_2[C_{1-5}\text{-Alkyl}]$, $-S(=O)_2-NH_2$,$-S(=O)_2-NH-C_{1-5}$-Alkyl, $-S(=O)_2-N(C_{1-5}\text{-Alkyl})_2$, Cyclopropyl, Cyclobutyl, Cyclopentyl, Cyclohexyl, (1,3)-Dioxolanyl, Pyrazolyl, Pyrrolyl, Phenyl, Furyl (Furanyl), Thiazolyl, Thiadiazolyl, Thiophenyl (Thienyl), Benzyl und Phenethyl substituiert sein, wobei die vorstehend genannten $C_{1-5}$-Alkyl-Reste jeweils linear oder verzweigt sein können und die zyklischen Substituenten bzw. die zyklischen Reste dieser Substituenten selbst mit ggf. 1, 2, 3, 4 oder 5, bevorzugt mit ggf. 1, 2, 3 oder 4, Substituenten unabhängig voneinander ausgewählt aus der Gruppe bestehend aus F, Cl, Br, I, -CN, $-NO_2$, -OH, -SH, $-NH_2$, -C(=O)-OH, $-C_{1-5}$-Alkyl, $-(CH_2)-O-C_{1-5}$-Alkyl, $-C_{2-5}$-Alkenyl, $-C_{2-5}$-Alkinyl, $C{\equiv}C-Si(CH_3)_3$, $-C{\equiv}C-Si(C_2H_5)_3$, $-S-C_{1-5}$-Alkyl, -S-Phenyl, $-S-CH_2$-Phenyl, $-O-C_{1-5}$-Alkyl, -O-Phenyl, -O-$CH_2$-Phenyl, $-CF_3$, $-CHF_2$, $-CH_2F$, $-O-CF_3$, $-O-CHF_2$, $-O-CH_2F$,$-C(=O)-CF_3$, $-S-CF_3$, $-S-CHF_2$ und $-S-CH_2F$ substituiert sein können.

Besonders bevorzugt können die Substituenten, jeweils unabhängig voneinander ausgewählt werden aus der Gruppe bestehend aus F, Cl, Br, I, -CN, $-CH_2-CN$, $-NO_2$, Methyl, Ethyl, n-Propyl, Isopropyl, n-Butyl, Isobutyl, 2-Butyl, tert-Butyl, n-Pentyl, neo-Pentyl, Ethenyl, Allyl, Ethinyl, Propinyl, $-C{\equiv}C-Si(CH_3)_3$, $-C{\equiv}C-Si(C_2H_5)_3$, $-CH_2-O-CH_3$, $-CH_2-O-C_2H_5$, -OH, $-CH_2-NH_2$, $-CH_2-OH$, -SH, $-SF_5$, $-NH_2$, -C(=O)-OH, $-S-CH_3$, $-S-C_2H_5$, $-S(=O)-CH_3$, $-S(=O)_2-CH_3$, $-S(=O)-C_2H_5$, $-S(=O)_2-C_2H_5$, $-O-CH_3$, $-O-C_2H_5$, $-O-C_3H_7$, $-O-C(CH_3)_3$, -CF3, $-CHF_2$, $-CH_2F$, $-O-CF_3$, $-O-CHF_2$, $-O-CH_2F$, $-C(=O)-CF_3$, $-S-CF_3$, $-S-CHF_2$, $-S-CH_2F$, $-S(=O)_2$-Phenyl, Pyrazolyl, $-N(CH_3)_2$, $-N(C_2H_5)_2$, $-NH-CH_3$,$-NH-C_2H_5$, $-CH_2-O-C(=O)$-Phenyl, $-NH-S(=O)_2-CH_3$, $-NH-S(=O)_2-C_2H_5$, $-C(=O)-O-CH_3$, $-C(=O)-O-C_2H_5$, $-C(=O)-O-C(CH_3)_3$, -C(=O)-H, $-C(=O)-CH_3$, $-C(=O)-C_2H_5$, $-NH-C(=O)-CH_3$, $-NH-C(=O)-C_2H_5$; -O-C(=O)-Phenyl, $-C(=O)-NH_2$, $-C(=O)-NH-CH_3$, $-C(=O)-N(CH_3)_2$, $-Si(Phenyl)_2[C(CH_3)_3]$, $-S(=O)_2-NH_2$, $-S(=O)_2-NH-CH_3$, $-S(=O)_2-N(CH_3)_2$,$-NH-C(=NH)-NH_2$, -NH-$S(=O)_2$-OH, (1,3)-Dioxolanyl, Pyrrolyl, Cyclopropyl, Cyclobutyl, Cyclopentyl, Cyclohexyl, Phenyl, Furyl (Furanyl), Thiadiazolyl, Thiophenyl (Thienyl) und Benzyl, wobei die zyklischen Substituenten bzw. die zyklischen Reste dieser Substituenten selbst jeweils mit ggf. 1, 2, 3, 4, oder 5, bevorzugt mit ggf. 1, 2, 3 oder 4, Substituenten unabhängig voneinander ausgewählt aus der Gruppe bestehend aus F, Cl, Br, I, -CN, $-NO_2$, -OH, -SH, $-NH_2$, -C(=O)-OH, Methyl, Ethyl, n-Propyl, Isopropyl, n-Butyl, Isobutyl, 2-Butyl, tert-Butyl, n-Pentyl, neo-Pentyl, Ethenyl, Allyl, Ethinyl, Propinyl, $-C{\equiv}C-Si(CH_3)_3$, $-C{\equiv}C-Si(C_2H_5)_3$, $-CH_2-O-CH_3$, $-CH_2-O-C_2H_5$, $-S-CH_3$, $-S-C_2H_5$, $-S(=O)-CH_3$, $-S(=O)_2-CH_3$, $-S(=O)-C_2H_5$, $-S(=O)_2-C_2H_5$, $-O-CH_3$, $-O-C_2H_5$,$-O-C_3H_7$, $-O-C(CH_3)_3$, $-CF_3$, $-CHF_2$, $-CH_2F$, $-O-CF_3$, $-O-CHF_2$, $-O-CH_2F$, $-C(=O)-CF_3$, $S-CF_3$, $-S-CHF_2$ und $-S-CH_2F$ substituiert sein können.

Ganz besonders bevorzugt kann ein substituierter Aryl-Rest aus der Gruppe bestehend aus 2-Methyl-phenyl, 3-Methyl-phenyl, 4-Methyl-phenyl, 2-Fluor-phenyl, 3-Fluor-phenyl, 4-Fluor-phenyl, 2-Cyano-phenyl, 3-Cyano-phenyl, 4-Cyano-phenyl, 2-Hydroxy-phenyl, 3-Hydroxy-phenyl, 4-Hydroxy-phenyl, 2-Amino-phenyl, 3-Amino-phenyl, 4-Amino-phenyl, 2-Dimethylamino-phenyl, 3-Dimethylamino-phenyl, 4-Dimethylamino-phenyl, 2-Methylamino-phenyl, 3-Methylamino-phenyl, 4-Methylamino-phenyl, 2-Acetyl-phenyl, 3-Acetyl-phenyl, 4-Acetyl-phenyl, 2-Methylsulfinyl-phenyl, 3-Methylsulfinyl-phenyl, 4-Methylsulfinyl-phenyl, 2-Methylsulfonyl-phenyl, 3-Methylsulfonyl-phenyl, 4-Methylsulfonyl-phenyl, 2-Methoxy-phenyl, 3-Methoxy-phenyl, 4-Methoxy-phenyl, 2-Chlor-phenyl, 3-Chlor-phenyl, 4-Chlor-phenyl, 2-Ethoxy-phenyl, 3-Ethoxy-phenyl, 4-Ethoxyphenyl, 2-Trifluormethyl-phenyl, 3-Trifluormethyl-phenyl, 4-Trifluormethyl-phenyl, 2-Difluormethyl-phenyl, 3-Difluormethyl-phenyl, 4-Difluormethyl-phenyl, 2-Fluormethyl-phenyl, 3-Fluormethyl-phenyl, 4-Fluormethyl-phenyl, 2-Nitro-phenyl, 3-Nitro-phenyl, 4-Nitro-phenyl, 2-Ethyl-phenyl, 3-Ethyl-phenyl, 4-Ethyl-phenyl, 2-Propyl-phenyl, 3-Propyl-phenyl, 4-Propyl-phenyl, 2-Isopropyl-phenyl, 3-Isopropyl-phenyl, 4-Isopropyl-phenyl, 2-tert-Butyl-phenyl, 3-tert-Butyl-phenyl, 4-tert-Butyl-phenyl, 2-Carboxyphenyl, 3-Carboxy-phenyl, 4-Carboxyphenyl, 2-Ethenyl-phenyl, 3-Ethenyl-phenyl, 4-Ethenyl-phenyl, 2-Ethinyl-phenyl, 3-Ethinyl-phenyl, 4-Ethinyl-phenyl, 2-Allyl-phenyl, 3-Allyl-phenyl, 4-Allyl-phenyl, 2-Trimethylsilanylethinyl-phenyl, 3-Trimethylsilanylethinyl-phenyl, 4-Trimethylsilanylethinyl-phenyl, 2-Formyl-phenyl, 3-Formyl-phenyl, 4-Formyl-phenyl, 2-Acetamino-phenyl, 3-Acetamino-phenyl, 4-Acetamino-phenyl, 2-Dimethylaminocarbonyl-phenyl, 3-Dimethylaminocarbonyl-phenyl, 4-Dimethylaminocarbonyl-phenyl, 2-Methoxymethyl-phenyl, 3-Methoxymethyl-phenyl, 4-Methoxymethyl-phenyl, 2-Ethoxymethyl-phenyl, 3-Ethoxymethyl-phenyl, 4-Ethoxymethyl-phenyl, 2-Aminocarbonyl-phenyl, 3-Aminocarbonyl-phenyl, 4-Aminocarbonyl-phenyl, 2-Methylaminocarbonyl-phenyl, 3-Methylaminocarbonyl-phenyl, 4-Methylaminocarbonyl-phenyl, 2-Carboxymethylester-phenyl, 3-Carboxymethylester-phenyl, 4-Carboxymethylester-phenyl, 2-Carboxyethylesterphenyl, 3-Carboxyethylester-phenyl, 4-Carboxyethylester-phenyl, 2-Carboxy-tert-butylester-phenyl, 3-Carboxy-tert-butylester-phenyl, 4-Carboxy-tert-butylester-phenyl, 2-Methylmercapto-phenyl, 3-Methylmercapto-phenyl, 4-Methylmercapto-phenyl, 2-Ethylmercapto-phenyl, 3-Ethylmercapto-phenyl, 4-Ethylmercaptophenyl, 2-Biphenyl, 3-Biphenyl, 4-Biphenyl, 2-Bromphenyl, 3-Bromphenyl, 4-

Bromphenyl, 2-Iod-phenyl, 3-Iodphenyl, 4-Iodphenyl, 2-Trifluormethoxy-phenyl, 3-Trifluormethoxy-phenyl, 4-Trifluormethoxy-phenyl, 2-Fluor-3-trifluormethylphenyl, 2-Fluor-4-methyl-phenyl, (2,3)-Difluorphenyl, (2,3)-Dimethyl-phenyl, (2,3)-Dichlorphenyl, 3-Fluor-2-trifluormethylphenyl, (2,4)-Dichlor-phenyl, (2,4)-Difluorphenyl, 4-Fluor-2-trifluormethylphenyl, (2,4)-Dimethoxyphenyl, 2-Chlor-4-fluor-phenyl, 2-Chlor-4-nitro-phenyl, 2-Chlor-4-methyl-phenyl, 2-Chlor-5-trifluormethyl-phenyl, 2-Chlor-5-methoxy-phenyl, 2-Brom-5-trifluormethyl-phenyl, 2-Brom-5-methoxy-phenyl, (2,4)-Dibromphenyl, (2,4)-Dimethyl-phenyl, 2-Fluor-4-trifluormethyl-phenyl, (2,5)-Difluor-phenyl, 2-Fluor-5-trifluormethyl-phenyl, 5-Fluor-2-trifluormethyl-phenyl, 5-Chlor-2-trifluormethyl-phenyl, 5-Brom-2-trifluormethyl-phenyl, (2,5)-Dimethoxy-phenyl, (2,5)-Bis-trifluormethyl-phenyl, (2,5)-Dichlor-phenyl, (2,5)-Dibrom-phenyl, 2-Methoxy-5-nitro-phenyl, 2-Fluor-6-trifluormethyl-phenyl, (2,6)-Dimethoxy-phenyl, (2,6)-Dimethyl-phenyl, (2,6)-Dichlor-phenyl, 2-Chlor-6-fluor-phenyl, 2-Brom-6-chlor-phenyl, 2-Brom-6-fluor-phenyl, (2,6)-Difluor-phenyl, (2,6)-Difluor-3-methyl-phenyl, (2,6)-Dibrom-phenyl, (2,6)-Dichlorphenyl, 3-Chlor-2-fluor-phenyl, 3-Chlor-5-methyl-phenyl, (3,4)-Dichlorphenyl, (3,4)-Dimethyl-phenyl, 3-Methyl-4-methoxy-phenyl, 4-Chlor-3-nitro-phenyl, (3,4)-Dimethoxy-phenyl, 4-Fluor-3-trifluormethylphenyl, 3-Fluor-4-trifluormethyl-phenyl, (3,4)-Difluor-phenyl, 3-Cyano-4-fluor-phenyl, 3-Cyano-4-methyl-phenyl, 3-Cyano-4-methoxy-phenyl, 3-Brom-4-fluor-phenyl, 3-Brom-4-methyl-phenyl, 3-Brom-4-methoxy-phenyl, 4-Chlor-2-fluor-phenyl, 4-Chlor-3-trifluormethyl, 4-Brom-3-methyl-phenyl, 4-Brom-5-methyl-phenyl, 3-Chlor-4-fluor-phenyl, 4-Fluor-3-nitro-phenyl, 4-Brom-3-nitro-phenyl, (3,4)-Dibrom-phenyl, 4-Chlor-3-methyl-phenyl, 4-Brom-3-methyl-phenyl, 4-Fluor-3-methyl-phenyl, 3-Fluor-4-methyl-phenyl, 3-Fluor-5-methyl-phenyl, 2-Fluor-3-methyl-phenyl, 4-Methyl-3-nitro-phenyl, (3,5)-Dimethoxy-phenyl, (3,5)-Dimethyl-phenyl, (3,5)-Bis-trifluormethyl-phenyl, (3,5)-Difluor-phenyl, (3,5)-Dinitro-phenyl, (3,5)-Dichlor-phenyl, 3-Fluor-5-trifluormethyl-phenyl, 5-Fluor-3-trifluormethyl-phenyl, (3,5)-Dibrom-phenyl, 5-Chlor-4-fluor-phenyl, 5-Chlor-4-fluor-phenyl, 5-Brom-4-methyl-phenyl, (2,3,4)-Trifluorphenyl, (2,3,4)-Trichlorphenyl, (2,3,6)-Trifluor-phenyl, 5-Chlor-2-methoxy-phenyl, (2,3)-Difluor-4-methyl, (2,4,5)-Trifluor-phenyl, (2,4,5)-Trichlorphenyl, (2,4)-Dichlor-5-fluor-phenyl, (2,4,6)-Trichlor-phenyl, (2,4,6)-Trimethylphenyl, (2,4,6)-Trifluor-phenyl, (2,4,6)-Trimethoxy-phenyl, (3,4,5)-Trimethoxy-phenyl, (2,3,4,5)-Tetrafluor-phenyl, 4-Methoxy-(2,3,6)-trimethyl-phenyl, 4-Methoxy-(2,3,6)-trimethyl-phenyl, 4-Chlor-2,5-dimethyl-phenyl, 2-Chlor-6-fluor-3-methyl-phenyl, 6-Chlor-2-fluor-3-methyl, (2,4,6)-Trimethylphenyl und (2,3,4,5,6)-Pentafluor-phenyl ausgewählt werden.

Ganz besonders bevorzugt kann ein substituierter Heteroaryl-Rest aus der Gruppe bestehend aus 3-Methyl-pyrid-2-yl, 4-Methyl-pyrid-2-yl, 5-Methyl-pyrid-2-yl, 6-Methyl-pyrid-2-yl, 2-Methyl-pyrid-3-yl, 4-Methyl-pyrid-3-yl, 5-Methyl-pyrid-3-yl, 6-Methyl-pyrid-3-yl, 2-Methyl-pyrid-4-yl, 3-Methyl-pyrid-4-yl, 3-Fluor-pyrid-2-yl, 4-Fluor-pyrid-2-yl, 5-Fluor-pyrid-2-yl, 6-Fluor-pyrid-2-yl, 3-Chlor-pyrid-2-yl, 4-Chlor-pyrid-2-yl, 5-Chlor-pyrid-2-yl, 6-Chlor-pyrid-2-yl, 3-Trifluormethyl-pyrid-2-yl, 4-Trifluormethyl-pyrid-2-yl, 5-Trifluormethyl-pyrid-2-yl, 6-Trifluormethyl-pyrid-2-yl, 3-Methoxy-pyrid-2-yl, 4-Methoxy-pyrid-2-yl, 5-Methoxy-pyrid-2-yl, 6-Methoxy-pyrid-2-yl, 4-Methyl-thiazol-2-yl, 5-Methyl-thiazol-2-yl, 4-Trifluormethyl-thiazol-2-yl, 5-Trifluormethyl-thiazol-2-yl, 4-Chlor-thiazol-2-yl, 5-Chlor-thiazol-2-yl, 4-Brom-thiazol-2-yl, 5-Brom-thiazol-2-yl, 4-Fluor-thiazol-2-yl, 5-Fluor-thiazol-2-yl, 4-Cyano-thiazol-2-yl, 5-Cyano-thiazol-2-yl, 4-Methoxy-thiazol-2-yl, 5-Methoxy-thiazol-2-yl, 4-Methyl-oxazol-2-yl, 5-Methyl-oxazol-2-yl, 4-Trifluormethyl-oxazol-2-yl, 5-Trifluormethyl-oxazol-2-yl, 4-Chlor-oxazol-2-yl, 5-Chlor-oxazol-2-yl, 4-Brom-oxazol-2-yl, 5-Brom-oxazol-2-yl, 4-Fluor-oxazol-2-yl, 5-Fluor-oxazol-2-yl, 4-Cyano-oxazol-2-yl, 5-Cyano-oxazol-2-yl, 4-Methoxy-oxazol-2-yl, 5-Methoxy-oxazol-2-yl, 2-Methyl-(1,2,4)-thiadiazol-5-yl, 2-Trifluormethyl-(1,2,4)-thiadiazol-5-yl, 2-Chlor-(1,2,4)-thiadiazol-5-yl, 2-Fluor-(1,2,4)-thiadiazol-5-yl, 2-Methoxy-(1,2,4)-thiadiazol-5-yl, 2-Cyano-(1,2,4)-thiadiazol-5-yl, 2-Methyl-(1,2,4)-oxadiazol-5-yl, 2-Trifluormethyl-(1,2,4)-oxadiazol-5-yl, 2-Chlor-(1,2,4)-oxadiazol-5-yl, 2-Fluor-(1,2,4)-oxadiazol-5-yl, 2-Methoxy-(1,2,4)-oxadiazol-5-yl und 2-Cyano-(1,2,4)-oxadiazol-5-yl ausgewählt werden.

Als geeignete Aryl-Reste seien beispielsweise Phenyl, 1-Naphthyl, 2-Naphthyl und Anthracenyl genannt. Ein geeigneter 6-gliedriger Aryl-Rest ist ein Phenyl-Rest.

Sofern einer oder mehrere der vorstehend genannten Substituenten für einen Heteroaryl-Rest stehen oder einen Heteroaryl-Rest aufweisen, können dessen Heteroatom(e), jeweils unabhängig voneinander, bevorzugt ausgewählt werden aus der Gruppe bestehend aus Sauerstoff, Schwefel und Stickstoff. Bevorzugt kann ein Heteroaryl-Rest ggf. 1, 2, 3, 4 oder 5, besonders bevorzugt 1, 2 oder 3 Heteroatome, aufweisen.

Als geeignete 5- oder 6-gliedrige Heteroaryl-Reste seien beispielsweise Furyl (Furanyl), Thienyl (Thiophenyl), Pyrazolyl, Imidazolyl, Thiazolyl, Thiadiazolyl, Triazolyl, Pyrrolyl, Oxazolyl, Oxadiazolyl, Isoxazolyl, Pyridinyl, Pyridazinyl, Pyrimidinyl, Pyrazinyl, Pyranyl, Diazinyl, Triazinyl, Tetrazinyl, Tetrazolyl, Purinyl, Dithiazolyl und Pentazolyl genannt.

Als geeignete 9- oder 10-gliedrige Heteroaryl-Reste seien beispielsweise Indolyl, Isoindolyl, Benzo[b]furanyl, Isobenzo[b]furanyl, Indazolyl, Indolizinyl, Chinolinyl, Isochinolinyl, Chinazolinyl, Benzo[b]thiophenyl und Isobenzo[b]thiophenyl genannt.

Aryl- oder Heteroaryl-Reste können im Sinne der vorliegenden Erfindung mit einem mono- bzw. bizyklischem Ringsystem kondensiert (anneliert) sein.

Sofern der 5- oder 6-gliedrige Heteroaryl-Rest mit einem unsubstituierten oder wenigstens einfach substituierten aromatischen mono- oder polyzyklischen Ringsystem kondensiert ist, können geeignete unsubstituierte oder wenigstens einfach substituierte Reste ausgewählt werden aus der Gruppe bestehend aus Indolyl, Isoindolyl, Benzo[b]furanyl, Isobenzo[b]furanyl, Indazolyl, Indolizinyl, Chinolinyl, Isochinolinyl, Chinazolinyl, Benzo[b]thiophenyl und Isobenzo[b]thiophenyl.

Beispielhaft für 6-gliedrige Aryl-Reste, die mit einem unsubstituierten oder wenigstens einfach substituierten gesättigten mono- oder polyzyklischen Ringsystem kondensiert sind, seien (1,2,3,4)-Tetrahydrochinolinyl, (1,2,3,4)-Tetrahydroisochinolinyl, (2,3)-Dihydro-1 H-isoindolyl, (1,2,3,4)-Tetrahydronaphthyl, (2,3)-Dihydro-benzo[1.4]dioxinyl, Benzo[1.3]dioxolyl und (3,4)-Dihydro-2H-benzo[1.4]oxazinyl genannt.

Unter einem mono- bzw. polyzyklischen Ringsystem werden im Sinne der vorliegenden Erfindung mono- bzw. polyzyklische Kohlenwasserstoffreste verstanden, die gesättigt, ungesättigt oder aromatisch sein und ggf. eines oder mehrere Heteroatome als Ringglieder aufweisen können. Ein solches mono- bzw. polyzyklisches Ringsystem kann beispielsweise mit einem cycloaliphatischen Rest, einem heterocycloaliphatischen Rest, einem Aryl-Rest oder einem Heteroaryl-Rest kondensiert (anneliert) sein.

Sofern ein polyzyklisches Ringsystem wie beispielsweise ein bizyklisches Ringsystem vorliegt, können die verschiedenen Ringe, jeweils unabhängig voneinander, einen unterschiedlichen Sättigungsgrad aufweisen, d.h. gesättigt, ungesättigt oder aromatisch sein. Die Heteroatome jedes Ringes können, jeweils unabhängig voneinander, bevorzugt ausgewählt werden aus der Gruppe bestehend aus Sauerstoff, Stickstoff und Schwefel. Vorzugsweise enthält ein Ring 0, 1, 2 oder 3 Heteroatome. Vorzugsweise sind die jeweiligen Ringe des mono- oder polyzyklischen Ringsystems 5-, 6- oder 7-gliedrig, besonders bevorzugt 5- oder 6-gliedrig.

Sofern einer oder mehrere der vorstehend genannten Substituenten ein gesättigtes, ungesättigtes oder aromatisches monozyklisches oder polyzyklisches Ringsystem aufweisen, das einfach oder mehrfach substituiert ist, kann dieses bevorzugt mit ggf. 1, 2, 3, 4 oder 5, besonders bevorzugt mit ggf. 1, 2 oder 3, Substituenten unabhängig voneinander ausgewählt aus der Gruppe bestehend aus Oxo (=O), Thioxo (=S), F, Cl, Br, I, -CN, $-NO_2$, -OH, $-CH_2$-OH, -SH, $-SF_5$, $-NH_2$, $-CH_2-NH_2$, -C(=O)-OH, $-C_{1-5}$-Alkyl, $-(CH_2)-O-C_{1-5}$-Alkyl, $-C_{2-5}$-Alkenyl, $-C_{2-5}$-Alkinyl, $-C\equiv C-Si(CH_3)_3$, $-C\equiv C-Si(C_2H_5)_3$, $-S-C_{1-5}$-Alkyl, -S-Phenyl, $-S-CH_2$-Phenyl, $-O-C_{1-5}$-Alkyl, -O-Phenyl, $-O-CH_2$-Phenyl,$-CF_3$, $-CHF_2$, $-CH_2F$, $-O-CF_3$, $-O-CHF_2$, $-O-CH_2F$, $-C(=O)-CF_3$, $-S-CF_3$, $-S-CHF_2$, $-S-CH_2F$, $-S(=O)_2$-Phenyl, $-S(=O)_2-C_{1-5}$-Alkyl, $-S(=O)-C_{1-5}$-Alkyl, $-NH-C_{1-5}$-Alkyl, $N(C_{1-5}alkyl)_2$, $-C(=O)-O-C_{1-5}$-Alkyl, -C(=O)-H; $-C(=O)-C_{1-5}$-Alkyl, $-CH_2$-O-C(=O)-Phenyl, -0-C(=O)-Phenyl, $-NH-S(=O)_2-C_{1-5}$-Alkyl, $-NH-C(=O)-C_{1-5}$-Alkyl, $-NH-C(=NH)-NH_2$, $-NH-S(=O)_2-OH$, $-C(=O)-NH_2$, $-C(=O)-NH-C_{1-5}$-Alkyl, $-C(=O)-N(C_{1-5}\text{-Alkyl})_2$,$-Si(Phenyl)_2[C_{1-5}$-Alkyl], $-S(=O)_2-NH_2$, $-S(=O)_2-NH-C_{1-5}$-Alkyl, $S(=O)_2-N(C_{1-5}\text{-Alkyl})_2$, Cyclopropyl, Cyclobutyl, Cyclopentyl, Cyclohexyl, (1,3)-Dioxolanyl, Pyrazolyl, Pyrrolyl, Phenyl, Furyl (Furanyl), Thiazolyl, Thiadiazolyl, Thiophenyl (Thienyl), Benzyl und Phenethyl substituiert sein, wobei die vorstehend genannten $C_{1-5}$-Alkyl-Reste jeweils linear oder verzweigt sein können und die zyklischen Substituenten bzw. die zyklischen Reste dieser Substituenten selbst mit ggf. 1, 2, 3, 4 oder 5, bevorzugt mit ggf. 1, 2, 3 oder 4, Substituenten unabhängig voneinander ausgewählt aus der Gruppe bestehend aus F, Cl, Br, I, -CN, $-NO_2$, -OH, -SH, $-NH_2$, -C(=O)-OH, $-C_{1-5}$-Alkyl, $-(CH_2)-O-C_{1-5}$-Alkyl, $-C_{2-5}$-Alkenyl, $-C_{2-5}$-Alkinyl, $-C\equiv C-Si(CH_3)_3$, $-C\equiv C-Si(C_2H_5)_3$, $-S-C_{1-5}$-Alkyl, -S-Phenyl, $-S-CH_2$-Phenyl, $-O-C_{1-5}$-Alkyl, -O-Phenyl, $-O-CH_2$-Phenyl,$-CF_3$,- $CHF_2$, $-CH_2F$, $-O-CF_3$, $-O-CHF_2$, $-O-CH_2F$, $-C(=O)-CF_3$, $-S-CF_3$, $-S-CHF_2$ und$-S-CH_2F$ substituiert sein können.

Besonders bevorzugt können die Substituenten, jeweils unabhängig voneinander ausgewählt werden aus der Gruppe bestehend aus Oxo (=0), Thioxo (=S), F, Cl, Br, I, -CN, $-NO_2$, Methyl, Ethyl, n-Propyl, Isopropyl, n-Butyl, Isobutyl, 2-Butyl, tert-Butyl, n-Pentyl, neo-Pentyl, Ethenyl, Allyl, Ethinyl, Propinyl, $-C\equiv C-Si(CH_3)_3$. $-C\equiv C-Si(C_2H_5)_3$, $-CH_2-O-CH_3$, $-CH_2-O-C_2H_5$, -OH, $-CH_2-NH_2$, $-CH_2$-OH, -SH, $-SF_5$, $-NH_2$, -C(=O)-OH,$-S-CH_3$, $-S-C_2H_5$, $-S(=O)-CH_3$, $-S(=O)_2-CH_3$, $-S(=O)-C_2H_5$, $-S(=O)_2-C_2H_5$, $-O-CH_3$, $-O-C_2H_5$, $-O-C_3H_7$, $-O-C(CH_3)_3$, $-CF_3$, $-CHF_2$, $-CH_2F$, $-O-CF_3$, $-O-CHF_2$, $-O-CH_2F$, $-C(=O)-CF_3$, $-S-CF_3$, $-S-CHF_2$, $-S-CH_2F$, $-S(=O)_2$-Phenyl, Pyrazolyl, $-N(CH_3)_2$, $-N(C_2H_5)_2$,$-NH-CH_3$, $-NH-C_2H_5$, $-CH_2$-O-C(=O)-Phenyl, $-NH-S(=O)_2-CH_3$, $-NH-S(=O)_2-C_2H_5$,$-C(=O)-O-CH_3$, $-C(=O)-O-C_2H_5$, $-C(=O)-O-C(CH_3)_3$, -C(=O)-H, $-C(=O)-CH_3$, $-C(=O)-C_2H_5$, $-NH-C(=O)-CH_3$, $-NH-C(=O)-C_2H_5$, -O-C(=O)-Phenyl, $-C(=O)-NH_2$, $-C(=O)-NH-CH_3$, $-C(=O)-N(CH_3)_2$, $-Si(Phenyl)_2[C(CH_3)_3]$, $-S(=O)_2-NH_2$, $-S(=O)_2-NH-CH_3$, $-S(=O)_2-N(CH_3)_2$, $-NH-C(=NH)-NH_2$, $-NH-S(=O)_2-OH$, (1,3)-Dioxolanyl, Pyrrolyl, Cyclopropyl, Cyclobutyl, Cyclopentyl, Cyclohexyl, Phenyl, Furyl (Furanyl), Thiadiazolyl, Thiophenyl (Thienyl) und Benzyl, wobei die zyklischen Substituenten bzw. die zyklischen Reste dieser Substituenten selbst jeweils mit ggf. 1, 2, 3, 4, oder 5, bevorzugt mit ggf. 1,2, 3 oder 4, Substituenten unabhängig voneinander ausgewählt aus der Gruppe bestehend aus F, Cl, Br, I, -CN, $-NO_2$, -OH, -SH, $-NH_2$, -C(=O)-OH, Methyl, Ethyl, n-Propyl, Isopropyl, n-Butyl, Isobutyl, 2-Butyl, tert-Butyl, n-Pentyl, neo-Pentyl, Ethenyl, Allyl, Ethinyl, Propinyl, $-C\equiv C-Si(CH_3)_3$, $-C\equiv C-Si(C_2H_5)_3$, $-CH_2-O-CH_3$, $-CH_2-O-C_2H_5$, $-S-CH_3$, $-S-C_2H_5$, $-S(=O)-CH_3$, $-S(=O)_2-CH_3$, $-S(=O)-C_2H_5$, $-S(=O)_2-C_2H_5$, $-O-CH_3$, $-O-C_2H_5$, $-O-C_3H_7$, $-O-C(CH_3)_3$, $-CF_3$, $-CHF_2$, $-CH_2F$, $-O-CF_3$, $-O-CHF_2$, $-O-CH_2F$, $-C(=O)-CF_3$, $-S-CF_3$, $-S-CHF_2$ und $-S-CH_2F$ substituiert sein können.

Sofern einer der vorstehend genannten Substituenten eine lineare oder verzweigte Alkylen-Gruppe aufweist, kann die Alkylen-Gruppe vorzugsweise ausgewählt werden aus der Gruppe bestehend aus $-(CH_2)-$, $-(CH_2)_2-$, $-C(H)(CH_3)-$, $-(CH_2)_3-$, $-(CH_2)_4-$,$-(CH_2)_5-$ und $-C(C_2H_5)(H)-$.

Besonders bevorzugt kann eine Alkylen-Gruppe mit 1, 2 oder 3 Substituenten unabhängig voneinander ausgewählt werden aus der Gruppe bestehend aus F, Cl, Br, I, $-NO_2$, -CN, -OH, -SH, $-NH_2$, $-N(CH_3)_2$, $-N(C_2H_5)_2$ und $-N(CH_3)(C_2H_5)$ substituiert sein.

Der Fachmann versteht, daß einige der erfindungsgemäßen substituierten Imidazo[2,1-b]thiazol-Verbindungen

der allgemeinen Formel I in Form von Tautomeren vorliegen können, die ebenfalls Gegenstand der vorliegenden Erfindung sind und jeweils auch als Wirkstoffe in den untenstehend beschriebenen Arzneimitteln vorliegen können.

Bevorzugt sind substituierte Imidazo[2,1-b]thiazol-Verbindungen der vorstehend angegebenen allgemeinen Formel I, worin

$R^1$ und $R^2$, unabhängig voneinander, jeweils für einen Wasserstoff-Rest; für einen Halogen-Rest; $-NO_2$; -CN; $-NH_2$; $-NHR^5$; $-NR^6R^7$; $-NH-C(=O)-R^8$; $-C(=O)-R^9$, $-C(=O)-NH_2$; $-C(=O)-NHR^{10}$; $-C(=O)-NR^{11}R^{12}$; $-C(=O)-OR^{13}$; $-(CH_2)_m-C(=O)-OR^{14}$ mit m = 1, 2, 3, 4 oder 5; $-O-C(=O)-R^{15}$; $-(CH_2)_n-O-C(=O)-R^{16}$ mit n = 1, 2, 3, 4 oder 5; $-OR^{17}$; $-(CH_2)_o-O-R^{18}$ mit o = 1, 2, 3; 4 oder 5; $-SR^{19}$; $-(CH_2)_p-S(=O)_t-R^{20}$ mit p = 1, 2, 3, 4 oder 5 und t = 0, 1 oder 2; $-NH-S(=O)_2-NR^{27}R^{28}$; $-S(=O)_2-NR^{29}R^{30}$; $-SF_5$; $-(CH_2)_u-O-S(=O)_2-R^{31}$ mit u = 1, 2, 3, 4 oder 5; $-(CH_2)_v-O-S(=O)_2-O-R^{32}$ mit v = 1, 2, 3, 4 oder 5; $-(CH2)_w-O-P(=O)(OR^{33})(OR^{34})$ mit w = 1, 2, 3, 4 oder 5; für einen linearen oder verzweigten, unsubstituierten oder wenigstens einfach substituierten $C_{1-10}$-Alkyl-Rest, $C_{2-6}$-Alkenyl-Rest oder $C_{2-6}$-Alkinyl-Rest;
für einen gesättigten oder ungesättigten, unsubstituierten oder wenigstens einfach substituierten, ggf. wenigstens ein Heteroatom als Ringglied aufweisenden cycloaliphatischen $C_{3-8}$-Rest, der über eine lineare oder verzweigte, unsubstituierte oder wenigstens einfach substituierte $C_{1-5}$-Alkylen-Gruppe gebunden sein kann; oder für einen unsubstituierten oder wenigstens einfach substituierten 5- oder 6-gliedrigen Aryl- oder Heteroaryl-Rest, der über eine lineare oder verzweigte, unsubstituierte oder wenigstens einfach substituierte $C_{1-5}$-Alkylen-Gruppe gebunden und/oder mit einem unsubstituierten oder wenigstens einfach substituierten mono-oder polyzyklischen Ringsystem kondensiert sein kann, wobei die Ringe des Ringsystems jeweils 5-, 6- oder 7-gliedrig sind, stehen;

$R^3$ und $R^4$, unabhängig voneinander, jeweils für einen Wasserstoff-Rest; $-C(=O)-R^{21}$; $-(CH_2)_q-C(=O)-R^{22}$ mit q = 1, 2, 3, 4 oder 5; $-C(=O)-O-R^{23}$; $-(CH_2)_r-C(=O)-O-R^{24}$ mit r = 1, 2, 3, 4 oder 5; $-C(=O)-NHR^{25}$; $-(CH_2)_s-C(=O)-NHR^{26}$ mit s = 1, 2, 3, 4 oder 5; für einen linearen oder verzweigten, unsubstituierten oder wenigstens einfach substituierten $C_{1-10}$-Alkyl-Rest, $C_{2-6}$-Alkenyl-Rest oder $C_{2-6}$-Alkinyl-Rest;
für einen gesättigten oder ungesättigten, unsubstituierten oder wenigstens einfach substituierten, ggf. wenigstens ein Heteroatom als Ringglied aufweisenden cycloaliphatischen $C_{3-8}$-Rest, der über eine lineare oder verzweigte, unsubstituierte oder wenigstens einfach substituierte $C_{1-5}$-Alkylen-Gruppe gebunden sein kann; oder für einen unsubstituierten oder wenigstens einfach substituierten 5- oder 6-gliedrigen Aryl- oder Heteroaryl-Rest, der über eine lineare oder verzweigte, unsubstituierte oder wenigstens einfach substituierte $C_{1-5}$-Alkylen-Gruppe gebunden und/oder mit einem unsubstituierten oder wenigstens einfach substituierten mono- oder polyzyklischen Ringsystem kondensiert sein kann, wobei die Ringe des Ringsystems jeweils 5-, 6- oder 7-gliedrig sind, stehen,
oder $R^3$ und $R^4$ zusammen mit dem sie verbindenden Stickstoffatom als Ringglied einen gesättigten oder ungesättigten, unsubstituierten oder wenigstens einfach substituierten, ggf. wenigstens ein weiteres Heteroatom als Ringglied aufweisenden heterocycloaliphatischen $C_{4-10}$-Rest bilden, der mit einem unsubstituierten oder wenigstens einfach substituierten mono- oder polyzyklischen Ringsystem kondensiert sein kann, wobei die Ringe des Ringsystems jeweils 5-, 6- oder 7-gliedrig sind;

$R^5$, $R^6$, $R^7$, $R^8$, $R^{10}$, $R^{11}$, $R^{12}$, $R^{15}$ und $R^{16}$, jeweils unabhängig voneinander, für einen linearen oder verzweigten, unsubstituierten oder wenigstens einfach substituierten $C_{1-10}$-Alkyl-Rest, $C_{2-6}$-Alkenyl-Rest oder $C_{2-6}$-Alkinyl-Rest; oder für einen unsubstituierten oder wenigstens einfach substituierten 5- bis 14-gliedrigen Aryl- oder Heteroaryl-Rest, der über eine lineare oder verzweigte, unsubstituierte oder wenigstens einfach substituierte $C_{1-5}$-Alkylen-Gruppe gebunden und/oder mit einem unsubstituierten oder wenigstens einfach substituierten mono- oder polyzyklischen Ringsystem kondensiert sein kann, wobei die Ringe des Ringsystems jeweils 5-, 6-oder 7-gliedrig sind, stehen;

$R^9$, $R^{13}$, $R^{14}$, $R^{17}$, $R^{18}$, $R^{19}$, $R^{20}$, $R^{21}$, $R^{22}$, $R^{23}$, $R^{24}$, $R^{25}$, $R^{26}$, $R^{27}$, $R^{28}$, $R^{29}$, $R^{30}$, $R^{31}$, $R^{32}$, $R^{33}$ und $R^{34}$, jeweils unabhängig voneinander, für einen Wasserstoff-Rest; für einen linearen oder verzweigten, unsubstituierten oder wenigstens einfach substituierten $C_{1-10}$-Alkyl-Rest, $C_{2-6}$-Alkenyl-Rest oder $C_{2-6}$-Alkinyl-Rest; oder für einen unsubstituierten oder wenigstens einfach substituierten 5- bis 14-gliedrigen Aryl- oder Heteroaryl-Rest, der über eine lineare oder verzweigte, unsubstituierte oder wenigstens einfach substituierte $C_{1-5}$-Alkylen-Gruppe gebunden und/oder mit einem unsubstituierten oder wenigstens einfach substituierten mono- oder polyzyklischen Ringsystem kondensiert sein kann, wobei die Ringe des Ringsystems jeweils 5-, 6-oder 7-gliedrig sind, stehen;

$M^1$ für einen 5- oder 6-gliedrigen Aryl- oder Heteroaryl-Rest steht, der mit wenigstens einem weiteren Substituenten substituiert sein kann und mit einem unsubsituierten oder wenigstens einfach substituierten mono- oder bizyklischem Ringsystem kondensiert sein kann, wobei die Ringe des Ringsystems jeweils 5-, 6- oder 7-gliedrig sind, und
und $M^2$ für einen 5- oder 6-gliedrigen Aryl- oder Heteroaryl-Rest steht, der unsubstituiert oder wenigstens einfach substituiert und mit einem unsubstituierten oder wenigstens einfach substituierten mono- oder bizyklischem Ringsystem kondensiert sein kann, wobei die Ringe des Ringsystems jeweils 5-, 6- oder 7-gliedrig sind;

wobei

die vorstehend genannten cycloaliphatischen Reste ggf. 1, 2, 3, 4 oder 5 Heteroatom(e) als Ringglied(er) aufweisen können, die jeweils unabhängig voneinander, ausgewählt werden können aus der Gruppe bestehend aus Stickstoff, Sauerstoff und Schwefel,

die vorstehend genannten heterocycloaliphatischen Reste ggf. weitere 1, 2, 3, 4 oder 5 Heteroatom(e) als Ringglied(er)

aufweisen können, die jeweils unabhängig voneinander, ausgewählt werden können aus der Gruppe bestehend aus Stickstoff, Sauerstoff und Schwefel,

die Ringe des mono- oder polyzyklischen Ringsystems jeweils ggf. 0, 1, 2 oder 3 Heteroatom(e) als Ringglied(er) aufweisen, die unabhängig voneinander aus der Gruppe bestehend aus Sauerstoff, Stickstoff und Schwefel ausgewählt sind;

und

die vorstehend genannten Heteroaryl-Reste ggf. 1, 2, 3, 4 oder 5 Heteroatom(e) als Ringglied(er) aufweisen können, die jeweils unabhängig voneinander, ausgewählt werden können aus der Gruppe bestehend aus Sauerstoff, Schwefel und Stickstoff.

Ferner bevorzugt sind substituierte Imidazo[2,1-b]thiazol-Verbindungen der vorstehend angegebenen allgemeinen Formel I, worin

$R^1$ und $R^2$, unabhängig voneinander, jeweils für einen Wasserstoff-Rest; für einen Halogen-Rest; $-NO_2$; -CN; $-NH_2$; $-NHR^5$; $-NR^6R^7$; $-NH-C(=O)-R^8$; $-C(=O)-R^9$, $-C(=O)-NH_2$; $-C(=O)-NHR^{10}$; $-C(=O)-NR^{11}R^{12}$; $-C(=O)-OR^{13}$; $-(CH_2)_m-C(=O)-OR^{14}$ mit m = 1, 2 oder 3; $-O-C(=O)-R^{15}$; $-(CH_2)_n-O-C(=O)-R^{16}$ mit n = 1, 2 oder 3; $-OR^{17}$; $-(CH_2)_o-O-R^{18}$, mit o = 1, 2 oder 3; $-SR^{19}$; $-(CH_2)_p-S(=O)_t-R^{20}$ mit p = 1, 2 oder 3 und t = 0, 1 oder 2; - $NH-S(=O)_2-NR^{27}R^{28}$; $-S(=O)_2-NR^{29}R^{30}$; $-SF_5$; $-(CH_2)_u-O-S(=O)_2-R^{31}$ mit u = 1, 2 oder 3; $-(CH_2)_v-O-S(=O)_2-O-R^{32}$ mit v = 1, 2 oder 3; $-(CH_2)_w-O-P(=O)(OR^{33})(OR^{34})$ mit w = 1, 2 oder 3; für einen Rest ausgewählt aus der Gruppe bestehend aus Methyl, Ethyl, n-Propyl, Isopropyl, n-Butyl, Isobutyl, 2-Butyl, tert-Butyl, n-Pentyl, 2-Pentyl, 3-Pentyl, iso-Pentyl, neo-Pentyl, n-Hexyl, n-Heptyl, n-Octyl, (2,4,4)-Trimethyl-pent-2-yl, $-CF_3$, - $CF_2H$, $-CFH_2$, $-(CH_2)-OH$, $-(CH_2)-NH_2$, $-(CH_2)-CN$, $-(CH_2)-(CF_3)$, $-(CH_2)-(CHF_2)$, - $(CH_2)-(CH_2F)$, $-(CH_2)-(CH_2)-OH$, $-(CH_2)-(CH_2)-NH_2$, $-(CH_2)-(CH_2)-CN$, $-(CF_2)-(CF_3)$, - $(CH_2)-(CH_2)-(CF_3)$ und $-(CH_2)-(CH_2)-(CH_2)-OH$; für einen Rest ausgewählt aus der Gruppe bestehend aus Cyclopropyl, Cyclobutyl, Cyclopentyl, Cyclohexyl, Cycloheptyl, Cyclooctyl, Cyclopentenyl, Cyclohexenyl, Cycloheptenyl, Cyclooctenyl, Imidazolidinyl, Tetrahydrofuranyl (Tetrahydrofuryl), Piperidinyl, Piperazinyl, Morpholinyl, Pyrrolidinyl, Tetrahydrothiophenyl, Tetrahydropyranyl, Thiomorpholinyl, Dioxolanyl, Azepanyl, Diazepanyl Azocanyl und Dithiolanyl, der jeweils unsubstituiert oder mit ggf. 1, 2, 3 oder 4 Substituenten unabhängig voneinander ausgewählt aus der Gruppe bestehend aus F, Cl, Br, I, -CN, $-NO_2$, Methyl, Ethyl, n-Propyl, Isopropyl, n-Butyl, Isobutyl, 2-Butyl, tert-Butyl, n-Pentyl, neo-Pentyl, Ethenyl, Allyl, Ethinyl, Propinyl, $-C\equiv C-Si(CH_3)_3$, $-C\equiv C-Si(C_2H_5)_3$, $-C\equiv C-Si(CH_3)_3$, $-C\equiv C-Si(C_2H_5)_3$, $-CH_2-O-CH_3$, $-CH_2-O-C_2H_5$, -OH, -SH, $-SF_5$, $-NH_2$, Oxo (=0), Thioxo (=S), -C(=O)-OH, $-S-CH_3$, $-S-C_2H_5$, $-S(=O)-CH_3$, $-S(=O)_2-CH_3$, $-S(=O)-C_2H_5$, $-S(=O)_2-C_2H_5$, $-O-CH_3$, $-O-C_2H_5$, $-O-C_3H_7$, $-O-C(CH_3)_3$, $-CF_3$, $-CHF_2$, $-CH_2F$, $-O-CF_3$, $-O-CHF_2$, $-O-CH_2F$, $-C(=O)-CF_3$, $-S-CF_3$, $-S-CHF_2$, $-S-CH_2F$, $-S(=O)_2$-Phenyl, Pyrazolyl, $-N(CH_3)_2$, $-N(C_2H_5)_2$, - $NH-CH_3$, $-NH-C_2H_5$, $-CH_2-O-C(=O)$-Phenyl, $-NH-S(=O)_2-CH_3$, $-C(=O)-O-CH_3$, $-C(=O)-O-C_2H_5$, $-C(=O)-O-C(CH_3)_3$, -C(=O)-H, $-C(=O)-CH_3$, $-C(=O)-C_2H_5$, $-NH-C(=O)-CH_3$, $NH-C(=O)-C_2H_5$, -O-C(=O)-Phenyl, $-C(=O)-NH_2$, $-C(=O)-NH-CH_3$, $-C(=O)-N(CH_3)_2$, Phenyl, Furyl (Furanyl), Thiadiazolyl, Thiophenyl (Thienyl) und Benzyl substituiert sein kann;

oder für einen Rest ausgewählt aus der Gruppe bestehend aus Phenyl, Benzyl, Phenethyl, (3-Phenyl)-prop-1-yl, Furyl (Furanyl), Thienyl (Thiophenyl), Pyrazolyl, Imidazolyl, Thiazolyl, Thiadiazolyl, Triazolyl, Pyrrolyl, Oxazolyl, Oxadiazolyl, Isoxazolyl, Pyridinyl, Pyridazinyl, Pyrimidinyl, Pyrazinyl, Pyranyl, Diazinyl, Triazinyl, Tetrazinyl, Tetrazolyl, Purinyl, Dithiazolyl, Pentazolyl, Indolyl, Isoindolyl, Benzo[b]furanyl, Isobenzo[b]furanyl, Indazolyl, Indolizinyl, Chinolinyl, Isochinolinyl, Chinazolinyl, Benzo[b]thiophenyl und Isobenzo[b]thiophenyl stehen, der jeweils unsubstituiert oder mit ggf. 1, 2, 3, 4 oder 5 Substituenten unabhängig voneinander ausgewählt aus der Gruppe bestehend aus F, Cl, Br, II, -CN, $-NO_2$, Methyl, Ethyl, n-Propyl, Isopropyl, n-Butyl, Isobutyl, 2-Butyl, tert-Butyl, n-Pentyl, neo-Pentyl, Ethenyl, Allyl, Ethinyl, Propinyl, $-C\equiv C-Si(CH_3)_3$, $-C\equiv C-Si(C_2H_5)_3$, $-CH_2-O-CH_3$, $-CH_2-O-C_2H_5$, - OH, -SH, $-SF_5$, $-NH_2$, -C(=O)-OH, $-S-CH_3$, $-S-C_2H_5$, $-S(=O)-CH_3$, $-S(=O)_2-CH_3$, - $S(=O)-C_2H_5$, $-S(=O)_2-C_2H_5$, $-O-CH_3$, $-O-C_2H_5$, $-O-C_3H_7$, $-O-C(CH_3)_3$, $-CF_3$, $-CHF_2$, - $CH_2F$, $-O-CF_3$, $-O-CHF_2$, $-O-CH_2F$, $-C(=O)-CF_3$, $-S-CF_3$, $-S-CHF_2$, $-S-CH_2F$, $-S(=O)_2$-Phenyl, Pyrazolyl, $-N(CH_3)_2$, $-N(C_2H_5)_2$, $-NH-CH_3$, $-NH-C_2H_5$, $-CH_2-O-C(=O)$-Phenyl, - $NH-S(=O)_2-CH_3$, $-C(=O)-O-CH_3$, $-C(=O)-O-C_2H_5$, $-C(=O)-O-C(CH_3)_3$, -C(=O)-H, - $C(=O)-CH_3$, $-C(=O)-C_2H_5$, $-NH-C(=O)-CH_3$, $-NH-C(=O)-C_2H_5$, -O-C(=O)-Phenyl, - $C(=O)-NH_2$, $-C(=O)-NH-CH_3$, $-C(=O)-N(CH_3)_2$, $-Si(Phenyl)_2[C(CH_3)_3]$, (1,3)-Dioxolanyl, Cyclopropyl, Cyclobutyl, Cyclopentyl, Cyclohexyl, Phenyl, Furyl (Furanyl), Thiadiazolyl, Thiophenyl (Thienyl) und Benzyl substituiert sein kann;

und jeweils die übrigen Reste die vorstehend genannte Bedeutung haben, jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Ebenfalls bevorzugt sind substituierte Imidazo[2,1-b]thiazol-Verbindungen der vorstehend angegebenen allgemeinen Formel I, worin

$R^3$ und $R^4$, unabhängig voneinander, jeweils für einen Wasserstoff-Rest; $-C(=O)-R^{21}$; $-(CH_2)_q-C(=O)-R^{22}$ mit q = 1, 2 oder 3; $-C(=O)-O-R^{23}$; $-(CH_2)_r-C(=O)-O-R^{24}$ mit r = 1, 2 oder 3; $-C(=O)-NHR^{25}$; $-(CH_2)_s-C(=O)-NHR^{26}$ mit s = 1, 2 oder 3; für einen Rest ausgewählt aus der Gruppe bestehend aus Methyl, Ethyl, n-Propyl, Isopropyl, n-Butyl, Isobutyl, 2-Butyl, tert-Butyl, n-Pentyl, 2-Pentyl, 3-Pentyl, iso-Pentyl, neo-Pentyl, n-Hexyl, n-Heptyl, n-Octyl, (2,4,4)-Trimethyl-pent-2-yl, $-CF_3$, - $CF_2H$, $-CFH_2$, $-(CH_2)-OH$, $-(CH_2)-NH_2$, $-(CH_2)-CN$, $-(CH_2)-(CF_3)$, $-(CH_2)-(CHF_2)$, - $(CH_2)-(CH_2F)$,

$-(CH_2)-(CH_2)-OH$, $-(CH_2)-(CH_2)-NH_2$, $-(CH_2)-(CH_2)-CN$, $-(CF_2)-(CF_3)$, - $(CH_2)-(CH_2)-(CF_3)$ und $-(CH_2)-(CH_2)-(CH_2)-OH$; für einen Rest ausgewählt aus der Gruppe bestehend aus Cyclopropyl, Cyclobutyl, Cyclopentyl, Cyclohexyl, Cycloheptyl, Cyclooctyl, Cyclopentenyl, Cyclohexenyl, Cycloheptenyl, Cyclooctenyl, Imidazolidinyl, Tetrahydrofuranyl (Tetrahydrofuryl), Piperidinyl, Piperazinyl, Morpholinyl, Pyrrolidinyl, Tetrahydrothiophenyl, Tetrahydropyranyl, Thiomorpholinyl, Dioxolanyl, Azepanyl, Diazepanyl, Azocanyl und Dithiolanyl, der jeweils unsubstituiert oder mit ggf. 1, 2, 3 oder 4 Substituenten unabhängig voneinander ausgewählt aus der Gruppe bestehend aus F, Cl, Br, I, -CN, $-NO_2$, Methyl, Ethyl, n-Propyl, Isopropyl, n-Butyl, Isobutyl, 2-Butyl, tert-Butyl, n-Pentyl, neo-Pentyl, Ethenyl, Allyl, Ethinyl, Propinyl, $-C{\equiv}C-Si(CH_3)_3$, $-C{\equiv}C-Si(C_2H_5)_3$, $-C{\equiv}C-Si(CH_3)_3$, $-C{\equiv}C-Si(C_2H_5)_3$, $-CH_2-O-CH_3$, $-CH_2-O-C_2H_5$, -OH, -SH, $-SF_5$, $-NH_2$, Oxo (=0), Thioxo (=S), -C(=O)-OH, $-S-CH_3$, $-S-C_2H_5$, $-S(=O)-CH_3$, $-S(=O)_2-CH_3$, $-S(=O)-C_2H_5$, $-S(=O)_2-C_2H_5$, $-O-CH_3$, $-O-C_2H_5$, $-O-C_3H_7$, $-O-C(CH_3)_3$, $-CF_3$, $-CHF_2$, $-CH_2F$, $-O-CF_3$, $-O-CHF_2$, $-O-CH_2F$, $-C(=O)-CF_3$, $-S-CF_3$, $-S-CHF_2$, $-S-CH_2F$, $-S(=O)_2$-Phenyl, Pyrazolyl, $-N(CH_3)_2$, $-N(C_2H_5)_2$, - $NH-CH_3$, $-NH-C_2H_5$, $-CH_2-O-C(=O)$-Phenyl, $-NH-S(=O)_2-CH_3$, $-C(=O)-O-CH_3$, $-C(=O)-O-C_2H_5$,- $C(=O)-O-C(CH_3)_3$, -C(=O)-H, $-C(=O)-CH_3$, $-C(=O)-C_2H_5$, $-NH-C(=O)-CH_3$, - $NH-C(=O)-C_2H_5$, -O-C(=O)-Phenyl, $-C(=O)-NH_2$, $-C(=O)-NH-CH_3$, $-C(=O)-N(CH_3)_2$, Phenyl, Furyl (Furanyl), Thiadiazolyl, Thiophenyl (Thienyl) und Benzyl substituiert und/oder über eine lineare oder verzweigte $C_{1-3}$-Alkylen-Gruppe gebunden sein kann; oder für einen Rest ausgewählt aus der Gruppe bestehend aus Phenyl, Furyl (Furanyl), Thienyl (Thiophenyl), Pyrazolyl, Imidazolyl, Thiazolyl, Thiadiazolyl, Triazolyl, Pyrrolyl, Oxazolyl, Oxadiazolyl, Isoxazolyl, Pyridinyl, Pyridazinyl, Pyrimidinyl, Pyrazinyl, Pyranyl, Diazinyl, Triazinyl, Tetrazinyl, Tetrazolyl, Purinyl, Dithiazolyl, Pentazolyl, Indolyl, Isoindolyl, Benzo[b]furanyl, Isobenzo[b]furanyl, Indazolyl, Indolizinyl, Chinolinyl, Isochinolinyl, Chinazolinyl, Benzo[b]thiophenyl und Isobenzo[b]thiophenyl stehen, der jeweils unsubstituiert oder mit ggf. 1, 2, 3, 4 oder 5 Substituenten unabhängig voneinander ausgewählt aus der Gruppe bestehend aus F, Cl, Br, I, -CN, $-NO_2$, Methyl, Ethyl, n-Propyl, Isopropyl, n-Butyl, Isobutyl, 2-Butyl, tert-Butyl, n-Pentyl, neo-Pentyl, Ethenyl, Allyl, Ethinyl, Propinyl, $-C{\equiv}C-Si(CH_3)_3$,$-C{\equiv}C-Si(C_2H_5)_3$, $-CH_2-O-CH_3$, $-CH_2-O-C_2H_5$, -OH, -SH, $-SF_5$, $-NH_2$, -C(=O)-OH, $-S-CH_3$, $-S-C_2H_5$, $-S(=O)-CH_3$, $-S(=O)_2-CH_3$, $-S(=O)-C_2H_5$, $-S(=O)_2-C_2H_5$, $-O-CH_3$, $-O-C_2H_5$, $-O-C_3H_7$, $-O-C(CH_3)_3$, $-CF_3$, $-CHF_2$, $-CH_2F$, $-O-CF_3$, $-O-CHF_2$, $-O-CH_2F$, $-C(=O)-CF_3$, $-S-CF_3$, $-S-CHF_2$, $-S-CH_2F$, $-S(=O)_2$-Phenyl, Pyrazolyl, $-N(CH_3)_2$, $-N(C_2H_5)_2$,$-NH-CH_3$, $-NH-C_2H_5$, $-CH_2-O-C(=O)$-Phenyl, $-NH-S(=O)_2-CH_3$, $-C(=O)-O-CH_3$, $-C(=O)-O-C_2H_5$, $-C(=O)-O-C(CH_3)_3$, -C(=O)-H, $-C(=O)-CH_3$, $-C(=O)-C_2H_5$, $-NH-C(=O)-CH_3$,$-NH-C(=O)-C_2H_5$, -O-C(=O)-Phenyl, $-C(=O)-NH_2$, $-C(=O)-NH-CH_3$, $-C(=O)-N(CH_3)_2$,$-Si(Phenyl)_2[C(CH_3)_3]$, (1,3)-Dioxolanyl, Cyclopropyl, Cyclobutyl, Cyclopentyl, Cyclohexyl, Phenyl, Furyl (Furanyl), Thiadiazolyl, Thiophenyl (Thienyl) und Benzyl substituiert und/oder über eine lineare oder verzweigte $C_{1-3}$-Alkylen-Gruppe gebunden sein kann;

oder $R^3$ und $R^4$ zusammen mit dem sie verbindenden Stickstoffatom als Ringglied einen Rest bilden ausgewählt aus der Gruppe bestehend aus Imidazolidinyl, Piperidinyl, Piperazinyl, Morpholinyl, Pyrrolidinyl, Thiomorpholinyl, Azepanyl, Diazepanyl und Azocanyl, der jeweils unsubstituiert oder mit ggf. 1, 2, 3, 4 oder 5 Substituenten unabhängig voneinander ausgewählt aus der Gruppe bestehend aus F, Cl, Br, I, -CN, Methyl, Ethyl, n-Propyl, Isopropyl, n-Butyl, 2-Butyl, Isobutyl, tert-Butyl, Ethenyl, Allyl, Ethinyl, Propinyl, $-C{\equiv}C-Si(CH_3)_3$, $-C{\equiv}C-Si(C_2H_5)_3$, -OH, Oxo, Thioxo, $-O-CH_3$, $-O-C_2H_5$, $-O-C_3H_7$, $-(CH_2)-O-CH_3$, $-(CH_2)-O-C_2H_5$, $-NH_2$, $-N(CH_3)_2$, - $N(C_2H_5)_2$, $-NH-CH_3$, $-NH-C_2H_5$, $-NO_2$, $-CF_3$, $-O-CF_3$, $-S-CF_3$, -SH, $-SF_5$, $-S-CH_3$, $-S-C_2H_5$, $-S(=O)-CH_3$, $-S(=O)_2-CH_3$, $-S(=O)-C_2H_5$, $-S(=O)_2-C_2H_5$, $-NH-S(=O)_2-CH_3$,-C(=O)-OH, -C(=0)-H;$-C(=O)-CH_3$,$-C(=O)-C_2H_5$,$-C(=O)-N(CH_3)_2$,$-C(=O)-NH-CH_3$,$-C(=O)-NH_2$,$-NH-C(=O)-CH_3$,$-NH-C(=O)-C_2H_5$, $-C(=O)-O-CH_3$, $-C(=O)-O-C_2H_5$,$-C(=O)-O-C(CH_3)_3$ und Phenyl substituiert sein kann;

und jeweils die übrigen Reste die vorstehend genannte Bedeutung haben, jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Ebenfalls bevorzugt sind substituierte Imidazo[2,1-b]thiazol-Verbindungen der vorstehend angegebenen allgemeinen Formel I, worin

$R^5$, $R^6$, $R^7$, $R^8$, $R^{10}$, $R^{11}$, $R^{12}$, $R^{15}$ und $R^{16}$, jeweils unabhängig voneinander, für einen Rest ausgewählt aus der Gruppe bestehend aus Methyl, Ethyl, n-Propyl, Isopropyl, n-Butyl, Isobutyl, 2-Butyl, tert-Butyl, n-Pentyl, 2-Pentyl, 3-Pentyl, iso-Pentyl, neo-Pentyl, n-Hexyl, n-Heptyl, n-Octyl, (2,4,4)-Trimethyl-pent-2-yl, $-CF_3$, $-CF_2H$, $-CFH_2$, $-(CH_2)-OH$, $-(CH_2)-NH_2$, $-(CH_2)-NH-CH_3$, $-(CH_2)-N(CH_3)_2$, $-(CH_2)-CN$, $-(CH_2)-(CF_3)$, $-(CH_2)-(CHF_2)$, $-(CH_2)-(CH_2F)$, $-(CH_2)-(CH_2)-OH$, $-(CH_2)-(CH_2)-NH_2$, $-(CH_2)-(CH_2)-CN$, $-(CF_2)-(CF_3)$, $-(CH_2)-(CH_2)-(CF_3)$, $-(CH_2)-(CH_2)-(CH_2)-OH$, $-(CH_2)-C(=O)-OH$, $-(CH_2)-C(=O)-O-CH_3$, $-(CH_2)-C(=O)-O-C_2H_5$, $-(CH_2)-(CH_2)-C(=O)-OH$, $-(CH_2)-(CH_2)-C(=O)-O-CH_3$ und $-(CH_2)-(CH_2)-C(=O)-O-C_2H_5$; oder für einen Rest ausgewählt aus der Gruppe bestehend aus Phenyl, Benzyl, Phenethyl, Furyl (Furanyl), Thienyl (Thiophenyl), Pyrazolyl, Imidazolyl, Thiazolyl, Thiadiazolyl, Triazolyl, Pyrrolyl, Oxazolyl, Oxadiazolyl, Isoxazolyl, Pyridinyl, Pyridazinyl, Pyrimidinyl, Pyrazinyl, Pyranyl, Diazinyl, Triazinyl, Tetrazinyl, Tetrazolyl, Purinyl, Dithiazolyl, Pentazolyl, Indolyl, Isoindolyl, Benzo[b]furanyl, Isobenzo[b]furanyl, Indazolyl, Indolizinyl, Chinolinyl, Isochinolinyl, Chinazolinyl, Benzo[b]thiophenyl und Isobenzo[b]thiophenyl stehen, der jeweils unsubstituiert oder mit ggf. 1, 2, 3, 4 oder 5 Substituenten unabhängig voneinander ausgewählt aus der Gruppe bestehend aus F, Cl, Br, I, -CN, $-NO_2$, Methyl, Ethyl, n-Propyl, Isopropyl, n-Butyl, Isobutyl, 2-Butyl, tert-Butyl, n-Pentyl, neo-Pentyl, Ethenyl, Allyl, Ethinyl, Propinyl, -OH, -SH, $-SF_5$, $-NH_2$, -C(=O)-OH, $-S-CH_3$, $-S-C_2H_5$, $-S(=O)-CH_3$, $-S(=O)_2-CH_3$, $-S(=O)-C_2H_5$,

$-S(=O)_2-C_2H_5$, $-O-CH_3$, $-O-C_2H_5$, $-O-C_3H_7$, $-O-C(CH_3)_3$, $-CF_3$, $-CHF_2$, $-CH_2F$, $-O-CF_3$, $-O-CHF_2$, $-O-CH_2F$, $-C(=O)-CF_3$, $-S-CF_3$, $-S-CHF_2$ und $-S-CH_2F$ substituiert sein kann;
und jeweils die übrigen Reste die vorstehend genannte Bedeutung haben, jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Ferner bevorzugt sind substituierte Imidazo[2,1-b]thiazol-Verbindungen der vorstehend angegebenen allgemeinen Formel I, worin
$R^9$, $R^{13}$, $R^{14}$, $R^{17}$, $R^{18}$, $R^{19}$, $R^{20}$ $R^{21}$, $R^{22}$, $R^{23}$, $R^{24}$, $R^{25}$, $R^{26}$, $R^{27}$, $R^{28}$, $R^{29}$, $R^{30}$, $R^{31}$, $R^{32}$, $R^{33}$ und $R^{34}$, jeweils unabhängig voneinander, für einen Wasserstoff-Rest; für einen Rest ausgewählt aus der Gruppe bestehend aus Methyl, Ethyl, n-Propyl, Isopropyl, n-Butyl, Isobutyl, 2-Butyl, tert-Butyl, n-Pentyl, 2-Pentyl, 3-Pentyl, iso-Pentyl, neo-Pentyl, n-Hexyl, n-Heptyl, n-Octyl, (2,4,4)-Trimethyl-pent-2-yl, $-CF_3$, $-CF_2H$, $-CFH_2$, $-(CH_2)-OH$, $-(CH_2)-NH_2$, $-(CH_2)-NH-CH_3$, $-(CH_2)-N(CH_3)_2$, $-(CH_2)-CN$, $-(CH_2)-(CF_3)$, $-(CH_2)-(CHF_2)$, $-(CH_2)-(CH_2F)$, $-(CH_2)-(CH_2)-OH$, $-(CH_2)-(CH_2)-NH_2$, $-(CH_2)-(CH_2)-CN$, $-(CF_2)-(CF_3)$, $-(CH_2)-(CH_2)-(CF_3)$, $-(CH_2)-(CH_2)-(CH_2)-OH$, $-(CH_2)-C(=O)-OH$, $-(CH_2)-C(=O)-O-CH_3$, $-(CH_2)-C(=O)-O-C_2H_5$, $-(CH_2)-(CH_2)-C(=O)-OH$, $-(CH_2)-(CH_2)-C(=O)-O-CH_3$ und-$(CH_2)-(CH_2)-C(=O)-O-C_2H_5$; oder für einen Rest ausgewählt aus der Gruppe bestehend aus Phenyl, Benzyl, Phenethyl, Furyl (Furanyl), Thienyl (Thiophenyl), Pyrazolyl, Imidazolyl, Thiazolyl, Thiadiazolyl, Triazolyl, Pyrrolyl, Oxazolyl, Oxadiazolyl, Isoxazolyl, Pyridinyl, Pyridazinyl, Pyrimidinyl, Pyrazinyl, Pyranyl, Diazinyl, Triazinyl, Tetrazinyl, Tetrazolyl, Purinyl, Dithiazolyl, Pentazolyl, Indolyl, Isoindolyl, Benzo[b]furanyl, Isobenzo[b]furanyl, Indazolyl, Indolizinyl, Chinolinyl, Isochinolinyl, Chinazolinyl, Benzo[b]thiophenyl und Isobenzo[b]thiophenyl stehen, der jeweils unsubstituiert oder mit ggf. 1, 2, 3, 4 oder 5 Substituenten unabhängig voneinander ausgewählt aus der Gruppe bestehend aus F, Cl, Br, I, -CN, $-NO_2$, Methyl, Ethyl, n-Propyl, Isopropyl, n-Butyl, Isobutyl, 2-Butyl, tert-Butyl, n-Pentyl, neo-Pentyl, Ethenyl, Allyl, Ethinyl, Propinyl, -OH, -SH, $-SF_5$, $-NH_2$, $-C(=O)-OH$, $-S-CH_3$, $-S-C_2H_5$, $-S(=O)-CH_3$, $-S(=O)_2-CH_3$, $-S(=O)-C_2H_5$, $-S(=O)_2-C_2H_5$, $-O-CH_3$, $-O-C_2H_5$, $-O-C_3H_7$, $-O-C(CH_3)_3$, $-CF_3$, $-CHF_2$, $-CH_2F$, $-O-CFH_3$, $-O-CHF_2$, $-O-CH_2F$, $-C(=O)-CF_3$, $-S-CF_3$, $-S-CHF_2$ und $-S-CH_2F$ substituiert sein kann;
und jeweils die übrigen Reste die vorstehend genannte Bedeutung haben, jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Ebenfalls bevorzugt sind substituierte Imidazo[2,1-b]thiazol-Verbindungen der vorstehend angegebenen allgemeinen Formel I, worin
$M^1$ für einen Rest ausgewählt aus der Gruppe bestehend aus Phenyl, Furanyl, Thiophenyl (Thienyl), Pyrazolyl, Pyrazinyl, Pyridazinyl, Pyrimidinyl, Pyridinyl, Pyrrolyl, Oxazolyl, Isoxazolyl, Thiazolyl, Thiadiazolyl, Imidazolyl, Oxadiazolyl, Triazolyl, Diazinyl, Triazinyl, Tetrazinyl und Tetrazolyl steht, der jeweils unsubstituiert oder mit ggf. 1, 2, 3 oder 4 Substituenten unabhängig voneinander ausgewählt aus der Gruppe bestehend aus F, Cl, Br, I, -CN, $-CH_2-CN$, $-NO_2$, Methyl, Ethyl, n-Propyl, Isopropyl, n-Butyl, Isobutyl, 2-Butyl, tert-Butyl, n-Pentyl, neo-Pentyl, Ethenyl, Allyl, Ethinyl, Propinyl, -OH, -SH, $-SF_5$, $-NH_2$, $-C(=O)-OH$, $-S-CH_3$, $-S-C_2H_5$, $-S(=O)-CH_3$,$-S(=O)_2-CH_3$, $-S(=O)-C_2H_5$, $-S(=O)_2-C_2H_5$, $-O-CH_3$, $-O-C_2H_5$, $-O-C_3H_7$, $-O-C(CH_3)_3$,$-CF_3$, $-CHF_2$, $-CH_2F$, $-O-CF_3$, $-O-CHF_2$, $-O-CH_2F$, $-C(=O)-CF_3$, $-S-CF_3$, $-S-CHF_2$ und-$S-CH_2F$ substituiert sein kann;
und jeweils die übrigen Reste die vorstehend genannte Bedeutung haben, jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Ferner bevorzugt sind substituierte Imidazo[2,1-b]thiazol-Verbindungen der vorstehend angegebenen allgemeinen Formel I, worin

$M^1$ für einen Rest ausgewählt aus der Gruppe bestehend aus den Resten 1 bis 38 steht,

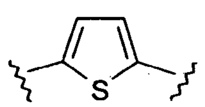
1

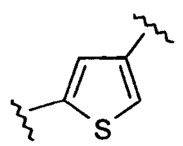
2

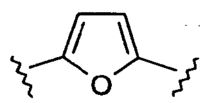
3

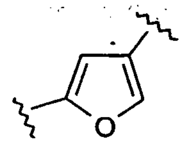
4

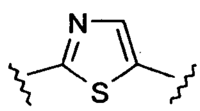
5

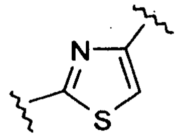
6

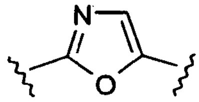
7

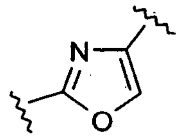
8

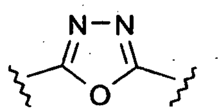
9

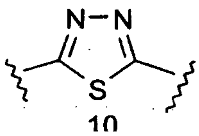
10

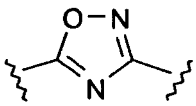
11

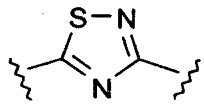
12

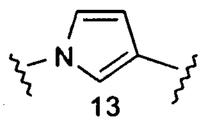
13

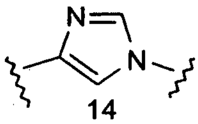
14

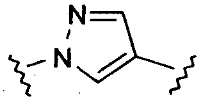
15

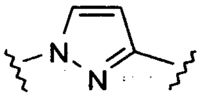
16

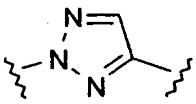
17

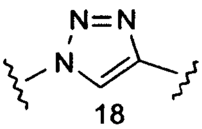
18

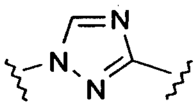
19

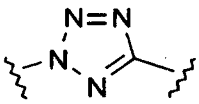
20

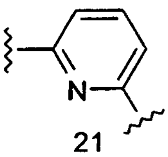
21

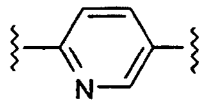
22

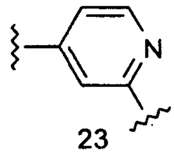
23

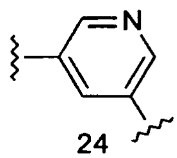
24

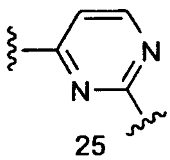
25

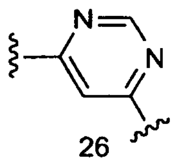
26

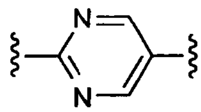
27

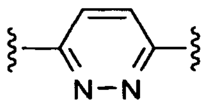
28

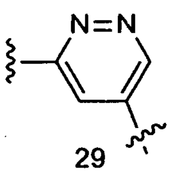
29

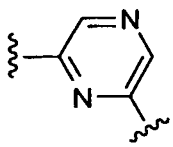
30

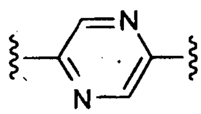
31

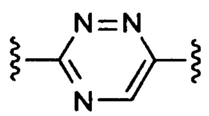
32

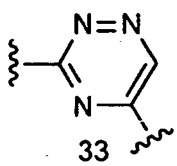
33

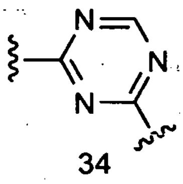
34

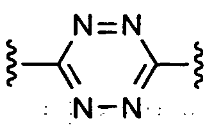
35

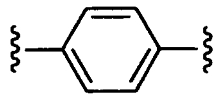
36

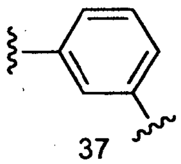 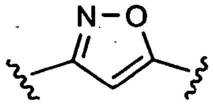

37 38

der jeweils unsubstituiert oder mit ggf. 1, 2, 3 oder 4 Substituenten unabhängig voneinander ausgewählt aus der Gruppe bestehend aus F, Cl, Br, I, -CN, $-CH_2-CN$,$-NO_2$, Methyl, Ethyl, n-Propyl, Isopropyl, n-Butyl, Isobutyl, 2-Butyl, tert-Butyl, n-Pentyl, neo-Pentyl, Ethenyl, Allyl, Ethinyl, Propinyl, -OH, -SH, $-SF_5$, $-NH_2$, -C(=O)-OH, $-S-CH_3$, $-S-C_2H_5$, $-S(=O)-CH_3$, $-S(=O)_2-CH_3$, $-S(=O)-C_2H_5$, $-S(=O)_2-C_2H_5$, $-O-CH_3$, $-O-C_2H_5$, $-O-C_3H_7$, $-O-C(CH_3)_3$, $-CF_3$, $-CHF_2$, $-CH_2F$, $-O-CF_3$, $-O-CHF_2$, $-O-CH_2F$, $-C(=O)-CF_3$, $-S-CF_3$, $-S-CHF_2$ und $-S-CH_2F$ substituiert sein kann, und der jeweils in beliebiger Richtung über die mit einer geschlängelten Linie gekennzeichneten Positionen mit dem Bizyklus und dem Kohlenstoffatom der Dreifachbindung verknüpft sein kann;

und jeweils die übrigen Reste die vorstehend genannte Bedeutung haben, jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Weiterhin bevorzugt sind substituierte Imidazo[2,1-b]thiazol-Verbindungen der vorstehend angegebenen allgemeinen Formel I, worin

$M^2$ für einen Rest ausgewählt aus der Gruppe bestehend aus Phenyl, Furanyl, Thiophenyl (Thienyl), Pyrazolyl, Pyrazinyl, Pyridazinyl, Pyrimidinyl, Pyridinyl, Pyrrolyl, Oxazolyl, Isoxazolyl, Thiazolyl, Thiadiazolyl, Oxadiazolyl, Triazolyl, Diazinyl, Triazinyl, Tetrazinyl, Tetrazolyl, Pentazolyl, Imidazolyl, Chinolinyl, Isochinolinyl, Naphthyl, Indolyl, Isoindolyl, Benzofuranyl, Isobenzofuranyl, Benzothiophenyl und Isobenzothiophenyl steht, der jeweils unsubstituiert oder mit ggf. 1, 2, 3, 4 oder 5 Substituenten unabhängig voneinander ausgewählt aus der Gruppe bestehend aus F, Cl, Br, I, -CN, $-NO_2$, Methyl, Ethyl, n-Propyl, Isopropyl, n-Butyl, Isobutyl, 2-Butyl, tert-Butyl, n-Pentyl, neo-Pentyl, Ethenyl, Allyl, Ethinyl, Propinyl, $-C{\equiv}C-Si(CH_3)_3$,$-C{\equiv}C-Si(C_2H_5)_3$, $-CH_2-O-CH_3$, $-CH_2-O-C_2H_5$, -OH, -SH, $-SF_5$, $-NH_2$, -C(=O)-OH, $-S-CH_3$, $-S-C_2H_5$, $-S(=O)-CH_3$, $-S(=O)_2-CH_3$, $-S(=O)-C_2H_5$, $-S(=O)_2-C_2H_5$, $-O-CH_3$, $-O-C_2H_5$, $-O-C_3H_7$, $-O-C(CH_3)_3$, $-CF_3$, $-CHF_2$, $-CH_2F$, $-O-CF_3$, $-O-CHF_2$, $-O-CH_2F$, $-C(=O)-CF_3$, $-S-CF_3$, $-S-CHF_2$, $-S-CH_2F$, $-S(=O)_2$-Phenyl, Pyrazolyl, $-N(CH_3)_2$, $-N(C_2H_5)_2$,$-NH-CH_3$, $-NH-C_2H_5$, $-CH_2-O-C(=O)$-Phenyl, $-NH-S(=O)_2-CH_3$, $-C(=O)-O-CH_3$, $-C(=O)-O-C_2H_5$, $-C(=O)-O-C(CH_3)_3$, -C(=O)-H, $-C(=O)-CH_3$, $-C(=O)-C_2H_5$, $-NH-C(=O)-CH_3$,$-NH-C(=O)-C_2H_5$, -O-C(=O)-Phenyl, $-C(=O)-NH_2$, $-C(=O)-NH-CH_3$, $-C(=O)-N(CH_3)_2$,$-Si(Phenyl)_2[C(CH_3)_3]$, $-CH_2-NH_2$, Pyrrolyl, $-NH-S(=O)_2-C_2H_5$, $-S(=O)_2-NH_2$, $-S(=O)_2-NH-CH_3$, $-CH_2-OH$, $-NH-C(=NH)-NH_2$, $-NH-S(=O)_2-OH$, $-S(=O)_2-N(CH_3)_2$, (1,3)-Dioxolanyl, Cyclopropyl, Cyclobutyl, Cyclopentyl, Cyclohexyl, Phenyl, Furyl (Furanyl), Thiadiazolyl, Thiophenyl (Thienyl) und Benzyl substituiert sein kann;

und jeweils die übrigen Reste die vorstehend genannte Bedeutung haben, jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Ebenfalls bevorzugt sind substituierte Imidazo[2,1-b]thiazol-Verbindungen der vorstehend angegebenen allgemeinen Formel I, worin

$M^2$ für einen Rest ausgewählt aus der Gruppe bestehend aus den Resten 1 bis 36 steht,

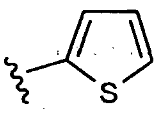 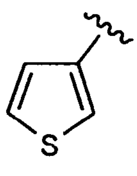 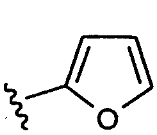 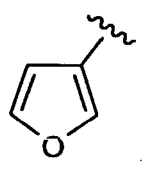 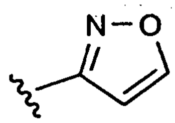

1 2 3 4 5

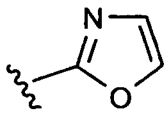
6
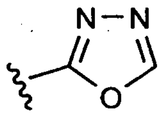
7
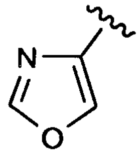
8
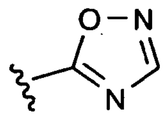
9
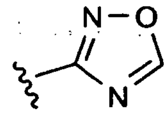
10

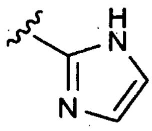
11
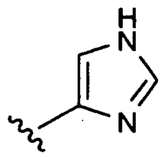
12
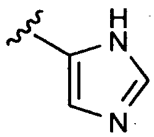
13
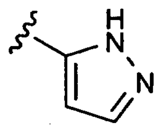
14
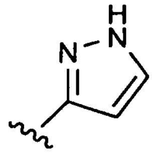
15

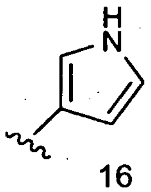
16
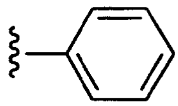
17
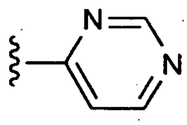
18
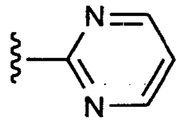
19
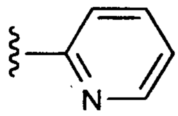
20

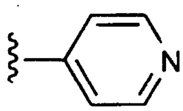
21
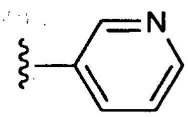
22
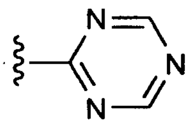
23
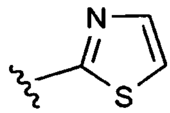
24
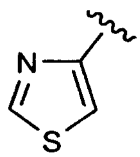
25

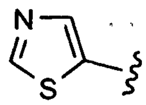
26
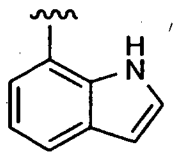
27
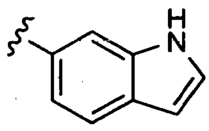
28
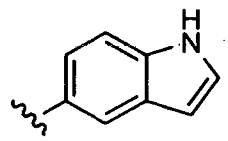
29

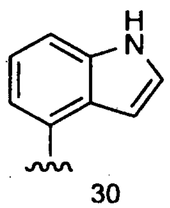
30
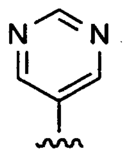
31
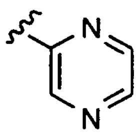
32
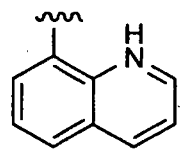
33

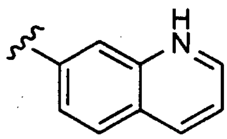
34
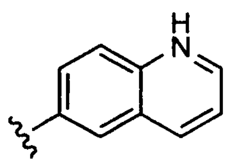
35
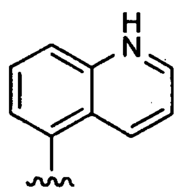
36

der jeweils über die mit einer geschlängelten Linie gekennzeichnete Position mit dem Kohlenstoffatom der Dreifachbindung verknüpft und unsubstituiert oder mit ggf. 1, 2, 3, 4 oder 5 Substituenten unabhängig voneinander ausgewählt aus der Gruppe bestehend aus F, Cl, Br, -CN, $-CF_3$, $-SF_5$, $-S-CH_3$, $-S-C_2H_5$, Methyl, Ethyl, n-Propyl, iso-Propyl, n-Butyl, sec-Butyl, iso-Butyl, tert-Butyl, Ethenyl, Propenyl, -OH, $-O-CH_3$,$-O-C_2H_5$, $-O-C_3H_7$, $-NH_2$, $-N(CH_3)_2$, $-NH-CH_3$, $-CH_2-NH_2$, $-N(C_2H_5)_2$, $-NO_2$, $-O-CF_3$, $-S-CF_3$, -SH, -C(=O)-H, $-C(=O)-O-CH_3$, $-C(=O)-O-C_2H_5$, Phenyl, Pyrrolyl, (1,3)-Dioxolanyl, $-Si(Phenyl)_2[C(CH_3)_3]$, $-C(=O)-CH_3$, $-C(=O)-C_2H_5$, $NH-S(=O)_2-CH_3$, $-NH-S(=O)_2-C_2H_5$, $-S(=O)_2-NH_2$, $-S(=O)_2-NH-CH_3$, $-CH_2-OH$, -C(=O)-OH, $-CH_2-O-CH_3$,$-C(=O)-NH_2$, $-C(=O)-NH-CH_3$, $-NH-C(=O)-CH_3$, $-NH-C(=NH)-NH_2$, $-NH-S(=O)_2-OH$ und $-S(=O)_2-N(CH_3)_2$ substituiert sein kann;

und jeweils die übrigen Reste die vorstehend genannte Bedeutung haben, jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Besonders bevorzugt sind substituierte Imidazo[2,1-b]thiazol-Verbindungen der vorstehend angegebenen allgemeinen Formel I, worin

$R^1$ und $R^2$, unabhängig voneinander, jeweils für einen Wasserstoff-Rest; -F; -Cl; -Br;-I; $-NO_2$; -CN; $-NH_2$; $-NHR^5$; $-NR^6R^7$; $-C(=O)-R^9$, $-C(=O)-NH_2$; $-C(=O)-NHR^{10}$; $-C(=O)-NR^{11}R^{12}$; $-C(=O)-OR^{13}$; $-(CH_2)_m-C(=O)-OR^{14}$ mit m = 1, 2 oder 3; $-O-C(=O)-R^{15}$;$-OR^{17}$; $-(CH_2)_o-O-R^{18}$ mit o = 1, 2 oder 3; $-S(=O)_2-NH_2$; $-SF_5$; $-(CH_2)_u-O-S(=O)_2-R^{31}$ mit u = 1, 2 oder 3; $-(CH_2)_v-O-S(=O)_2-O-R^{32}$ mit v = 1, 2 oder 3; $-(CH_2)_w-O-P(=O)(OR^{33})(OR^{34})$ mit w = 1, 2 oder 3; für einen Rest ausgewählt aus der Gruppe bestehend aus Methyl, Ethyl, n-Propyl, Isopropyl, n-Butyl, Isobutyl, 2-Butyl, tert-Butyl, n-Pentyl, 2-Pentyl, 3-Pentyl, iso-Pentyl, neo-Pentyl, n-Hexyl, n-Heptyl, n-Octyl, (2,4,4)-Trimethyl-pent-2-yl, $-CF_3$, $-CF_2H$, $-CFH_2$, $-(CH_2)-(CF_3)$, $-(CH_2)-(CHF_2)$, $-(CH_2)-(CH_2F)$ und $-(CF_2)-(CF_3)$; für einen Rest ausgewählt aus der Gruppe bestehend aus Cyclopropyl, Cyclobutyl, Cyclopentyl, Cyclohexyl, Cycloheptyl und Cyclooctyl, der jeweils unsubstituiert oder mit ggf. 1, 2, 3 oder 4 Substituenten unabhängig voneinander ausgewählt aus der Gruppe bestehend aus F, Cl, Br, I, -CN, $-NO_2$, Methyl, Ethyl, n-Propyl, Isopropyl, n-Butyl, Isobutyl, 2-Butyl, tert-Butyl, n-Pentyl, neo-Pentyl, -OH, Oxo (=0), Thioxo (=S), $-O-CH_3$, $-O-C_2H_5$, $-O-C_3H_7$, $-O-C(CH_3)_3$, $-CF_3$,$-CHF_2$, $-CH_2F$, $-O-CF_3$, $-O-CHF_2$ und $-O-CH_2F$ substituiert sein kann; oder für einen Rest ausgewählt aus der Gruppe bestehend aus Phenyl, Benzyl, Phenethyl, (3-Phenyl)-prop-1-yl, Furyl (Furanyl), Thienyl (Thiophenyl), Pyrazolyl, Imidazolyl, Thiazolyl, Thiadiazolyl, Pyridinyl, Pyridazinyl, Pyrimidinyl und Pyrazinyl stehen, der jeweils unsubstituiert oder mit ggf. 1, 2, 3, 4 oder 5 Substituenten unabhängig voneinander ausgewählt aus der Gruppe bestehend aus F, Cl, Br, I,-CN, $-NO_2$, Methyl, Ethyl, n-Propyl, Isopropyl, n-Butyl, Isobutyl, 2-Butyl, tert-Butyl, n-Pentyl, neo-Pentyl, Ethenyl, Allyl, Ethinyl, Propinyl, -OH, -SH, $-SF_5$, $-NH_2$, -C(=O)-OH, $-S-CH_3$, $-S-C_2H_5$, $-O-CH_3$, $-O-C_2H_5$, $-O-C_3H_7$, $-O-C(CH_3)_3$, $-CF_3$, $-CHF_2$, $-CH_2F$, $-O-CF_3$, $-O-CHF_2$, $-O-CH_2F$, $-C(=O)-CF_3$, $-S-CF_3$, $-S-CHF_2$, $-S-CH_2F$, $-C(=O)-O-CH_3$,$-C(=O)-O-C_2H_5$, $-C(=O)-O-C(CH_3)_3$, Cyclopropyl, Cyclobutyl, Cyclopentyl, Cyclohexyl und Phenyl substituiert sein kann;

$R^3$ und $R^4$, unabhängig voneinander, jeweils für einen Wasserstoff-Rest; $-C(=O)-R^{21}$; $-(CH_2)_q-C(=O)-R^{22}$ mit q = 1, 2 oder 3; $-C(=O)-O-R^{23}$; $-(CH_2)_r-C(=O)-O-R^{24}$ mit r = 1, 2 oder 3; $-C(=O)-NHR^{25}$; $-(CH_2)_sC(=O)-NHR^{26}$ mit s = 1, 2 oder 3; für einen Rest ausgewählt aus der Gruppe bestehend aus Methyl, Ethyl, n-Propyl, Isopropyl, n-Butyl, Isobutyl, 2-Butyl, tert-Butyl, n-Pentyl, 2-Pentyl, 3-Pentyl, iso-Pentyl, neo-Pentyl, n-Hexyl, n-Heptyl, n-Octyl und (2,4,4)-Trimethyl-pent-2-yl; für einen Rest ausgewählt aus der Gruppe bestehend aus Cyclopropyl, Cyclobutyl, Cyclopentyl, Cyclohexyl, Cycloheptyl und Cyclooctyl, der jeweils unsubstituiert oder mit ggf. 1, 2, 3 oder 4 Substituenten unabhängig voneinander ausgewählt aus der Gruppe bestehend aus F, Cl, Br, I, -CN, $-NO_2$, Methyl, Ethyl, n-Propyl, Isopropyl, n-Butyl, Isobutyl, 2-Butyl, tert-Butyl, n-Pentyl, neo-Pentyl, -OH, Oxo (=0), Thioxo (=S), - $O-CH_3$, $-O-C_2H_5$, $-O-C_3H_7$ und $-O-C(CH_3)_3$ substituiert und/oder über eine lineare oder verzweigte $C_{1-3}$-Alkylen-Gruppe gebunden sein kann;

oder für einen Rest ausgewählt aus der Gruppe bestehend aus Phenyl, Furyl (Furanyl), Thienyl (Thiophenyl), Pyrazolyl, Imidazolyl, Thiazolyl, Thiadiazolyl, Pyridinyl, Pyridazinyl, Pyrimidinyl und Pyrazinyl stehen, der jeweils unsubstituiert oder mit ggf. 1, 2, 3, 4 oder 5 Substituenten unabhängig voneinander ausgewählt aus der Gruppe bestehend aus F, Cl, Br, I, -CN, $-NO_2$, Methyl, Ethyl, n-Propyl, Isopropyl, n-Butyl, Isobutyl, 2-Butyl, tert-Butyl, n-Pentyl, neo-Pentyl, Ethenyl, Allyl, -OH, -SH,$-SF_5$, $-NH_2$, -C(=O)-0H, $-S-CH_3$, $-S-C_2H_5$, $-O-CH_3$, $-O-C_2H_5$, $-O-C_3H_7$, $-O-C(CH_3)_3$,$-CF_3$, $-CHF_2$, $-CH_2F$, $-O-CF_3$, $-O-CHF_2$, $-O-CH_2F$, $-C(=O)-CF_3$, $-S-CF_3$, $-S-CHF_2$, $-S-CH_2F$, $-N(CH_3)_2$, $-N(C_2H_5)_2$, $-NH-CH_3$, $-NH-C_2H_5$, Cyclopropyl, Cyclobutyl und Cyclopentyl substituiert und/oder über eine lineare oder verzweigte $C_{1-3}$-AlkylenGruppe gebunden sein kann;

oder $R^3$ und $R^4$ zusammen mit dem sie verbindenden Stickstoffatom als Ringglied einen Rest bilden ausgewählt aus der Gruppe bestehend aus Imidazolidinyl, Piperidinyl, Piperazinyl, Morpholinyl, Pyrrolidinyl, Thiomorpholinyl, Azepanyl, Diazepanyl und Azocanyl, der jeweils unsubstituiert oder mit ggf. 1, 2, 3, 4 oder 5 Substituenten unabhängig voneinander ausgewählt aus der Gruppe bestehend aus F, Cl, Br, I, -CN, Methyl, Ethyl, n-Propyl, Isopropyl, n-Butyl, 2-Butyl, Isobutyl, tert-Butyl, -OH, Oxo, Thioxo, $-O-CH_3$, $-O-C_2H_5$, $-O-C_3H_7$, $-NH_2$, $-N(CH_3)_2$, $-N(C_2H_5)_2$, $-NH-CH_3$, $-NH-C_2H_5$, $-NO_2$, $-CF_3$, $-O-CF_3$, $-S-CF_3$, -SH, $-SF_5$, $-S-CH_3$, $-S-C_2H_5$, $-S(=O)-CH_3$, $-S(=O)_2-CH_3$, $-S(=O)-C_2H_5$, $-S(=O)_2-C_2H_5$, $-C(=O)-CH_3$, $-C(=O)-C_2H_5$, $-C(=O)-N(CH_3)_2$, $-C(=O)-NH-CH_3$, $-C(=O)-NH_2$, $-C(=O)-O-CH_3$, $-C(=O)-O-C_2H_5$, $-C(=O)-O-C(CH_3)_3$ und Phenyl substituiert sein kann;

$R^5$, $R^6$, $R^7$, $R^{10}$, $R^{11}$, $R^{12}$ , $R^{15}$ und $R^{16}$, jeweils unabhängig voneinander, für einen Rest ausgewählt aus der Gruppe

bestehend aus Methyl, Ethyl, n-Propyl, Isopropyl, n-Butyl, Isobutyl, 2-Butyl, tert-Butyl, n-Pentyl, 2-Pentyl, 3-Pentyl, iso-Pentyl, neo-Pentyl, n-Hexyl, n-Heptyl, n-Octyl, (2,4,4)-Trimethyl-pent-2-yl, $-CF_3$, $-CF_2H$, $-CFH_2$, $-(CH_2)-(CHF_2)$, $-(CH_2)-(CH_2F)$, $-(CF_2)-(CF_3)$, $-(CH_2)-(CH_2)-C(=O)-OH$, $-(CH_2)-(CH_2)-C(=O)-O-CH_3$ und-$(CH_2)-(CH_2)-C(=O)-O-C_2H_5$; oder für einen Rest ausgewählt aus der Gruppe bestehend aus Phenyl, Benzyl, Phenethyl, Furyl (Furanyl), Thienyl (Thiophenyl), Pyrazolyl, Imidazolyl, Thiazolyl, Thiadiazolyl, Pyridinyl, Pyridazinyl, Pyrimidinyl und Pyrazinyl stehen, der jeweils unsubstituiert oder mit ggf. 1, 2, 3, 4 oder 5 Substituenten unabhängig voneinander ausgewählt aus der Gruppe bestehend aus F, Cl, Br, I, -CN, $-NO_2$, Methyl, Ethyl, n-Propyl, Isopropyl, n-Butyl, Isobutyl, 2-Butyl, tert-Butyl, n-Pentyl, neo-Pentyl, -OH, -SH, $-SF_5$, $-NH_2$, -C(=O)-OH,$-S-CH_3$, $-S-C_2H_5$, $-O-CH_3$, $-O-C_2H_5$, $-O-C_3H_7$, $-O-C(CH_3)_3$, $-CF_3$, $-CHF_2$, $-CH_2F$, $-O-CF_3$, $-O-CHF_2$, $-O-CH_2F$ und $-C(=O)-CF_3$ substituiert sein kann;

$R^9$, $R^{13}$, $R^{14}$, $R^{17}$, $R^{18}$, $R^{21}$, $R^{22}$, $R^{23}$ , $R^{24}$, $R^{25}$, $R^{26}$, $R^{31}$, $R^{32}$, $R^{33}$ und $R^{34}$ , jeweils unabhängig voneinander, für einen Wasserstoff-Rest; für einen Rest ausgewählt aus der Gruppe bestehend aus Methyl, Ethyl, n-Propyl, Isopropyl, n-Butyl, Isobutyl, 2-Butyl, tert-Butyl, n-Pentyl, 2-Pentyl, 3-Pentyl, iso-Pentyl, neo-Pentyl, n-Hexyl, n-Heptyl, n-Octyl, (2,4,4)-Trimethyl-pent-2-yl, $-CF_3$, $-CF_2H$, $-CFH_2$, $-(CH_2)-(CF_3)$, $-(CH_2)-(CHF_2)$, $-(CH_2)-(CH_2F)$ und $-(CF_2)-(CF_3)$; oder für einen Rest ausgewählt aus der Gruppe bestehend aus Phenyl, Benzyl, Phenethyl, Furyl (Furanyl), Thienyl (Thiophenyl), Pyrazolyl, Imidazolyl, Thiazolyl, Thiadiazolyl, Pyridinyl, Pyridazinyl, Pyrimidinyl und Pyrazinyl stehen, der jeweils unsubstituiert oder mit ggf. 1, 2, 3, 4 oder 5 Substituenten unabhängig voneinander ausgewählt aus der Gruppe bestehend aus F, Cl, Br, I, -CN, $-NO_2$, Methyl, Ethyl, n-Propyl, Isopropyl, n-Butyl, Isobutyl, 2-Butyl, tert-Butyl, n-Pentyl, neo-Pentyl, -OH, -SH, $-SF_5$, $-NH_2$, -C(=O)-OH,$-S-CH_3$, $-S-C_2H_5$, $-O-CH_3$, $-O-C_2H_5$, $-O-C_3H_7$, $-O-C(CH_3)_3$, $-CF_3$, $-CHF_2$, $-CH_2F$, $-O-CF_3$, $-O-CHF_2$, $-O-CH_2F$ und $-C(=O)-CF_3$ substituiert sein kann;

$M^1$ für einen Rest ausgewählt aus der Gruppe bestehend aus den Resten aus den Resten 1 bis 9, 11, 21, 22 und 36 bis 38 steht,

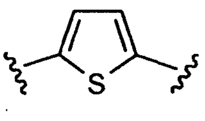
1

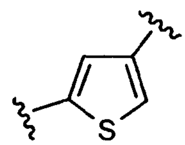
2

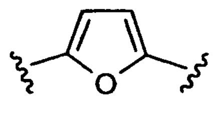
3

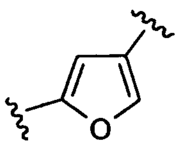
4

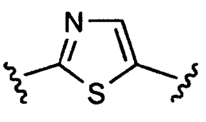
5

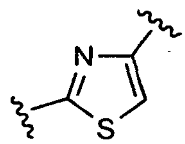
6

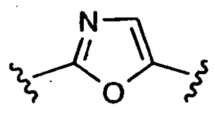
7

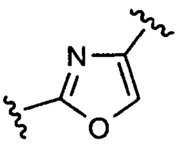
8

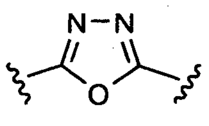
9

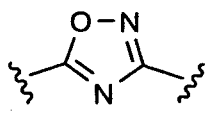
11

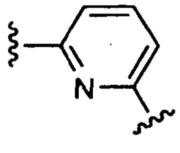
21

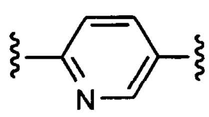
22

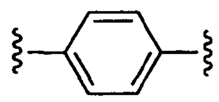
36

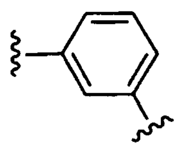
37

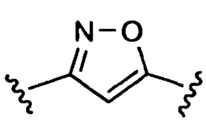
38

der jeweils unsubstituiert oder mit ggf. 1 oder 2 Substituenten unabhängig voneinander ausgewählt aus der Gruppe bestehend aus F, Cl, Br, I, -CN, $-NO_2$, Methyl, Ethyl, n-Propyl, Isopropyl, n-Butyl, Isobutyl, 2-Butyl, tert-Butyl, n-Pentyl, neo-Pentyl, Ethenyl, Allyl, Ethinyl, Propinyl, -OH, -SH, $-SF_5$, $-NH_2$, -C(=O)-OH, $-S-CH_3$, $-S-C_2H_5$, $-S(=O)-CH_3$,

$-S(=O)_2-CH_3$, $-S(=O)-C_2H_5$, $-S(=O)_2-C_2H_5$, $-O-CH_3$, $-O-C_2H_5$, $-O-C_3H_7$, $-O-C(CH_3)_3$, $-CF_3$, $-CHF_2$, $-CH_2F$, $-O-CF_3$, $-O-CHF_2$, $-O-CH_2F$, $-C(=O)-CF_3$, $-S-CF_3$, $-S-CHF_2$ und $-S-CH_2F$ substituiert sein kann, und der jeweils in beliebiger Richtung über die mit einer geschlängelten Linie gekennzeichneten Positionen mit dem Bizyklus und dem Kohlenstoffatom der Dreifachbindung verknüpft sein kann;

und $M^2$ für einen Rest ausgewählt aus der Gruppe bestehend aus den Resten 1 bis 36 steht,

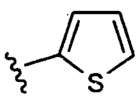
1
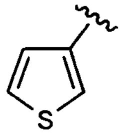
2
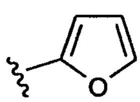
3
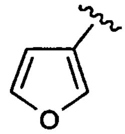
4
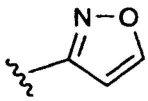
5

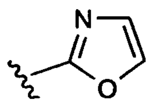
6
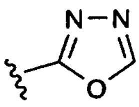
7
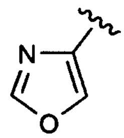
8
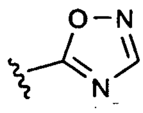
9
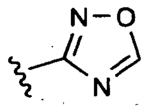
10

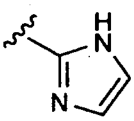
11
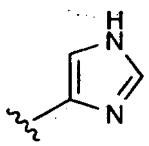
12
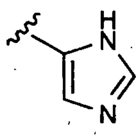
13
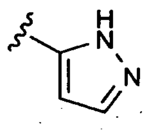
14
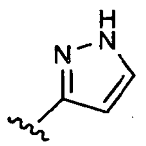
15

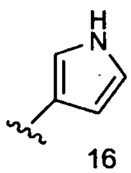
16
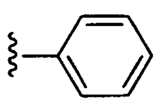
17
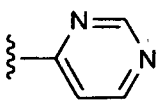
18
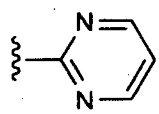
19
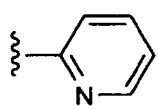
20

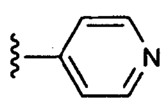
21
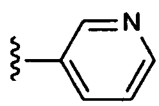
22
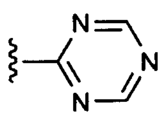
23
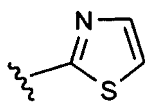
24
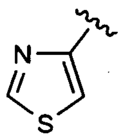
25

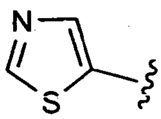
26
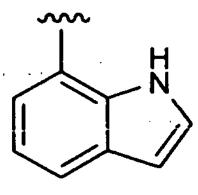
27
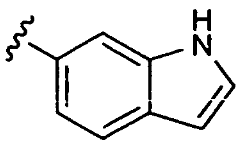
28
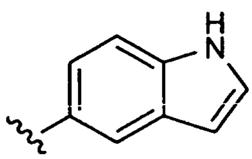
29

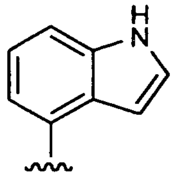
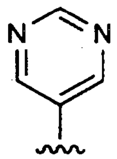
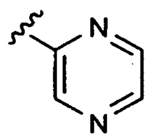
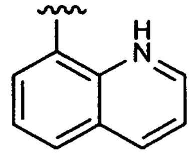

30 31 32 33

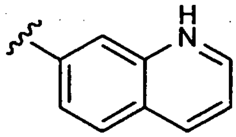
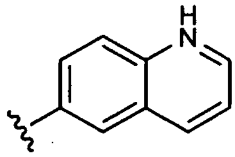
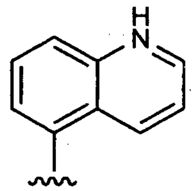

34 35 36

der jeweils über die mit einer geschlängelten Linie gekennzeichnete Position mit dem Kohlenstoffatom der Dreifachbindung verknüpft und unsubstituiert oder mit ggf. 1, 2, 3, 4 oder 5 Substituenten unabhängig voneinander ausgewählt aus der Gruppe bestehend aus F, Cl, Br, -CN, $-CF_3$, $-SF_5$, $-S-CH_3$, $-S-C_2H_5$, Methyl, Ethyl, n-Propyl, iso-Propyl, n-Butyl, sec-Butyl, iso-Butyl, tert-Butyl, Ethenyl, Propenyl, -OH, $-O-CH_3$,$-O-C_2H_5$, $-O-C_3H_7$, $-NH_2$, $-N(CH_3)_2$, $-NH-CH_3$, $-CH_2-NH_2$, $-N(C_2H_5)_2$, $-NO_2$, $-O-CF_3$, $-S-CF_3$, -SH, -C(=O)-H, $-C(=O)-O-CH_3$, $-C(=O)-O-C_2H_5$, Phenyl, Pyrrolyl, (1,3)-Dioxolanyl, $-Si(Phenyl)_2[C(CH_3)_3]$, $-C(=O)-CH_3$, $-C(=O)-C_2H_5$, $NH-S(=O)_2-CH_3$, $-NH-S(=O)_2-C_2H_5$, $-S(=O)_2-NH_2$, $-S(=O)_2-NH-CH_3$, $-CH_2-OH$, -C(=O)-OH, $-CH_2-O-CH_3$,$-C(=O)-NH_2$, $-C(=O)-NH-CH_3$, $-NH-C(=O)-CH_3$, $-NH-C(=NH)-NH_2$, $-NH-S(=O)_2-OH$ und $-S(=O)_2-N(CH_3)_2$ substituiert sein kann;

jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Ganz besonders bevorzugt sind substituierte Imidazo[2,1-b]thiazol-Verbindungen der vorstehend angegebenen allgemeinen Formel I, worin

$R^1$ und $R^2$, unabhängig voneinander, jeweils für einen Wasserstoff-Rest; -F; -Cl; -Br;-I; $-NO_2$: -CN; $-NHR^5$; $-NR^6R^7$; -C(=0)-R9, $-C(=O)-OR^{13}$; $-(CH_2)-O-C(=O)-R^{16}$; $-OR^{17}$;$-(CH_2)-O-S(=O)_2-R^{31}$; $-(CH_2)-O-S(=O)_2-O-R^{32}$; $-(CH_2)-O-P(=O)(OR^{33})(OR^{34})$; $-SF_5$; für einen Rest ausgewählt aus der Gruppe bestehend aus Methyl, Ethyl, n-Propyl, Isopropyl, n-Butyl, Isobutyl, 2-Butyl, tert-Butyl, n-Pentyl, $-CF_3$, $-CF_2H$, $-CFH_2$, $-(CH_2)-(CF_3)$, $-(CH_2)-(CHF_2)$, $-(CH_2)-(CH_2F)$ und $-(CF_2)-(CF_3)$; für einen Rest ausgewählt aus der Gruppe bestehend aus Cyclopropyl, Cyclobutyl, Cyclopentyl, Cyclohexyl, Cycloheptyl und Cyclooctyl;

oder für einen Rest ausgewählt aus der Gruppe bestehend aus Phenyl, Benzyl, Phenethyl und (3-Phenyl)-prop-1-yl stehen, der jeweils unsubstituiert oder mit ggf. 1, 2, 3, 4 oder 5 Substituenten unabhängig voneinander ausgewählt aus der Gruppe bestehend aus F, Cl, Br, I, -CN, $-NO_2$, Methyl, Ethyl, n-Propyl, Isopropyl, n-Butyl, Isobutyl, 2-Butyl, tert-Butyl, $-O-CH_3$, $-O-C_2H_5$, $-O-C_3H_7$, $-O-C(CH_3)_3$, $-CF_3$, $-CHF_2$,$-CH_2F$, $-O-CF_3$, $-O-CHF_2$ und $-O-CH_2F$ substituiert sein kann;

$R^3$ und $R^4$, unabhängig voneinander, jeweils für einen Wasserstoff-Rest; $-C(=O)-R^{21}$; $-(CH_2)_q-C(=O)-R^{22}$ mit q = 1, 2 oder 3; $-(CH_2)_r-C(=O)-O-R^{24}$ mit r = 1, 2 oder 3; für einen Rest ausgewählt aus der Gruppe bestehend aus Methyl, Ethyl, n-Propyl, Isopropyl, n-Butyl, Isobutyl, 2-Butyl, tert-Butyl, n-Pentyl, 2-Pentyl, 3-Pentyl, iso-Pentyl, neo-Pentyl, n-Hexyl, n-Heptyl, n-Octyl und (2,4,4)-Trimethyl-pent-2-yl; für einen Rest ausgewählt aus der Gruppe bestehend aus Cyclopropyl, Cyclobutyl, Cyclopentyl, Cyclohexyl, Cycloheptyl und Cyclooctyl, der über eine $-(CH_2)-$, $-(CH_2)_2-$, $-(CH(CH_3))-$ oder $-(CH_2)_3$-Gruppe gebunden sein kann;

oder für einen Rest ausgewählt aus der Gruppe bestehend aus Phenyl, Furyl (Furanyl), Thienyl (Thiophenyl), Pyridinyl, Pyridazinyl, Pyrimidinyl und Pyrazinyl stehen, der jeweils unsubstituiert oder mit ggf. 1, 2, 3, 4 oder 5 Substituenten unabhängig voneinander ausgewählt aus der Gruppe bestehend aus F, Cl, Br, I,-CN, $-NO_2$, Methyl, Ethyl, n-Propyl, Isopropyl, n-Butyl, Isobutyl, 2-Butyl, tert-Butyl, $-O-CH_3$, $-O-C_2H_5$, $-O-C_3H_7$, $-O-C(CH_3)_3$, $-CF_3$, $-CHF_2$, $-CH_2F$, $-O-CF_3$, $-O-CHF_2$ und $-O-CH_2F$ substituiert und/oder über eine$-(CH_2)-$, $-(CH_2)_2-$, $-(CH(CH_3))-$ oder $-(CH_2)_3$-Gruppe gebunden sein kann;

oder $R^3$ und $R^4$ zusammen mit dem sie verbindenden Stickstoffatom als Ringglied einen Rest bilden ausgewählt aus der Gruppe bestehend aus Piperidinyl, Piperazinyl, Morpholinyl und Pyrrolidinyl, der jeweils unsubstituiert oder mit ggf. 1, 2, 3, 4 oder 5 Substituenten unabhängig voneinander ausgewählt aus der Gruppe bestehend aus Methyl, Ethyl, n-Propyl, Isopropyl, n-Butyl, 2-Butyl, Isobutyl, tert-Butyl, $-S(=O)-CH_3$,$-S(=O)_2-CH_3$, $-S(=O)-C_2H_5$, $-S(=O)_2-C_2H_5$, $-C(=O)-CH_3$, $-C(=O)-C_2H_5$, $-C(=O)-N(CH_3)_2$, $-C(=O)-NH-CH_3$, $-C(=O)-NH_2$, $-C(=O)-O-CH_3$, $-C(=O)-O-C_2H_5$ und $-C(=O)-O-C(CH_3)_3$ substituiert sein kann;

$R^5$, $R^6$, $R^7$ und $R^{16}$, jeweils unabhängig voneinander, für einen Rest ausgewählt aus der Gruppe bestehend aus Methyl, Ethyl, n-Propyl, Isopropyl, n-Butyl, Isobutyl, 2-Butyl, tert-Butyl, n-Pentyl, 2-Pentyl, 3-Pentyl, iso-Pentyl, neo-Pentyl, n-Hexyl, n-Heptyl, n-Octyl, (2,4,4)-Trimethyl-pent-2-yl, $-CF_3$, $-CF_2H$, $-CFH_2$, $-(CH_2)-(CHF_2)$,$-(CH_2)-(CH_2F)$, $-(CF_2)-(CF_3)$, $-(CH_2)-(CH_2)-C(=O)-OH$; $-(CH_2)-(CH_2)-C(=O)-O-CH_3$ und $-(CH_2)-(CH_2)-C(=O)-O-C_2H_5$; oder für einen Rest ausgewählt aus der Gruppe bestehend aus Phenyl, Benzyl und Phenethyl stehen, der jeweils unsubstituiert oder mit ggf. 1, 2, 3, 4 oder 5 Substituenten unabhängig voneinander ausgewählt aus der Gruppe bestehend aus F, Cl, Br, I, -CN, $-NO_2$, Methyl, Ethyl, n-Propyl, Isopropyl, n-Butyl, Isobutyl, 2-Butyl, tert-Butyl, $-O-CH_3$ und $-O-C_2H_5$ substituiert sein kann;

$R^9$, $R^{13}$, $R^{17}$, $R^{21}$, $R^{22}$, $R^{24}$, $R^{31}$, $R^{32}$, $R^{33}$ und $R^{34}$, jeweils unabhängig voneinander, für einen Wasserstoff-Rest; für einen Rest ausgewählt aus der Gruppe bestehend aus Methyl, Ethyl, n-Propyl, Isopropyl, n-Butyl, Isobutyl, 2-Butyl, tert-Butyl, n-Pentyl, 2-Pentyl, 3-Pentyl, iso-Pentyl, neo-Pentyl, n-Hexyl, n-Heptyl, n-Octyl, (2,4,4)-Trimethyl-pent-2-yl, $-CF_3$, $-CF_2H$, $-CFH_2$, $-(CH_2)-(CF_3)$, $-(CH_2)-(CHF_2)$, $-(CH_2)-(CH_2F)$ und-$(CF_2)-(CF_3)$; oder für einen Rest ausgewählt aus der Gruppe bestehend aus Phenyl, Benzyl und Phenethyl stehen, der jeweils unsubstituiert oder mit ggf. 1, 2, 3, 4 oder 5 Substituenten unabhängig voneinander ausgewählt aus der Gruppe bestehend aus F, Cl, Br, I, -CN, $-NO_2$, Methyl, Ethyl, n-Propyl, Isopropyl, n-Butyl, Isobutyl, 2-Butyl, tert-Butyl,$-O-CH_3$ und $-O-C_2H_5$ substituiert sein kann;

$M^1$ für einen Rest ausgewählt aus der Gruppe bestehend aus den Resten 1 bis 6, 21, 22, 36 und 37 steht,

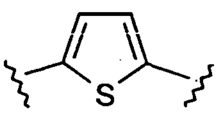
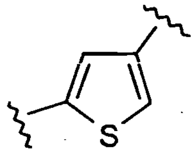
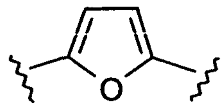
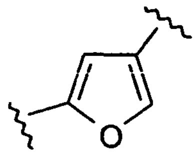

1 2 3 4

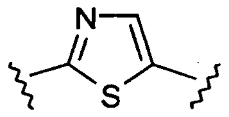
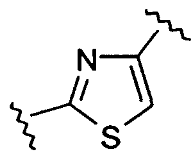

5 6

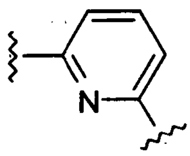
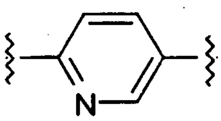
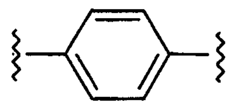
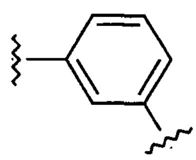

21 22 36 37

der jeweils unsubstituiert oder mit ggf. 1 oder 2 Substituenten unabhängig voneinander ausgewählt aus der Gruppe bestehend aus F, Cl, Br, I, -CN, $-CH_2-CN$,$-NO_2$, Methyl, Ethyl, n-Propyl, isopropyl, n-Butyl, Isobutyl, 2-Butyl, tert-Butyl, n-Pentyl, neo-Pentyl, $-SF_5$, $-O-CH_3$, $-O-C_2H_5$, $-O-C_3H_7$, $-O-C(CH_3)_3$, $-CF_3$, $-CHF_2$, $-CH_2F$, $-O-CF_3$, $-O-CHF_2$ und $-O-CH_2F$ substituiert sein kann, und der jeweils in beliebiger Richtung über die mit einer geschlängelten Linie gekennzeichneten Positionen mit dem Bizyklus und dem Kohlenstoffatom der Dreifachbindung verknüpft sein kann;

und $M^2$ für einen Rest ausgewählt aus der Gruppe bestehend aus Phenyl, 2-Pyridinyl, 3-Pyridinyl, 4-Pyridinyl, 2-Thiazolyl, 4-Thiazolyl, 5-Thiazolyl, 4-Indolyl, 5-Indolyl, 6-Indolyl, 7-Indolyl, 2-Thiophenyl (2-Thienyl), 3-Thiophenyl (3-Thienyl), 2-Imidazolyl, 4-Imidazolyl, 5-Imidazolyl, 2-Pyrimidinyl, 4-Pyrimidinyl, 5-Pyrimidinyl, 2-Pyrazinyl, 5-Chinolinyl, 6-Chinolinyl, 7-Chinolinyl und 8-Chinolinyl steht, der jeweils unsubstituiert oder mit ggf. 1, 2, 3, 4 oder 5 Substituenten unabhängig voneinander ausgewählt aus der Gruppe bestehend aus F, Cl, Br, -CN, $-CF_3$, $-SF_5$,$-S-CH_3$, $-S-C_2H_5$, Methyl, Ethyl, n-Propyl, iso-Propyl, n-Butyl, sec-Butyl, iso-Butyl, tert-Butyl, Ethenyl, Propenyl, -OH, $-O-CH_3$, $-O-C_2H_5$, $-O-C_3H_7$, $-NH_2$,

$-N(CH_3)_2$, $-NH-CH_3$,$-CH_2-NH_2$, $-N(C_2H_5)_2$, $-NO_2$, $-O-CF_3$, $-S-CF_3$, -SH, -C(=O)-H, $-C(=O)-O-CH_3$, $-C(=O)-O-C_2H_5$, Phenyl, Pyrrolyl, (1,3)-Dioxolanyl, $-Si(Phenyl)_2[C(CH_3)_3]$, $-C(=O)-CH_3$, - $C(=O)-C_2H_5$, $NH-S(=O)_2-CH_3$, $-NH-S(=O)_2-C_2H_5$, $-S(=O)_2-NH_2$, $-S(=O)_2-NH-CH_3$,$-CH_2-OH$, -C(=O)-OH, $-CH_2-O-CH_3$, $-C(=O)-NH_2$, $-C(=O)-NH-CH_3$, $-NH-C(=O)-CH_3$,$-NH-C(=NH)-NH_2$, $-NH-S(=O)_2-OH$ und $-S(=O)_2-N(CH_3)_2$ substituiert sein kann;
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Ebenfalls ganz besonders bevorzugt sind substituierte Imidazo[2,1-b]thiazol-Verbindungen der allgemeinen Formel Ia,

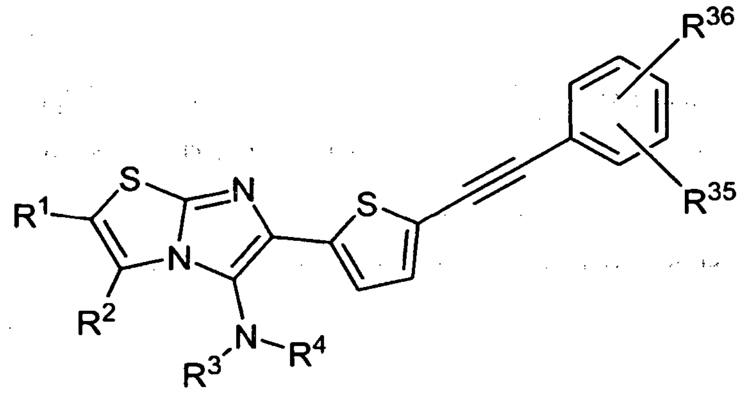

Ia,

worin

$R^1$, $R^2$, $R^3$ und $R^4$ die vorstehend genannte Bedeutung haben,

und $R^{35}$ und $R^{36}$, unabhängig voneinander, jeweils für einen Rest ausgewählt aus der Gruppe bestehend aus H, F, Cl, Br, -CN, $-CF_3$, $-SF_5$, $-S-CH_3$, $-S-C_2H_5$, Methyl, Ethyl, n-Propyl, iso-Propyl, n-Butyl, sec-Butyl, iso-Butyl, tert-Butyl, Ethenyl, Propenyl, -OH,$-O-CH_3$, $-O-C_2H_5$, $-O-C_3H_7$, $-NH_2$, $-N(CH_3)_2$, $-NH-CH_3$, $-CH_2-NH_2$, $-N(C_2H_5)_2$, $-NO_2$, $-O-CF_3$, $-S-CF_3$, -SH, -C(=O)-H, $-C(=O)-O-CH_3$, $-C(=O)-O-C_2H_5$, Phenyl, Pyrrolyl, (1,3)-Dioxolanyl, $-Si(Phenyl)_2[C(CH_3)_3]$, $-C(=O)-CH_3$, $-C(=O)-C_2H_5$, $NH-S(=O)_2-CH_3$, $-NH-S(=O)_2-C_2H_5$, $-S(=O)_2-NH_2$, $-S(=O)_2-NH-CH_3$, $-CH_2-OH$, -C(=O)-OH, $-CH_2-O-CH_3$,$-C(=O)-NH_2$,- $C(=O)-NH-CH_3$, $-NH-C(=O)-CH_3$, $-NH-C(=NH)-NH_2$, $-NH-S(=O)_2-OH$ und $-S(=O)_2-N(CH_3)_2$ stehen;

jeweils ggf. in Form entsprechender Salze, oder jeweils ggf. in Form entsprechender Solvate.

Ebenfalls ganz besonders bevorzugt sind substituierte Imidazo[2,1-b]thiazol-Verbindungen der allgemeinen Formel 1b

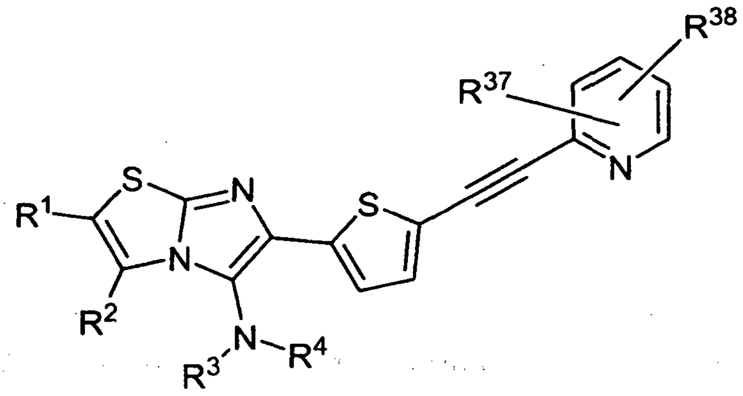

**Ib,**

worin

$R^1$, $R^2$, $R^3$ und $R^4$ die vorstehend genannte Bedeutung haben,

und $R^{37}$ und $R^{38}$, unabhängig voneinander, jeweils für einen Rest ausgewählt aus der Gruppe bestehend aus H, F, Cl, Br, -CN, $-CF_3$, $-SF_5$, $-S-CH_3$, $-S-C_2H_5$, Methyl, Ethyl, n-Propyl, iso-Propyl, n-Butyl, sec-Butyl, iso-Butyl, tert-Butyl, Ethenyl, Propenyl, -OH,$-O-CH_3$, $-O-C_2H_5$, $-O-C_3H_7$, $-NH_2$, $-N(CH_3)_2$, $-NH-CH_3$, $-CH_2-NH_2$, $-N(C_2H_5)_2$, $-NO_2$, $-O-CF_3$, $-S-CF_3$, -SH, -C(=O)-H, $-C(=O)-O-CH_3$, $-C(=O)-O-C_2H_5$, Phenyl, Pyrrolyl, (1,3)-Dioxolanyl, $-Si(Phenyl)_2[C(CH_3)_3]$, $-C(=O)-CH_3$, $-C(=O)-C_2H_5$, $NH-S(=O)_2-CH_3$, $-NH-S(=O)_2-C_2H_5$, $-S(=O)_2-NH_2$, $-S(=O)_2-NH-CH_3$, $-CH_2-OH$, -C(=O)-OH, $-CH_2-O-CH_3$,$-C(=O)-NH_2$, $-C(=O)-NH-CH_3$, $-NH-C(=O)-CH_3$, $-NH-C(=NH)-NH_2$, $-NH-S(=O)_2-OH$ und $-S(=O)_2-N(CH_3)_2$ stehen;

jeweils ggf. in Form entsprechender Salze, oder jeweils ggf. in Form entsprechender Solvate.

Ebenfalls ganz besonders bevorzugt sind substituierte Imidazo[2,1-b]thiazol-Verbindungen der allgemeinen Formel Ic

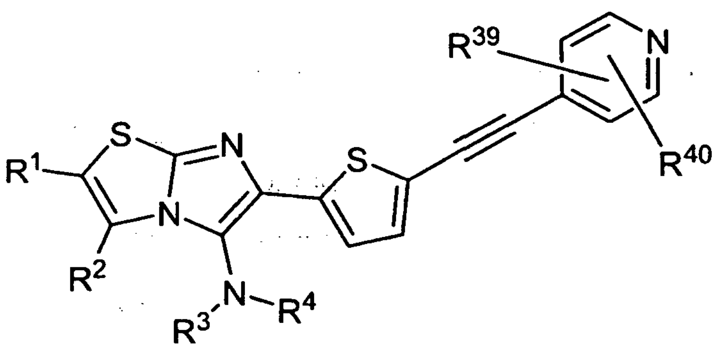

**Ic,**

worin

$R^1$, $R^2$, $R^3$ und $R^4$ die vorstehend genannte Bedeutung haben,

und $R^{39}$ und $R^{40}$, unabhängig voneinander, jeweils für einen Rest ausgewählt aus der Gruppe bestehend aus H, F, Cl, Br, -CN, $-CF_3$, $-SF_5$, $-S-CH_3$, $-S-C_2H_5$, Methyl, Ethyl, n-Propyl, iso-Propyl, n-Butyl, sec-Butyl, iso-Butyl, tert-Butyl, Ethenyl, Propenyl, -OH,$-O-CH_3$, $-O-C_2H_5$, $-O-C_3H_7$, $-NH_2$, $-N(CH_3)_2$, $-NH-CH_3$, $-CH_2-NH_2$, -N(C2H5)$_2$, $-NO_2$, $-O-CF_3$, $-S-CF_3$, -SH, -C(=O)-H, $-C(=O)-O-CH_3$, $-C(=O)-O-C_2H_5$; Phenyl, Pyrrolyl, (1,3)-Dioxolanyl, $-Si(Phenyl)_2[C(CH_3)_3]$, $-C(=O)-CH_3$, $-C(=O)-C_2H_5$, $NH-S(=O)_2-CH_3$, $-NH-S(=O)_2-C_2H_5$, $-S(=O)_2-NH_2$, $-S(=O)_2-NH-CH_3$,

$-CH_2-OH$, $-C(=O)-OH$, $-CH_2-O-CH_3$,$-C(=O)-NH_2$, $-C(=O)-NH-CH_3$, $-NH-C(=O)-CH_3$, $-NH-C(=NH)-NH_2$, $-NH-S(=O)_2-OH$ und $-S(=O)_2-N(CH_3)_2$ stehen;

jeweils ggf. in Form entsprechender Salze, oder jeweils ggf. in Form entsprechender Solvate.

Ebenfalls ganz besonders bevorzugt sind substituierte Imidazo[2,1-b]thiazol-Verbindungen der allegemeinen Formel Id,

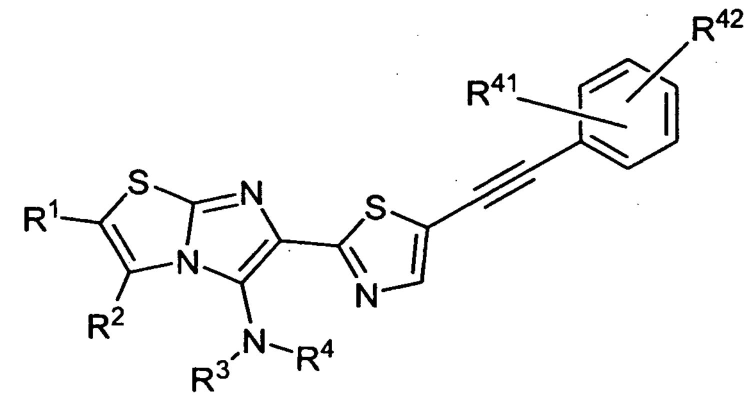

**Id,**

worin

$R^1$, $R^2$, $R^3$ und $R^4$ die vorstehend genannte Bedeutung haben,

und $R^{41}$ und $R^{42}$, unabhängig voneinander, jeweils für einen Rest ausgewählt aus der Gruppe bestehend aus H, F, Cl, Br, -CN, $-CF_3$, $-SF_5$, $-S-CH_3$, $-S-C_2H_5$, Methyl, Ethyl, n-Propyl, iso-Propyl, n-Butyl, sec-Butyl, iso-Butyl, tert-Butyl, Ethenyl, Propenyl, -OH,$-O-CH_3$, $-O-C_2H_5$, $-O-C_3H_7$, $-NH_2$, $-N(CH_3)_2$, $-NH-CH_3$, $-CH_2-NH_2$, $-N(C_2H_5)_2$, $-NO_2$, $-O-CF_3$, $-S-CF_3$, -SH, -C(=O)-H, $-C(=O)-O-CH_3$, $-C(=O)-O-C_2H_5$, Phenyl, Pyrrolyl, (1,3)-Dioxolanyl, -Si(Phenyl)$_2$[C(CH$_3$)$_3$], $-C(=O)-CH_3$, $-C(=O)-C_2H_5$; $NH-S(=O)_2-CH_3$, $-NH-S(=O)_2-C_2H_5$; $-S(=O)_2-NH_2$, $-S(=O)_2-NH-CH_3$, $-CH_2-OH$, -C(=O)-OH, $-CH_2-O-CH_3$,$-C(=O)-NH_2$, $-C(=O)-NH-CH_3$, $-NH-C(=O)-CH_3$, $-NH-C(=NH)-NH_2$, $-NH-S(=O)_2-OH$ und $-S(=O)_2-N(CH_3)_2$ stehen;

jeweils ggf. in Form entsprechender Salze, oder jeweils ggf. in Form entsprechender Solvate.

Ebenfalls ganz besonders bevorzugt sind substituierte Imidazo[2,1-b]thiazol-Verbindungen der allgemeinen Formel Ie,

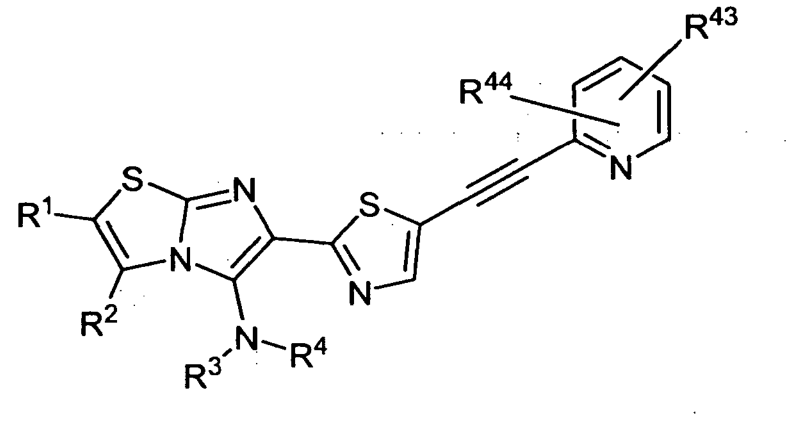

**Ie,**

worin

$R^1$, $R^2$, $R^3$ und $R^4$ die vorstehend genannte Bedeutung haben,

und $R^{43}$ und $R^{44}$, unabhängig voneinander, jeweils für einen Rest ausgewählt aus der Gruppe bestehend aus H, F, Cl, Br, -CN, $-CF_3$, $-SF_5$, $-S-CH_3$, $-S-C_2H_5$, Methyl, Ethyl, n-Propyl, iso-Propyl, n-Butyl, sec-Butyl, iso-Butyl, tert-Butyl, Ethenyl, Propenyl, -OH,$-O-CH_3$, $-O-C_2H_5$, $-O-C_3H_7$, $-NH_2$, $-N(CH_3)_2$, $-NH-CH_3$, $-CH_2-NH_2$, $-N(C_2H_5)_2$, $-NO_2$, $-O-CF_3$, $-S-CF_3$, -SH, -C(=O)-H, $-C(=O)-O-CH_3$, $-C(=O)-O-C_2H_5$, Phenyl, Pyrrolyl, (1,3)-Dioxolanyl, -Si(Phenyl)$_2$[C(CH$_3$)$_3$], $-C(=O)-CH_3$, $-C(=O)-C_2H_5$, $NH-S(=O)_2-CH_3$, $-NH-S(=O)_2-C_2H_5$, $-S(=O)_2-NH_2$, $-S(=O)_2-NH-CH_3$, $-CH_2-OH$, -C(=O)-OH, $-CH_2-O-CH_3$,$-C(=O)-NH_2$, $-C(=O)-NH-CH_3$, $-NH-C(=O)-CH_3$, $-NH-C(=NH)-NH_2$, -NH-$S(=O)_2$-OH und $-S(=O)_2-N(CH_3)_2$ stehen;

jeweils ggf. in Form entsprechender Salze, oder jeweils ggf. in Form entsprechender Solvate.

Ebenfalls ganz besonders bevorzugt sind substituierte Imidazo[2,1-b]thiazol-Verbindungen der allgemeinen Formel If,

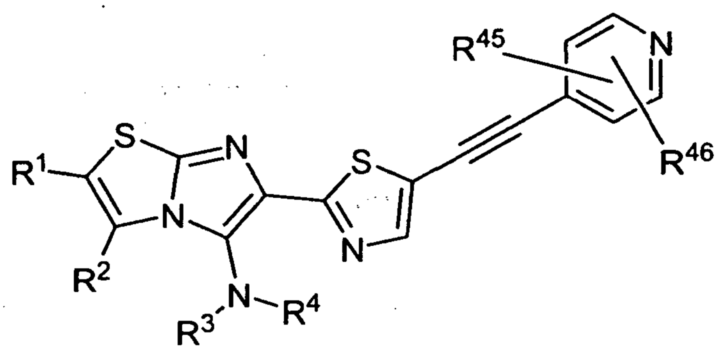

If,

worin

$R^1$, $R^2$, $R^3$ und $R^4$ die vorstehend genannte Bedeutung haben,

und $R^{45}$ und $R^{46}$, unabhängig voneinander, jeweils für einen Rest ausgewählt aus der Gruppe bestehend aus H, F, Cl, Br, -CN, $-CF_3$, $-SF_5$, $-S-CH_3$, $-S-C_2H_5$, Methyl, Ethyl, n-Propyl, iso-Propyl, n-Butyl, sec-Butyl, iso-Butyl, tert-Butyl, Ethenyl, Propenyl, -OH,$-O-CH_3$, $-O-C_2H_5$, $-O-C_3H_7$, $-NH_2$, $-N(CH_3)_2$, $-NH-CH_3$, $-CH_2-NH_2$, $-N(C_2H_5)_2$, $-NO_2$, $-O-CF_3$, $-S-CF_3$, -SH, -C(=O)-H, $-C(=O)-O-CH_3$, $-C(=O)-O-C_2H_5$, Phenyl, Pyrrolyl, (1,3)-Dioxolanyl, -Si(Phenyl)$_2$[C(CH$_3$)$_3$], $=C(=O)-CH_3$, $-C(=O)-C_2H_5$, $NH-S(=O)_2-CH_3$, $-NH-S(=O)_2-C_2H_5$, $-S(=O)_2-NH_2$, $-S(=O)_2-NH-CH_3$, $-CH_2-OH$, -C(=O)-OH, $-CH_2-O-CH_3$, - $C(=O)-NH_2$, $-C(=O)-NH-CH_3$, $-NH-C(=O)-CH_3$, $-NH-C(=NH)-NH_2$, -NH-$S(=O)_2$-OH und $-S(=O)_2-N(CH_3)_2$ stehen;

jeweils ggf. in Form entsprechender Salze, oder jeweils ggf. in Form entsprechender Solvate.

Ebenfalls ganz besonders bevorzugt sind substituierte Imidazo[2,1-b]thiazol-Verbindungen der allgemeinen Formel Ig,

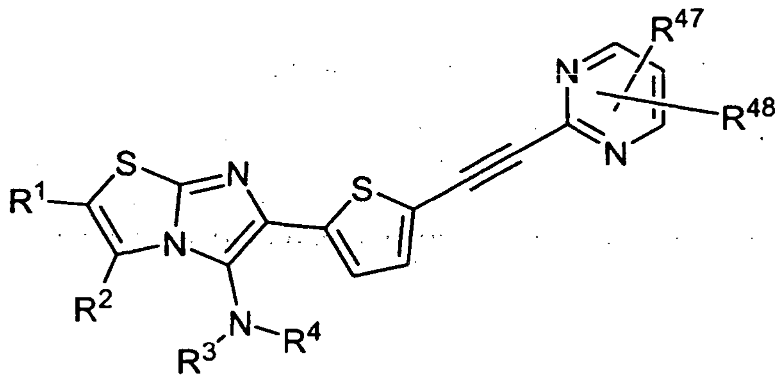

Ig,

worin

$R^1$, $R^2$, $R^3$ und $R^4$ die vorstehend genannte Bedeutung haben,

und $R^{47}$ und $R^{48}$, unabhängig voneinander, jeweils für einen Rest ausgewählt aus der Gruppe bestehend aus H, F, Cl, Br, -CN, $-CF_3$, $-SF_5$, $-S-CH_3$, $-S-C_2H_5$, Methyl, Ethyl, n-Propyl, iso-Propyl, n-Butyl, sec-Butyl, iso-Butyl, tert-Butyl, Ethenyl, Propenyl, -OH,$-O-CH_3$, $-O-C_2H_5$, $-O-C_3H_7$, $-NH_2$, $-N(CH_3)_2$, $-NH-CH_3$, $-CH_2-NH_2$, $-N(C_2H_5)_2$, $-NO_2$, $-O-CF_3$, $-S-CF_3$, -SH, -C(=O)-H, $-C(=O)-O-CH_3$, $-C(=O)-O-C_2H_5$, Phenyl, Pyrrolyl, (1,3)-Dioxolanyl, -Si(Phenyl)$_2$[C(CH$_3$)$_3$], $-C(=O)-CH_3$, $-C(=O)-C_2H_5$, $NH-S(=O)_2-CH_3$, $-NH-S(=O)_2-C_2H_5$, $-S(=O)_2-NH_2$, $-S(=O)_2-NH-CH_3$, $-CH_2-OH$, -C(=O)-OH, $-CH_2-O-CH_3$,$-C(=O)-NH_2$, $-C(=O)-NH-CH_3$, $-NH-C(=O)-CH_3$, $-NH-C(=NH)-NH_2$, $-NH-S(=O)_2-OH$ und $-S(=O)_2-N(CH_3)_2$ stehen;

jeweils ggf. in Form entsprechender Salze, oder jeweils ggf. in Form entsprechender Solvate.

Ebenfalls ganz besonders bevorzugt sind substituierte Imidazo[2,1-b]thiazol-Verbindungen der allgemeinen Formel Ih,

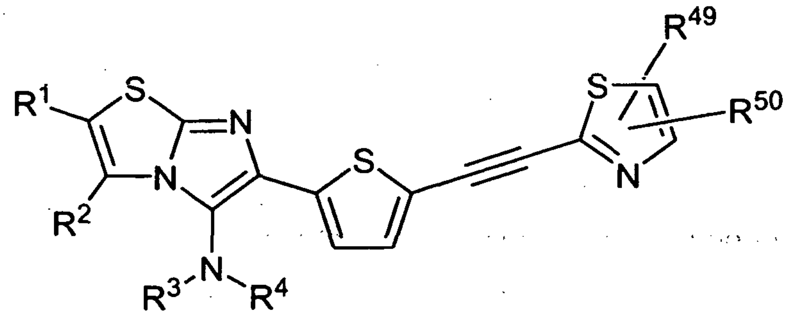

Ih,

worin

$R^1$, $R^2$, $R^3$ und $R^4$ die vorstehend genannte Bedeutung haben,

und $R^{49}$ und $R^{50}$, unabhängig voneinander, jeweils für einen Rest ausgewählt aus der Gruppe bestehend aus H, F, Cl, Br, -CN, $-CF_3$, $-SF_5$, $-S-CH_3$, $-S-C_2H_5$, Methyl, Ethyl, n-Propyl, iso-Propyl, n-Butyl, sec-Butyl, iso-Butyl, tert-Butyl, Ethenyl, Propenyl, -OH,$-O-CH_3$, $-O-C_2H_5$, $-O-C_3H_7$, $-NH_2$, $-N(CH_3)_2$, $-NH-CH_3$, $-CH_2-NH_2$, $-N(C_2H_5)_2$, $-NO_2$, $-O-CF_3$, $-S-CF_3$, -SH, -C(=O)-H, $-C(=O)-O-CH_3$, $-C(=O)-O-C_2H_5$, Phenyl, Pyrrolyl, (1,3)-Dioxolanyl, -Si(Phenyl)$_2$[C(CH$_3$)$_3$], $-C(=O)-CH_3$, $-C(=O)-C_2H_5$, $NH-S(=O)_2-CH_3$, $-NH-S(=O)_2-C_2H_5$, $-S(=O)_2-NH_2$, $-S(=O)_2-NH-CH_3$,

$-CH_2-OH$, $-C(=O)-OH$, $-CH_2-O-CH_3$,$-C(=O)-NH_2$, $-C(=O)-NH-CH_3$, $-NH-C(=O)-CH_3$, $-NH-C(=NH)-NH_2$, $-NH-S(=O)_2-OH$ und $-S(=O)_2-N(CH_3)_2$ stehen;

jeweils ggf. in Form entsprechender Salze, oder jeweils ggf. in Form entsprechender Solvate.

Noch weiter bevorzugt sind substituierte Imidazo[2,1-b]thiazol-Verbindungen der vorstehend angegebenen allgemeinen Formeln Ia, Ib, Ic, Id, Ie, If, Ig und Ih, worin
$R^1$ für einen Wasserstoff-Rest; -F; -Cl; -Br; -CN; $-C(=O)-OR^{13}$; oder für einen Rest ausgewählt aus der Gruppe bestehend aus Methyl, Ethyl, n-Propyl, Isopropyl, n-Butyl, Isobutyl, 2-Butyl, tert-Butyl und n-Pentyl steht;
$R^2$ für einen Wasserstoff-Rest; -F; -Cl; -Br; -CN; $-C(=O)-OR^{13}$; oder für einen Rest ausgewählt aus der Gruppe bestehend aus Methyl, Ethyl, n-Propyl, Isopropyl, n-Butyl, Isobutyl, 2-Butyl, tert-Butyl und n-Pentyl steht;
$R^3$ für einen Wasserstoff-Rest steht;
$R^4$ für einen Wasserstoff-Rest; für $-C(=O)-R^{21}$ oder für einen Rest ausgewählt aus der Gruppe bestehend aus Methyl, Ethyl, n-Propyl, Isopropyl, n-Butyl, Isobutyl, 2-Butyl, tert-Butyl, n-Pentyl und (2,4,4)-Trimethyl-pent-2-yl steht;
$R^{13}$ für einen Rest ausgewählt aus der Gruppe bestehend aus Methyl, Ethyl, n-Propyl, Isopropyl, n-Butyl, Isobutyl, 2-Butyl, tert-Butyl, n-Pentyl, 2-Pentyl, 3-Pentyl und (2,4,4)-Trimethyl-pent-2-yl steht;
$R^{21}$ für einen Rest ausgewählt aus der Gruppe bestehend aus Methyl, Ethyl, n-Propyl, Isopropyl, n-Butyl, Isobutyl, 2-Butyl, tert-Butyl, n-Pentyl und (2,4,4)-Trimethyl-pent-2-yl oder für einen Phenyl-Rest steht, der jeweils unsubstituiert ist;
und $R^{35}$, $R^{36}$, $R^{37}$, $R^{38}$, $R^{39}$, $R^{40}$, $R^{41}$, $R^{42}$, $R^{43}$, $R^{44}$, $R^{45}$, $R^{46}$ $R^{47}$, $R^{48}$, $R^{49}$ und $R^{50}$ unabhängig voneinander jeweils für einen Rest ausgewählt aus der Gruppe bestehend aus F, Cl, Br, -CN, -OH, $-CF_3$, Methyl, Ethyl, n-Propyl, iso-Propyl, n-Butyl, sec-Butyl, iso-Butyl und tert-Butyl stehen;
jeweils ggf. in Form entsprechender Salze, oder jeweils in Form entsprechender Solvate.

Ebenfalls ganz besonders bevorzugt sind substituierte Imidazo[2,1-b]thiazol-Verbindungen der vorstehend angegebenen allgemeinen Formel I, worin
$R^1$ für einen Wasserstoff-Rest; -F; -Cl; -Br; -CN; $-O-CH_3$; $-C(=O)-R^9$, $-C(=O)-OR^{13}$;$-(CH_2)-O-C(=O)-R^{16}$; $-(CH_2)-O-S(=O)_2-R^{31}$; $-(CH_2)-O-S(=O)_2-O-R^{32}$; $-(CH_2)-O-P(=O)(OR^{33})(OR^{34})$; oder für einen Rest ausgewählt aus der Gruppe bestehend aus Methyl, Ethyl, n-Propyl, Isopropyl, n-Butyl, Isobutyl, 2-Butyl, tert-Butyl, n-Pentyl, $-CF_3$, $-CF_2H$, $-CFH_2$, $-(CH_2)-(CF_3)$, $-(CH_2)-(CHF_2)$, $-(CH_2)-(CH_2F)$ und $-(CF_2)-(CF_3)$ steht;
$R^2$ für einen Wasserstoff-Rest; -F; -Cl; -Br; -CN; $-O-CH_3$; $-C(=O)-R^9$, $-C(=O)-OR^{13}$;$-(CH_2)-O-C(=O)-R^{16}$; $-(CH_2)-O-S(=O)_2-R^{31}$; $-(CH_2)-O-S(=O)_2-O-R^{32}$; $-(CH_2)-O-P(=O)(OR^{33})(OR^{34})$; oder für einen Rest ausgewählt aus der Gruppe bestehend aus Methyl, Ethyl, n-Propyl, Isopropyl, n-Butyl, Isobutyl, 2-Butyl, tert-Butyl, n-Pentyl, $-CF_3$, $-CF_2H$, $-CFH_2$, $-(CH_2)-(CF_3)$, $-(CH_2)-(CHF_2)$, $-(CH_2)-(CH_2F)$ und $-(CF_2)-(CF_3)$ steht;
$R^3$ für einen Wasserstoff-Rest oder für einen Rest ausgewählt aus der Gruppe bestehend aus Methyl, Ethyl und Isopropyl steht;
$R^4$ für einen Wasserstoff-Rest; für $-C(=O)-R^{21}$ oder für einen Rest ausgewählt aus der Gruppe bestehend aus Methyl, Ethyl, n-Propyl, Isopropyl, n-Butyl, Isobutyl, 2-Butyl, tert-Butyl, n-Pentyl, 2-Pentyl, 3-Pentyl, iso-Pentyl, neo-Pentyl, n-Hexyl, n-Heptyl, n-Octyl und (2,4,4)-Trimethyl-pent-2-yl steht;
oder $R^3$ und $R^4$ zusammen mit dem sie verbindenden Stickstoffatom als Ringglied einen Rest ausgewählt aus der Gruppe bestehend aus Pyrrolidinyl, Piperidinyl und Morpholinyl bilden;
$R^{16}$ für einen Rest ausgewählt aus der Gruppe bestehend aus $-(CH_2)-(CH_2)-C(=O)-OH$; $-(CH_2)-(CH_2)-C(=O)-O-CH_3$ und $-(CH_2)-(CH_2)-C(=O)-O-C_2H_5$ steht;
$R^9$, $R^{13}$, $R^{31}$, $R^{32}$, $R^{33}$ und $R^{34}$, jeweils unabhängig voneinander, für einen Wasserstoff-Rest; oder für einen Rest ausgewählt aus der Gruppe bestehend aus Methyl, Ethyl, n-Propyl, Isopropyl, n-Butyl, Isobutyl, 2-Butyl, tert-Butyl, n-Pentyl, 2-Pentyl, 3-Pentyl, iso-Pentyl, neo-Pentyl, n-Hexyl, n-Heptyl, n-Octyl, (2,4,4)-Trimethyl-pent-2-yl, $-CF_3$, $-CF_2H$, $-CFH_2$, $-(CH_2)-(CF_3)$, $-(CH_2)-(CHF_2)$, $-(CH_2)-(CH_2F)$ und - $(CF_2)-(CF_3)$ stehen;
$R^{21}$ für einen Rest ausgewählt aus der Gruppe bestehend aus Methyl, Ethyl, n-Propyl, Isopropyl, n-Butyl, Isobutyl, 2-Butyl, tert-Butyl, n-Pentyl, 2-Pentyl, 3-Pentyl, iso-Pentyl, neo-Pentyl, n-Hexyl, n-Heptyl, n-Octyl, (2,4,4)-Trimethyl-pent-2-yl, $-CF_3$, - $CF_2H$, $-CFH_2$, $-(CH_2)-(CF_3)$, $-(CH_2)-(CHF_2)$, $-(CH_2)-(CH_2F)$ und $-(CF_2)-(CF_3)$ oder für einen Phenyl-Rest steht, der jeweils unsubstituiert oder mit ggf. 1, 2, 3, 4 oder 5 Substituenten unabhängig voneinander ausgewählt aus der Gruppe bestehend aus F, Cl, Br, I, -CN, $-NO_2$, Methyl, Ethyl, n-Propyl, Isopropyl, n-Butyl, Isobutyl, 2-Butyl, tert-Butyl,$-O-CH_3$ und $-O-C_2H_5$ substituiert sein kann;
$M^1$ für einen Rest ausgewählt aus der Gruppe bestehend aus den Resten 1, 3, 5, 22, 36 und 37,

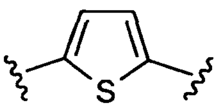

1

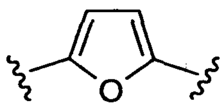

3

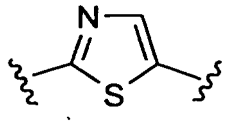

5

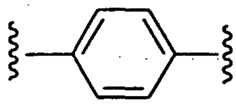

36

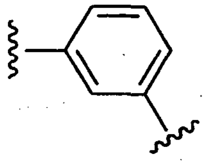

37

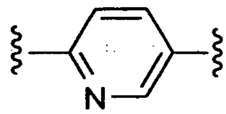

22

der jeweils unsubstituiert ist, und der jeweils in beliebiger Richtung über die mit einer geschlängelten Linie gekennzeichneten Positionen mit dem Bizyklus und dem Kohlenstoffatom der Dreifachbindung verknüpft sein kann;
$M^2$ für einen Rest ausgewählt aus der Gruppe bestehend aus Phenyl, 2-Pyrimidinyl, 5-Pyrimidinyl, 2-Thiophenyl (2-Thienyl), 3-Thiophenyl (3-Thienyl), 2-Pyridinyl, 3-Pyridinyl, 4-Pyridinyl, 2-Thiazolyl und 4-Thiazolyl steht, der jeweils unsubstituiert oder mit ggf. 1, 2, 3, 4 oder 5 Substituenten unabhängig voneinander ausgewählt aus der Gruppe bestehend aus F, Cl, Br, -CN, -O-$CH_3$, -OH, -$CF_3$, Methyl, Ethyl, n-Propyl, iso-Propyl, n-Butyl, sec-Butyl, iso-Butyl und tert-Butyl substituiert sein kann;
jeweils ggf. in Form entsprechender Salze, oder jeweils in Form entsprechender Solvate.

Ebenfalls ganz besonders bevorzugt sind substituierte Imidazo[2,1-b]thiazol-Verbindungen der vorstehend angegebenen allgemeinen Formel I, worin
$R^1$ für einen Wasserstoff-Rest; -F; -Cl; -Br; -CN; -C(=O)-$OR^{13}$; oder für einen Rest ausgewählt aus der Gruppe bestehend aus Methyl, Ethyl, n-Propyl, Isopropyl, n-Butyl, Isobutyl, 2-Butyl, tert-Butyl und n-Pentyl steht;
$R^2$ für einen Wasserstoff-Rest; -F; -Cl; -Br; -CN; -C(=O)-$OR^{13}$; oder für einen Rest ausgewählt aus der Gruppe bestehend aus Methyl, Ethyl, n-Propyl, Isopropyl, n-Butyl, Isobutyl, 2-Butyl, tert-Butyl und n-Pentyl steht;
$R^3$ für einen Wasserstoff-Rest steht;
$R^4$ für einen Wasserstoff-Rest; für -C(=O)-$R^{21}$ oder für einen Rest ausgewählt aus der Gruppe bestehend aus Methyl, Ethyl, n-Propyl, Isopropyl, n-Butyl, Isobutyl, 2-Butyl, tert-Butyl, n-Pentyl und (2,4,4)-Trimethyl-pent-2-yl steht;
$R^{13}$ für einen Rest ausgewählt aus der Gruppe bestehend aus Methyl, Ethyl, n-Propyl, Isopropyl, n-Butyl, Isobutyl, 2-Butyl, tert-Butyl, n-Pentyl, 2-Pentyl, 3-Pentyl und (2,4,4)-Trimethyl-pent-2-yl steht;
$R^{21}$ für einen Rest ausgewählt aus der Gruppe bestehend aus Methyl, Ethyl, n-Propyl, Isopropyl, n-Butyl, Isobutyl, 2-Butyl, tert-Butyl, n-Pentyl und (2,4,4)-Trimethyl-pent-2-yl oder für einen Phenyl-Rest steht, der jeweils unsubstituiert ist;
$M^1$ für einen Rest ausgewählt aus der Gruppe bestehend aus den Resten 1, 3, 5 und 22,

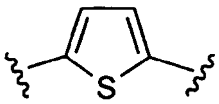

1

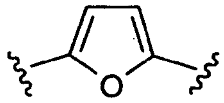

3

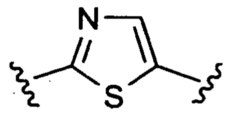

5

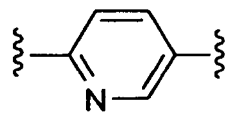

22

der jeweils unsubstituiert ist, und der jeweils in beliebiger Richtung über die mit einer geschlängelten Linie gekennzeichneten Positionen mit dem Bizyklus und dem Kohlenstoffatom der Dreifachbindung verknüpft sein kann;
und $M^2$ für einen Rest ausgewählt aus der Gruppe bestehend aus Phenyl, 2-Pyrimidinyl, 5-Pyrimidinyl, 2-Thiophenyl (2-Thienyl), 3-Thiophenyl (3-Thienyl), 2-Pyridinyl, 3-Pyridinyl, 4-Pyridinyl, 2-Thiazolyl und 4-Thiazolyl steht, des jeweils unsubstituiert oder mit ggf. 1, 2, 3, 4 oder 5 Substituenten unabhängig voneinander ausgewählt aus der Gruppe bestehend aus F, Cl, Br, -CN, -OH, -$CF_3$, Methyl, Ethyl, n-Propyl, iso-Propyl, n-Butyl, sec-Butyl, iso-Butyl und tert-Butyl substituiert sein kann;
jeweils ggf. in Form entsprechender Salze, oder jeweils in Form entsprechender Solvate.

Noch weiter bevorzugt sind substituierte Imidazo[2,1-b]thiazol-Verbindungen der vorstehend angegebenen allgemeinen Formel I ausgewählt aus der Gruppe bestehend aus

[1] 6-(5-(Phenylethinyl)thiophen-2-yl)-N-(2,4,4-trimethylpentan-2-yl)-imidazo[2,1-b]thiazol-5-amin,
[2] N-tert-Butyl-6-(5-(pyridin-2-ylethinyl)thiophen-2-yl)-imidazo[2,1-b]thiazol-5-amin,
[3] N-tert-Butyl-3-methyl-6-(5-(pyridin-2-ylethinyl)thiophen-2-yl)-imidazo[2,1-b]thiazol-5-amin Hydrochlorid,
[4] N-tert-Butyl-2-methyl-6-(5-(pyridin-2-ylethinyl)thiophen-2-yl)-imidazo[2,1-b]thiazol-5-amin,
[5] N-tert-Butyl-2,3-dimethyl-6-(5-(pyridin-2-ylethinyl)thiophen-2-yl)-imidazo[2,1-b]thiazol-5-amin Hydrochlorid,
[6] N-tert-Butyl-2-chlor-6-(5-(pyridin-2-ylethinyl)thiophen-2-yl)-imidazo[2,1-b]thiazol-5-amin Hydrochlorid,
[7] N-tert-Butyl-6-(5-(pyridin-4-ylethinyl)thiophen-2-yl)imidazo[2,1-b]thiazol-5-amin,
[8] 5-(tert-Butylamino)-6-(5-(pyridin-2-ylethinyl)thiophen-2-yl)-imidazo[2,1-b]thiazol-2-carbonsäuremethylester Hydrochlorid,
[9] N-tert-Butyl-6-(5-(pyridin-2-ylethinyl)thiazol-2-yl)imidazo[2,1-b]thiazol-5-amin,
[10] 6-(5-Pyridin-2-ylethinyl)thiophen-2-yl)-N-(2,4,4-trimethylpentan-2-yl)imidazo[2,1-b]thiazol-5-amin,
[11] N-tert-Butyl-2-methyl-6-(4-(pyridin-2-ylethinyl)phenyl)imidazo[2,1-b]thiazol-5-amin,
[12] N-tert-Butyl-6-(5-(pyridin-2-ylethinyl)furan-2-yl)imidazo[2,1-b]thiazol-5-amin,
[13] N-tert-Butyl-3-methyl-6-(5-(phenylethinyl)thiophen-2-yl)imidazo[2,1-b]thiazol-5-amin,
[14] 6-(5-(Pyridin-2-ylethinyl)thiophen-2-yl)imidazo[2,1-b]thiazol-5-amin,
[15] N-tert-Butyl-6-(5-(pyrimidin-2-ylethinyl)thiophen-2-yl)imidazo[2,1-b]thiazol-5-amin,
[16] N-tert-Butyl-6-(5-((3-fluorpyridin-2-yl)ethinyl)thiophen-2-yl)imidazo[2,1-b]thiazol-5-amin,
[17] N-tert-Butyl-6-(5-((2-fluorpyridin-4-yl)ethinyl)thiophen-2-yl)imidazo[2,1-b]thiazol-5-amin,
[18] N-tert-Butyl-6-(5-(thiophen-2-ylethinyl)thiophen-2-yl)imidazo[2,1-b]thiazol-5-amin,
[19] N-tert-Butyl-6-(5-(thiazol-2-ylethinyl)thiophen-2-yl)imidazo[2,1-b]thiazol-5-amin,
[20] 3-((5-(5-(tert-Butylamino)imidazo[2,1-b]thiazol-6-yl)thiophen-2-yl)ethinyl)phenol,
[21] 3-((5-(5-(tert-Butylamino)imidazo[2,1-b]thiazol-6-yl)thiophen-2-yl)ethinyl)benzonitril,
[22] N-Ethyl-6-(6-(phenylethinyl)pyridin-3-yl)imidazo[2,1-b]thiazol-5-amin,
[23] N-tert-Butyl-6-(5-((3-methylpyridin-2-yl)ethinyl)thiophen-2-yl)imidazo[2,1-b]thiazol-5-amin,
[24] N-(6-(5-(Pyridin-2-ylethinyl)thiophen-2-yl)imidazo[2,1-b]thiazol-5-yl)acetamid und
[25] N-(6-(5-(Pyridin-2-ylethinyl)thiophen-2-yl)imidazo[2,1-b]thiazol-5-yl)benzamid;

jeweils ggf. in Form entsprechender Salze, oder jeweils in Form entsprechender Solvate.

Ebenfalls besonders bevorzugt sind substituierte Imidazo[2,1-b]thiazol-Verbindungen der vorstehend angegebenen allgemeinen Formel I, die nach 60 Minuten Inkubation in 450 µg Protein aus Schweinehirnhomogenat bei einer Temperatur zwischen 20 °C und 25 °C in einer Konzentration kleiner 2500 nM, bevorzugt kleiner 1000 nM, besonders bevorzugt kleiner 700 nM, ganz besonders bevorzugt kleiner 100 nM, noch weiter bevorzugt kleiner 70 nM, eine 50-prozentige Verdrängung von [$^3$H]-2-Methyl-6-(3-methoxyphenyl)-ethinylpyridin bewirken, dass in einer Konzentration von 5 nM vorliegt.

Die Bestimmung der Verdrängung von [$^3$H]-2-Methyl-6-(3-methoxyphenyl)-ethinylpyridin erfolgt dabei wie im Abschnitt Pharmakologische Methoden, I. Methode zur Bestimmung der Hemmung der [$^3$H]-MPEP-Bindung im mGluR5-Rezeptor-Bindungsassay beschrieben.

Ein weiterer Gegenstand der vorliegenden Erfindung ist ein Verfahren zur Herstellung von Verbindungen der vorstehend angegebenen allgemeinen Formel I, gemäß dem wenigstens eine Verbindung der allgemeinen Formel II,

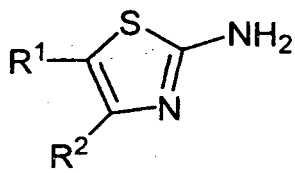

II,

worin $R^1$ und $R^2$ die vorstehend angegebene Bedeutung haben, in einem Reaktionsmedium, ggf. in Gegenwart wenigstens einer organischen oder anorganischen Säure oder wenigstens eines Übergangsmetallsalzes mit wenigstens einem Isocyanid der allgemeinen Formel III,

$R^3$-N≡C III,

worin $R^3$ die vorstehend angegebene Bedeutung hat, und wenigstens einem Aldehyd der allgemeinen Formel IV,

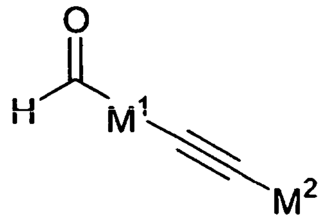

IV,

worin $M^1$ und $M^2$ die vorstehend angegebene Bedeutung haben, umgesetzt und die so erhaltene Verbindung der allgemeinen Formel V,

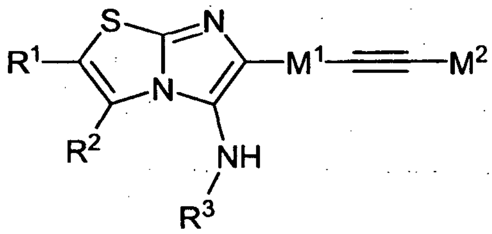

V,

worin $R^1$, $R^2$, $R^3$, $M^1$ und $M^2$ die vorstehend genannte Bedeutung haben, ggf. gereinigt und/oder isoliert wird, und ggf. in ein entsprechendes Salz überführt und dieses ggf. gereinigt und/oder isoliert wird,
oder wenigstens eine Verbindung der allgemeinen Formel II,

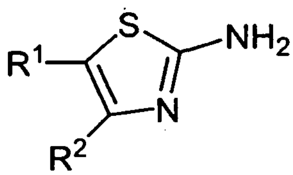

II,

worin $R^1$ und $R^2$ die vorstehend angegebene Bedeutung haben, in einem Reaktionsmedium, ggf. in Gegenwart wenigstens einer organischen oder anorganischen Säure oder wenigstens eines Übergangsmetallsalzes mit wenigstens einem Isocyanid der allgemeinen Formel III,

$R^3$-N≡C III,

worin $R^3$ die vorstehend angegebene Bedeutung hat, und wenigstens einem Aldehyd der allgemeine Formel VII,

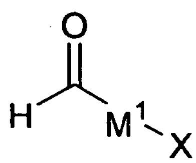

VI,

worin $M^1$ die vorstehend angegebene Bedeutung hat und X für eine Abgangsgruppe, vorzugsweise für einen Halogen-Rest oder für einen Sulfonsäureester, besonders bevorzugt für Chlor, Brom oder Trifluormethansulfonat, steht, umgesetzt und die so erhaltene Verbindung der allgemeinen Formel VII,

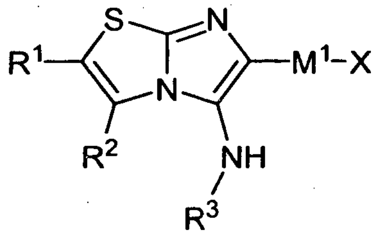

VII,

worin $R^1$, $R^2$, $R^3$, $M^1$ und X die vorstehend genannte Bedeutung haben, ggf. gereinigt und/oder isoliert wird, und ggf. in ein entsprechendes Salz überführt und dieses ggf. gereinigt und/oder isoliert wird, und
durch Umsetzung mit wenigstens einem Acetylen der allgemeinen Formel XI,

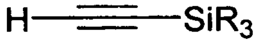

XI,

worin R, unabhängig voneinander, jeweils für einen linearen oder verzweigten Alkyl-Rest oder für einen unsubstituierten Phenyl-Rest steht, in einem Reaktionsmedium, ggf. in Gegenwart wenigstens eines geeigneten Katalysators, ggf. in Gegenwart wenigstens eines Kupfer(I)salzes, bevorzugt in Gegenwart von Kupfer-(I)-iodid, und ggf. in Gegenwart wenigstens einer anorganischen und/oder organischen Base in eine entsprechend substituierte Verbindung der allgemeinen Formel XII,

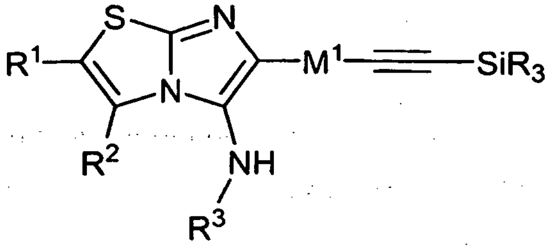

XII,

worin $R^1$, $R^2$, $R^3$ und $M^1$ die vorstehend genannte Bedeutung haben und R, unabhängig voneinander, jeweils für einen linearen oder verzweigten Alkyl-Rest oder für einen unsubstituierten Phenyl-Rest steht, überführt und ggf. gereinigt und/oder isoliert wird, und ggf. in ein entsprechendes Salz überführt und dieses ggf. gereinigt und/oder isoliert wird,
und wenigstens eine Verbindung der allgemeinen Formel XII in einem Reaktionsmedium, ggf. in Gegenwart wenigstens einer anorganischen und/oder organischen Base, ggf. in Gegenwart wenigstens eines anorganischen Salzes, und ggf. in Gegenwart wenigstens eines Ammoniumsalzes, in eine entsprechend substituierte Verbindung der allgemeinen Formel XIII,

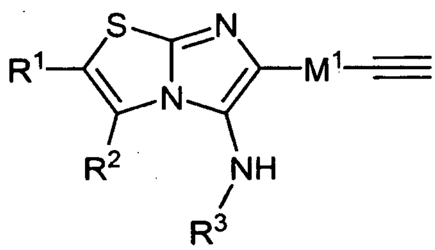

XIII,

worin $R^1$, $R^2$, $R^3$ und $M^1$ die vorstehend genannte Bedeutung haben, überführt und ggf. gereinigt und/oder isoliert wird, und ggf. in ein entsprechendes Salz überführt und dieses ggf. gereinigt und/oder isoliert wird,
und wenigstens eine Verbindung der allgemeinen Formel XIII und/oder wenigstens eine Verbindung der allgemeinen Formel XII durch Umsetzung mit wenigstens einer Verbindung der allgemeinen Formel $M^2$-X, worin $M^2$ die vorstehend genannte Bedeutung hat und X für eine Abgangsgruppe, vorzugsweise für einen Halogen-Rest oder für einen Sulfonsäureester, besonders bevorzugt für Chlor, Brom oder Trifluormethansulfonat steht, in einem Reaktionsmedium, ggf. in Gegenwart wenigstens eines geeigneten Katalysators, ggf. in Gegenwart wenigstens einer anorganischen und/oder organischen Base, ggf. in Gegenwart wenigstens eines anorganischen Salzes und ggf. in Gegenwart wenigstens eines Ammoniumsalzes in eine entsprechend substituierte Verbindung der allgemeinen Formel V,

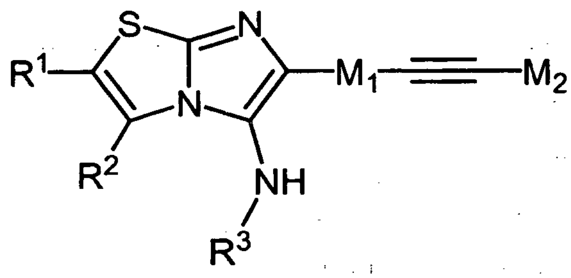

V,

worin $R^1$, $R^2$, $R^3$, $M^1$ und $M^2$ die vorstehend genannte Bedeutung haben überführt und ggf. gereinigt und/oder isoliert wird, und ggf. in ein entsprechendes Salz überführt und dieses ggf. gereinigt und/oder isoliert wird,
oder eine Verbindung der allgemeinen Formel VII durch Umsetzung mit wenigstens einem Acetylen der allgemeinen Formel VIII,

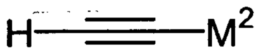

VIII,

worin $M^2$ die vorstehend angegebene Bedeutung hat, in einem Reaktionsmedium, ggf. in Gegenwart wenigstens eines geeigneten Katalysators, ggf. in Gegenwart wenigstens eines Kupfer(I)salzes, bevorzugt in Gegenwart von Kupfer-(I)-iodid und ggf. in Gegenwart wenigstens einer anorganischen und/oder organischen Base in eine entsprechend substituierte Verbindung der allgemeinen Formel V,

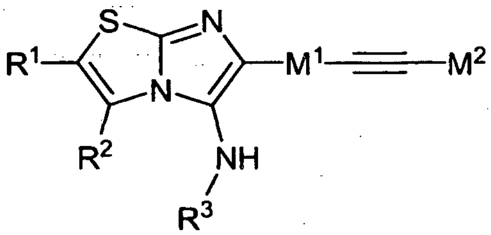

V,

worin $R^1$, $R^2$, $R^3$, $M^1$ und $M^2$ die vorstehend genannte Bedeutung haben überführt und ggf. gereinigt und/oder isoliert wird, und ggf. in ein entsprechendes Salz überführt und dieses ggf. gereinigt und/oder isoliert wird,
und ggf. die Verbindung der allgemeinen Formel V durch Umsetzung mit wenigstens einer Verbindung der allgemeinen Formel $R^4$-X, worin $R^4$ die vorstehend angegebene Bedeutung hat und X für eine Abgangsgruppe, bevorzugt für einen Halogen-Rest oder für einen Sulfonsäureester, besonders bevorzugt für Chlor, steht, in einem Reaktionsmedium, in Gegenwart wenigstens einer organischen oder anorganischen Base, vorzugsweise in Gegenwart wenigstens eines Metallhydridsalzes,

oder durch Umsetzung mit wenigstens einer Verbindung der allgemeinen Formel $R^{21}$-C(=O)-OH, worin $R^{21}$ die vorstehend angegebene Bedeutung hat, in einem Reaktionsmedium, ggf. in Gegenwart wenigstens einer organischen oder anorganischen Base und/oder in Gegenwart wenigstens eines Kopplungsmittels, oder durch Umsetzung mit wenigstens einer Verbindung der allgemeinen Formel $R^{21}$-C(=O)-X, worin $R^{21}$ die vorstehend angegebene Bedeutung hat und X für eine Abgangsgruppe, bevorzugt für einen Halogen-Rest oder für einen Sulfonsäureester, besonders bevorzugt für Chlor, Brom oder Trifluormethansulfonat, steht, in einem Reaktionsmedium, ggf. in Gegenwart wenigstens einer organischen oder anorganischen Base,
oder durch Umsetzung mit wenigstens einer Verbindung der allgemeinen Formel $R^{21}$-C(=O)-H, worin $R^{21}$ die vorstehend angegebene Bedeutung hat, in einem Reaktionsmedium, ggf. in Gegenwart wenigstens eines Reduktionsmittels, in eine Verbindung der allgemeinen Formel I, ggf. in Form eines entsprechenden Salzes, überführt wird,

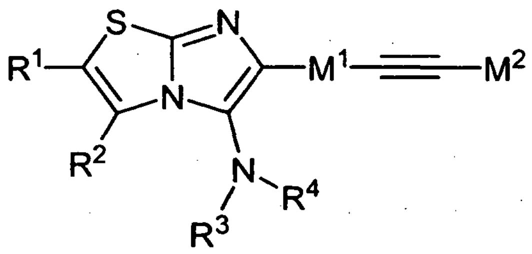

I,

worin $R^1$, $R^2$, $R^3$, $R^4$, $M^1$ und $M^2$ die vorstehend angegebene Bedeutung haben, und diese ggf. gereinigt und/oder isoliert wird.

Ebenfalls ein Gegenstand der vorliegenden Erfindung ist ein Verfahren zur Herstellung von Verbindungen der vorstehend angegebenen allgemeinen Formel I gemäß dem wenigstens eine Verbindung der allgemeinen Formel V,

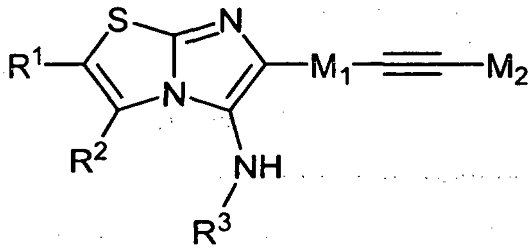

V,

worin $R^1$, $R^2$, $R^3$, $M^1$ und $M^2$ die vorstehend genannte Bedeutung haben, ggf. in einem Reaktionsmedium in Gegenwart wenigstens einer organischen oder anorganischen Säure, umgesetzt, und die so erhaltene Verbindung der allgemeinen Formel IX,

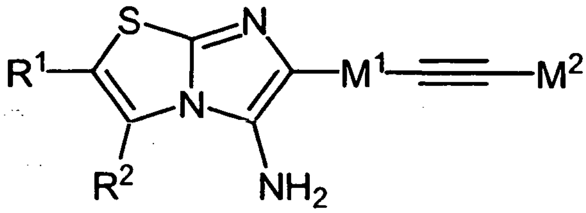

IX,

worin $R^1$, $R^2$, $M^1$ und $M^2$ die vorstehend genannte Bedeutung haben, ggf. gereinigt und/oder isoliert wird, und ggf. in ein entsprechendes Salz überführt und dieses ggf. gereinigt und/oder isoliert wird und in einem Reaktionsmedium, in

Gegenwart wenigstens einer anorganischen oder organischen Base, vorzugsweise in Gegenwart wenigstens eines Metallhydridsalzes, mit wenigstens einer Verbindung der allgemeinen Formel $R^3$-X, worin $R^3$ die vorstehend angegebene Bedeutung hat und X für eine Abgangsgruppe, bevorzugt für einen Halogen-Rest oder für einen Sulfonsäureester, besonders bevorzugt für Chlor, steht, oder
in einem Reaktionsmedium, ggf. in Gegenwart wenigstens einer organischen oder anorganischen Base und/oder ggf. in Gegenwart wenigstens eines Kopplungsmittels mit wenigstens einer Verbindung der allgemeinen Formel $R^{21}$-C(=O)-OH, worin $R^{21}$ die vorstehend genannte Bedeutung hat,
oder in einem Reaktionsmedium, ggf. in Gegenwart wenigstens einer organischen oder anorganischen Base mit wenigstens einer Verbindung der allgemeinen Formel $R^{21}$-C(=O)-X, worin $R^{21}$ die vorstehend genannte Bedeutung hat und X für eine Abgangsgruppe, vorzugsweise für einen Halogen-Rest oder für einen Sulfonsäureester, besonders bevorzugt für Chlor, Brom oder Trifluormethansulfonat, steht,
oder in einem Reaktionsmedium, ggf. in Gegenwart wenigstens eines Reduktionsmittels mit wenigstens einer Verbindung der allgemeinen Formel $R^{21}$-C(=O)-H, worin $R^{21}$ die vorstehend genannte Bedeutung hat,
in eine entsprechende Verbindung der allgemeinen Formel X, ggf. in Form eines entsprechenden Salzes, überführt wird,

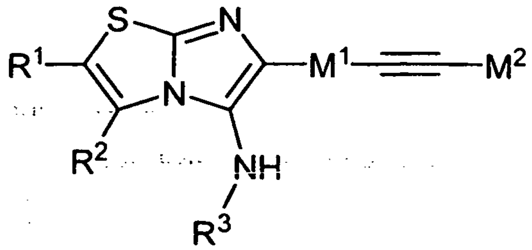

X,

worin $R^1$, $R^2$, $R^3$, $M^1$ und $M^2$ die vorstehend genannte Bedeutung haben, und diese ggf. gereinigt und/oder isoliert wird, und ggf. die Verbindung der allgemeinen Formel X durch Umsetzung mit wenigstens einer Verbindung der allgemeinen Formel $R^4$-X, worin $R^4$ die vorstehend genannte Bedeutung hat und X für eine Abgangsgruppe, bevorzugt für einen Halogen-Rest oder für einen Sulfonsäureester, besonders bevorzugt für Chlor, steht, in einem Reaktionsmedium, in Gegenwart wenigstens einer organischen oder anorganischen Base, vorzugsweise in Gegenwart wenigstens eines Metallhydridsalzes,
oder durch Umsetzung mit wenigstens einer Verbindung der allgemeinen Formel $R^{21}$-C(=O)-OH, worin $R^{21}$ vorstehend genannte Bedeutung hat, in einem Reaktionsmedium, ggf. in Gegenwart wenigstens einer organischen oder anorganischen Base und/oder in Gegenwart wenigstens eines Kopplungsmittels, oder durch Umsetzung mit wenigstens einer Verbindung der allgemeinen Formel $R^{21}$-C(=O)-X, worin $R^{21}$ die vorstehend genannte Bedeutung hat und X für eine Abgangsgruppe, bevorzugt für einen Halogen-Rest oder für einen Sulfonsäureester, besonders bevorzugt für Chlor, Brom oder Trifluormethansulfonat, steht, in einem Reaktionsmedium, ggf. in Gegenwart wenigstens einer organischen oder anorganischen Base,
oder durch Umsetzung mit wenigstens einer Verbindung der allgemeinen Formel $R^{21}$-C(=O)-H, worin $R^{21}$ die vorstehend genannte Bedeutung hat, in einem Reaktionsmedium, ggf. in Gegenwart wenigstens eines Reduktionsmittels,
in eine Verbindung der vorstehend angegebenen allgemeinen Formel I, ggf. in Form eines entsprechenden Salzes, überführt wird,

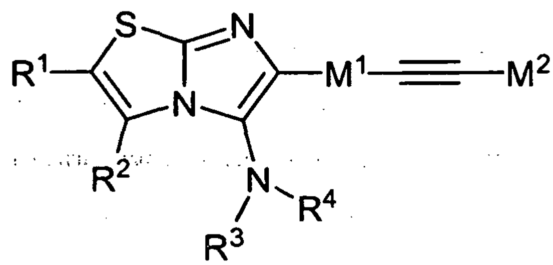

I,

worin $R^1$, $R^2$, $R^3$, $R^4$, $M^1$ und $M^2$ die vorstehend genannte Bedeutung haben, und diese ggf. gereinigt und/oder isoliert wird.

Die erfindungsgemäßen Verfahren zur Herstellung substituierter Imidazo[2,1-b]thiazol-Verbindungen der vorstehend angegebenen allgemeinen Formel I sind auch in den nachfolgenden Schemata 1 bis 4 angegeben.

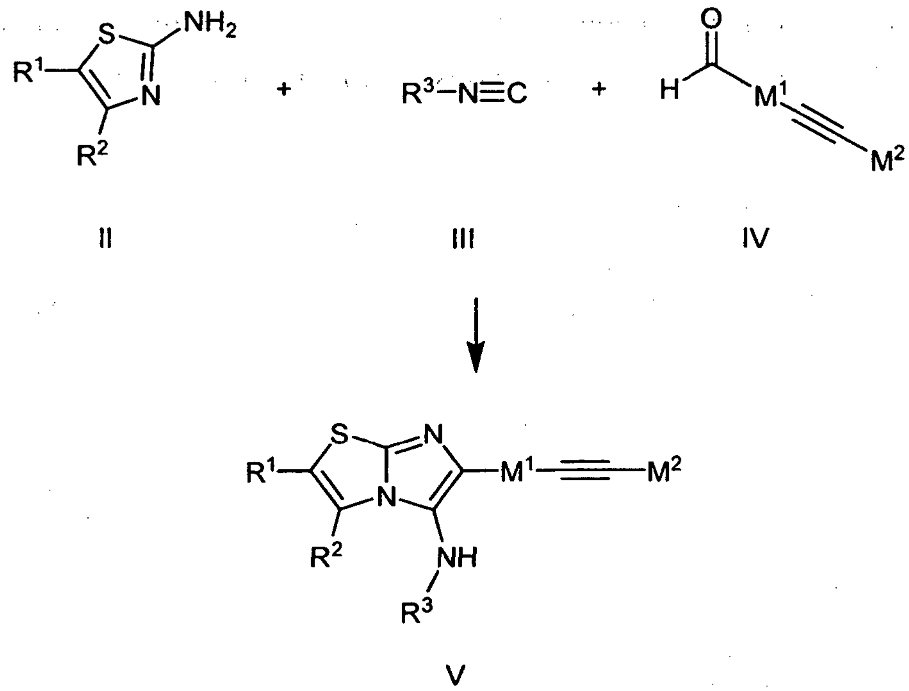

II III IV

V

Schema 1.

In einer Drei-Komponenten-Kopplungsreaktion werden Amine der allgemeinen Formel II mit Isocyaniden der allgemeinen Formel III und Aldehyden der allgemeinen Formel IV in einem Reaktionsmedium, vorzugsweise ausgewählt aus der Gruppe bestehend aus Chloroform, Dichlormethan, Acetonitril, Methanol und Ethanol, unter Zusatz wenigstens einer organischen oder anorganischen Säure, vorzugsweise Trifluoressigsäure oder Perchlorsäure, oder unter Zusatz wenigstens eines Übergangsmetallsalzes, vorzugsweise unter Zusatz wenigstens eines Übergangsmetalltriflats (Übergangsmetalltrifluormethansulfonats), besonders bevorzugt unter Zusatz wenigstens eines Übergangsmetalltriflats ausgewählt aus der Gruppe bestehend aus Scandium(III)trifluormethansulfonat, Ytterbiumtrifluormethansulfonat und Indium(III)trifluormethansulfonat, vorzugsweise bei Temperaturen von 0°C bis 150°C, ggf. in Gegenwart von Mikrowellenstrahlung zu Verbindungen der allgemeinen Formel V umgesetzt.

Ein weiteres Verfahren zur Herstellung substituierter Imidazo[2,1-b]thiazol-Verbindungen der vorstehend angegebenen allgemeinen Formel I via Verbindungen der vorstehend angegebenen allgemeinen Formel V ist in Schema 2 wiedergegeben.

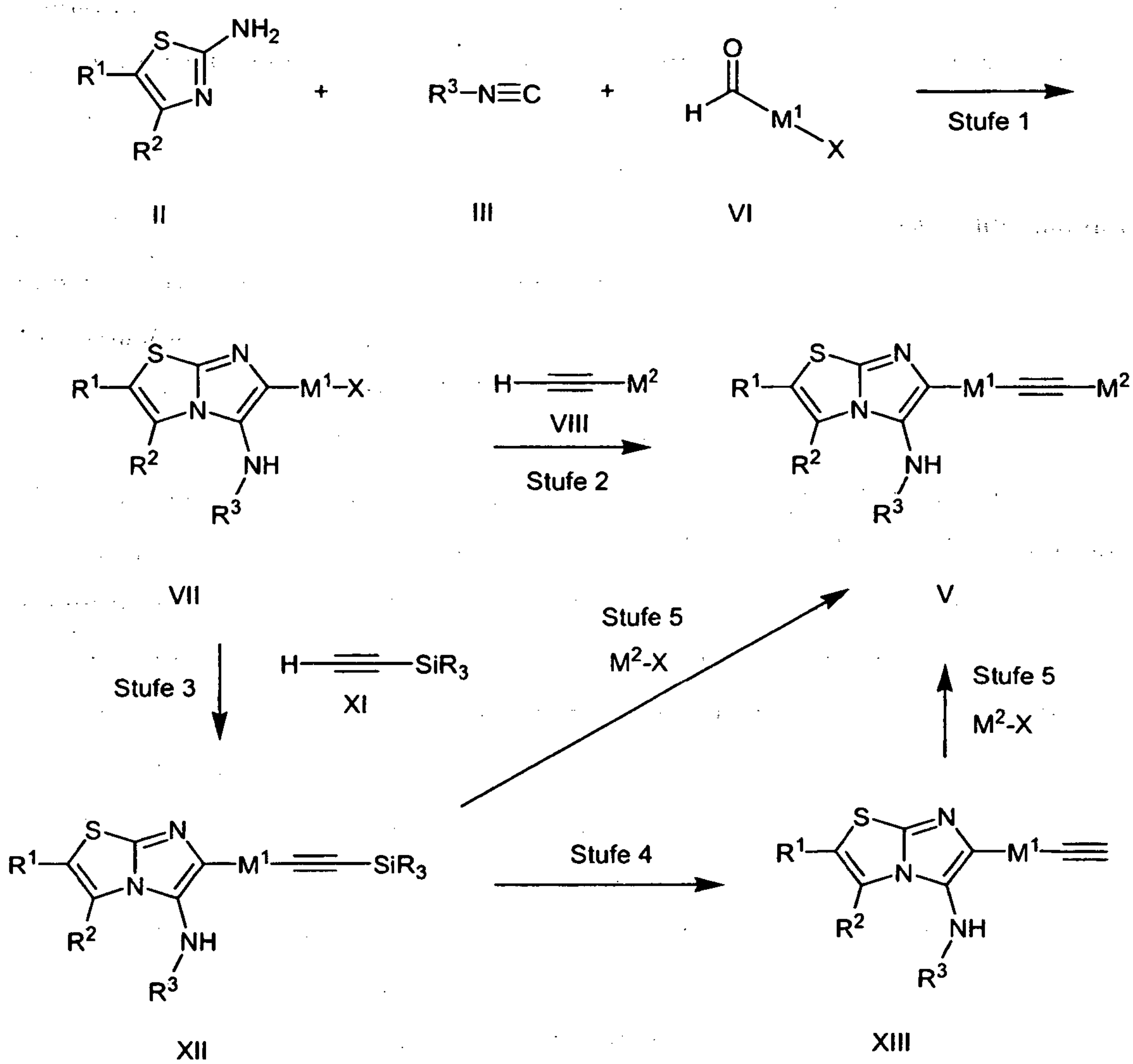


Schema 2.

In Stufe 1 werden in einer Drei-Komponenten-Kopplungsreaktion Amine der allgemeinen Formel II mit Isocyaniden der allgemeinen Formel III und Aldehyden der allgemeinen Formel VI, worin X für eine Abgangsgruppe, vorzugsweise für einen Halogen-Rest oder für einen Sulfonsäureester, besonders bevorzugt für Chlor, Brom oder Trifluormethansulfonat, steht, in einem Reaktionsmedium, vorzugsweise ausgewählt aus der Gruppe bestehend aus Chloroform, Dichlormethan, Acetonitril, Methanol und Ethanol, unter Zusatz wenigstens einer organischen oder anorganischen Säure, vorzugsweise ausgewählt aus der Gruppe bestehend aus Trifluoressigsäure oder Perchlorsäure, oder unter Zusatz wenigstens eines Übergangsmetallsalzes, vorzugsweise unter Zusatz wenigstens eines Übergangsmetalltriflats (Übergangsmetalltrifluormethansulfonats), besonders bevorzugt unter Zusatz wenigstens eines Übergangsmetalltrifluormethansulfonats ausgewählt aus der Gruppe bestehend aus Scandium(III)trifluormethansulfonat, Ytterbiumtrifluormethansulfonat und Indium(III) trifluormethansulfonat, vorzugsweise bei Temperaturen von 0°C bis 150°C, ggf. in Gegenwart von Mikrowellenstrahlung zu Verbindungen der allgemeinen Formel VII, worin X für eine Abgangsgruppe, vorzugsweise für einen Halogen-Rest oder für einen Sulfonsäureester, besonders bevorzugt für Chlor, Brom oder Trifluormethansulfonat, steht, umgesetzt.

In Stufe 2 werden Verbindungen der vorstehend angegebenen allgemeinen Formel VII, worin X für einen Halogen-Rest oder einen Sulfonsäureester, besonders bevorzugt für Chlor, Brom oder Trifluormethansulfonat, steht, mit Acetylenen der allgemeinen Formel VIII in einem Reaktionsmedium, vorzugsweise ausgewählt aus der Gruppe bestehend aus Methanol, Ethylacetat, Ethanol, Isopropanol, n-Butanol, Dioxan, Chloroform, Dichlormethan, Pyridin, Dimethylsulfoxid, Toluol, Tetrahydrofuran, Dimethylformamid, Acetonitril, Diethylether, Wasser und entsprechenden Mischungen, besonders bevorzugt ausgewählt aus der Gruppe bestehend aus Dimethylformamid, Ethylacetat, Tetrahydrofuran, Wasser und entsprechenden Mischungen, vorzugsweise unter Zusatz wenigstens eines Palladiumkatalysators, vorzugsweise ausgewählt aus der Gruppe bestehend aus Palladium(II)-dichlorid [$PdCl_2$], Bis(triphenylphos-

phin)-palladium(II)-acetat [$Pd(PPh_3)_2(OAc)_2$], Bis(triphenylphosphin)-palladium(II)-chlorid [$PdCl_2(PPh_3)_2$], Palladium(II)-acetat [$Pd(OAc)_2$; Ac = Acetat], Bis(acetonitril)-palladium(II)-chlorid [$(CH_3CN)_2)PdCl_2$], Bis(benzonitril)-palladium(II)-chlorid [$(PhCN)_2PdCl_2$] und Tetrakis(triphenylphosphin)palladium [$(PPh_3)_4Pd$], besonders bevorzugt ausgewählt aus der Gruppe bestehend aus $Pd(PPh_3)_2(OAc)_2$, $(PPh_3)_4Pd$ und $PdCl_2(PPh_3)_2$, ggf. in Gegenwart wenigstens eines Kupfer(I)salzes, vorzugsweise in Gegenwart von Kupfer(I)-iodid, ggf. in Gegenwart wenigstens eines Phosphins, vorzugsweise eines Phosphins ausgewählt aus der Gruppe bestehend aus Triphenylphosphin, Tri-(tert-butyl)-phosphin, Triphenylarsin und Tri-(ortho-toluyl)-phosphin, besonders bevorzugt in Gegenwart von Triphenylphosphin, ggf. unter Zusatz wenigstens eines anorganischen Salzes, bevorzugt unter Zusatz von Lithium- und/oder Zinkchlorid, ggf. unter Zusatz wenigstens einer organischen Base, vorzugsweise einer organischen Base ausgewählt aus der Gruppe bestehend aus Triethylamin, Diisopropylamin, Diisopropylethylamin und [1,4]-Diazabicyclo-[2.2.2]octan und/oder Zusatz wenigstens einer anorganischen Base, vorzugsweise ausgewählt aus der Gruppe bestehend aus Kaliumcarbonat, Natriumhydrogencarbonat und Cäsiumcarbonat, wobei insbesondere die organische Base auch das Reaktionsmedium sein kann, bei Temperaturen von vorzugsweise -70 °C bis 300 °C, besonders bevorzugt von -70°C bis 150°C, ,ggf. in Gegenwart von Mikrowellenstrahlung zu Verbindungen der allgemeinen Formel V umgesetzt.

In Stufe 3 werden Verbindungen der vorstehend angegeben allgemeinen Formel VII mit Verbindungen der vorstehend angegebenen allgemeinen Formel XI unter den in Schema 2, Stufe 2, genannten Bedingungen zu Verbindungen der allgemeinen Formel XII umgesetzt.

In Stufe 4 werden Verbindungen der vorstehend angegebenen allgemeinen Formel XII in einem Reaktionsmedium, vorzugsweise ausgewählt aus der Gruppe bestehend aus Methanol, Ethylacetat, Ethanol, Isopropanol, n-Butanol, Dioxan, Chloroform, Dichlormethan, Pyridin, Dimethylsulfoxid, Toluol, Tetrahydrofuran, Dimethylformamid, Acetonitril, Diethylether, Wasser und entsprechenden Mischungen, besonders bevorzugt ausgewählt aus der Gruppe bestehend aus Dimethylformamid, Ethylacetat, Tetrahydrofuran, Wasser und entsprechenden Mischungen, ggf. in Gegenwart wenigstens einer anorganischen Base, bevorzugt in Gegenwart wenigstens einer anorganischen Base ausgewählt aus der Gruppe bestehend aus Kaliumcarbonat, Natriumhydroxid, Kaliumhydrogencarbonat, Natriumhydrogencarbonat, Kaliumhydroxid und Lithiumhydroxid, ggf. in Gegenwart wenigstens einer anorganischen Base, bevorzugt wenigstens einer anorganischen Base ausgewählt aus der Gruppe bestehend aus Triethylamin und Pyridin, ggf. in Gegenwart wenigstens eines anorganischen Salzes, bevorzugt in Gegenwart wenigstens eines Ammoniumsalzes oder in Gegenwart von Kalium- und/oder Natriumfluorid, besonders bevorzugt in Gegenwart wenigstens eines Ammoniumsalzes ausgewählt aus der Gruppe bestehend aus Tetra-n-butylammoniumfluorid, Tetra-n-butyl-ammoniumiodid und Tetrabutylammoniumbromid, bei Temperaturen von vorzugsweise -70 °C bis 300 °C, besonders bevorzugt von -70°C bis 150°C, , ggf. in Gegenwart von Mikrowellenstrahlung, zu Verbindungen der allgemeinen Formel XIII umgesetzt.

In Stufe 5 werden Verbindungen der vorstehend angegebenen allgemeinen Formeln XII und XIII mit Verbindungen der allgemeinen Formel $M^2$-X, worin X für einen Halogen-Rest oder einen Sulfonsäureester, besonders bevorzugt für Chlor, Brom oder Trifluormethansulfonat, steht, in einem Reaktionsmedium, vorzugsweise ausgewählt aus der Gruppe bestehend aus Methanol, Ethylacetat, Ethanol, Isopropanol, n-Butanol, Dioxan, Chloroform, Dichlormethan, Pyridin, Dimethylsulfoxid, Toluol, Tetrahydrofuran, Dimethylformamid, Acetonitril, Diethylether, Wasser und entsprechenden Mischungen, besonders bevorzugt ausgewählt aus der Gruppe bestehend aus Dimethylformamid, Ethylacetat, Tetrahydrofuran, Wasser und entsprechenden Mischungen, vorzugsweise unter Zusatz wenigstens eines Palladiumkatalysators, vorzugsweise ausgewählt aus der Gruppe bestehend aus Palladium(II)-dichlorid [$PdCl_2$], Bis(triphenylphosphin)-palladium(II)-acetat [$Pd(PPh_3)_2(OAc)_2$], Bis(triphenylphosphin)-palladium(II)-chlorid [$PdCl_2(PPh_3)_2$], Palladium(II)-acetat [$Pd(OAc)_2$; Ac = Acetat], Bis(acetonitril)-palladium(II)-chlorid [$(CH_3CN)_2)PdCl_2$], Bis(benzonitril)-palladium(II)-chlorid [$(PhCN)_2PdCl_2$] und Tetrakis(triphenylphosphin)palladium [$(PPh_3)_4Pd$], besonders bevorzugt ausgewählt aus der Gruppe bestehend aus $Pd(PPh_3)_2(OAc)_2$, $(PPh_3)_4Pd$ und $PdCl_2(PPh_3)_2$, ggf. in Gegenwart wenigstens eines Kupfer(I)salzes, vorzugsweise in Gegenwart von Kupfer(I)-iodid, ggf. in Gegenwart wenigstens eines Phosphins, vorzugsweise eines Phosphins ausgewählt aus der Gruppe bestehend aus Triphenylphosphin, Tri-(tert-butyl)-phosphin, Triphenylarsin und Tri-(ortho-toluyl)-phosphin, besonders bevorzugt in Gegenwart von Triphenylphosphin, ggf. unter Zusatz wenigstens eines anorganischen Salzes, bevorzugt unter Zusatz von Lithium-und/oder Zinkchlorid, ggf. in Gegenwart wenigstens eines Ammoniumsalzes oder in Gegenwart von Kalium- und/oder Natriumfluorid, bevorzugt in Gegenwart wenigstens eines Ammoniumsalzes ausgewählt aus der Gruppe bestehend aus Tetra-n-butylammoniumfluorid, Tetra-n-butyl-ammoniumiodid und Tetrabutylammoniumbromid, ggf. unter Zusatz wenigstens einer organischen Base, vorzugsweise einer organischen Base ausgewählt aus der Gruppe bestehend aus Triethylamin, Diisopropylamin, Diisopropylethylamin und [1,4]-Diazabicyclo-[2.2.2]octan und/oder Zusatz wenigstens einer anorganischen Base, vorzugsweise ausgewählt aus der Gruppe bestehend aus Kaliumcarbonat, Natriumhydrogencarbonat und Cäsiumcarbonat, wobei insbesondere die organische Base auch das Reaktionsmedium sein kann, bei Temperaturen von vorzugsweise - 70 °C bis 300 °C, besonders bevorzugt von -70°C bis 150°C, ggf. in Gegenwart von Mikrowellenstrahlung zu Verbindungen der allgemeinen Formel V umgesetzt.

Die Umsetzung von Verbindungen der allgemeinen Formel XII mit Verbindungen der allgemeinen Formel $M^2$-X erfolgt vorzugsweise in Gegenwart wenigstens eines Ammoniumsalzes oder in Gegenwart von Kalium- und/oder Natriumfluorid.

Die Verbindungen der allgemeinen Formel V lassen sich wie in Schema 3 dargestellt zu Verbindungen der Formel X umsetzen.

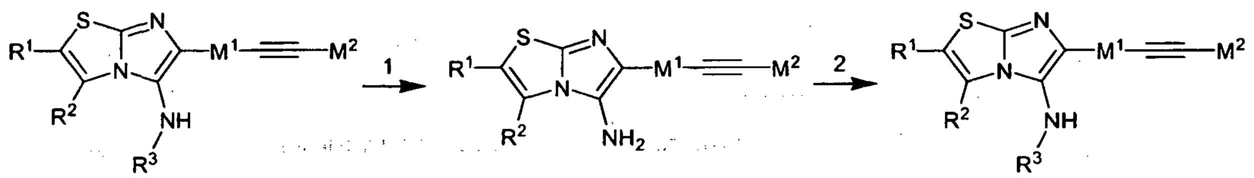


V IX X

Schema 3.

In Stufe 1 werden Verbindungen der vorstehend angegebenen allgemeinen Formel V in einem Reaktionsmedium, vorzugsweise ausgewählt aus der Gruppe bestehend aus Ethanol, Methanol und Aceton, unter Zusatz wenigstens einer organischen Säure, vorzugsweise Essigsäure oder Trifluoressigsäure und/oder unter Zusatz wenigstens einer anorganischen Säure, vorzugsweise Salzsäure oder Schwefelsäure, bei Temperaturen von vorzugsweise 0°C bis 80°C, ggf. in Gegenwart von Mikrowellenstrahlung zu Verbindungen der allgemeinen Formel IX umgesetzt. In Stufe 2 werden Verbindungen der vorstehend angegebenen allgemeinen Formel IX mit Carbonsäuren der allgemeinen Formel $R^{21}$-C(=O)-OH, worin $R^{21}$ die vorstehend genannte Bedeutung hat, in einem Reaktionsmedium, vorzugsweise ausgewählt aus der Gruppe bestehend aus Diethylether, Tetrahydrofuran, Acetonitril, Methanol, Ethanol, Dimethylformamid und Dichlormethan, ggf. in Gegenwart wenigstens eines Kupplungsreagenzes, vorzugsweise ausgewählt aus der Gruppe bestehend aus 1-Benzotriazolyloxy-tris-(dimethylamino)-phosphonium hexafluorophosphat (BOP), Dicyclohexylcarbodiimid (DCC), N'-(3-Dimethylaminopropyl)-N-ethylcarbodiimid (EDCI), N-[(Dimethyamino)-1H-1, 2, 3-triazolo[4, 5-b]pyridino-1-ylmethylen]-N-methylmethanaminium hexafluorophosphat N-oxid (HATU), O-(Benzotriazol-1-yl)-N,N,N',N'-tetramethyluroniom hexafluorophosphat (HBTU) und 1-Hydroxy-7-azabenzotriazol (HOAt), ggf. in Gegenwart wenigstens einer anorganischen Base, vorzugsweise ausgewählt aus der Gruppe bestehend aus Kaliumcarbonat und Cäsiumcarbonat, oder einer organischen Base, vorzugsweise ausgewählt aus der Gruppe bestehend aus Triethylamin, Pyridin, Dimethylaminopyridin und Diisopropylethylamin vorzugsweise bei Temperaturen von -70°C bis 100°C, ggf. in Gegenwart von Mikrowellenstrahlung zu Verbindungen der allgemeinen Formel X umgesetzt.

Alternativ werden Verbindungen der allgemeinen Formel IX mit Carbonsäurederivaten bzw. Kohlensäurederivaten der allgemeinen Formel $R^{21}$-C(=O)-X, wobei X für einen Halogen-Rest, vorzugsweise Chlor oder Brom, steht, in einem Reaktionsmedium, vorzugsweise ausgewählt aus der Gruppe bestehend aus Diethylether, Tetrahydrofuran, Acetonitril, Methanol, Ethanol, Dimethylformamid und Dichlormethan, mit oder ohne Zusatz wenigstens einer organischen oder anorganischen Base, beispielsweise Triethylamin, Dimethylaminopyridin, Pyridin oder Diisopropylamin, ggf. in Gegenwart wenigstens einer organischen Base, vorzugsweise ausgewählt aus der Gruppe bestehend aus Triethylamin, Dimethylaminopyridin, Pyridin und Diisopropylamin, oder einer anorganischen Base bei Temperaturen von vorzugsweise -70°C bis 100°C, ggf. in Gegenwart von Mikrowellenstrahlung zu Verbindungen der allgemeinen Formel X umgesetzt.

Als weitere Alternative werden Verbindungen der allgemeinen Formel IX mit Aldehyden der allgemeinen Formel $R^{21}$-C(=O)-H in einem Reaktionsmedium, vorzugsweise ausgewählt aus der Gruppe bestehend aus Diethylether, Tetrahydrofuran, Methanol, Ethanol, Dichlormethan und Toluol, unter Zusatz wenigstens eines Reduktionsmittels, vorzugsweise ausgewählt aus der Gruppe bestehend aus Natriumborhydrid, Natriacetoxybofiydrid oder Natriumcyanoborhydrid, bei Temperaturen von vorzugsweise -70°C bis 100°C, ggf. in Gegenwart von Mikrowellenstrahlung zu Verbindungen der allgemeinen Formel X umgesetzt.

Ebenfalls lassen sich Verbindungen der allgemeinen Formel IX mit Verbindungen der allgemeinen Formel $R^3$-X, worin X für einen Halogen-Rest, bevorzugt Chlor, steht, in einem Reaktionsmedium, vorzugsweise ausgewählt aus der Gruppe bestehend aus Toluol, Tetrahydrofuran und Diethylether, unter Zusatz wenigstens eines Metallhydridsalzes, vorzugsweise unter Zusatz wenigstens eines Metallhydridsalzes ausgewählt aus der Gruppe bestehend aus Natriumhydrid, Kaliumhydrid und Lithiumhydrid, bei Temperaturen von vorzugsweise 0 °C bis 40 °C zu Verbindungen der allgemeinen Formel X umsetzen.

Verbindungen der allgemeinen Formeln X und V lassen sich wie in Schema 4 angegeben weiter zu den Verbindungen der allgemeinen Formel I umsetzen, wobei die gleichen Methoden, wie unter Schema 3, Stufe 2, beschrieben, verwendet werden können.

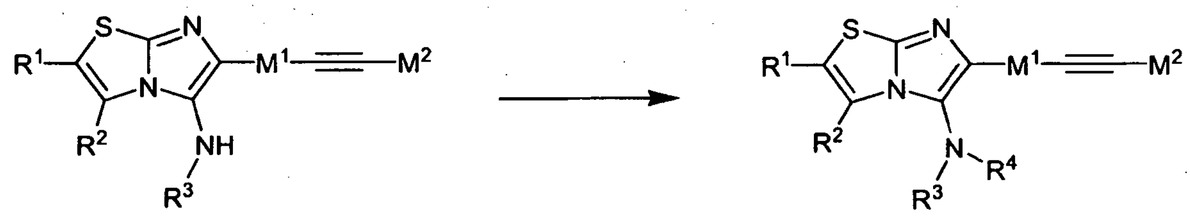

V, X

I

Schema 4.

Die Verbindungen der vorstehend angegebenen Formeln II, III, IV, VI und VIII, sowie der vorstehend genannten allgemeinen Formeln $R^3$-X, $R^4$-X, $R^{21}$-C(=O)-OH, $R^{21}$-C(=O)-X und $R^{21}$-C(=O)-H sind jeweils am Markt käuflich erhältlich und/oder können nach den üblichen, dem Fachmann bekannten Verfahren hergestellt werden.

Die vorstehend beschriebenen Umsetzungen können jeweils unter üblichen, dem Fachmann geläufigen Bedingungen, beispielsweise in Hinblick auf Druck oder Reihenfolge der Zugabe der Komponenten durchgeführt werden. Ggf. kann die unter den jeweiligen Bedingungen optimale Verfahrensführung vom Fachmann durch einfache Vorversuche ermittelt werden.

Die nach den vorstehend beschriebenen Umsetzungen erhaltenen Zwischen- und Endprodukte können jeweils, falls gewünscht und/oder erforderlich, nach üblichen, dem Fachmann bekannten Methoden gereinigt und/oder isoliert werden. Geeignete Reinigungsverfahren sind beispielsweise Extraktionsverfahren und chromatographische Verfahren wie Säulenchromatographie oder präparative Chromatographie.

Sämtliche der vorstehend beschriebenen Verfahrensschritte sowie jeweils auch die Reinigung und/oder Isolierung von Zwischen- oder Endprodukten können teilweise oder vollständig unter einer Inertgasatmossphäre, vorzugsweise unter Stickstoffatmossphäre, durchgeführt werden.

Die erfindungsgemäßen substituierten Imidazo[2,1-b]thiazol-Verbindungen der vorstehend genannten allgemeinen Formeln I, Ia, Ib, Ic, Id, Ie, If, Ig und Ih, im Folgenden nur als Verbindungen der allgemeinen Formel I bezeichnet, sowie entsprechende Stereoisomere können sowohl in Form ihrer freien Basen, ihrer freien Säuren wie auch in Form entsprechender Salze, insbesondere physiologisch verträglicher Salze, isoliert werden.

Die freien Basen der jeweiligen erfindungsgemäßen substituierten Imidazo[2,1-b]thiazol-Verbindungen der vorstehend genannten allgemeinen Formel I sowie entsprechender Stereoisomere können beispielsweise durch Umsetzung mit einer anorganischen oder organischen Säure, vorzugsweise mit Salzsäure, Bromwasserstoffsäure, Schwefelsäure, Phosphorsäure, Methansulfonsäure, p-Toluolsulfonsäure, Kohlensäure, Ameisensäure, Essigsäure, Oxalsäure, Bernsteinsäure, Weinsäure, Mandelsäure, Fumarsäure, Milchsäure, Citronensäure, Glutaminsäure oder Asparaginsäure, in die entsprechenden Salze, vorzugsweise physiologisch verträglichen Salze, überführt werden.

Die freien Basen der jeweiligen substituierten Imidazo[2,1-b]thiazol-Verbindungen der vorstehend genannten allgemeinen Formel I und entsprechender Stereoisomere können ebenfalls mit der freien Säure oder einem Salz eines Zuckerersatzstoffes, wie z.B. Saccharin, Cyclamat oder Acesulfam, in die entsprechenden physiologisch verträglichen Salze überführt werden.

Entsprechend können die freien Säuren der substituierten Imidazo[2,1-b]thiazol-Verbindungen der vorstehend genannten allgemeinen Formel I und entsprechender Stereoisomere durch Umsetzung mit einer geeigneten Base in die entsprechenden physiologisch verträglichen Salze überführt werden. Beispielhaft seien die Alkalimetallsalze, Erdalkalimetallsalze oder Ammoniumsalze $[NH_xR_{4-x}]^+$, worin x = 0, 1, 2, 3 oder 4 ist und R für einen linearen oder verzweigten $C_{1-4}$-Alkyl-Rest steht, genannt.

Die erfindungsgemäßen substituierten Imidazo[2,1-b]thiazol-Verbindungen der vorstehend genannten allgemeinen Formel I und entsprechende Stereoisomere können ggf., ebenso wie die entsprechenden Säuren, die entsprechenden Basen oder Salze dieser Verbindungen, nach üblichem, dem Fachmann bekannten Methoden auch in Form ihrer Solvate, vorzugsweise in Form ihrer Hydrate, erhalten werden.

Sofern die erfindungsgemäßen substituierten Imidazo[2,1-b]thiazol-Verbindungen der vorstehend genannten allgemeinen Formel I nach ihrer Herstellung in Form einer Mischung ihrer Stereoisomeren, vorzugsweise in Form ihrer Racemate oder anderer Mischungen ihrer verschiedenen Enantiomeren und/oder Diastereomeren erhalten werden, können diese nach üblichen, dem Fachmann bekannten Verfahren getrennt und ggf. isoliert werden. Beispielhaft seien chromatographische Trennverfahren, insbesondere Flüssigkeitschromatographie-Verfahren unter Normaldruck oder unter erhöhtem Druck, bevorzugt MPLC- und HPLC-Verfahren, sowie Verfahren der fraktionierten Kristallisation genannt. Dabei können insbesondere einzelne Enantiomeren, z.B. mittels HPLC an chiraler stationärer Phase oder mittels Kristallisation mit chiralen Säuren, etwa (+)-Weinsäure, (-)-Weinsäure oder (+)-10-Camphersulfonsäure, gebildete diastereomere Salze voneinander getrennt werden.

Die erfindungsgemäßen substituierten Imidazo[2,1-b]thiazol-Verbindungen der vorstehend genannten allgemeinen Formel I und entsprechende Stereoisomere sowie jeweils die entsprechenden Säuren, Basen, Salze und Solvate sind toxikologisch unbedenklich und eignen sich daher als pharmazeutische Wirkstoffe in Arzneimitteln.

Ein weiterer Gegenstand der vorliegenden Erfindung ist daher ein Arzneimittel enthaltend wenigstens eine erfindungsgemäße Imidazo[2,1-b]thiazol-Verbindung der vorstehend angegebenen allgemeinen Formel I, jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form eines entsprechenden Salzes, oder jeweils in Form eines entsprechenden Solvates, sowie ggf. einen oder mehrere pharmazeutisch verträgliche Hilfsstoffe.

Das erfindungsgemäße Arzneimittel eignet sich zur mGluR5-Rezeptor-Regulation, insbesondere zur Inhibierung des mGluR5-Rezeptors.

Bevorzugt eignet sich das erfindungsgemäße Arzneimittel zur Prophylaxe und/oder Behandlung von Störungen und/oder Krankheiten, die zumindest teilweise durch mGluR5-Rezeptoren vermittelt werden.

Besonders bevorzugt eignet sich das erfindungsgemäße Arzneimittel daher zur Behandlung und/oder Prophylaxe von Schmerz, vorzugsweise von Schmerz ausgewählt aus der Gruppe bestehend aus akutem Schmerz, chronischem Schmerz, neuropathischem Schmerz und visceralem Schmerz; Migräne; Depressionen; neurodegenerativen Erkrankungen, vorzugsweise ausgewählt aus der Gruppe bestehend aus Multipler Sklerose, Morbus Alzheimer, Morbus Parkinson und Morbus Huntington; kognitiven Erkrankungen, vorzugsweise kognitiven Mangelzuständen, besonders bevorzugt des Aufmerksamkeit-Defizit-Syndroms (ADS); psychiatrische Störungen, vorzugsweise ausgewählt aus der Gruppe bestehend aus Angstzuständen und Panikattacken; Epilepsie; Husten; Harninkontinenz, Diarrhöe; Pruritus; Schizophrenie; cerebralen Ischämien; Muskelspasmen; Krämpfen; Lungenkrankheiten, vorzugsweise ausgewählt aus der Gruppe bestehend aus Asthma und Pseudokrupp; Regurgitation (Erbrechen); Schlaganfall; Dyskinesie; Retinopathie; Antriebslosigkeit; Schläfrigkeit; Lustlosigkeit; Kehlkopfentzündung (Laryngitis); Störungen der Nahrungsaufnahme, vorzugsweise ausgewählt aus der Gruppe bestehend aus Bulimie, Kachexie, Anorexie und Fettleibigkeit; Alkoholabhängigkeit; Medikamentenabhängigkeit; Drogenabhängigkeit, vorzugsweise Nikotin- und/oder Kokainabhängigkeit; Alkoholmißbrauch; Medikamentenmißbrauch; Drogenmißbrauch; vorzugsweise Nikotin- und/oder Kokainmißbrauch; Entzugserscheinungen bei Alkohol-, Medikamenten- und/oder Drogen- (insbesondere Nikotin- und/oder Kokain-)abhängigkeit; Toleranzentwicklung gegenüber Medikamenten, vorzugsweise gegenüber natürlichen oder synthetischen Opioiden; Magen-Ösaphagus-Reflux-Syndrom; gastroösophageale Refluxkrankheit; Reizdarmsyndrom; zur Diurese; zur Antinatriurese; zur Beeinflussung des kardiovaskulären Systems; zur Vigilanzsteigerung; zur Libidosteigerung; zur Modulation der Bewegungsaktivität oder zur Lokalanästhesie.

Ganz besonders bevorzugt eignet sich das erfindungsgemäße Arzneimittel zur Prophylaxe von Schmerz, vorzugsweise von Schmerz ausgewählt aus der Gruppe bestehend aus akutem Schmerz, chronischem Schmerz, neuropathischem Schmerz und visceralem Schmerz; psychiatrische Störungen, vorzugsweise ausgewählt aus der Gruppe bestehend aus Angstzuständen und Panikattacken; Alkoholabhängigkeit; Medikamentenabhängigkeit; Störungen der Nahrungsaufnahme, vorzugsweise ausgewählt aus der Gruppe bestehend aus Bulimie, Kachexie, Anorexie und Fettleibigkeit; Drogenabhängigkeit, vorzugsweise Nikotin- und/oder Kokainabhängigkeit; Alkoholmißbrauch; Medikamentenmißbrauch; Drogenmißbrauch; vorzugsweise Nikotin- und/oder Kokainmißbrauch; Entzugserscheinungen bei Alkohol-, Medikamenten- und/oder Drogen-(insbesondere Nikotin- und/oder Kokain-)abhängigkeit; Toleranzentwicklung gegenüber Medikamenten und/oder Drogen, insbesondere gegenüber natürlichen oder synthetischen Opioiden; Magen-Ösaphagus-Reflux-Syndrom, gastroösophageale Refluxkrankheit und Reizdarmsyndrom.

Noch weiter bevorzugt eignet sich das erfindungsgemäße Arzneimittel zur Prophylaxe und/oder Behandlung von Schmerz, vorzugsweise von Schmerz ausgewählt aus der Gruppe bestehend aus akutem Schmerz, chronischem Schmerz, neuropathischem Schmerz und visceralem Schmerz.

Ebenfalls noch weiter bevorzugt eignet sich das erfindungsgemäße Arzneimittel zur Prophylaxe und/oder Behandlung von psychiatrischen Störungen, vorzugsweise ausgewählt aus der Gruppe bestehend aus Angstzuständen und Panikattacken.

Am weitesten bevorzugt eignet sich das erfindungsgemäße Arzneimittel zur Prophylaxe und/oder Behandlung von Schmerzen, vorzugsweise von akuten Schmerzen, chronischen Schmerzen, neuropathischen Schmerzen oder visceralen Schmerzen.

Ein weiterer Gegenstand der vorliegenden Erfindung ist die Verwendung wenigstens einer erfindungsgemäßen substituierten Imidazo[2,1-b]thiazol-Verbindung der vorstehend angegebenen allgemeinen Formel I, jeweils ggf. in Form eines seiner reinen Stereoisomeren, insbesondere Enantiomeren oder Diastereomeren, seiner Racemate oder in Form einer Mischung von Stereoisomeren, insbesondere der Enantiomeren und/oder Diastereomeren, in einem beliebigen Mischungsverhältnis, oder jeweils in Form eines entsprechenden Salzes, oder jeweils in Form eines entsprechenden Solvates, sowie ggf. eines oder mehrerer pharmazeutisch verträglicher Hilfsstoffe zur Herstellung eines Arzneimittels

zur mGluR5-Rezeptor-Regulation, vorzugsweise zur Inhibierung des mGluR5-Rezeptors.

Bevorzugt ist die Verwendung wenigstens einer erfindungsgemäß substituierten Imidazo[2,1-b]thiazol-Verbindung der vorstehend angegebenen allgemeinen Formel I, jeweils ggf. in Form eines seiner reinen Stereoisomeren, insbesondere Enantiomeren oder Diastereomeren, seiner Racemate oder in Form einer Mischung von Stereoisomeren, insbesondere der Enantiomeren und/oder Diastereomeren, in einem beliebigen Mischungsverhältnis, oder jeweils in Form eines entsprechenden Salzes, oder jeweils in Form eines entsprechenden Solvates, sowie ggf. eines oder mehrerer pharmazeutisch verträglicher Hilfsstoffe zur Herstellung eines Arzneimittels zur Prophylaxe und/oder Behandlung von Störungen und/oder Krankheiten, die zumindest teilweise durch mGluR5-Rezeptoren vermittelt werden.

Besonders bevorzugt ist die Verwendung wenigstens einer erfindungsgemäßen substituierten Imidazo[2,1-b]thiazol-Verbindung der vorstehend angegebenen allgemeinen Formel I, jeweils ggf. in Form eines seiner reinen Stereoisomeren, insbesondere Enantiomeren oder Diastereomeren, seiner Racemate oder in Form einer Mischung von Stereoisomeren, insbesondere der Enantiomeren und/oder Diastereomeren, in einem beliebigen Mischungsverhältnis, oder jeweils in Form eines entsprechenden Salzes, oder jeweils in Form eines entsprechenden Solvates, sowie ggf. eines oder mehrerer pharmazeutisch verträglicher Hilfsstoffe zur Herstellung eines Arzneimittels zur Prophylaxe und/oder Behandlung von Schmerz, vorzugsweise von Schmerz ausgewählt aus der Gruppe bestehend aus akutem Schmerz, chronischem Schmerz, neuropathischem Schmerz und visceralem Schmerz; Migräne; Depressionen; neurodegenerativen Erkrankungen, vorzugsweise ausgewählt aus der Gruppe bestehend aus Multipler Sklerose, Morbus Alzheimer, Morbus Parkinson und Morbus Huntington; kognitiven Erkrankungen, vorzugsweise kognitiven Mangelzuständen, besonders bevorzugt des Aufmerksamkeit-Defizit-Syndroms (ADS); psychiatrische Störungen, vorzugsweise ausgewählt aus der Gruppe bestehend aus Angstzuständen und Panikattacken; Epilepsie; Husten; Harninkontinenz; Diarrhöe; Pruritus; Schizophrenie; cerebralen Ischämien; Muskelspasmen; Krämpfen; Lungenkrankheiten, vorzugsweise ausgewählt aus der Gruppe bestehend aus Asthma und Pseudokrupp; Regurgitation (Erbrechen); Schlaganfall; Dyskinesie; Retinopathie; Antriebslosigkeit; Schläfrigkeit; Lustlosigkeit; Kehlkopfentzündung (Laryngitis); Störungen der Nahrungsaufnahme, vorzugsweise ausgewählt aus der Gruppe bestehend aus Bulimie, Kachexie, Anorexie und Fettleibigkeit; Alkoholabhängigkeit; Medikamentenabhängigkeit; Drogenabhängigkeit, vorzugsweise Nikotin- und/oder Kokainabhängigkeit; Alkoholmißbrauch; Medikamentenmißbrauch; Drogenmißbrauch; vorzugsweise Nikotin- und/oder Kokainmißbrauch; Entzugserscheinungen bei Alkohol-, Medikamenten- und/oder Drogen- (insbesondere Nikotin- und/oder Kokain-)abhängigkeit; Toleranzentwicklung gegenüber Medikamenten, insbesondere gegenüber natürlichen oder synthetischen Opioiden; Magen-Ösaphagus-Reflux-Syndrom; gastroösophageale Refluxkrankheit; Reizdarmsyndrom; zur Diurese; zur Antinatriurese; zur Beeinflussung des kardiovaskulären Systems; zur Vigilanzsteigerung; zur Libidosteigerung; zur Modulation der Bewegungsaktivität oder zur Lokalanästhesie.

Ganz besonders bevorzugt ist die Verwendung wenigstens einer erfindungsgemäßen substituierten Imidazo[2,1-b]thiazol-Verbindung der vorstehend angegebenen allgemeinen Formel I, jeweils ggf. in Form eines seiner reinen Stereoisomeren, insbesondere Enantiomeren oder Diastereomeren, seiner Racemate oder in Form einer Mischung von Stereoisomeren, insbesondere der Enantiomeren und/oder Diastereomeren, in einem beliebigen Mischungsverhältnis, oder jeweils in Form eines entsprechenden Salzes, oder jeweils in Form eines entsprechenden Solvates, sowie ggf. eines oder mehrerer pharmazeutisch verträglicher Hilfsstoffe zur Herstellung eines Arzneimittels zur Prophylaxe und/oder Behandlung von Schmerz, vorzugsweise von Schmerz ausgewählt aus der Gruppe bestehend aus akutem Schmerz, chronischem Schmerz, neuropathischem Schmerz und visceralem Schmerz; psychiatrische Störungen, vorzugsweise ausgewählt aus der Gruppe bestehend aus Angstzuständen und Panikattacken; Alkoholabhängigkeit; Medikamentenabhängigkeit; Störungen der Nahrungsaufnahme, vorzugsweise ausgewählt aus der Gruppe bestehend aus Bulimie, Kachexie, Anorexie und Fettleibigkeit; Drogenabhängigkeit, vorzugsweise Nikotin- und/oder Kokainabhängigkeit; Alkoholmißbrauch; Medikamentenmißbrauch; Drogenmißbrauch; vorzugsweise Nikotin- und/oder Kokainmißbrauch; Entzugserscheinungen bei Alkohol-, Medikamenten- und/oder Drogen-(insbesondere Nikotin- und/oder Kokain-) abhängigkeit; Toleranzentwicklung gegenüber Medikamenten und/oder Drogen, insbesondere gegenüber natürlichen oder synthetischen Opioiden; Magen-Ösaphagus-Reflux-Syndrom, gastroösophageale Refluxkrankheit und Reizdarmsyndrom.

Noch weiter bevorzugt ist die Verwendung wenigstens einer erfindungsgemäßen substituierten Imidazo[2,1-b]thiazol-Verbindung der vorstehend angegebenen allgemeinen Formel I, jeweils ggf. in Form eines seiner reinen Stereoisomeren, insbesondere Enantiomeren oder Diastereomeren, seiner Racemate oder in Form einer Mischung von Stereoisomeren, insbesondere der Enantiomeren und/oder Diastereomeren, in einem beliebigen Mischungsverhältnis, oder jeweils in Form eines entsprechenden Salzes, oder jeweils in Form eines entsprechenden Solvates, sowie ggf. eines oder mehrerer pharmazeutisch verträglicher Hilfsstoffe zur Herstellung eines Arzneimittels zur Prophylaxe und/oder Behandlung von Schmerz, vorzugsweise von Schmerz ausgewählt aus der Gruppe bestehend aus akutem Schmerz, chronischem Schmerz, neuropathischem Schmerz und visceralem Schmerz.

Noch weiter bevorzugt ist die Verwendung wenigstens einer erfindungsgemäßen substituierten Imidazo[2,1-b]thiazol-Verbindung der vorstehend angegebenen allgemeinen Formel I, jeweils ggf. in Form eines seiner reinen Stereoisomeren, insbesondere Enantiomeren oder Diastereomeren, seiner Racemate oder in Form einer Mischung von Stereoisomeren, insbesondere der Enantiomeren und/oder Diastereomeren, in einem beliebigen Mischungsverhältnis,

oder jeweils in Form eines entsprechenden Salzes, oder jeweils in Form eines entsprechenden Solvates, sowie ggf. eines oder mehrerer pharmazeutisch verträglicher Hilfsstoffe zur Herstellung eines Arzneimittels zur Prophylaxe und/oder Behandlung von psychiatrischen Störungen, vorzugsweise ausgewählt aus der Gruppe bestehend aus Angstzuständen und Panikattacken.

Das erfindungsgemäße Arzneimittel eignet sich zur Verabreichung an Erwachsene und Kinder einschließlich Kleinkindern und Säuglingen.

Das erfindungsgemäße Arzneimittel kann als flüssige, halbfeste oder feste Arzneiform, beispielsweise in Form von Injektionslösungen, Tropfen, Säften, Sirupen, Sprays, Suspensionen, Tabletten, Patches, Kapseln, Pflastern, Zäpfchen, Salben, Cremes, Lotionen, Gelen, Emulsionen, Aerosolen oder in multipartikulärer Form, beispielsweise in Form von Pellets oder Granulaten, ggf. zu Tabletten verpreßt, in Kapseln abgefüllt oder in einer Flüssigkeit suspendiert, vorliegen und als solche auch verabreicht werden.

Neben wenigstens einer substituierten Imidazo[2,1-b]thiazol-Verbindung der vorstehend angegebenen allgemeinen Formel I, ggf. in Form eines ihrer reinen Stereoisomeren, insbesondere Enantiomeren oder Diastereomeren, ihres Racemates oder in Form von Mischungen der Stereoisomeren, insbesondere der Enantiomeren oder Diastereomeren, in einem beliebigen Mischungsverhältnis, oder ggf. in Form eines entsprechenden Salzes oder jeweils in Form eines entsprechendes Solvates, enthält das erfindungsgemäße Arzneimitteln üblicherweise weitere physiologisch verträgliche pharmazeutische Hilfsstoffe, die bevorzugt ausgewählt werden können aus der Gruppe bestehend aus Trägermaterialien, Füllstoffen, Lösungsmitteln, Verdünnungsmitteln, oberflächenaktiven Stoffen, Farbstoffen, Konservierungsstoffen, Sprengmitteln, Gleitmitteln, Schmiermitteln, Aromen und Bindemitteln.

Die Auswahl der physiologisch verträglichen Hilfsstoffe sowie die einzusetzenden Mengen derselben hängt davon ab, ob das Arzneimittel oral, subkutan, parenteral, intravenös, intraperitoneal, intradermal, intramuskulär, intranasal, buccal, rectal oder örtlich, zum Beispiel auf Infektionen an der Haut, der Schleimhäute und an den Augen, appliziert werden soll. Für die orale Applikation eignen sich bevorzugt Zubereitungen in Form von Tabletten, Dragees, Kapseln, Granulaten, Pellets, Tropfen, Säften und Sirupen, für die parenterale, topische und inhalative Applikation Lösungen, Suspensionen, leicht rekonstituierbare Trockenzubereitungen sowie Sprays.

Die in dem erfindungsgemäßen Arzneimittel zum Einsatz kommenden erfindungsgemäßen substituierten Imidazo[2,1-b]thiazol-Verbindungen in einem Depot in gelöster Form oder in einem Pflaster, gegebenenfalls unter Zusatz von die Hautpenetration fördernden Mitteln, sind geeignete perkutane Applikationszubereitungen.

Oral oder perkutan anwendbare Zubereitungsformen können die jeweilige erfindungsgemäße substituierte Imidazo[2,1-b]thiazol-Verbindung auch verzögert freisetzen.

Die Herstellung der erfindungsgemäßen Arzneimittel erfolgt mit Hilfe von üblichen, aus dem Stande der Technik bekannten Mitteln, Vorrichtungen, Methoden und Verfahren, wie sie beispielsweise in "Remington's Pharmaceutical Sciences", Herausgeber A.R. Gennaro, 17. Auflage, Mack Publishing Company, Easton, Pa, 1985, insbesondere in Teil 8, Kapitel 76 bis 93 beschrieben sind.

Die an den Patienten zu verabreichende Menge der jeweiligen erfindungsgemäßen substituierten Imidazo[2,1-b]thiazol-Verbindungen der vorstehend angegebenen allgemeinen Formel I kann variieren und ist beispielsweise abhängig vom Gewicht oder Alter des Patienten sowie von der Applikationsart, der Indikation und dem Schweregrad der Erkrankung. Üblicherweise werden 0,005 bis 2000 mg/kg, vorzugsweise 0,05 bis 500 mg/kg, besonders bevorzugt 0,05 bis 100 mg/kg Körpergewicht des Patienten wenigstens einer solchen erfindungsgemäßen Verbindung pro Tag appliziert.

**Pharmakologische Methoden:**

**1. Methode zur Bestimmung der Affinität zum mGluR5-Rezeptor**

Schweinehirnhomogenat wird durch Homogenisieren (Polytron PT 3000, Kinematica AG, 10.000 Umdrehungen pro Minute für 90 Sekunden) von Schweinehirnhälften ohne Medulla, Cerebellum und Pons in Puffer pH 8.0 (30mM Hepes, Sigma, Bestellnr. H3375 + 1 Tablette Complete auf 100ml, Roche Diagnostics, Bestellnr. 1836145) im Verhältnis 1:20 (Hirngewicht/Volumen) und Differentialzentrifugation bei 900 x g und 40.000 x g hergestellt. In 250 μl Inkubationsansätzen in 96 Well Mikrotiterplatten werden jeweils 450 μg Protein aus Hirnhomogenat mit 5nM $^3$[H]-MPEP (Tocris, Bestellnr. R1212) (MPEP = 2-Methyl-6-(3-methoxyphenyl)-ethinylpyridin) und die zu untersuchenden Verbindungen (10 μM im Test) in Puffer (wie oben) bei Raumtemperatur für 60 min inkubiert.

Danach werden die Ansätze mit Hilfe eines Brandel Cell Harvesters (Brandel, TYP Robotic 9600) auf Unifilterplates mit Glasfaserfiltermatten (Perkin Elmer, Bestellnr. 6005177) filtriert und anschließend mit Puffer (wie oben) 3-mal mit je 250 μl pro Probe gewaschen. Die Filterplatten werden anschließend für 60 min bei 55 °C getrocknet. Anschließend werden pro Well 30 μL Ultima Gold™ Szintillator (Packard BioScience, Bestellnr.6013159) zugegeben und nach 3 Stunden werden die Proben am ß-Counter (Mikrobeta, Perkin Elmer) gemessen. Die unspezifische Bindung wird durch Zugabe von 10 μM MPEP (Tocris, Bestellnr.1212) bestimmt.

**2a. Formalin-Test an der Ratte**

Der Formalintest (Dubuisson, D. and Dennis, S.G., 1977, Pain, 4, 161 - 174) stellt ein Modell für den akuten sowie den chronischen Schmerz dar. Durch eine einmalige Formalin-Injektion in die dorsale Seite einer Hinterpfote wird bei freibeweglichen Versuchstieren eine biphasische nozizeptive Reaktion induziert, die durch Beobachtung von drei deutlich voneinander unterscheidbaren Verhaltensmustern erfasst wird. Die Reaktion ist zweiphasisch: Phase 1 = Sofortreaktion (Dauer bis 10 min; Pfotenschütteln, Lecken), Phase 2 = Spätreaktion (nach einer Ruhephase; ebenfalls Pfotenschütteln, Lecken; Dauer bis 60 min). Die 1. Phase reflektiert eine direkte Stimulation der peripheren Nozisensoren mit hohem spinalen nozizeptiven Input bzw. Glutamatfreisetzung (akute Schmerzphase); die 2. Phase reflektiert eine spinale und periphere Hypersensibilisierung (chronische Schmerzphase). In den hier vorgestellten Untersuchungen wurde die chronische Schmerzkomponente (Phase 2) ausgewertet.

Formalin wird mit dem Volumen von 50μl und einer Konzentration von 5 % subkutan in die dorsale Seite der rechten Hinterpfote jedes Tieres appliziert. Die zu testenden Substanzen werden 30 min vor der Formalininjektion oral (p.o.), intravenös (i.v.) oder intraperitoneal (i.p.) appliziert. Die spezifischen Verhaltensänderungen, wie Anheben und Schütteln der Pfote, Gewichtsverlagerungen des Tieres sowie Beiß- und Leckreaktionen werden im Beobachtungszeitraum von 21 bis 27 min nach Formalininjektion beobachtet und registriert. Die Zusammenfassung der verschiedenen Verhaltensweisen erfolgt in der so genannten Pain-Rate (PR), die, auf die Teilintervalle von 3 min bezogen, die Berechnung einer mittleren Nozizeptionsreaktion darstellt. Die Berechnung der PR erfolgt aufgrund einer numerischen Gewichtung (= jeweils Faktor 1, 2, 3) der beobachteten Verhaltensweisen (entsprechend Verhaltensscore 1, 2, 3) und wird mit folgender Formel berechnet:

$$PR = [(T_0 \times 0) + (T_1 \times 1) + (T_2 \times 2) + (T_3 \times 3)] / 180$$

wobei $T_0$, $T_1$, $T_2$, und $T_3$ jeweils der Zeit in Sekunden entspricht, in der das Tier die Verhaltensweisen 0, 1, 2 oder 3 zeigt. Die Gruppengröße beträgt 10 Tiere (n=10).

**2b. Formalin-Test an der Maus**

Formalin wird mit dem Volumen von 20 μl und einer Konzentration von 1 % subkutan in die dorsale Seite der rechten Hinterpfote jedes Tieres appliziert. Die zu testenden Substanzen werden 15 min vor der Formalin-Injektion intraperitoneal (i.p.) appliziert. Die spezifischen Verhaltensänderungen, wie Anheben und Schütteln der Pfote (score 3, Dubuisson & Dennis, 1977), werden im Beobachtungszeitraum von 21 bis 24 min nach Formalin-Injektion beobachtet und registriert. Die Gruppengröße beträgt 10 Tiere (n=10).

**3. Neuropathischer Schmerz an der Ratte**

Die Untersuchung auf Wirksamkeit im neuropathischen Schmerz wurde im Bennett-Modell (chronic constriction injury; Bennett und Xie, 1988, Pain 33: 87-107) durchgeführt. Die entsprechenden Teile der Literatur gelten hiermit als Teil der vorliegenden Offenbarung.

Sprague-Dawley Ratten mit einem Gewicht von 140-160 g werden unter Nembutal-Narkose mit vier losen Ligaturen des rechten nervus ischiaticus versehen. Die Tiere entwickeln an der vom geschädigten Nerv innervierten Pfote eine Überempfindlichkeit, die nach einer Erholungsphase von einer Woche über etwa vier Wochen mittels einer 4°C kalten Metallplatte quantifiziert wird (Kälte-Allodynie). Die Tiere werden für einen Zeitraum von 2 min. auf dieser Platte beobachtet und die Anzahl der Wegziehreaktionen der geschädigten Pfote wird gemessen. Bezogen auf den Vorwert vor Substanzapplikation wird die Substanzwirkung über einen Zeitraum von einer Stunde an vier Zeitpunkten (15, 30, 45, 60 min. nach Applikation) bestimmt und die resultierende Fläche unter der Kurve (AUC) sowie die Hemmung der Kälte-Allodynie zu den einzelnen Meßpunkten in Prozent Wirkung zur Vehikelkontrolle (AUC) bzw. zum Ausgangswert (Einzelmeßpunkte) ausgedrückt. Die Gruppengröße beträgt n = 10. Die Signifikanz einer anti-allodynischen Wirkung wird anhand der AUC-Werte über einen gepaarten T-Test (* $0.05 \geq p > 0.01$; ** $0.01 \geq p > 0.001$; *** $p \leq 0.001$; Armitage und Berry, 1987, Stat. Methods in Medical Research, London: Blackwell Scientific Publications) bestimmt.

**4. "Elevated Plus Maze" Modell**

Im "elevated plus maze" (EPM) Modell werden Verbindungen auf mögliche anxiolytische Effekte getestet. Die Tests werden bei männlichen Sprague-Dawley Ratten (200-250 g) durchgeführt und 2 "elevated plus mazes" (Med Associates) mit elektronisch gesteuerten Infrarot-Lichtschranken zur Bestimmung des Aufenthaltsortes der Tiere im

Labyrinth werde verwendet. Jedes Labyrinth besitzt 2 offene und 2 geschlossene Arme und eine zentrale Plattform. Die Ränder der offenen Arme werden von schmalen Leisten begrenzt. Das gesamte Labyrinth ist auf einem Metallständer montiert.

Zu Beginn eines 5-min Tests wird jedes Tier einzeln mit dem Kopf in Richtung eines geschlossenen Armes auf die zentrale Plattform gesetzt.

Die folgenden Parameter werden bestimmt bzw. berechnet und ausgewertet: Anzahl und Prozent Eintritte in die offenen und geschlossenen Arme, sowie Prozent Zeit in den offenen und geschlossenen Armen und auf der zentralen Plattform.

Die Daten werden mittels einer 1-faktoriellen ANOVA (Vergleich Behandlungsgruppen versus Vehikel-Gruppe) analysiert. Das Signifikanz-Niveau wird bei $p < 0.05$ festgelegt. Alle Gruppen haben eine Größe von N = 10.

Der Test ist ebenfalls in Hogg, S. (1996) A review of the validity and variability of the elevated plus-maze as an animal model of anxiety. Pharmacol. Biochem. Behav. 54, 21-30 und Rodgers, R.J., Cole, J.C. (1994) The elevated plus-maze: pharmacology, methodology and ethology. In: Cooper, S.J., Hendrie, C.A. (eds.) Ethology and Psychopharmacology. Wiley & Sons; pp. 9-44 beschrieben. Die entsprechenden Stellen der Literatur gelten hiermit als Teil der Offenbarung.

### 5. Beschreibung des funktionellen $Ca^{2+}$-Influx Assay

20.000 CHO-hmGluR5 Zellen/well (Euroscreen, Gosselies, Belgium) werden in 96 well Platten (BD Biosciences, Heidelberg, Deutschland, Ref 356640, clear bottom, 96 well, Poly-D-Lysine) aus pipettiert und über Nacht in HBSS-Puffer (Gibco Nr. 14025-050) mit folgenden Zusätzen: 10% FCS (GIBCO, 10270-106) und Doxycyclin (BD Biosciences Clontech 631311 600ng/ml) inkubiert.

Zur funktionellen Untersuchung wurden die Zellen mit 2 μM Fluo-4 und 0,01 Vol.-% Pluronic F127 (Molecular Probes Europe BV, Leiden Niederlande) in HBSS-Puffer (Hank's buffered saline solution, Gibco Invitrogen GmbH, Karlsruhe, Deutschland) mit Probenicid (Sigma P8761, 0.69 mg/ml) für 30 min bei 37 °C beladen.

Die Zellen werden dann 3 mal mit Waschpuffer (HBSS-Puffer, Gibco Nr. 14025-050, mit Probenicid (Sigma P8761, 0.69 mg/ml) gewaschen und anschließend mit dem gleichen Puffer ad 100 μl aufgenommen. Nach 15 min. werden die Platten für die Bestimmung von $Ca^{2+}$-Messungen in Gegenwart von DHPG ((S)-3,5-Dihydroxyphenylalycine, Tocris Biotrend Chemikalien GmbH, Köln, Deutschland, finale DHPG-Konzentration: 10 μM) sowie in Gegenwart oder Abwesenheit von Testsubstanzen in einen Fuorometric Imaging Plate Reader (FLIPR, Molecular Devices, Sunnyvale, CA) transferiert.

Die $Ca^{2+}$-abhängige Fluoreszenz wird dabei vor und nach Zugabe von Testsubstanzen gemessen. Die Quantifizierung erfolgt durch die Messung der höchsten Fluoreszenzintensität über die Zeit.

Nach der Aufnahme einer Fluoreszenzbasislinie für 10 sec. werden 50 μl Testsubstanzlösung (verschiedene Testsubstanzkonzentrationen in HBSS-Puffer mit 1% DMSO und 0.02% Tween 20, Sigma) zugegeben und das Fluoreszenzsignal wird für 6 min gemessen. Anschließend werden 50 μl DHPG-Lösung ((S)-3,5-Dihydroxyphenylglycine, Tocris Biotrend Chemikalien GmbH, Köln, Deutschland, finale DHPG-Konzentration: 10 μM) zugegeben und der Einstrom von $Ca^{2+}$ wird simultan für 60 sec gemessen. Die finale DMSO-Konzentration beträgt 0.25% und der finale Tween 20 Gehalt beträgt 0.005%. Die Daten werden mit Microsoft Excel und GraphPad Prism analysiert. Die Dosis-Wirkungskurven werden mit nicht-linearer Regression berechnet und $IC_{50}$-Werte ermittelt. Jeder Datenpunkt wird 3-fach bestimmt und $IC_{50}$-Werte werden aus minimal 2 unabhängigen Messungen gemittelt.

Ki-Werte werden nach folgender Formel berechnet: $Ki = IC50/(1+(AG_{Konz}/EC50))$.

$AG_{Konz.}$ = 10 μM; EC50 entspricht der DHPG-Konzentration, die für den halbmaximalen Einstrom von $Ca^{2+}$ notwendig ist.

### Allgemeine Vorschriften für die Herstellung beispielgemäßer substituierter Imidazo[2,1-b]thiazole

## Allgemeines Syntheseschema 1:

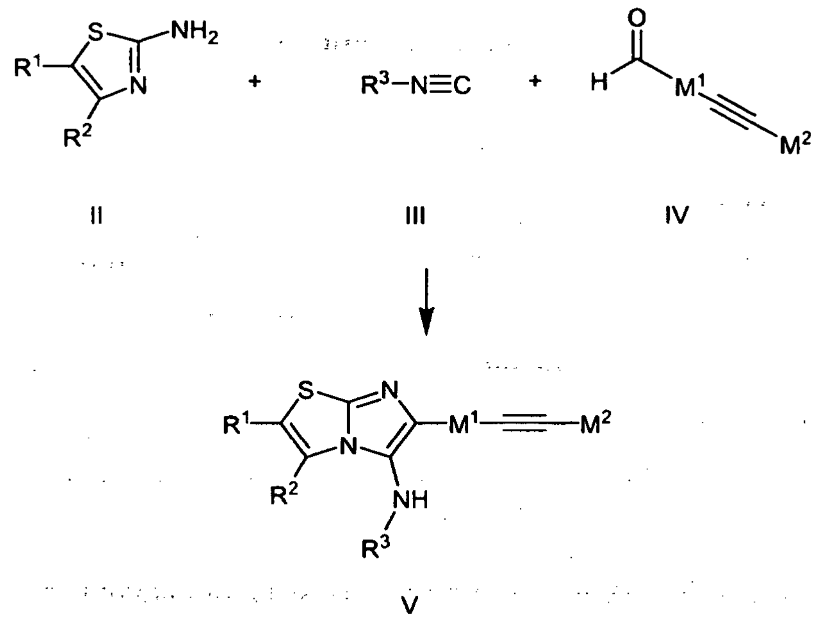

II III IV

V

Die Umsetzung von Aminen der allgemeinen Formel II mit Isocyaniden der allgemeinen Formel III und Aldehyden der allgemeinen Formel IV zu Verbindungen der allgemeinen Formel V wurde in organischen Lösungsmitteln oder Lösungsmittelgemischen, beispielsweise von Chloroform, DCM, MeCN, MeOH oder EtOH, unter Zusatz einer organischen oder anorganischen Säure, beispielsweise Trifluoressigsäure oder Perchlorsäure, oder unter Zusatz eines Übergangsmetalltriflats, beispielsweise Scandium(III)triflat, Ytterbiumtriflat oder Indium(III)triflat, bei Temperaturen von 0°C bis 150°C durchgeführt.

## Allgemeines Syntheseschema 2:

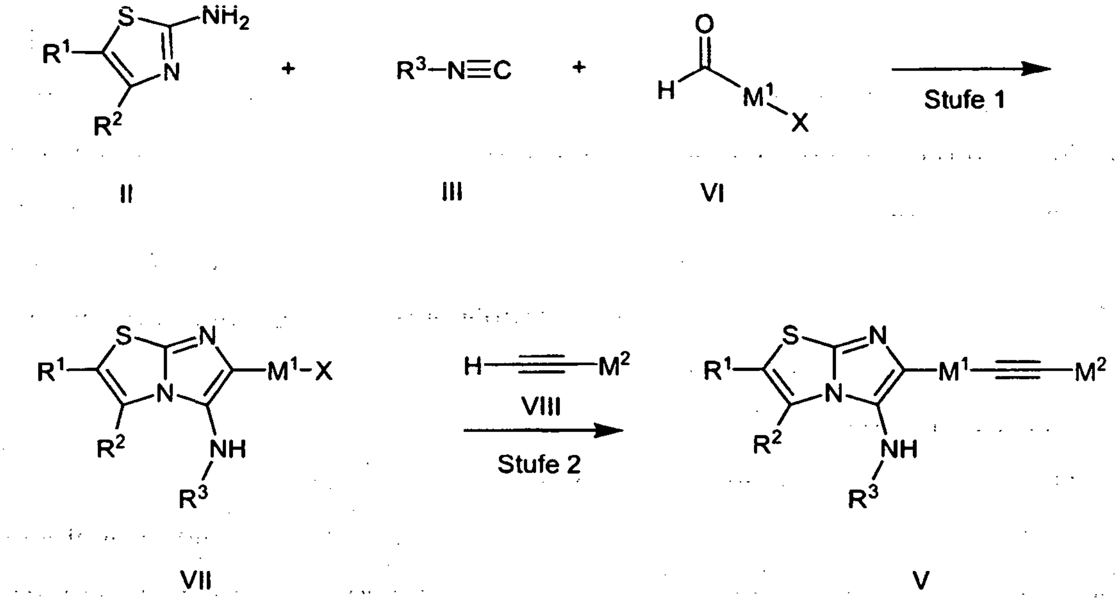


II III VI

VII V

In Stufe 1 wurde die Umsetzung von Aminen der allgemeinen Formel II mit Isocyaniden der allgemeinen Formel III und Aldehyden der allgemeinen Formel VI, worin X für einen Halogen-Rest steht, zu Verbindungen der allgemeinen Formel VII in organischen Lösungsmitteln oder Lösungsmittelgemischen, beispielsweise von Chloroform, DCM, MeCN, MeOH oder EtOH, unter Zusatz einer organischen oder anorganischen Säure, beispielsweise Trifluoressigsäure oder

Perchlorsäure, oder unter Zusatz eines Übergangsmetalltriflats, beispielsweise Scandium(III)triflat, Ytterbiumtriflat oder Indium(III)triflat, bei Temperaturen von 0°C bis 150°C durchgeführt.

In Stufe 2 erfolgte die Umsetzung von Verbindungen der allgemeinen Formel VII, worin X für einen Halogen-Rest steht, mit Acetylenen der allgemeinen Formel VIII zu Verbindungen der allgemeinen Formel V in einem Lösungsmittel oder Lösungsmittelgemisch, beispielsweise von Toluol, THF, DMF, MeCN, Ether, $NEt_3$ oder Diisopropylamin, unter Zusatz eines Palladiumkatalysators, beispielsweise Bis(triphenylphosphin)-palladium(II)-chlorid, von Kupfer(I)-iodid und einer organischen Base, beispielsweise $NEt_3$ oder Diisopropylamin, und/oder anorganischen Base, beispielsweise Kaliumcarbonat oder Cäsiumcarbonat, bei Temperaturen von -70°C bis 150°C.

## Allgemeines Syntheseschema 3:

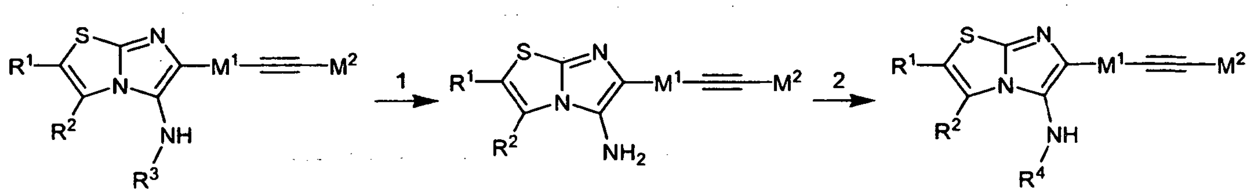

V IX X

In Stufe 1 wurden Verbindungen der allgemeinen Formel V zu Aminen der allgemeinen Formel IX in einem Lösungsmittel oder Lösungsmittelgemisch, beispielsweise von EtOH, MeOH oder Aceton, unter Zusatz einer organischen oder anorganischen Säure, beispielsweise Essigsäure, Trifluoressigsäure, Salzsäure oder Schwefelsäure, bei Temperaturen von 0°C bis 80°C umgesetzt.

In Stufe 2 wurden Verbindungen der allgemeinen Formel IX (1 Äquivalent) mit Carbonsäuren (1 Äquivalent) der allgemeinen Formel $R^{21}$-C(=O)-OH in einem Lösungsmittel oder Lösungsmittelgemisch, beispielsweise von Ether, THF, MeCN, MeOH, EtOH, DMF oder DCM, mit oder ohne Zusatz eines Kupplungsreagenz (1 Äquivalent), beispielsweise DCC, BOP, HATU oder EDCI und ggf. in Gegenwart wenigstens einer anorganischen oder organischen Base, beispielsweise $NEt_3$ oder Diisopropylethylamin, bei Temperaturen von -70°C bis 100°C zu Verbindungen der allgemeinen Formel X umgesetzt.

Alternativ wurden Verbindungen der allgemeinen Formel IX (1 Äquivalent) mit Carbonsäurehalogeniden (1 Äquivalent) bzw. Kohlensäurederivaten der allgemeinen Formel $R^{21}$-C(=O)-X, wobei X für einen Halogen-Rest steht, in einem Lösungsmitteln oder Lösungsmittelgemisch, beispielsweise von Ether, THF, MeCN, MeOH, EtOH, DMF oder DCM, mit oder ohne Zusatz einer organischen oder anorganischen Base, beispielsweise $NEt_3$, DMAP, Pyridin oder Diisopropylamin, bei Temperaturen von - 70°C bis 100°C zu Verbindungen der allgemeinen Formel X umgesetzt.

Als weitere Alternative wurden Verbindungen der allgemeinen Formel IX (1 Äquivalent) mit Aldehyden (1 Äquivalent) der allgemeinen Formel $R^{21}$-C(=O)-H in einem Lösungsmittel oder Lösungsmittelgemisch, beispielsweise von Ether, THF, MeOH, EtOH, DCM oder Toluol, und nachfolgender Zugabe eines Reduktionsmittels, beispielsweise Natriumborhydrid, Natriacetoxyborhydrid oder Natriumcyanoborhydrid, bei Temperaturen von -70°C bis 100°C zu Verbindungen der allgemeinen Formel X umgesetzt.

Ebenfalls wurden Verbindungen der allgemeinen Formel IX (1 Äquivalent) mit Verbindungen der allgemeinen Formel $R^4$-X (1.1 Äquivalente), worin X für einen Halogen-Rest, bevorzugt Chlor, steht, in einem Lösungsmittel oder Lösungsmittelgemisch, beispielsweise von Toluol, THF, oder Ether, unter Zusatz eines Metallhydridsalzes (1.1 Äquivalente), vorzugsweise unter Zusatz von Natriumhydrid, zu Verbindungen der allgemeinen Formel X bei Temperaturen von 0 °C bis 40 °C umgesetzt.

## Allgemeines Syntheseschema 4:

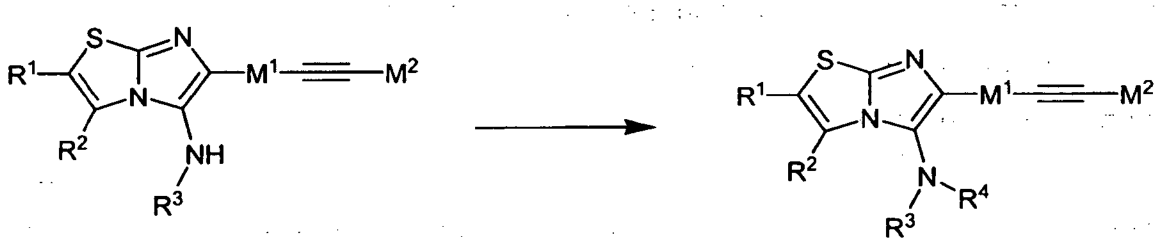

V, X

I

Die Verbindungen der allgemeinen Formel V oder X lassen sich mit den gleichen Methoden, wie im Allgemeinen Syntheseschema 3, Stufe 2, beschrieben, zu Verbindungen der allgemeinen Formel I umsetzen.

## Allgemeines Syntheseschema 5:

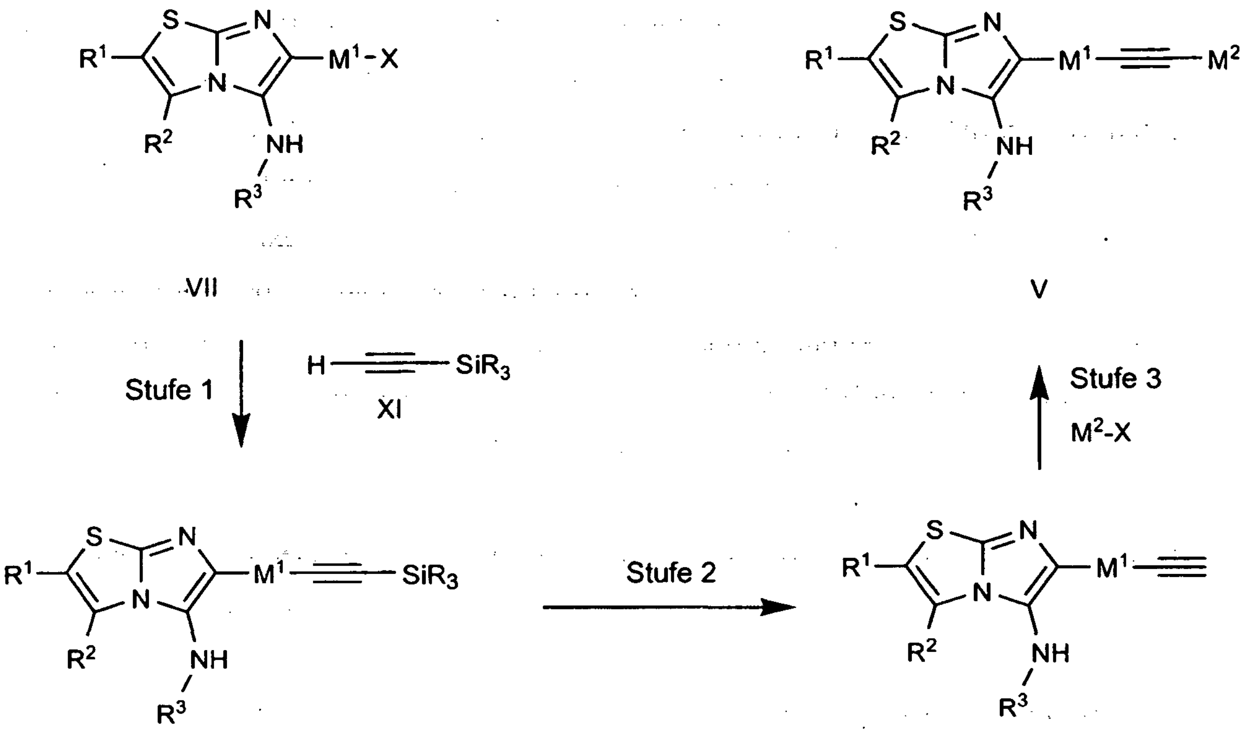


VII

V

XI

XII

XIII

In Stufe 1 werden Verbindungen der vorstehend angegebenen allgemeinen Formel VII (1.0 Äquivalent), worin X für einen Halogen-Rest oder einen Sulfonsäureester, besonders bevorzugt für Chlor, Brom oder Trifluormethansulfonat, steht, mit Acetylenen der allgemeinen Formel VIII (5.0 Äquivalente) in Acetonitril unter Zusatz von Tetrakis(triphenylphosphin)palladium [$(PPh_3)_4Pd$] (10 Mol.-%) und unter Zusatz von [1,4]-Diazabicyclo-[2.2.2]octan (2.0 Äquivalente) unter Rückfluß zu Verbindungen der allgemeinen Formel V umgesetzt.

In Stufe 2 werden Verbindungen der vorstehend angegebenen allgemeinen Formel XII (1.0 Äquivalente) in einem Reaktionsmedium, vorzugsweise ausgewählt aus der Gruppe bestehend aus Methanol und Dichlormethan und entsprechenden Mischungen in Gegenwart von Kaliumcarbonat (10 Mol-%) bei Temperaturen von 20 °C bis 30 °C zu Verbindungen der allgemeinen Formel XIII umgesetzt.

In Stufe 3 werden Verbindungen der vorstehend angegebenen allgemeinen Formel XIII (1.0 Äquivalent) mit

Verbindungen der allgemeinen Formel $M^2$-X (1.25 Äquivalente), worin X für einen Halogen-Rest oder einen Sulfonsäureester, besonders bevorzugt für Chlor, Brom oder Trifluormethansulfonat, steht, in Ethylacetat, unter Zusatz von Bis(triphenylphosphin)-palladium(II)-chlorid $[PdCl_2(PPh_3)_2]$ (5 Mol-%), in Gegenwart von Kupfer(I)-iodid (6 Mol-%), unter Zusatz von Triethylamin (2.0 Äquivalente), bei Temperaturen von 40 °C bis 60 °C, zu Verbindungen der allgemeinen Formel V umgesetzt.

Im Folgenden werden die vorstehend beschriebenen Vorschriften für die Herstellung substituierter Imidazo[2,1-b]thiazole anhand einiger Beispielverbindungen detailliert erläutert.

Die folgenden Beispiele dienen zur näheren Erläuterung der Erfindung.

## Beispiele

Die Ausbeuten der hergestellten Verbindungen sind nicht optimiert.

Alle Temperaturen sind unkorrigiert.

## Abkürzungen:

| | |
|---|---|
| aq. | wäßrig |
| d | Tage |
| Brine | gesättigte, wässrige NaCl-Lösung |
| DCM | Dichlormethan |
| DMF | N,N-Dimethylformamid |
| EE | Essigsäureethylester |
| Ether | Diethylether |
| ges. | gesättigt |
| $NEt_3$ | Triethylamin |
| RT | Raumtemperatur |
| SC | Säulenchromatographie |
| TBME | tertiär Butyl-methyl-ether |

Die eingesetzten Chemikalien und Lösungsmittel wurden kommerziell bei den herkömmlichen Anbietern (Acros, Avocado, Aldrich, Bachem, Fluka, Lancaster, Maybridge, Merck, Sigma, TCI, etc.) bezogen oder nach den für den Fachmann bekannten Methoden synthetisiert.

Als stationäre Phase für die Säulenchromathographie wurde Kieselgel 60 (0.040 - 0.063 mm) der Firma E. Merck, Darmstadt, eingesetzt.

Die dünnschicht-chromatographischen Untersuchungen wurden mit HPTLC-Fertigplatten, Kieselgel 60 F 254, der Firma E. Merck, Darmstadt, durchgeführt.

Die Mischungsverhältnisse von Lösungsmitteln, Laufmitteln oder für chromatographische Untersuchungen sind stets in Volumen/Volumen angegeben.

Die Analytik aller Beispiele erfolgte durch Massenspektroskopie und NMR-Spektroskopie.

### Beispiel 1: Synthese von 6-(5-(Phenylethinyl)thiophen-2-yl)-N-(2,4,4-trimethylpentan-2-yl)imidazo[2,1-b]thiazol-5-amin

Eine Lösung von 400 mg (4.0 mmol) 2-Aminothiazol, 1018 mg (4.8 mmol) 5-Phenylethinyl-thiophen-2-carbaldehyd, 557 mg (4.0 mmol) (2,4,4-Trimethylpentan-2-yl)-isocyanid und 400 μl einer 20%-igen aq. Perchlorsäure in DCM (8 ml) wurde 5 d bei RT gerührt. Anschließend wurde mit einer 1 molaren aq. $Na_2CO_3$-Lsg. versetzt. Die Phasen wurden getrennt und die wässrige Phase wurde mit DCM extrahiert. Die gesammelten organischen Phasen wurden mit Brine gewaschen und über $MgSO_4$ getrocknet. Nach Filtrieren und Entfernen der Lösungsmittel im Vakuum wurde mit dem Rückstand eine SC (EE/Hexan 1:4) durchgeführt, wobei 637 mg (1.47 mmol, 37%) 6-(5-(Phenylethinyl)thiophen-2-yl)-N-(2,4,4-trimethylpentan-2-yl)imidazo[2,1-b]thiazol-5-amin erhalten wurden.

### Beispiel 2: Synthese von N-tert-Butyl-6-(5-(pyridin-2-ylethinyl)thiophen-2-yl)imidazo[2,1-b]thiazol-5-amin

Eine Lösung von 400 mg (4.0 mmol) 2-Aminothiazol, 938 mg (4.4 mmol) 5-(Pyridin-2-yl-ethinyl)-thiophen-2-carbaldehyd, 332 mg (4.0 mmol) tert.Butyl-isocyanid und 400 μl einer 20%-igen aq. Perchlorsäure in Chloroform (10 ml) wurde 10 d bei RT gerührt. Anschließend wurde mit einer 1 molaren aq. $Na_2CO_3$-Lsg versetzt. Die Phasen wurden

getrennt und die wässrige Phase wurde mit Chloroform extrahiert. Die gesammelten organischen Phasen wurden mit Brine gewaschen und über $MgSO_4$ getrocknet. Nach Filtrieren und Entfernen der Lösungsmittel im Vakuum wurde mit dem Rückstand eine SC (EE/DCM 15:85) durchgeführt, wobei 223 mg verunreinigtes Rohprodukt erhalten wurden. Aus diesem wurden durch Kristallisation aus EE 156 mg (0.41 mmol, 10%) N-tert-Butyl-6-(5-(pyridin-2-ylethinyl)thiophen-2-yl)imidazo[2,1-b]thiazol-5-amin erhalten.

**Beispiel 3: Synthese von N-tert-Butyl-3-methyl-6-(5-(pyridin-2-ylethinyl)thiophen-2-yi)imidazo[2,1-b]thiazol-5-amin Hydrochlorid**

Eine Lösung von 256 mg (2.25 mmol) 2-Amino-4-methyl-thiazol, 527 mg (2.48 mmol) 5-(Pyridin-2-yl-ethinyl)-thiophen-2-carbaldehyd, 205 mg (2.48 mmol) tert.Butyl-isocyanid und 58 μl einer 70%-igen aq. Perchlorsäure in Chloroform (2 ml) wurde 16 h bei RT gerührt. Anschließend wurde mit DCM (20 ml) verdünnt und mit einer 1 molaren aq. $Na_2CO_3$-Lsg (10 ml) versetzt. Nach 10 min Rühren bei RT wurden die Phasen getrennt. Die wässrige Phase wurde mit DCM extrahiert. Die gesammelten organischen Phasen wurden über $MgSO_4$ getrocknet. Nach Filtrieren und Entfernen der Lösungsmittel im Vakuum wurde mit dem Rückstand eine SC (EE/DCM 25:75) durchgeführt, wobei 41 mg verunreinigtes Rohprodukt erhalten wurden. Dieses wurde in Aceton (1 ml) gelöst und nacheinander mit 1 μl Wasser und 11 μl Trimethylchlorsilan versetzt. Der entstandene Niederschlag wurde abgesaugt und mit Ether nach gewaschen. Dabei wurden 18 mg (0.04 mmol, 2%) N-tert-Butyl-3-methyl-6-(5-(pyridin-2-ylethinyl)thiophen-2-yl)imidazo[2,1-b]thiazol-5-amin Hydrochlorid erhalten.

**Beispiel 4: Synthese von N-tert-Butyl-2-methyl-6-(5-(pyridin-2-ylethinyl)thiophen-2-yl)imidazo[2,1-b]thiazol-5-amin**

Eine Lösung von 256 mg (2.25 mmol) 2-Amino-5-methyl-thiazol, 527 mg (2.48 mmol) 5-(Pyridin-2-yl-ethinyl)-thiophen-2-carbaldehyd, 205 mg (2.48 mmol) tert.Butyl-isocyanid und 58 μl einer 70%-igen aq. Perchlorsäure in Chloroform (2 ml) wurde 16 h bei RT gerührt. Anschließend wurde mit DCM (20 ml) verdünnt und mit einer 1 molaren aq. $Na_2CO_3$-Lsg (10 ml) versetzt. Nach 10 min Rühren bei RT wurden die Phasen getrennt. Die wässrige Phase wurde mit DCM extrahiert. Die gesammelten organischen Phasen wurden über $MgSO_4$ getrocknet. Nach Filtrieren und Entfernen der Lösungsmittel im Vakuum wurde mit dem Rückstand eine SC (EE/DCM 25:75) durchgeführt, wobei 367 mg verunreinigtes Rohprodukt erhalten wurden. Aus diesem wurden durch Kristallisation aus EE 48 mg (0.12 mmol, 5%) N-tert-Butyl-2-methyl-6-(5-(pyridin-2-ylethinyl)thiophen-2-yl)imidazo[2,1-b]thiazol-5-amin erhalten.

**Beispiel 5: Synthese von N-tert-Butyl-2,3-dimethyl-6-(5-(pyridin-2-ylethinyl)thiophen-2-yl)-imidazo[2,1-b]thiazol-5-amin Hydrochlorid**

**Beispiel 6: Synthese von N-tert-Butyl-2-chlor-6-(5-(pyridin-2-ylethinyl)thiophen-2-yl)-imidazo[2,1-b]thiazol-5-amin Hydrochlorid**

Die Synthese der **Beispiele 5** und **6** erfolgte nach dem für Beispiel 3 beschriebenen Verfahren.

**Beispiel 7: N-tert-Butyl-6-(5-(pyridin-4-ylethinyl)thiophen-2-yl)imidazo[2,1-b]thiazol-5-amin**

**Beispiel 8: 5-(tert-Butylamino)-6-(5-(pyridin-2-ylethinyl)thiophen-2-yl)-imidazo[2,1-b]thiazol-2-carbonsäuremethylester Hydrochlorid**

Die Synthese der **Beispiele 7** und **8** erfolgte nach dem für Beispiel 4 beschriebenen Verfahren.

**Beispiel 9: N-tert-Butyl-6-(5-(pyridin-2-ylethinyl)thiazol-2-yl)imidazo[2,1-b]thiazol-5-amin**

**a) Synthese von 6-(5-Bromthiazol-2-yl)-N-tert-butylimidazo[2,1-b]thiazol-5-amin**

Eine Lösung von 100 mg (1.0 mmol) 2-Amino-thiazol, 191 mg (1.0 mmol) 5-Brom-thiazole-2-carbaldehyd und 97 mg (1.17 mmol) tert.Butylisonitril in Ethanol (4 ml) wurde mit einer 1 molaren Perchlorsäure (10 μl) versetzt und 5 min in der Mikrowelle (Biotage Initiator), erhitzt. Anschließend wurde die Reaktionslösung im Vakuum eingeengt. Mit dem Rückstand wurde eine SC (TBME/Hexan 1:1) durchgeführt, wobei 71 mg (0.2 mmol, 20%) 6-(5-Bromthiazol-2-yl)-N-tert-butylimidazo[2,1-b]thiazol-5-amin ([MH+] 357.0)erhalten wurden.

**b) Synthese von N-tert-Butyl-6-(5-(pyridin-2-ylethinyl)thiazol-2-yl)imidazo[2,1-b]thiazol-5-amin**

Eine Mischung aus 235 mg (0.66 mmol) 6-(5-Bromthiazol-2-yl)-N-tert-butylimidazo[2,1-b]thiazol-5-aminl, 22 mg (0.033 mmol) Bis(triphenylphosphin)-palladium-(II)-chlorid, 12 mg (0.066mmol) Kupfer-(I)-iodid, 80 μl (0.79 mmol) 2-Ethinylpyridin und 731 μl (5.28 mmol) $NEt_3$ in DMF (2 ml) wurde in der Mikrowelle (Biotage Initiator) 10 min auf 120°C erhitzt. Die Reaktionslösung wurde mit Wasser verdünnt und mit EE mehrmals extrahiert. Die gesammelten organischen Phasen wurden mit Brine gewaschen und über $MgSO_4$ getrocknet. Nach Filtrieren und Entfernen der Lösungsmittel im Vakuum wurde mit dem Rückstand eine SC (1.TBME, 2.MeOH) durchgeführt, wobei 43 mg (0.11 mmol, 17%) N-tert-Butyl-6-(5-(pyridin-2-ylethinyl)thiazol-2-yl)imidazo[2,1-b]thiazol-5-amin erhalten wurden.

**Beispiel 10: Synthese von 6-(5-Pyridin-2-ylethinyl)thiophen-2-yl)-N-(2,4,4-trimethylpentan-2-yl)imidazo[2,1-b]thiazol-5-amin**

Eine Lösung von 5.0 g (50.0 mmol) 2-Amino-thiazol, 10.7 g (50.0 mmol) 5-(Pyridin-2-yl-ethinyl)-thiophen-2-carbaldehyd, 7.0 g (50.0 mmol) (2,4,4-Trimethylpentan-2-yl)-isocyanid und 1.0 ml einer 70%-igen aq. Perchlorsäure in Chloroform (25 ml) wurde unter Rühren 5 d auf 50 °C erhitzt. Anschließend wurde mit DCM verdünnt und mit einer 1 molaren aq. $Na_2CO_3$-Lsg versetzt. Nach 10 min Rühren bei RT wurden die Phasen getrennt. Die wässrige Phase wurde mit DCM extrahiert. Die gesammelten organischen Phasen wurden über $Na_2SO_4$ getrocknet, filtriert und im Vakuum eingeengt. Durch mehrfache Kristallisation des Rückstands aus EE wurden 11.86 g (27.3 mmol, 55%) 6-(5-Pyridin-2-ylethinyl)thiophen-2-yl)-N-(2,4,4-trimethylpentan-2-yl)imidazo[2,1-b]thiazol-5-amin erhalten.

**Beispiel 11: N-tert-Butyl-2-methyl-6-(4-(pyridin-2-ylethinyl)phenyl)imidazo[2,1-b]thiazol-5-amin**

Die Synthese des **Beispiels 11** erfolgte nach dem für Beispiel 10 beschriebenen Verfahren.

**Beispiel 12: Synthese von N-tert-Butyl-6-(5-(pyridin-2-ylethinyl)furan-2-yl)imidazo[2,1-b]thiazol-5-amin**

Eine Lösung von 380 mg (3.8 mmol) 2-Amino-thiazol, 749 mg (3.8 mmol) 5-(Pyridin-2-yl-ethinyl)-furan-2-carbaldehyd, 378 mg (3.8 mmol) tert.Butyl-isonitril und 73 μl einer 70%-igen aq. Perchlorsäure in Chloroform (2 ml) wurde unter Rühren 16 h auf 45 °C erhitzt. Anschließend wurde mit DCM verdünnt und mit einer 1 molaren aq. $Na_2CO_3$-Lsg versetzt. Nach 10 min Rühren bei RT wurden die Phasen getrennt. Die wässrige Phase wurde mit DCM extrahiert. Die gesammelten organischen Phasen wurden über $Na_2SO_4$ getrocknet, filtriert und im Vakuum eingeengt. Durch SC (EE) mit dem Rückstand wurden 472 mg (1.3 mmol, 34%) N-tert-Butyl-6-(5-(pyridin-2-ylethinyl)furan-2-yl)imidazo[2,1-b]thiazol-5-amin erhalten.

**Beispiel 13: N-tert-Butyl-3-methyl-6-(5-(phenylethinyl)thiophen-2-yl)imidazo[2,1-b]thiazol-5-amin**

Die Synthese des Beispiels 13 erfolgte nach dem für Beispiel 12 beschriebenen Verfahren.

**Beispiel 14: Synthese von 6-(5-(Pyridin-2-ylethinyl)thiophen-2-yl)imidazo[2,1-b]thiazol-5-amin**

Eine Lösung von 2.5 g (5.75 mmol) 6-(5-Pyridin-2-ylethinyl)thiophen-2-yl)-N-(2,4,4-trimethylpentan-2-yl)imidazo[2,1-b]thiazol-5-amin (Beispiel 10) in DCM (30 ml) wurde mit Trifluoressigsäure (30 ml) versetzt und 25 min bei RT gerührt. Anschließend wurden unter Kühlen (Eisbad) mit einer 12 molaren aq. NaOH-Lsg basisch gestellt (pH > 12). Der entstandene Rückstand wurde abfiltriert und in einem Gemisch aus EE (200 ml) und DCM (50 ml) gelöst und mit Wasser (15 ml) gewaschen und über $Na_2SO_4$ getrocknet. Nach Entfernen der Lösungsmittel im Vakuum wurden 773 mg (2.40 mmol, 42%) 6-(5-(Pyridin-2-ylethinyl)thiophen-2-yl)imidazo[2,1-b]thiazol-5-amin erhalten.

**Beispiel 16: N-tert-Butyl-6-(5-((3-fluorpyridin-2-yl)ethinyl)thiophen-2-yl)imidazo[2,1-b]thiazol-5-amin**

**a) Synthese von 6-(5-Bromthiophen-2-yl)-N-tert-butylimidazo[2,1-b]thiazol-5-amin**

Zu einer Lösung von 12.0 g (119.9 mmol) 2-Amino-thiazol in Chloroform (60 ml) wurden 22.9 g (119.9 mmol) 5-Brom-thiophen-2-carbaldehyd und 11.0 g (131.9 mmol) tert.-Butyl-isonitril gegeben. Die Reaktionslösung wurde mit einer 1 molaren Perchlorsäure (2.3 ml) versetzt und unter Rühren 5 d auf 50 °C erhitzt. Nach Abkühlen auf RT wurde mit DCM verdünnt und mit einer 1 molaren aq. $Na_2CO_3$-Lsg versetzt. Nach 10 min Rühren bei RT wurden die Phasen getrennt. Die organische Phase wurde über $Na_2SO_4$ getrocknet, filtriert und im Vakuum eingeengt. Durch Kristallisation des Rückstands aus EE wurden 6.6 g (18.5 mmol, 15%) 6-(5-Bromthiophen-2-yl)-N-tert-butylimidazo[2,1-b]thiazol-5-amin ([MH+] 356.0) erhalten.

**b) Synthese von N-tert-Butyl-6-(5-((trimethylsilyl)ethinyl)thiophen-2-yl)imidazo[2,1-b]thiazol-5-amin**

Zu einer Suspension von 12.5 g (35.1 mmol) 6-(5-Bromthiophen-2-yl)-N-tert-butylimidazo[2,1-b]thiazol-5-amin in Acetonitril (150 ml) wurden nacheinander 4.1 g (3.5 mmol) Tetrakis(triphenyl)phosphin-palladium(0), 17.2 g (175.4 mmol) Trimethylsilylacetylen und 7.9 g (70.2 mmol) 1,4-Diazabicyclo[2.2.2]octan gegeben. Die Reaktionslösung wurde 72 h unter Rückfluss erhitzt und anschließend im Vakuum aufkonzentriert. Durch SC (DCM/EE 95:5) wurden 3.0 g (7.9 mmol, 23%) N-tert-Butyl-6-(5-((trimethylsilyl)ethinyl)thiophen-2-yl)imidazo[2,1-b]thiazol-5-amin ([MH+] 374.1) erhalten.

**c) Synthese von N-tert-Butyl-6-(5-ethinylthiophen-2-yl)imidazo[2,1-b]thiazol-5-amin**

Eine Suspension von 3.0 g (7.9 mmol) N-tert-Butyl-6-(5-((trimethylsilyl)-ethinyl)thiophen-2-yl)imidazo[2,1-b]thiazol-5-amin in einer Mischung aus DCM (12 ml) und Methanol (58 ml) wurde auf 35 °C erwärmt und mit 108 mg (0.79 mmol) Kaliumcarbonat versetzt. Nach 100 min Rühren bei RT wurde Wasser und DCM zugegeben. Die Phasen wurden getrennt und die wässrige Phase wurde mit DCM extrahiert. Die gesammelten organischen Phasen wurden mit Wasser gewaschen, über $Na_2SO_4$ getrocknet, filtriert und im Vakuum eingeengt. Die erhaltenen 2.17 g (7.2 mmol, 91%) Rohprodukt an N-tert-Butyl-6-(5-ethinylthiophen-2-yl)imidazo[2,1-b]thiazol-5-amin ([MH+] 302.1) wurde ohne weitere Reinigung im nächsten Schritt umgesetzt.

**d) Synthese von N-tert-Butyl-6-(5-((3-fluorpyridin-2-yl)ethinyl)thiophen-2-yl)imidazo[2,1-b]thiazol-5-amin**

Eine Lösung von 450 mg (1.49 mmol) N-tert-Butyl-6-(5-ethinylthiophen-2-yl)imidazo[2,1-b]thiazol-5-amin, 333 mg (1.90 mmol) 3-Fluor-2-iod-pyridin, 38 mg (0.06 mmol) Bis(triphenylphosphin)-palladium-(II)-chlorid, 19 mg (0.07 mmol) Kupfer-(I)-iodid und 389 μl (2.80 mmol) $NEt_3$ in EE (21 ml) wurde unter Rühren 20 h auf 50 °C erhitzt. Anschließend wurde im Vakuum eingeengt und mit dem Rückstand eine SC (EE) durchgeführt, wobei 456 mg (1.15 mmol, 77%) N-tert-Butyl-6-(5-{(3-fluorpyridin-2-yl)ethinyl)thiophen-2-yl)imidazo[2,1-b]thiazol-5-amin erhalten wurden.

**Beispiel 15: N-tert-Butyl-6-(5-(pyrimidin-2-ylethinyl)thiophen-2-yl)imidazo[2,1-b]thiazol-5-amin**

**Beispiel 17: N-tert-Butyl-6-(5-((2-fluorpyridin-4-yl)ethinyl)thiophen-2-yl)imidazo[2,1-b]thiazol-5-amin**

**Beispiel 18: N-tert-Butyl-6-(5-(thiophen-2-ylethinyl)thiophen-2-yl)imidazo[2,1-b]thiazol-5-amin**

**Beispiel 19: N-tert-Butyl-6-(5-(thiazol-2-ylethinyl)thiophen-2-yl)imidazo[2,1-b]thiazol-5-amin**

**Beispiel 20: 3-((5-(5-(tert-Butylamino)imidazo[2,1-b]thiazol-6-yl)thiophen-2-yl)ethinyl)phenol**

**Beispiel 21: 3-((5-(5-(tert-Butylamino)imidazo[2,1-b)thiazol-6-yl)thiophen-2-yl)ethinyl)benzonitril**

**Beispiel 23 N-tert-Butyl-6-(5-((3-methylpyridin-2-yl)ethinyl)thiophen-2-yl)imidazo[2,1-b]thiazol-5-amin**

Die Synthese der **Beispiele 15, 17, 18, 19, 20, 21 und 23** erfolgte nach dem für Beispiel 16 beschriebenen Verfahren.

**Beispiel 25: Synthese von N-(6-(5-(pyridin-2-ylethinyl)thiophen-2-yl)imidazo[2,1-b]thiazol-5-yl)benzamid**

Zu einer Lösung von 185 mg (0.57 mmol) 6-(5-(Pyridin-2-ylethinyl)thiophen-2-yl)imidazo[2,1-b]thiazol-5-amin (Beispiel 14) und 174 μl (1.26 mmol) $NEt_3$ in DCM (3 ml) wurden unter Kühlen (Eisbad) 59 μl (0.52 mmol) Benzoylchlorid zugetropft. Nach 16 h Rühren bei RT wurde mit EE verdünnt. Anschließend wurde nacheinander mit einer gesättigten, wässrigen Natriumcarbonatlösung und mit einer gesättigten, wässrigen Natriumchloridiösung gewaschen. Die organische Phase wurde über $Na_2SO_4$ getrocknet, filtriert und im Vakuum eingeengt. Mit dem Rückstand wurde eine SC (EE) durchgeführt, wobei 10 mg (0.02 mmol, 4%) N-(6-(5-(Pyridin-2-ylethinyl)thiophen-2-yl)imidazo[2,1-b]thiazol-5-yl)benzamid erhalten wurden.

**Beispiel 22: N-Ethyl-6-(6-(phenylethinyl)pyridin-3-yl)imidazo[2,1-b]thiazol-5-amin**

Die Synthese des **Beispiels 22** erfolgte nach dem für Beispiel 4 beschriebenen Verfahren.

**Beispiel 24: N-(6-(5-(Pyridin-2-ylethinyl)thiophen-2-yl)imidazo[2,1-b]thiazol-5-yl)acetamid**

Die Synthese des **Beispiels 24** erfolgte nach dem für Beispiel 25 beschriebenen Verfahren.

In der folgenden Tabelle sind die mittels Massenspektrometrie erhaltenen Werte aufgeführt.

| Beispiel | Masse [$MH^+$] |
|---|---|
| 1 | 434,2 |
| 2 | 379,1 |
| 3 | 393,1 |
| 4 | 393,1 |
| 5 | 407,1 |
| 6 | 413,1 |
| 7 | 379,1 |
| 8 | 437,1 |
| 9 | 380,1 |
| 10 | 435,2 |
| 11 | 387,2 |
| 12 | 363,1 |
| 13 | 392,1 |
| 14 | 323,0 |
| 15 | 380,1 |
| 16 | 397,1 |
| 17 | 397,1 |
| 18 | 384,1 |
| 19 | 385,1 |
| 20 | 394,1 |
| 21 | 403,1 |
| 22 | 345,1 |
| 23 | 393,1 |
| 24 | 365,0 |
| 25 | 427,1 |

**Pharmakologische Daten:**

1. Die Affinität der erfindungsgemäßen substituierten Imidazo[2,1-b]thiazol-Verbindungen der allgemeinen Formel I für den mGluR5-Rezeptor wurde wie vorstehend beschrieben bestimmt.

Die erfindungsgemäßen substituierten Imidazo[2,1-b]thiazol-Verbindungen zeigen eine ausgezeichnete Affinität für den mGluR5-Rezeptor.

In der nachfolgenden Tabelle 1 sind die pharmakologischen Daten für die substituierten Imidazo[2,1-b]thiazol-Verbindungen gemäß den Beispielen 1 bis 4 wiedergegeben:

Tabelle 1:

| Bsp. | $IC_{50}$ [$^3$H]-MPEP-Bindung mGluR5 Rezeptor (Schwein) [μM] | mGluR5 Rezeptor (Schwein) (10 μM) Hemmung (%) | $ED_{50}$ Formalintest (Ratte) i.v. [mg/kg] |
|---|---|---|---|
| 1 | 2,1800 | | |
| 2 | 0,0063 | | 0,47 |
| 3 | 0,0120 | | |
| 4 | 0,0055 | | |
| 5 | 0,0170 | | |
| 6 | 0,0099 | | |
| 7 | 0,0030 | | |
| 8 | 0,1600 | | |
| 10 | 0,1000 | | |
| 11 | 0,0200 | | |
| 12 | 0,0870 | | |
| 13 | | 48 | |
| 14 | 0,1400 | | |
| 15 | 0,0100 | | |
| 16 | 0,0130 | | |
| 17 | 0,0180 | | |
| 18 | 0,0450 | | |
| 19 | 0,0240 | | |
| 20 | 0,1700 | | |
| 21 | 0,0260 | | |
| 22 | | 82 | |
| 23 | 0,2300 | | |

2. Die erfindungsgemäßen substituierten Imidazo[2,1-b]thiazol-Verbindungen zeigen ebenfalls eine ausgezeichnete Wirkung im Formalin-Test an der Ratte wie in der nachfolgenden Tabelle 2 wieder gegeben ist.

Tabelle 2:

| Bsp. | $ED_{50}$ Formalintest (Ratte) i.v. [mg/kg] | Formalintest (Ratte) p.o. Reduzierung des nozizeptiven Verhaltens gegenüber Kontrollen bei 10 mg/kg [%] |
|---|---|---|
| **2** | 0,47 | 66 |
| **4** | | 62 |

3. Die erfindungsgemäßen substituierten Imidazo[2,1-b]thiazol-Verbindungen zeigen eine ausgezeichnete Affinität für den humanen mGluR5-Rezeptor (Tabelle 3.).

Tabelle 3.

| Bsp. | $K_i$ mGluR5 Rezeptor (Mensch) $Ca^{2+}$-Influx [μM] |
|---|---|
| 4 | 0,00031 |

(fortgesetzt)

| Bsp. | $K_i$ mGluR5 Rezeptor (Mensch) $Ca^{2+}$-Influx [μM] |
|---|---|
| 7 | 0,00026 |

**Patentansprüche**

**1.** Substituierte Imidazo[2,1-b]thiazol-Verbindungen der allgemeinen Formel I,

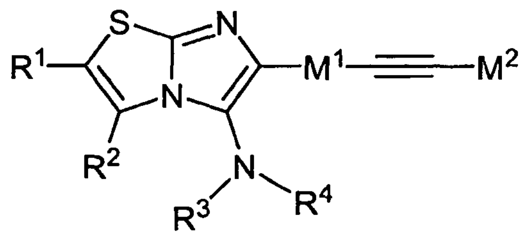

I,

worin

$R^1$ und $R^2$, unabhängig voneinander, jeweils für einen Wasserstoff-Rest; für einen Halogen-Rest; $-NO_2$; -CN; $-NH_2$; $-NHR^5$; $-NR^6R^7$; $-NH-C(=O)-R^3$; $-C(=O)-R^9$, $-C(=O)-NH_2$; $-C(=O)-NHR^{10}$; $-C(=O)-NR^{11}R^{12}$; $-C(=O)-OR^{13}$; $-(CH_2)_m-C(=O)-OR^{14}$ mit m = 1, 2, 3, 4 oder 5; $-O-C(=O)-R^{15}$; $-(CH_2)_n-O-C(=O)-R^{16}$ mit n = 1, 2, 3, 4 oder 5; $-OR^{17}$; $-(CH_2)_o-O-R^{18}$ mit o = 1, 2, 3; 4 oder 5; $-SR^{19}$;$-(CH_2)_p-S(=O)_t-R^{20}$ mit p = 1, 2, 3, 4 oder 5 und t = 0, 1 oder 2; $-NH-S(=O)_2-NR^{27}R^{28}$; $-S(=O)_2-NR^{29}R^{30}$; $-SF_5$; $-(CH_2)_u-O-S(=O)_2-R^{31}$ mit u = 1, 2, 3, 4 oder 5; $-(CH_2)_v-O-S(=O)_2-O-R^{32}$ mit v = 1, 2, 3, 4 oder 5; $-(CH_2)_w-O-P(=O)(OR^{33})(OR^{34})$ mit w =1, 2, 3, 4 oder 5;
für einen linearen oder verzweigten, gesättigten oder ungesättigten, unsubstituierten oder wenigstens einfach substituierten aliphatischen Rest;
für einen gesättigten oder ungesättigten, unsubstituierten oder wenigstens einfach substituierten, ggf. wenigstens ein Heteroatom als Ringglied aufweisenden cycloaliphatischen Rest, der über eine lineare oder verzweigte, unsubstituierte oder wenigstens einfach substituierte Alkylen-Gruppe gebunden und/oder mit einem unsubstituierten oder wenigstens einfach substituierten mono- oder polyzyklischen Ringsystem kondensiert sein kann;
oder für einen unsubstituierten oder wenigstens einfach substituierten Aryl- oder Heteroaryl-Rest, der über eine lineare oder verzweigte, unsubstituierte oder wenigstens einfach substituierte Alkylen-Gruppe gebunden und/oder mit einem unsubstituierten oder wenigstens einfach substituierten mono- oder polyzyklischen Ringsystem kondensiert sein kann, stehen;
$R^3$ und $R^4$, unabhängig voneinander, jeweils für einen Wasserstoff-Rest; $-C(=O)-R^{21}$; $-(CH_2)_q-C(=O)-R^{22}$ mit q = 1, 2, 3, 4 oder 5; $-C(=O)-O-R^{23}$; $-(CH_2)_r-C(=O)-O-R^{24}$ mit r = 1, 2, 3, 4 oder 5; $-C(=O)-NHR^{25}$; $-(CH_2)_s-C(=O)-NHR^{26}$ mit s = 1, 2, 3, 4 oder 5; für einen linearen oder verzweigten, gesättigten oder ungesättigten, unsubstituierten oder wenigstens einfach substituierten aliphatischen Rest; für einen gesättigten oder ungesättigten, unsubstituierten oder wenigstens einfach substituierten, ggf. wenigstens ein Heteroatom als Ringglied aufweisenden cycloaliphatischen Rest, der über eine lineare oder verzweigte, unsubstituierte oder wenigstens einfach substituierte Alkylen-Gruppe gebunden und/oder mit einem unsubstituierten oder wenigstens einfach substituierten mono- oder polyzyklischen Ringsystem kondensiert sein kann; oder für einen unsubstituierten oder wenigstens einfach substituierten Aryl- oder Heteroaryl-Rest, der über eine lineare oder verzweigte, unsubstituierte oder wenigstens einfach substituierte Alkylen-Gruppe gebunden und/oder mit einem unsubstituierten oder wenigstens einfach substituierten mono- oder polyzyklischen Ringsystem kondensiert sein kann, stehen,
oder $R^3$ und $R^4$ zusammen mit dem sie verbindenden Stickstoffatom als Ringglied einen gesättigten oder ungesättigten, unsubstituierten oder wenigstens einfach substituierten, ggf. wenigstens ein weiteres Heteroatom als Ringglied aufweisenden heterocycloaliphatischen Rest bilden, der mit einem unsubstituierten oder wenigstens einfach substituierten mono- oder polyzyklischen Ringsystem kondensiert sein kann;
$R^5$, $R^6$, $R^7$, $R^8$, $R^{10}$, $R^{11}$, $R^{12}$, $R^{15}$ und $R^{16}$, jeweils unabhängig voneinander, für einen linearen oder verzweigten,

gesättigten oder ungesättigten, unsubstituierten oder wenigstens einfach substituierten aliphatischen Rest oder für einen unsubstituierten oder wenigstens einfach substituierten Aryl- oder Heteroaryl-Rest, der über eine lineare oder verzweigte, unsubstituierte oder wenigstens einfach substituierte Alkylen-Gruppe gebunden und/oder mit einem unsubstituierten oder wenigstens einfach substituierten mono- oder polyzyklischen Ringsystem kondensiert sein kann, stehen;

$R^9$, $R^{13}$, $R^{14}$, $R^{17}$, $R^{18}$, $R^{19}$, $R^{20}$, $R^{21}$, $R^{22}$, $R^{23}$, $R^{24}$, $R^{25}$, $R^{26}$, $R^{27}$, $R^{28}$, $R^{29}$, $R^{30}$, $R^{31}$, $R^{32}$, $R^{33}$ und $R^{34}$, jeweils unabhängig voneinander, für einen Wasserstoff-Rest; für einen linearen oder verzweigten, gesättigten oder ungesättigten, unsubstituierten oder wenigstens einfach substituierten aliphatischen Rest oder für einen unsubstituierten oder wenigstens einfach substituierten Aryl- oder Heteroaryl-Rest, der über eine lineare oder verzweigte, unsubstituierte oder wenigstens einfach substituierte Alkylen-Gruppe gebunden und/oder mit einem unsubstituierten oder wenigstens einfach substituierten mono- oder polyzyklischen Ringsystem kondensiert sein kann, stehen;

$M^1$ für einen Aryl- oder Heteroaryl-Rest steht, der mit wenigstens einem weiteren Substituenten substituiert und/oder mit einem unsubstituierten oder wenigstens einfach substituierten mono- oder polyzyklischen Ringsystem kondensiert sein kann;

und $M^2$ für einen Aryl- oder Heteroaryl-Rest steht, der unsubstituiert oder wenigstens einfach substituiert und mit einem unsubstituierten oder wenigstens einfach substituierten mono- oder polyzyklischen Ringsystem kondensiert sein kann;

jeweils in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

**2.** Verbindungen gemäß Anspruch 1, **dadurch gekennzeichnet, dass**
$R^1$ und $R^2$, unabhängig voneinander, jeweils für einen Wasserstoff-Rest; für einen Halogen-Rest; $-NO_2$; -CN; $-NH_2$; $-NHR^5$; $-NR^6R^7$; $-NH-C(=O)-R^8$; $-C(=O)-R^9$, $-C(=O)-NH_2$; $-C(=O)-NHR^{10}$; $-C(=O)-NR^{11}R^{12}$; $-C(=O)-OR^{13}$; $-(CH_2)_m-C(=O)-OR^{14}$ mit m = 1, 2, 3, 4 oder 5; $-O-C(=O)-R^{15}$; $-(CH_2)_n-O-C(=O)-R^{16}$ mit n = 1, 2, 3, 4 oder 5; $-OR^{17}$; $-(CH_2)_o-O-R^{18}$ mit o = 1, 2, 3; 4 oder 5; $-SR^{19}$;$-(CH_2)_p-S(=O)_t-R^{20}$ mit p = 1, 2, 3, 4 oder 5 und t = 0, 1 oder 2; $-NH-S(=O)_2-NR^{27}R^{28}$;$-S(=O)_2-NR^{29}R^{30}$; $-SF_5$; $-(CH_2)_u-O-S(=O)_2-R^{31}$ mit u = 1, 2, 3, 4 oder 5; $-(CH_2)_v-O-S(=O)_2-O-R^{32}$ mit v = 1, 2, 3, 4 oder 5; $-(CH_2)_w-O-P(=O)(OR^{33})(OR^{34})$ mit w = 1, 2, 3, 4 oder 5; für einen linearen oder verzweigten, unsubstituierten oder wenigstens einfach substituierten $C_{1-10}$-Alkyl-Rest, $C_{2-6}$-Alkenyl-Rest oder $C_{2-6}$-Alkinyl-Rest;
für einen gesättigten oder ungesättigten, unsubstituierten oder wenigstens einfach substituierten, ggf. wenigstens ein Heteroatom als Ringglied aufweisenden cycloaliphatischen $C_{3-8}$-Rest, der über eine lineare oder verzweigte, unsubstituierte oder wenigstens einfach substituierte $C_{1-5}$-Alkylen-Gruppe gebunden sein kann;
oder für einen unsubstituierten oder wenigstens einfach substituierten 5- oder 6-gliedrigen Aryl- oder Heteroaryl-Rest, der über eine lineare oder verzweigte, unsubstituierte oder wenigstens einfach substituierte $C_{1-5}$-Alkylen-Gruppe gebunden und/oder mit einem unsubstituierten oder wenigstens einfach substituierten mono- oder polyzyklischen Ringsystem kondensiert sein kann, wobei die Ringe des Ringsystems jeweils 5-, 6- oder 7-gliedrig sind, stehen;
$R^3$ und $R^4$, unabhängig voneinander, jeweils für einen Wasserstoff-Rest; $-C(=O)-R^{21}$; $-(CH_2)_q-C(=O)-R^{22}$ mit q = 1, 2, 3, 4 oder 5; $-C(=O)-O-R^{23}$; $-(CH_2)_r-C(=O)-O-R^{24}$ mit r = 1, 2, 3, 4 oder 5; $-C(=O)-NHR^{25}$; $-(CH_2)_s-C(=O)-NHR^{26}$ mit s = 1, 2, 3, 4 oder 5; für einen linearen oder verzweigten, unsubstituierten oder wenigstens einfach substituierten $C_{1-10}$-Alkyl-Rest, $C_{2-6}$-Alkenyl-Rest oder $C_{2-6}$-Alkinyl-Rest;
für einen gesättigten oder ungesättigten, unsubstituierten oder wenigstens einfach substituierten, ggf. wenigstens ein Heteroatom als Ringglied aufweisenden cycloaliphatischen $C_{3-8}$-Rest, der über eine lineare oder verzweigte, unsubstituierte oder wenigstens einfach substituierte $C_{1-5}$-Alkylen-Gruppe gebunden sein kann; oder für einen unsubstituierten oder wenigstens einfach substituierten 5- oder 6-gliedrigen Aryl- oder Heteroaryl-Rest, der über eine lineare oder verzweigte, unsubstituierte oder wenigstens einfach substituierte $C_{1-5}$-Alkylen-Gruppe gebunden und/oder mit einem unsubstituierten oder wenigstens einfach substituierten mono- oder polyzyklischen Ringsystem kondensiert sein kann, wobei die Ringe des Ringsystems jeweils 5-, 6- oder 7-gliedrig sind, stehen,
oder $R^3$ und $R^4$ zusammen mit dem sie verbindenden Stickstoffatom als Ringglied einen gesättigten oder ungesättigten, unsubstituierten oder wenigstens einfach substituierten, ggf. wenigstens ein weiteres Heteroatom als Ringglied aufweisenden heterocycloaliphatischen $C_{4-10}$-Rest bilden, der mit einem unsubstituierten oder wenigstens einfach substituierten mono- oder polyzyklischen Ringsystem kondensiert sein kann, wobei die Ringe des Ringsystems jeweils 5-, 6- oder 7-gliedrig sind;
$R^5$, $R^6$, $R^7$, $R^8$, $R^{10}$, $R^{11}$, $R^{12}$, $R^{15}$ und $R^{16}$, jeweils unabhängig voneinander, für einen linearen oder verzweigten, unsubstituierten oder wenigstens einfach substituierten $C_{1-10}$-Alkyl-Rest, $C_{2-6}$-Alkenyl-Rest oder $C_{2-6}$-Alkinyl-Rest;
oder für einen unsubstituierten oder wenigstens einfach substituierten 5- bis 14-gliedrigen Aryl- oder Heteroaryl-Rest, der über eine lineare oder verzweigte, unsubstituierte oder wenigstens einfach substituierte $C_{1-5}$-Alkylen-Gruppe gebunden und/oder mit einem unsubstituierten oder wenigstens einfach substituierten mono- oder polyzyklischen Ringsystem kondensiert sein kann, wobei die Ringe des Ringsystems jeweils 5-, 6- oder 7-gliedrig sind, stehen;

$R^9$, $R^{13}$, $R^{14}$, $R^{17}$, $R^{18}$, $R^{19}$, $R^{20}$, $R^{21}$, $R^{22}$, $R^{23}$, $R^{24}$, $R^{25}$, $R^{26}$, $R^{27}$, $R^{28}$, $R^{29}$, $R^{30}$, $R^{31}$, $R^{32}$, $R^{33}$ und $R^{34}$, jeweils unabhängig voneinander, für einen Wasserstoff-Rest; für einen linearen oder verzweigten, unsubstituierten oder wenigstens einfach substituierten $C_{1-10}$-Alkyl-Rest, $C_{2-6}$-Alkenyl-Rest oder $C_{2-6}$-AlkinylRest; oder für einen unsubstituierten oder wenigstens einfach substituierten 5-bis 14-gliedrigen Aryl- oder Heteroaryl-Rest, der über eine lineare oder verzweigte, unsubstituierte oder wenigstens einfach substituierte $C_{1-5}$-Alkylen-Gruppe gebunden und/oder mit einem unsubstituierten oder wenigstens einfach substituierten mono- oder polyzyklischen Ringsystem kondensiert sein kann, wobei die Ringe des Ringsystems jeweils 5-, 6- oder 7-gliedrig sind, stehen;

$M^1$ für einen 5- oder 6-gliedrigen Aryl- oder Heteroaryl-Rest steht, der mit wenigstens einem weiteren Substituenten substituiert sein kann und mit einem unsubsituierten oder wenigstens einfach substituierten mono- oder bizyklischem Ringsystem kondensiert sein kann, wobei die Ringe des Ringsystems jeweils 5-, 6- oder 7-gliedrig sind, und

und $M^2$ für einen 5- oder 6-gliedrigen Aryl- oder Heteroaryl-Rest steht, der unsubstituiert oder wenigstens einfach substituiert und mit einem unsubstituierten oder wenigstens einfach substituierten mono- oder bizyklischem Ringsystem kondensiert sein kann, wobei die Ringe des Ringsystems jeweils 5-, 6- oder 7-gliedrig sind;

wobei

die vorstehend genannten cycloaliphatischen Reste ggf. 1, 2, 3, 4 oder 5 Heteroatom(e) als Ringglied(er) aufweisen können, die jeweils unabhängig voneinander, ausgewählt werden können aus der Gruppe bestehend aus Stickstoff, Sauerstoff und Schwefel,

die vorstehend genannten heterocycloaliphatischen Reste ggf. weitere 1, 2, 3, 4 oder 5 Heteroatom(e) als Ringglied(er) aufweisen können, die jeweils unabhängig voneinander, ausgewählt werden können aus der Gruppe bestehend aus Stickstoff, Sauerstoff und Schwefel,

die Ringe des mono- oder polyzyklischen Ringsystems jeweils ggf. 0, 1, 2 oder 3 Heteroatom(e) als Ringglied(er) aufweisen, die unabhängig voneinander aus der Gruppe bestehend aus Sauerstoff, Stickstoff und Schwefel ausgewählt sind;

und

die vorstehend genannten Heteroaryl-Reste ggf. 1, 2, 3, 4 oder 5 Heteroatom(e) als Ringglied(er) aufweisen können, die jeweils unabhängig voneinander, ausgewählt werden können aus der Gruppe bestehend aus Sauerstoff, Schwefel und Stickstoff.

**3.** Verbindungen gemäß Anspruch 1 oder 2, **dadurch gekennzeichnet, dass**

$R^1$ und $R^2$, unabhängig voneinander, jeweils für einen Wasserstoff-Rest; -F; - Cl; -Br; -I; $-NO_2$; -CN; $-NH_2$; $-NHR^5$; $-NR^6R^7$; $-C(=O)-R^9$, $-C(=O)-NH_2$; $-C(=0)-NHR^{10}$; $-C(=O)-NR^{11}R^{12}$; $-C(=O)-OR^{13}$; $-(CH_2)_m-C(=O)-OR^{14}$ mit m = 1, 2 oder 3; $-O-C(=O)-R^{15}$; $-OR^{17}$; $-(CH_2)_o-O-R^{18}$ mit o = 1, 2 oder 3; $-S(=O)_2-NH_2$; $-SF_5$;$-(CH_2)_u-O-S(=O)_2-R^{31}$ mit u = 1, 2 oder 3; $-(CH_2)_v-O-S(=O)_2-O-R^{32}$ mit v = 1, 2 oder 3; $-(CH_2)_w-O-P(=O)(OR^{33})(OR^{34})$ mit w = 1, 2 oder 3; für einen Rest ausgewählt aus der Gruppe bestehend aus Methyl, Ethyl, n-Propyl, Isopropyl, n-Butyl, Isobutyl, 2-Butyl, tert-Butyl, n-Pentyl, 2-Pentyl, 3-Pentyl, iso-Pentyl, neo-Pentyl, n-Hexyl, n-Heptyl, n-Octyl, (2,4,4)-Trimethyl-pent-2-yl, $-CF_3$,$-CF_2H$, $-CFH_2$, $-(CH_2)-(CF_3)$, $-(CH_2)-(CHF_2)$, $-(CH_2)-(CH_2F)$ und $-(CF_2)-(CF_3)$; für einen Rest ausgewählt aus der Gruppe bestehend aus Cyclopropyl, Cyclobutyl, Cyclopentyl, Cyclohexyl, Cycloheptyl und Cyclooctyl, der jeweils unsubstituiert oder mit ggf. 1, 2, 3 oder 4 Substituenten unabhängig voneinander ausgewählt aus der Gruppe bestehend aus F, Cl, Br, I, -CN, - $NO_2$, Methyl, Ethyl, n-Propyl, Isopropyl, n-Butyl, Isobutyl, 2-Butyl, tert-Butyl, n-Pentyl, neo-Pentyl, -OH, Oxo (=O), Thioxo (=S), $-O-CH_3$, $-O-C_2H_5$, $-O-C_3H_7$,$-O-C(CH_3)_3$, $-CF_3$, $-CHF_2$, $-CH_2F$, $-O-CF_3$, $-O-CHF_2$ und $-O-CH_2F$ substituiert sein kann;

oder für einen Rest ausgewählt aus der Gruppe bestehend aus Phenyl, Benzyl, Phenethyl, (3-Phenyl)-prop-1-yl, Furyl (Furanyl), Thienyl (Thiophenyl), Pyrazolyl, Imidazolyl, Thiazolyl, Thiadiazolyl, Pyridinyl, Pyridazinyl, Pyrimidinyl und Pyrazinyl stehen, der jeweils unsubstituiert oder mit ggf. 1, 2, 3, 4 oder 5 Substituenten unabhängig voneinander ausgewählt aus der Gruppe bestehend aus F, Cl, Br, I, -CN, $-NO_2$, Methyl, Ethyl, n-Propyl, Isopropyl, n-Butyl, Isobutyl, 2-Butyl, tert-Butyl, n-Pentyl, neo-Pentyl, Ethenyl, Allyl, Ethinyl, Propinyl, -OH, -SH, $-SF_5$, $-NH_2$, -C(=O)-OH, $-S-CH_3$, $-S-C_2H_5$, $-O-CH_3$, $-O-C_2H_5$, $-O-C_3H_7$, $-O-C(CH_3)_3$, $-CF_3$, $-CHF_2$, $-CH_2F$, $-O-CF_3$, $-O-CHF_2$, $-O-CH_2F$,$-C(=O)-CF_3$, $-S-CF_3$, $-S-CHF_2$, $-S-CH_2F$, $-C(=O)-O-CH_3$, $-C(=O)-O-C_2H_5$,$-C(=O)-O-C(CH_3)_3$, Cyclopropyl, Cyclobutyl, Cyclopentyl, Cyclohexyl und Phenyl substituiert sein kann;

$R^3$ und $R^4$, unabhängig voneinander, jeweils für einen Wasserstoff-Rest; $-C(=O)-R^{21}$; $-(CH_2)_q-C(=O)-R^{22}$ mit q = 1, 2 oder 3; $-C(=O)-O-R^{23}$; $-(CH_2)_r-C(=O)-O-R^{24}$ mit r = 1, 2 oder 3; $-C(=O)-NHR^{25}$; $-(CH_2)_s-C(=O)-NHR^{26}$ mit s = 1, 2 oder 3; für einen Rest ausgewählt aus der Gruppe bestehend aus Methyl, Ethyl, n-Propyl, Isopropyl, n-Butyl, Isobutyl, 2-Butyl, tert-Butyl, n-Pentyl, 2-Pentyl, 3-Pentyl, iso-Pentyl, neo-Pentyl, n-Hexyl, n-Heptyl, n-Octyl und (2,4,4)-Trimethyl-pent-2-yl; für einen Rest ausgewählt aus der Gruppe bestehend aus Cyclopropyl, Cyclobutyl,

Cyclopentyl, Cyclohexyl, Cycloheptyl und Cyclooctyl, der jeweils unsubstituiert oder mit ggf. 1, 2, 3 oder 4 Substituenten unabhängig voneinander ausgewählt aus der Gruppe bestehend aus F, Cl, Br, I, -CN, $-NO_2$, Methyl, Ethyl, n-Propyl, Isopropyl, n-Butyl, Isobutyl, 2-Butyl, tert-Butyl, n-Pentyl, neo-Pentyl, -OH, Oxo (=0), Thioxo (=S), $-O-CH_3$, $-O-C_2H_5$, $-O-C_3H_7$ und $-O-C(CH_3)_3$ substituiert und/oder über eine lineare oder verzweigte $C_{1-3}$-Alkylen-Gruppe gebunden sein kann; oder für einen Rest ausgewählt aus der Gruppe bestehend aus Phenyl, Furyl (Furanyl), Thienyl (Thiophenyl), Pyrazolyl, Imidazolyl, Thiazolyl, Thiadiazolyl, Pyridinyl, Pyridazinyl, Pyrimidinyl und Pyrazinyl stehen, der jeweils unsubstituiert oder mit ggf. 1, 2, 3, 4 oder 5 Substituenten unabhängig voneinander ausgewählt aus der Gruppe bestehend aus F, Cl, Br, I, -CN, $-NO_2$, Methyl, Ethyl, n-Propyl, Isopropyl, n-Butyl, Isobutyl, 2-Butyl, tert-Butyl, n-Pentyl, neo-Pentyl, Ethenyl, Allyl, -OH,-SH, $-SF_5$, $-NH_2$, -C(=O)-OH, $-S-CH_3$, $-S-C_2H_5$, $-O-CH_3$, $-O-C_2H_5$, $-O-C_3H_7$, $-O-C(CH_3)_3$, $-CF_3$, $-CHF_2$, $-CH_2F$, $-O-CF_3$, $-O-CHF_2$, $-O-CH_2F$, $-C(=O)-CF_3$, $-S-CF_3$, $-S-CHF_2$, $-S-CH_2F$, $-N(CH_3)_2$, $-N(C_2H_5)_2$, $-NH-CH_3$, $-NH-C_2H_5$, Cyclopropyl, Cyclobutyl und Cyclopentyl substituiert und/oder über eine lineare oder verzweigte $C_{1-3}$-Alkylen-Gruppe gebunden sein kann;

oder $R^3$ und $R^4$ zusammen mit dem sie verbindenden Stickstoffatom als Ringglied einen Rest bilden ausgewählt aus der Gruppe bestehend aus Imidazolidinyl, Piperidinyl, Piperazinyl, Morpholinyl, Pyrrolidinyl, Thiomorpholinyl, Azepanyl, Diazepanyl und Azocanyl, der jeweils unsubstituiert oder mit ggf. 1, 2, 3, 4 oder 5 Substituenten unabhängig voneinander ausgewählt aus der Gruppe bestehend aus F, Cl, Br, I, -CN, Methyl, Ethyl, n-Propyl, Isopropyl, n-Butyl, 2-Butyl, Isobutyl, tert-Butyl, -OH, Oxo, Thioxo, $-O-CH_3$, $-O-C_2H_5$, $-O-C_3H_7$, $-NH_2$, $-N(CH_3)_2$, $-N(C_2H_5)_2$, $-NH-CH_3$,-$NH-C_2H_5$, $-NO_2$, $-CF_3$, $-O-CF_3$, $-S-CF_3$, -SH, $-SF_5$, $-S-CH_3$, $-S-C_2H_5$, $-S(=O)-CH_3$, $-S(=O)_2-CH_3$, $-S(=O)-C_2H_5$, $-S(=O)_2-C_2H_5$, $-C(=O)-CH_3$, $-C(=O)-C_2H_5$,$-C(=O)-N(CH_3)_2$, $-C(=O)-NH-CH_3$, $-C(=O)-NH_2$, $-C(=O)-O-CH_3$, $-C(=O)-O-C_2H_5$, $-C(=O)-O-C(CH_3)_3$ und Phenyl substituiert sein kann;

$R^5$, $R^6$, $R^7$, $R^{10}$, $R^{11}$, $R^{12}$ und $R^{15}$, jeweils unabhängig voneinander, für einen Rest ausgewählt aus der Gruppe bestehend aus Methyl, Ethyl, n-Propyl, Isopropyl, n-Butyl, Isobutyl, 2-Butyl, tert-Butyl, n-Pentyl, 2-Pentyl, 3-Pentyl, iso-Pentyl, neo-Pentyl, n-Hexyl, n-Heptyl, n-Octyl, (2,4,4)-Trimethyl-pent-2-yl,$-CF_3$, $-CF_2H$, $-CFH_2$, $-(CH_2)-(CHF_2)$, $-(CH_2)-(CH_2F)$, $-(CF_2)-(CF_3)$, $-(CH_2)-(CH_2)-C(=O)-OH$, $-(CH_2)-(CH_2)-C(=O)-O-CH_3$ und $-(CH_2)-(CH_2)-C(=O)-O-C_2H_5$; oder für einen Rest ausgewählt aus der Gruppe bestehend aus Phenyl, Benzyl, Phenethyl, Furyl (Furanyl), Thienyl (Thiophenyl), Pyrazolyl, Imidazolyl, Thiazolyl, Thiadiazolyl, Pyridinyl, Pyridazinyl, Pyrimidinyl und Pyrazinyl stehen, der jeweils unsubstituiert oder mit ggf. 1, 2, 3, 4 oder 5 Substituenten unabhängig voneinander ausgewählt aus der Gruppe bestehend aus F, Cl, Br, I, -CN, $-NO_2$, Methyl, Ethyl, n-Propyl, Isopropyl, n-Butyl, Isobutyl, 2-Butyl, tert-Butyl, n-Pentyl, neo-Pentyl, -OH, -SH, $-SF_5$, $-NH_2$, -C(=O)-OH, $-S-CH_3$, $-S-C_2H_5$, $-O-CH_3$, $-O-C_2H_5$, $-O-C_3H_7$, $-O-C(CH_3)_3$, $-CF_3$, $-CHF_2$, $-CH_2F$, $-O-CF_3$,$-O-CHF_2$, $-O-CH_2F$ und $-C(=O)-CF_3$ substituiert sein kann;

$R^9$, $R^{13}$, $R^{14}$, $R^{17}$, $R^{18}$, $R^{21}$, $R^{22}$, $R^{23}$, $R^{24}$, $R^{25}$, $R^{26}$, $R^{31}$, $R^{32}$, $R^{33}$ und $R^{34}$, jeweils unabhängig voneinander, für einen Wasserstoff-Rest; für einen Rest ausgewählt aus der Gruppe bestehend aus Methyl, Ethyl, n-Propyl, Isopropyl, n-Butyl, Isobutyl, 2-Butyl, tert-Butyl, n-Pentyl, 2-Pentyl, 3-Pentyl, iso-Pentyl, neo-Pentyl, n-Hexyl, n-Heptyl, n-Octyl, (2,4,4)-Trimethyl-pent-2-yl, $-CF_3$,$-CF_2H$, $-CFH_2$, $-(CH_2)-(CF_3)$, $-(CH_2)-(CHF_2)$, $-(CH_2)-(CH_2F)$ und $-(CF_2)-(CF_3)$; oder für einen Rest ausgewählt aus der Gruppe bestehend aus Phenyl, Benzyl, Phenethyl, Furyl (Furanyl), Thienyl (Thiophenyl), Pyrazolyl, Imidazolyl, Thiazolyl, Thiadiazolyl, Pyridinyl, Pyridazinyl, Pyrimidinyl und Pyrazinyl stehen, der jeweils unsubstituiert oder mit ggf. 1, 2, 3, 4 oder 5 Substituenten unabhängig voneinander ausgewählt aus der Gruppe bestehend aus F, Cl, Br, I, -CN, $-NO_2$, Methyl, Ethyl, n-Propyl, Isopropyl, n-Butyl, Isobutyl, 2-Butyl, tert-Butyl, n-Pentyl, neo-Pentyl, -OH, -SH, $-SF_5$, $-NH_2$, -C(=O)-OH, $-S-CH_3$, $-S-C_2H_5$,- $O-CH_3$, $-O-C_2H_5$, $-O-C_3H_7$, $-O-C(CH_3)_3$, $-CF_3$, $-CHF_2$, $-CH_2F$, $-O-CF_3$,$-O-CHF_2$, $-O-CH_2F$ und $-C(=O)-CF_3$ substituiert sein kann;

$M^1$ für einen Rest ausgewählt aus der Gruppe bestehend aus den Resten aus den Resten 1 bis 9, 11, 21, 22 und 36 bis 38 steht,

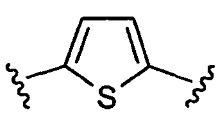

1

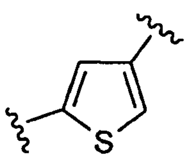

2

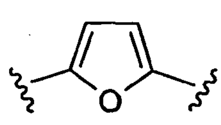

3

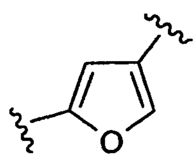

4

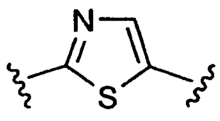
5

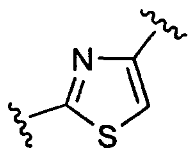
6

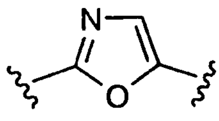
7

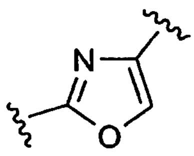
8

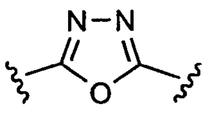
9

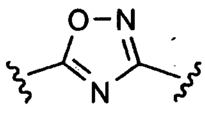
11

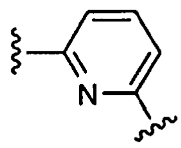
21

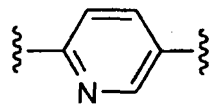
22

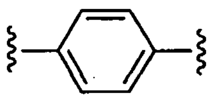
36

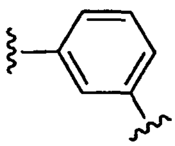
37

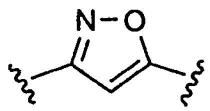
38

der jeweils unsubstituiert oder mit ggf. 1 oder 2 Substituenten unabhängig voneinander ausgewählt aus der Gruppe bestehend aus F, Cl, Br, I, -CN, - $NO_2$, Methyl, Ethyl, n-Propyl, Isopropyl, n-Butyl, Isobutyl, 2-Butyl, tert-Butyl, n-Pentyl, neo-Pentyl, Ethenyl, Allyl, Ethinyl, Propinyl, -OH, -SH, -$SF_5$, -$NH_2$,-C(=O)-OH, -S-$CH_3$, -O-$C_2H_5$, -S(=O)-$CH_3$, -S(=O)$_2$-$CH_3$, -S(=O)-$C_2H_5$, -S(=O)$_2$-$C_2H_5$, -O-$CH_3$, -O-$C_2H_5$, -O-$C_3H_7$, -O-C($CH_3$)$_3$, -$CF_3$, -$CHF_2$, -$CH_2F$, -O-$CF_3$,-O-$CHF_2$, -O-$CH_2F$, -C(=O)-$CF_3$, -S-$CF_3$, -S-$CHF_2$ und -S-$CH_2F$ substituiert sein kann, und der jeweils in beliebiger Richtung über die mit einer geschlängelten Linie gekennzeichneten Positionen mit dem Bizyklus und dem Kohlenstoffatom der Dreifachbindung verknüpft sein kann;

und $M^2$ für einen Rest ausgewählt aus der Gruppe bestehend aus den Resten 1 bis 36 steht,

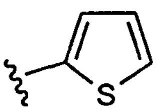
1

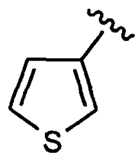
2

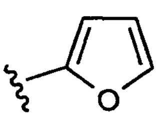
3

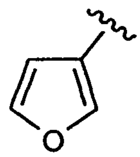
4

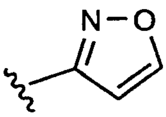
5

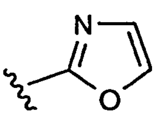
6

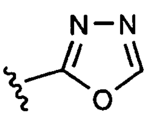
7

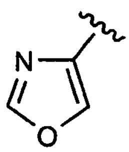
8

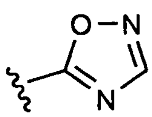
9

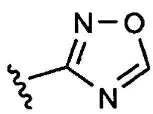
10

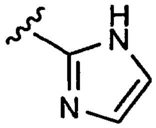
11

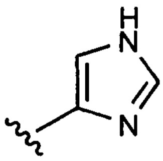
12

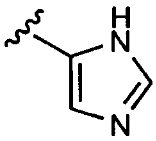
13

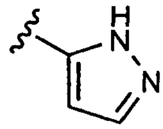
14

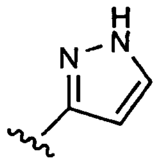
15

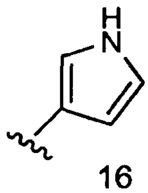
16

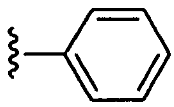
17

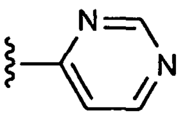
18

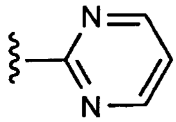
19

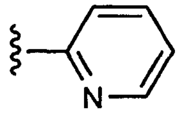
20

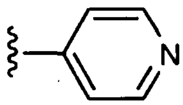
21

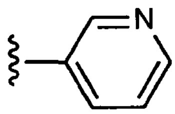
22

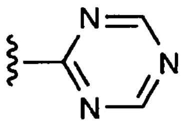
23

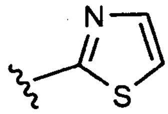
24

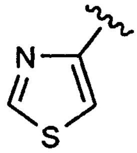
25

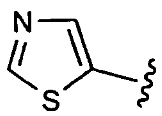
26

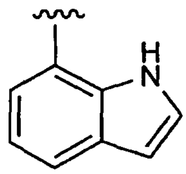
27

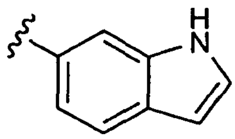
28

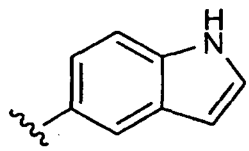
29

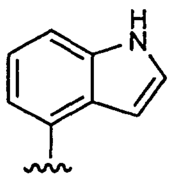
30

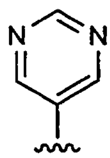
31

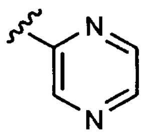
32

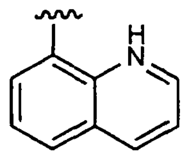
33

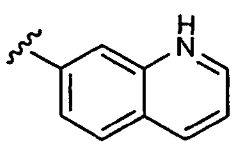
34

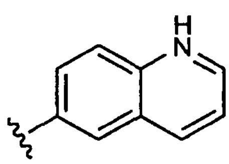
35

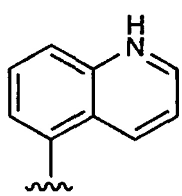
36

der jeweils über die mit einer geschlängelten Linie gekennzeichnete Position mit dem Kohlenstoffatom der Dreifachbindung verknüpft und unsubstituiert oder mit ggf. 1, 2, 3, 4 oder 5 Substituenten unabhängig voneinander ausgewählt aus der Gruppe bestehend aus F, Cl, Br, -CN, $-CF_3$, $-SF_5$, $-S-CH_3$, $-S-C_2H_5$, Methyl, Ethyl, n-Propyl, iso-Propyl, n-Butyl, sec-Butyl, iso-Butyl, tert-Butyl, Ethenyl, Propenyl, -OH, $-O-CH_3$, $-O-C_2H_5$, $-O-C_3H_7$, $-NH_2$, $-N(CH_3)_2$,$-NH-CH_3$, $-CH_2-NH_2$, $-N(C_2H_5)_2$, $-NO_2$, $-O-CF_3$, $-S-CF_3$, -SH, -C(=O)-H, $-C(=O)-O-CH_3$, $-C(=O)-O-C_2H_5$, Phenyl, Pyrrolyl, (1,3)-Dioxolanyl, - $Si(Phenyl)_2[C(CH_3)_3]$, $-C(=O)-CH_3$, $-C(=O)-C_2H_5$, $NH-S(=O)_2-CH_3$, $-NH-S(=O)_2-C_2H_5$, $-S(=O)_2-NH_2$, $-S(=O)_2-NH-CH_3$, $-CH_2-OH$, -C(=O)-OH, $-CH_2-O-CH_3$,$-C(=O)-NH_2$, $-C(=O)-NH-CH_3$, $-NH-C(=O)-CH_3$, $-NH-C(=NH)-NH_2$, $-NH-S(=O)_2-OH$ und $-S(=O)_2-N(CH_3)_2$ substituiert sein kann;
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

**4.** Verbindungen gemäß einem oder mehreren der Ansprüche 1 bis 3, **dadurch gekennzeichnet, dass**
$R^1$ und $R^2$, unabhängig voneinander, jeweils für einen Wasserstoff-Rest; -F;-Cl; -Br; -I; $-NO_2$; -CN; $-NHR^5$; $-NR^6R^7$; $-C(=O)-R^9$, $-C(=O)-OR^{13}$; $-(CH_2)-O-C(=O)-R^{16}$; $-OR^{17}$; $-(CH_2)-O-S(=O)_2-R^{31}$; $-(CH_2)-O-S(=O)_2-O-R^{32}$; $-(CH_2)-O-P(=O)(OR^{33})(OR^{34})$; $-SF_5$; für einen Rest ausgewählt aus der Gruppe bestehend aus Methyl, Ethyl, n-Propyl, Isopropyl, n-Butyl, Isobutyl, 2-Butyl, tert-Butyl, n-Pentyl, $-CF_3$, $-CF_2H$, $-CFH_2$, $-(CH_2)-(CF_3)$, $-(CH_2)-(CHF_2)$, $-(CH_2)-(CH_2F)$ und $-(CF_2)-(CF_3)$; für einen Rest ausgewählt aus der Gruppe bestehend aus Cyclopropyl, Cyclobutyl, Cyclopentyl, Cyclohexyl, Cycloheptyl und Cyclooctyl;
oder für einen Rest ausgewählt aus der Gruppe bestehend aus Phenyl, Benzyl, Phenethyl und (3-Phenyl)-prop-1-yl stehen, der jeweils unsubstituiert oder mit ggf. 1, 2, 3, 4 oder 5 Substituenten unabhängig voneinander ausgewählt aus der Gruppe bestehend aus F, Cl, Br, I, -CN, $-NO_2$, Methyl, Ethyl, n-Propyl, Isopropyl, n-Butyl, Isobutyl, 2-Butyl, tert-Butyl, $-O-CH_3$, $-O-C_2H_5$, $-O-C_3H_7$, $-O-C(CH_3)_3$, $-CF_3$, $-CHF_2$, $-CH_2F$, $-O-CF_3$, $-O-CHF_2$ und $-O-CH_2F$ substituiert sein kann;
$R^3$ und $R^4$, unabhängig voneinander, jeweils für einen Wasserstoff-Rest; $-C(=O)-R^{21}$; $-(CH_2)_q-C(=O)-R^{22}$ mit q = 1, 2 oder 3; $-(CH_2)_r-C(=O)-O-R^{24}$ mit r = 1, 2 oder 3; für einen Rest ausgewählt aus der Gruppe bestehend aus Methyl, Ethyl, n-Propyl, Isopropyl, n-Butyl, Isobutyl, 2-Butyl, tert-Butyl, n-Pentyl, 2-Pentyl, 3-Pentyl, iso-Pentyl, neo-Pentyl, n-Hexyl, n-Heptyl, n-Octyl und (2,4,4)-Trimethyl-pent-2-yl; für einen Rest ausgewählt aus der Gruppe bestehend aus Cyclopropyl, Cyclobutyl, Cyclopentyl, Cyclohexyl, Cycloheptyl und Cyclooctyl, der über eine $-(CH_2)-$, $-(CH_2)_2-$, $-(CH(CH_3))-$ oder $-(CH_2)_3$-Gruppe gebunden sein kann;
oder für einen Rest ausgewählt aus der Gruppe bestehend aus Phenyl, Furyl (Furanyl), Thienyl (Thiophenyl), Pyridinyl, Pyridazinyl, Pyrimidinyl und Pyrazinyl stehen, der jeweils unsubstituiert oder mit ggf. 1, 2, 3, 4 oder 5 Substituenten unabhängig voneinander ausgewählt aus der Gruppe bestehend aus F, Cl, Br, I, -CN, $-NO_2$, Methyl, Ethyl, n-Propyl, Isopropyl, n-Butyl, Isobutyl, 2-Butyl, tert-Butyl, $-O-CH_3$, $-O-C_2H_5$, $-O-C_3H_7$, $-O-C(CH_3)_3$,$-CF_3$, $-CHF_2$, $-CH_2F$, $-O-CF_3$, $-O-CHF_2$ und $-O-CH_2F$ substituiert und/oder über eine-$(CH_2)$-, $-(CH_2)_2-$, $-(CH(CH_3))-$ oder $-(CH_2)_3$-Gruppe gebunden sein kann;
oder $R^3$ und $R^4$ zusammen mit dem sie verbindenden Stickstoffatom als Ringglied einen Rest bilden ausgewählt aus der Gruppe bestehend aus Piperidinyl, Piperazinyl, Morpholinyl und Pyrrolidinyl, der jeweils unsubstituiert oder mit ggf. 1, 2, 3, 4 oder 5 Substituenten unabhängig voneinander ausgewählt aus der Gruppe bestehend aus Methyl, Ethyl, n-Propyl, Isopropyl, n-Butyl, 2-Butyl, Isobutyl, tert-Butyl, $-S(=O)-CH_3$, $-S(=O)_2-CH_3$, $-S(=O)-C_2H_5$,$-S(=O)_2-C_2H_5$, $-C(=O)-CH_3$, $-C(=O)-C_2H_5$, $-C(=O)-N(CH_3)_2$, $-C(=O)-NH-CH_3$,$-C(=O)-NH_2$, $-C(=O)-O-CH_3$, $-C(=O)-O-C_2H_5$ und $-C(=O)-O-C(CH_3)_3$ substituiert sein kann;
$R^5$, $R^6$, $R^7$ und $R^{16}$, jeweils unabhängig voneinander, für einen Rest ausgewählt aus der Gruppe bestehend aus Methyl, Ethyl, n-Propyl, Isopropyl, n-Butyl, Isobutyl, 2-Butyl, tert-Butyl, n-Pentyl, 2-Pentyl, 3-Pentyl, iso-Pentyl, neo-Pentyl, n-Hexyl, n-Heptyl, n-Octyl, (2,4,4)-Trimethyl-pent-2-yl, $-CF_3$,$-CF_2H$, $-CFH_2$, $-(CH_2)-(CHF_2)$, $-(CH_2)-(CH_2F)$, $-(CF_2)-(CF_3)$, $-(CH_2)-(CH_2)-C(=O)-OH$; $-(CH_2)-(CH_2)-C(=O)-O-CH_3$ und $-(CH_2)-(CH_2)-C(=O)-O-C_2H_5$; oder für einen Rest ausgewählt aus der Gruppe bestehend aus Phenyl, Benzyl und Phenethyl stehen, der jeweils unsubstituiert oder mit ggf. 1, 2, 3, 4 oder 5 Substituenten unabhängig voneinander ausgewählt aus der Gruppe bestehend aus F, Cl, Br, I, -CN, $-NO_2$, Methyl, Ethyl, n-Propyl" Isopropyl, n-Butyl, Isobutyl, 2-Butyl, tert-Butyl, $-O-CH_3$ und $-O-C_2H_5$ substituiert sein kann;
$R^9$, $R^{13}$, $R^{17}$, $R^{21}$, $R^{22}$, $R^{24}$, $R^{31}$, $R^{32}$, $R^{33}$ und $R^{34}$, jeweils unabhängig voneinander, für einen Wasserstoff-Rest; für einen Rest ausgewählt aus der Gruppe bestehend aus Methyl, Ethyl, n-Propyl, Isopropyl, n-Butyl, Isobutyl, 2-Butyl, tert-Butyl, n-Pentyl, 2-Pentyl, 3-Pentyl, iso-Pentyl, neo-Pentyl, n-Hexyl, n-Heptyl, n-Octyl, (2,4,4)-Trimethyl-pent-2-yl, $-CF_3$, $-CF_2H$, $-CFH_2$, $-(CH_2)-(CF_3)$, $-(CH_2)-(CHF_2)$, $-(CH_2)-(CH_2F)$ und $-(CF_2)-(CF_3)$; oder für einen Rest ausgewählt aus der Gruppe bestehend aus Phenyl, Benzyl und Phenethyl stehen, der jeweils unsubstituiert oder mit ggf. 1, 2, 3, 4 oder 5 Substituenten unabhängig voneinander ausgewählt aus der Gruppe bestehend aus F, Cl, Br, I, -CN, $-NO_2$, Methyl, Ethyl, n-Propyl, Isopropyl, n-Butyl, Isobutyl, 2-Butyl, tert-Butyl,$-O-CH_3$ und $-O-C_2H_5$ substituiert sein kann;

$M^1$ für einen Rest ausgewählt aus der Gruppe bestehend aus den Resten 1 bis 6, 21, 22, 36 und 37 steht,

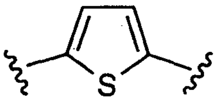
1

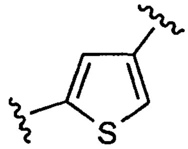
2

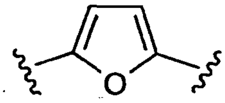
3

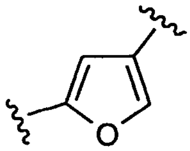
4

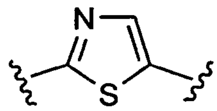
5

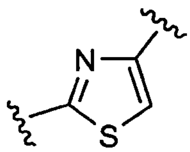
6

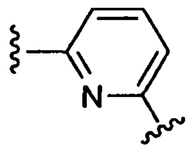
21

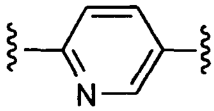
22

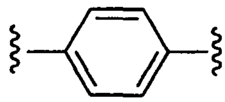
36

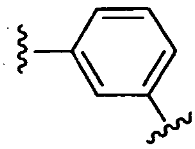
37

der jeweils unsubstituiert oder mit ggf. 1 oder 2 Substituenten unabhängig voneinander ausgewählt aus der Gruppe bestehend aus F, Cl, Br, I, -CN,-$CH_2$-CN, -$NO_2$, Methyl, Ethyl, n-Propyl, Isopropyl, n-Butyl, Isobutyl, 2-Butyl, tert-Butyl, n-Pentyl, neo-Pentyl, -$SF_5$, -O-$CH_3$, -O-$C_2H_5$, -O-$C_3H_7$, -O-C$(CH_3)_3$,-$CF_3$, -$CHF_2$, -$CH_2F$, -O-$CF_3$, -O-$CHF_2$ und -O-$CH_2F$ substituiert sein kann, und der jeweils in beliebiger Richtung über die mit einer geschlängelten Linie gekennzeichneten Positionen mit dem Bizyklus und dem Kohlenstoffatom der Dreifachbindung verknüpft sein kann;
und $M^2$ für einen Rest ausgewählt aus der Gruppe bestehend aus Phenyl, 2-Pyridinyl, 3-Pyridinyl, 4-Pyridinyl, 2-Thiazolyl, 4-Thiazolyl, 5-Thiazolyl, 4-Indolyl, 5-Indolyl, 6-Indolyl, 7-Indolyl, 2-Thiophenyl (2-Thienyl), 3-Thiophenyl (3-Thienyl), 2-Imidazolyl, 4-Imidazolyl, 5-Imidazolyl, 2-Pyrimidinyl, 4-Pyrimidinyl, 5-Pyrimidinyl, 2-Pyrazinyl, 5-Chinolinyl, 6-Chinolinyl, 7-Chinolinyl und 8-Chinolinyl steht, der jeweils unsubstituiert oder mit ggf. 1, 2, 3, 4 oder 5 Substituenten unabhängig voneinander ausgewählt aus der Gruppe bestehend aus F, Cl, Br, -CN, -$CF_3$, -$SF_5$, -S-$CH_3$, -S-$C_2H_5$, Methyl, Ethyl, n-Propyl, iso-Propyl, n-Butyl, sec-Butyl, iso-Butyl, tert-Butyl, Ethenyl, Propenyl, - OH, -O-$CH_3$, -O-$C_2H_5$, -O-$C_3H_7$, -$NH_2$, -N$(CH_3)_2$, -NH-$CH_3$, -$CH_2$-$NH_2$,-N$(C_2H_5)_2$, -$NO_2$, -O-$CF_3$, -S-$CF_3$, -SH, -C(=O)-H, -C(=O)-O-$CH_3$, -C(=O)-O-$C_2H_5$, Phenyl, Pyrrolyl, (1,3)-Dioxolanyl, -Si(Phenyl)$_2$[C$(CH_3)_3$], -C(=O)-$CH_3$,-C(=O)-$C_2H_5$, NH-S$(=O)_2$-$CH_3$, -NH-S$(=O)_2$-$C_2H_5$, -S$(=O)_2$-$NH_2$, -S$(=O)_2$-NH-$CH_3$, -$CH_2$-OH, -C(=O)-OH, -$CH_2$-O-$CH_3$, -C(=O)-$NH_2$, -C(=O)-NH-$CH_3$, -NH-C(=O)-$CH_3$, -NH-C(=NH)-$NH_2$, -NH-S$(=O)_2$-OH und -S$(=O)_2$-N$(CH_3)_2$ substituiert sein kann;
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

**5.** Verbindungen der allgemeinen Formel Ia gemäß Anspruch 4,

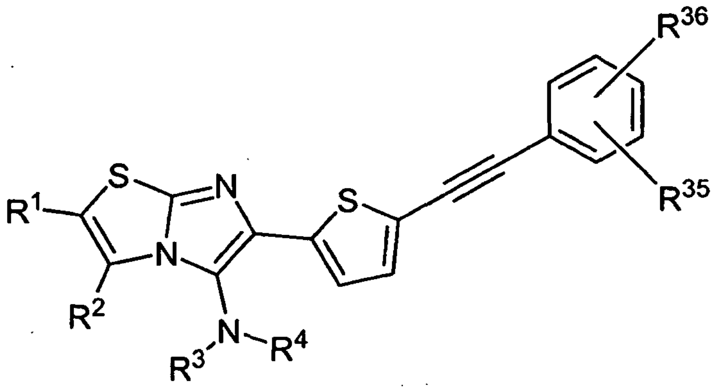

**Ia,**

worin
$R^1$, $R^2$, $R^3$ und $R^4$ die Bedeutung gemäß Anspruch 4 haben,
und $R^{35}$ und $R^{36}$, unabhängig voneinander, jeweils für einen Rest ausgewählt aus der Gruppe bestehend aus H, F, Cl, Br, -CN, $-CF_3$, $-SF_5$, $-S-CH_3$, $-S-C_2H_5$, Methyl, Ethyl, n-Propyl, iso-Propyl, n-Butyl, sec-Butyl, iso-Butyl, tert-Butyl, Ethenyl, Propenyl, -OH, $-O-CH_3$, $-O-C_2H_5$, $-O-C_3H_7$, $-NH_2$, $-N(CH_3)_2$, $-NH-CH_3$, $-CH_2-NH_2$, $-N(C_2H_5)_2$, $-NO_2$, $-O-CF_3$, $-S-CF_3$, -SH, -C(=O)-H, $-C(=O)-O-CH_3$,$-C(=O)-O-C_2H_5$, Phenyl, Pyrrolyl, (1,3)-Dioxolanyl, -Si(Phenyl)$_2$[C(CH$_3$)$_3$],$-C(=O)-CH_3$, $-C(=O)-C_2H_5$, $NH-S(=O)_2-CH_3$, $-NH-S(=O)_2-C_2H_5$, $-S(=O)_2-NH_2$,$-S(=O)_2-NH-CH_3$, $-CH_2-OH$, -C(=O)-OH, $-CH_2-O-CH_3$, $-C(=O)-NH_2$, $-C(=O)-NH-CH_3$, $-NH-C(=O)-CH_3$, $-NH-C(=NH)-NH_2$, $-NH-S(=O)_2-OH$ und $-S(=O)_2-N(CH_3)_2$ stehen;
jeweils ggf. in Form entsprechender Salze, oder jeweils ggf. in Form entsprechender Solvate.

**6.** Verbindungen der allgemeinen Formel Ib gemäß Anspruch 4,

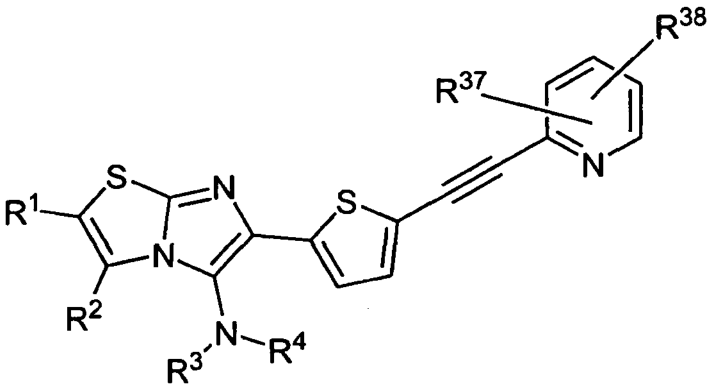

**Ib,**

worin

$R^1$, $R^2$, $R^3$ und $R^4$ die Bedeutung gemäß Anspruch 4 haben,
und $R^{37}$ und $R^{38}$, unabhängig voneinander, jeweils für einen Rest ausgewählt aus der Gruppe bestehend aus H, F, Cl, Br, -CN, $-CF_3$, $-SF_5$, $-S-CH_3$, $-S-C_2H_5$, Methyl, Ethyl, n-Propyl, iso-Propyl, n-Butyl, sec-Butyl, iso-Butyl, tert-Butyl, Ethenyl, Propenyl, -OH, $-O-CH_3$, $-O-C_2H_5$, $-O-C_3H_7$, $-NH_2$, $-N(CH_3)_2$, $-NH-CH_3$, $-CH_2-NH_2$, $-N(C_2H_5)_2$, $-NO_2$, $-O-CF_3$, $-S-CF_3$, -SH, -C(=O)-H, $-C(=O)-O-CH_3$,$-C(=O)-O-C_2H_5$, Phenyl, Pyrrolyl, (1,3)-Dioxolanyl, -Si(Phenyl)$_2$[C(CH$_3$)$_3$],$-C(=O)-CH_3$, $-C(=O)-C_2H_5$, $NH-S(=O)_2-CH_3$, $-NH-S(=O)_2-C_2H_5$, $-S(=O)_2-NH_2$,$-S(=O)_2-NH-CH_3$, $-CH_2-OH$, -C(=O)-OH, $-CH_2-O-CH_3$, $-C(=O)-NH_2$, $-C(=O)-NH-CH_3$, $-NH-C(=O)-CH_3$, $-NH-C(=NH)-NH_2$, $-NH-S(=O)_2-OH$ und $-S(=O)_2-N(CH_3)_2$ stehen;
jeweils ggf. in Form entsprechender Salze, oder jeweils ggf. in Form entsprechender Solvate.

**7.** Verbindungen der allgemeinen Formel Ic gemäß Anspruch 4,

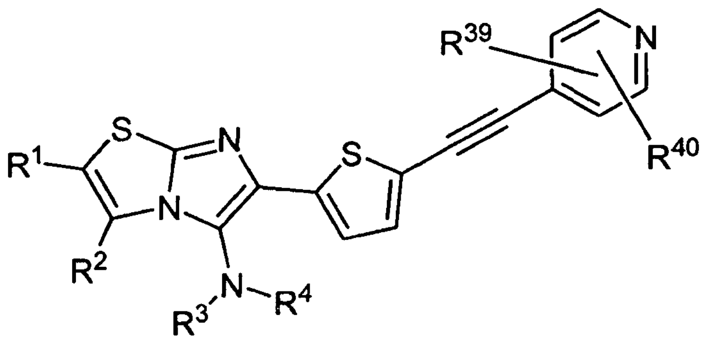

**Ic,**

worin

$R^1$, $R^2$, $R^3$ und $R^4$ die Bedeutung gemäß Anspruch 4 haben,
und $R^{39}$ und $R^{40}$, unabhängig voneinander, jeweils für einen Rest ausgewählt aus der Gruppe bestehend aus H, F, Cl, Br, -CN, $-CF_3$, $-SF_5$, $-S-CH_3$, $-S-C_2H_5$, Methyl, Ethyl, n-Propyl, iso-Propyl, n-Butyl, sec-Butyl, iso-Butyl, tert-Butyl, Ethenyl, Propenyl, -OH, $-O-CH_3$, $-O-C_2H_5$, $-O-C_3H_7$, $-NH_2$, $-N(CH_3)_2$, $-NH-CH_3$, $-CH_2-NH_2$, $-N(C_2H_5)_2$, $-NO_2$, $-O-CF_3$, $-S-CF_3$, -SH, -C(=O)-H, $-C(=O)-O-CH_3$,$-C(=O)-O-C_2H_5$, Phenyl, Pyrrolyl, (1,3)-Dioxolanyl, $-Si(Phenyl)_2[C(CH_3)_3]$, - $C(=O)-CH_3$, $-C(=O)-C_2H_5$, $NH-S(=O)_2-CH_3$, $-NH-S(=O)_2-C_2H_5$, $-S(=O)_2-NH_2$, - $S(=O)_2-NH-CH_3$, $-CH_2-OH$, -C(=O)-OH, $-CH_2-O-CH_3$, $-C(=O)-NH_2$, $-C(=O)-NH-CH_3$, $-NH-C(=O)-CH_3$, -NH-$C(=NH)-NH_2$, $-NH-S(=O)_2-OH$ und $-S(=O)_2-N(CH_3)_2$ stehen;
jeweils ggf. in Form entsprechender Salze, oder jeweils ggf. in Form entsprechender Solvate.

**8.** Verbindungen der allgemeinen Formel Id gemäß Anspruch 4,

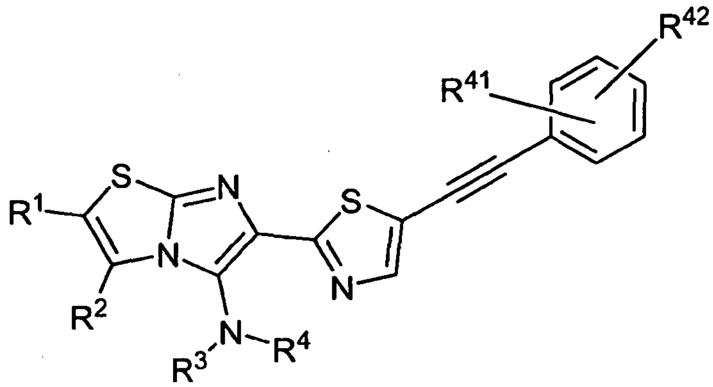

**Id,**

worin

$R^1$, $R^2$, $R^3$ und $R^4$ die Bedeutung gemäß Anspruch 4 haben,
und $R^{41}$ und $R^{42}$, unabhängig voneinander, jeweils für einen Rest ausgewählt aus der Gruppe bestehend aus H, F, Cl, Br, -CN, $-CF_3$, $-SF_5$, $-S-CH_3$, $-S-C_2H_5$, Methyl, Ethyl, n-Propyl, iso-Propyl, n-Butyl, sec-Butyl, iso-Butyl, tert-Butyl, Ethenyl, Propenyl, -OH, $-O-CH_3$, $-O-C_2H_5$, $-O-C_3H_7$, $-NH_2$, $-N(CH_3)_2$, $-NH-CH_3$, $-CH_2-NH_2$, $-N(C_2H_5)_2$, $-NO_2$, $-O-CF_3$, $-S-CF_3$, -SH, -C(=O)-H, $-C(=O)-O-CH_3$, - $C(=O)-O-C_2H_5$, Phenyl, Pyrrolyl, (1,3)-Dioxolanyl, $-Si(Phenyl)_2[C(CH_3)_3]$, - $C(=O)-CH_3$, $-C(=O)-C_2H_5$, $NH-S(=O)_2-CH_3$, $-NH-S(=O)_2-C_2Hs$, $-S(=O)_2-NH_2$, - $S(=O)_2-NH-CH_3$, $-CH_2-OH$, -C(=O)-OH, $-CH_2-O-CH_3$, $-C(=O)-NH_2$, $-C(=O)-NH-CH_3$, $-NH-C(=O)-CH_3$, -NH-

$C(=NH)-NH_2$, $-NH-S(=O)_2-OH$ und $-S(=O)_2-N(CH_3)_2$ stehen; jeweils ggf. in Form entsprechender Salze, oder jeweils ggf. in Form entsprechender Solvate.

**9.** Verbindungen der allgemeinen Formel Ie gemäß Anspruch 4,

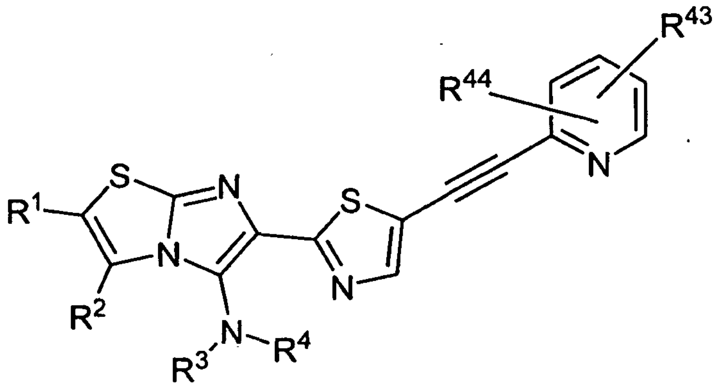

**Ie,**

worin

$R^1$, $R^2$, $R^3$ und $R^4$ die Bedeutung gemäß Anspruch 4 haben, und $R^{43}$ und $R^{44}$, unabhängig voneinander, jeweils für einen Rest ausgewählt aus der Gruppe bestehend aus H, F, Cl, Br, -CN, $-CF_3$, $-SF_5$, $-S-CH_3$, $-S-C_2H_5$, Methyl, Ethyl, n-Propyl, iso-Propyl, n-Butyl, sec-Butyl, iso-Butyl, tert-Butyl, Ethenyl, Propenyl, -OH, $-O-CH_3$, $-O-C_2H_5$, $-O-C_3H_7$, $-NH_2$, $-N(CH_3)_2$, $-NH-CH_3$, $-CH_2-NH_2$, $-N(C_2H_5)_2$, $-NO_2$, $-O-CF_3$, $-S-CF_3$, -SH, -C(=O)-H, $-C(=O)-O-CH_3$, - $C(=O)-O-C_2H_5$, Phenyl, Pyrrolyl, (1,3)-Dioxolanyl, $-Si(Phenyl)_2[C(CH_3)_3]$, - $C(=O)-CH_3$, $-C(=O)-C_2H_5$, $NH-S(=O)_2-CH_3$, $-NH-S(=O)_2-C_2H_5$, $-S(=O)_2-NH_2$,$-S(=O)_2-NH-CH_3$, $-CH_2-OH$, -C(=O)-OH, $-CH_2-O-CH_3$, $-C(=O)-NH_2$, $-C(=O)-NH-CH_3$, -NH-$C(=O)-CH_3$, $-NH-C(=NH)-NH_2$, $-NH-S(=O)_2-OH$ und $-S(=O)_2-N(CH_3)_2$ stehen;
jeweils ggf. in Form entsprechender Salze, oder jeweils ggf. in Form entsprechender Solvate.

**10.** Verbindungen der allgemeinen Formel If gemäß Anspruch 4,

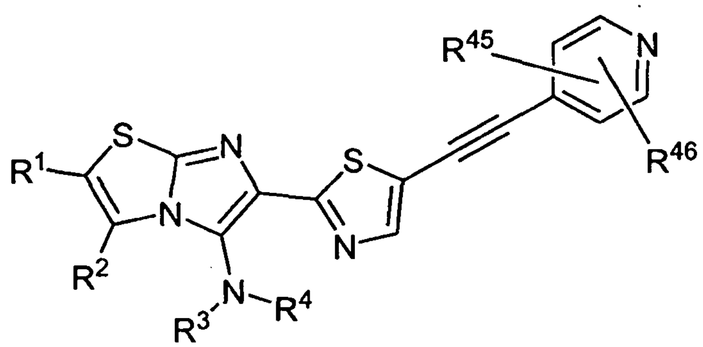

**If,**

worin

$R^1$, $R^2$, $R^3$ und $R^4$ die Bedeutung gemäß Anspruch 4 haben, und $R^{45}$ und $R^{46}$, unabhängig voneinander, jeweils für einen Rest ausgewählt aus der Gruppe bestehend aus H, F, Cl, Br, -CN, $-CF_3$, $-SF_5$, $-S-CH_3$, $-S-C_2H_5$, Methyl, Ethyl, n-Propyl, iso-Propyl, n-Butyl, sec-Butyl, iso-Butyl, tert-Butyl, Ethenyl, Propenyl, -OH, $-O-CH_3$, $-O-C_2H_5$, $-O-C_3H_7$, $-NH_2$, $-N(CH_3)_2$, $-NH-CH_3$, $-CH_2-NH_2$, $-N(C_2H_5)_2$,

$-NO_2$, $-O-CF_3$, $-S-CF_3$, -SH, -C(=O)-H, $-C(=O)-O-CH_3$, - $C(=O)-O-C_2H_5$, Phenyl, Pyrrolyl, (1,3)-Dioxolanyl, $-Si(Phenyl)_2[C(CH_3)_3]$, - $C(=O)-CH_3$, $-C(=O)-C_2H_5$, $NH-S(=O)_2-CH_3$, $-NH-S(=O)_2-C_2H_5$, $-S(=O)_2-NH_2$, - $S(=O)_2-NH-CH_3$, $-CH_2-OH$, -C(=O)-OH, $-CH_2-O-CH_3$, $-C(=O)-NH_2$, $-C(=O)-NH-CH_3$, $-NH-C(=O)-CH_3$, -NH-$C(=NH)-NH_2$, $-NH-S(=O)_2-OH$ und $-S(=O)_2-N(CH_3)_2$ stehen;
jeweils ggf. in Form entsprechender Salze, oder jeweils ggf. in Form entsprechender Solvate.

**11.** Verbindungen gemäß einem oder mehreren der Ansprüche 1 bis 10, **dadurch gekennzeichnet, dass**
$R^1$ für einen Wasserstoff-Rest; -F; -Cl; -Br; -CN; $-O-CH_3$; $-C(=O)-R^9$, $-C(=0)-OR^{13}$; $-(CH_2)-O-C(=O)-R^{16}$; $-(CH_2)-O-S(=O)_2-R^{31}$; $-(CH_2)-O-S(=O)_2-O-R^{32}$;$-(CH_2)-O-P(=O)(OR^{33})(OR^{34})$; oder für einen Rest ausgewählt aus der Gruppe bestehend aus Methyl, Ethyl, n-Propyl, Isopropyl, n-Butyl, Isobutyl, 2-Butyl, tert-Butyl, n-Pentyl, $-CF_3$, $-CF_2H$, $-CFH_2$, $-(CH_2)-(CF_3)$, $-(CH_2)-(CHF_2)$, $-(CH_2)-(CH_2F)$ und $-(CF_2)-(CF_3)$ steht;
$R^2$ für einen Wasserstoff-Rest; -F; -Cl; -Br; -CN; $-O-CH_3$; $-C(=O)-R^9$, $-C(=O)-OR^{13}$; $-(CH_2)-O-C(=O)-R^{16}$; $-(CH_2)-O-S(=O)_2-R^{31}$; $-(CH_2)-O-S(=O)_2-O-R^{32}$; - $(CH_2)-O-P(=O)(OR^{33})(OR^{34})$; oder für einen Rest ausgewählt aus der Gruppe bestehend aus Methyl, Ethyl, n-Propyl, Isopropyl, n-Butyl, Isobutyl, 2-Butyl, • tert-Butyl, n-Pentyl, $-CF_3$, $-CF_2H$, $-CFH_2$, $-(CH_2)-(CF_3)$, $-(CH_2)-(CHF_2)$, $-(CH_2)-(CH_2F)$ und $-(CF_2)-(CF_3)$ steht;
$R^3$ für einen Wasserstoff-Rest oder für einen Rest ausgewählt aus der Gruppe bestehend aus Methyl, Ethyl und Isopropyl steht;
$R^4$ für einen Wasserstoff-Rest; für $-C(=O)-R^{21}$ oder für einen Rest ausgewählt aus der Gruppe bestehend aus Methyl, Ethyl, n-Propyl, Isopropyl, n-Butyl, Isobutyl, 2-Butyl, tert-Butyl, n-Pentyl, 2-Pentyl, 3-Pentyl, iso-Pentyl, neo-Pentyl, n-Hexyl, n-Heptyl, n-Octyl und (2,4,4)-Trimethyl-pent-2-yl steht;
oder $R^3$ und $R^4$ zusammen mit dem sie verbindenden Stickstoffatom als Ringglied einen Rest ausgewählt aus der Gruppe bestehend aus Pyrrolidinyl, Piperidinyl und Morpholinyl bilden;
$R^{16}$ für einen Rest ausgewählt aus der Gruppe bestehend aus $-(CH_2)-(CH_2)-C(=O)-OH$; $-(CH_2)-(CH_2)-C(=O)-O-CH_3$ und $-(CH_2)-(CH_2)-C(=O)-O-C_2H_5$ steht;
$R^9$, $R^{13}$, $R^{31}$, $R^{32}$, $R^{33}$ und $R^{34}$, jeweils unabhängig voneinander, für einen Wasserstoff-Rest; oder für einen Rest ausgewählt aus der Gruppe bestehend aus Methyl, Ethyl, n-Propyl, Isopropyl, n-Butyl, Isobutyl, 2-Butyl, tert-Butyl, n-Pentyl, 2-Pentyl, 3-Pentyl, iso-Pentyl, neo-Pentyl, n-Hexyl, n-Heptyl, n-Octyl, (2,4,4)-Trimethyl-pent-2-yl, $-CF_3$, $-CF_2H$, $-CFH_2$, $-(CH_2)-(CF_3)$, $-(CH_2)-(CHF_2)$,$-(CH_2)-(CH_2F)$ und $-(CF_2)-(CF_3)$ stehen;
$R^{21}$ für einen Rest ausgewählt aus der Gruppe bestehend aus Methyl, Ethyl, n-Propyl, Isopropyl, n-Butyl, Isobutyl, 2-Butyl, tert-Butyl, n-Pentyl, 2-Pentyl, 3-Pentyl, iso-Pentyl, neo-Pentyl, n-Hexyl, n-Heptyl, n-Octyl, (2,4,4)-Trimethyl-pent-2-yl, $-CF_3$, $-CF_2H$, $-CFH_2$, $-(CH_2)-(CF_3)$, $-(CH_2)-(CHF_2)$, $-(CH_2)-(CH_2F)$ und $-(CF_2)-(CF_3)$ oder für einen Phenyl-Rest steht, der jeweils unsubstituiert oder mit ggf. 1, 2, 3, 4 oder 5 Substituenten unabhängig voneinander ausgewählt aus der Gruppe bestehend aus F, Cl, Br, I, -CN, $-NO_2$, Methyl, Ethyl, n-Propyl, Isopropyl, n-Butyl, Isobutyl, 2-Butyl, tert-Butyl,$-O-CH_3$ und $-O-C_2H_5$ substituiert sein kann;
$M^1$ für einen Rest ausgewählt aus der Gruppe bestehend aus den Resten 1, 3, 5, 22, 36 und 37,

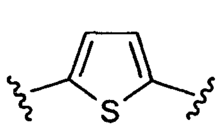
1

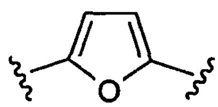
3

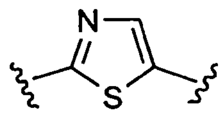
5

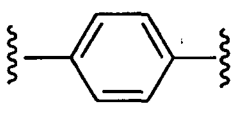
36

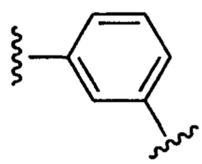
37

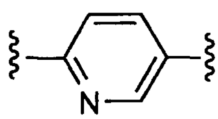
22

der jeweils unsubstituiert ist, und der jeweils in beliebiger Richtung über die mit einer geschlängelten Linie gekennzeichneten Positionen mit dem Bizyklus und dem Kohlenstoffatom der Dreifachbindung verknüpft sein kann;
$M^2$ für einen Rest ausgewählt aus der Gruppe bestehend aus Phenyl, 2-Pyrimidinyl, 5-Pyrimidinyl, 2-Thiophenyl (2-Thienyl), 3-Thiophenyl (3-Thienyl), 2-Pyridinyl, 3-Pyridinyl, 4-Pyridinyl, 2-Thiazolyl und 4-Thiazolyl steht, der jeweils

unsubstituiert oder mit ggf. 1, 2, 3, 4 oder 5 Substituenten unabhängig voneinander ausgewählt aus der Gruppe bestehend aus F, Cl, Br, -CN, -O-$CH_3$, -OH, -$CF_3$, Methyl, Ethyl, n-Propyl, iso-Propyl, n-Butyl, sec-Butyl, iso-Butyl und tert-Butyl substituiert sein kann;
jeweils ggf. in Form entsprechender Salze, oder jeweils in Form entsprechender Solvate.

**12.** Verbindungen gemäß einem oder mehreren der Ansprüche 1 bis 11, **dadurch gekennzeichnet, dass**
$R^1$ für einen Wasserstoff-Rest; -F; -Cl; -Br; -CN; -C(=O)-$OR^{13}$; oder für einen Rest ausgewählt aus der Gruppe bestehend aus Methyl, Ethyl, n-Propyl, Isopropyl, n-Butyl, Isobutyl, 2-Butyl, tert-Butyl und n-Pentyl steht;
$R^2$ für einen Wasserstoff-Rest; -F; -Cl; -Br; -CN; -C(=O)-$OR^{13}$; oder für einen Rest ausgewählt aus der Gruppe bestehend aus Methyl, Ethyl, n-Propyl, Isopropyl, n-Butyl, Isobutyl, 2-Butyl, tert-Butyl und n-Pentyl steht;
$R^3$ für einen Wasserstoff-Rest steht;
$R^4$ für einen Wasserstoff-Rest; für -C(=O)-$R^{21}$ oder für einen Rest ausgewählt aus der Gruppe bestehend aus Methyl, Ethyl, n-Propyl, Isopropyl, n-Butyl, Isobutyl, 2-Butyl, tert-Butyl, n-Pentyl und (2,4,4)-Trimethyl-pent-2-yl steht;
$R^{13}$ für einen Rest ausgewählt aus der Gruppe bestehend aus Methyl, Ethyl, n-Propyl, Isopropyl, n-Butyl, Isobutyl, 2-Butyl, tert-Butyl, n-Pentyl, 2-Pentyl, 3-Pentyl und (2,4,4)-Trimethyl-pent-2-yl steht;
$R^{21}$ für einen Rest ausgewählt aus der Gruppe bestehend aus Methyl, Ethyl, n-Propyl, Isopropyl, n-Butyl, Isobutyl, 2-Butyl, tert-Butyl, n-Pentyl und (2,4,4)-Trimethyl-pent-2-yl oder für einen Phenyl-Rest steht, der jeweils unsubstituiert ist;
$M^1$ für einen Rest ausgewählt aus der Gruppe bestehend aus den Resten 1, 3, 5, 22 und 36,

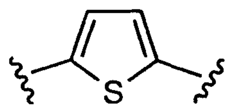

1

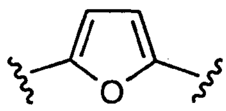

3

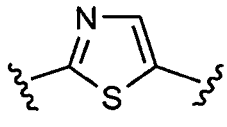

5

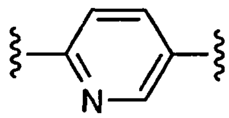

22

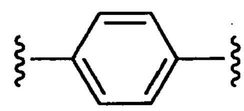

36

der jeweils unsubstituiert ist, und der jeweils in beliebiger Richtung über die mit einer geschlängelten Linie gekennzeichneten Positionen mit dem Bizyklus und dem Kohlenstoffatom der Dreifachbindung verknüpft sein kann;
und $M^2$ für einen Rest ausgewählt aus der Gruppe bestehend aus Phenyl, 2-Pyrimidinyl, 5-Pyrimidinyl, 2-Thiophenyl (2-Thienyl), 3-Thiophenyl (3-Thienyl), 2-Pyridinyl, 3-Pyridinyl, 4-Pyridinyl, 2-Thiazolyl und 4-Thiazolyl steht, der jeweils unsubstituiert oder mit ggf. 1, 2, 3, 4 oder 5 Substituenten unabhängig voneinander ausgewählt aus der Gruppe bestehend aus F, Cl, Br, -CN, -OH,-$CF_3$, Methyl, Ethyl, n-Propyl, iso-Propyl, n-Butyl, sec-Butyl, iso-Butyl und tert-Butyl substituiert sein kann;
jeweils ggf. in Form entsprechender Salze, oder jeweils in Form entsprechender Solvate.

**13.** Verbindungen gemäß einem oder mehreren der Ansprüche 1 bis 12 ausgewählt aus der Gruppe bestehend aus

[1] 6-(5-(Phenylethinyl)thiophen-2-yl)-N-(2,4,4-trimethylpentan-2-yl)-imidazo[2,1-b]thiazol-5-amin,
[2] N-tert-Butyl-6-(5-(pyridin-2-ylethinyl)thiophen-2-yl)-imidazo[2,1-b]thiazol-5-amin,
[3] N-tert-Butyl-3-methyl-6-(5-(pyridin-2-ylethinyl)thiophen-2-yl)-imidazo[2,1-b]thiazol-5-amin,
[4] N-tert-Butyl-2-methyl-6-(5-(pyridin-2-ylethinyl)thiophen-2-yl)-imidazo[2,1-b]thiazol-5-amin,
[5] N-tert-Butyl-2,3-dimethyl-6-(5-(pyridin-2-ylethinyl)thiophen-2-yl)-imidazo[2,1-b]thiazol-5-amin,
[6] N-tert-Butyl-2-chlor-6-(5-(pyridin-2-ylethinyl)thiophen-2-yl)-imidazo[2,1-b]thiazol-5-amin,
[7] N-tert-Butyl-6-(5-(pyridin-4-ylethinyl)thiophen-2-yl)imidazo[2,1-b]thiazol-5-amin,
[8] 5-(tert-Butylamino)-6-(5-(pyridin-2-ylethinyl)thiophen-2-yl)-imidazo[2,1-b]thiazol-2-carbonsäuremethylester,
[9] N-tert-Butyl-6-(5-(pyridin-2-ylethinyl)thiazol-2-yl)imidazo[2,1-b]thiazol-5-amin,
[10] 6-(5-Pyridin-2-ylethinyl)thiophen-2-yl)-N-(2,4,4-trimethylpentan-2-yl)imidazo[2,1-b]thiazol-5-amin,

[11] N-tert-Butyl-2-methyl-6-(4-(pyridin-2-ylethinyl)phenyl)imidazo[2,1-b]thiazol-5-amin,
[12] N-tert-Butyl-6-(5-(pyridin-2-ylethinyl)furan-2-yl)imidazo[2,1-b]thiazol-5-amin,
[13] N-tert-Butyl-3-methyl-6-(5-(phenylethinyl)thiophen-2-yl)imidazo[2,1-b]thiazol-5-amin,
[14] 6-(5-(Pyridin-2-ylethinyl)thiophen-2-yl)imidazo[2,1-b]thiazol-5-amin,
[15] N-tert-Butyl-6-(5-(pyrimidin-2-ylethinyl)thiophen-2-yl)imidazo[2,1-b]thiazol-5-amin,
[16] N-tert-Butyl-6-(5-((3-fluorpyridin-2-yl)ethinyl)thiophen-2-yl)imidazo[2,1-b]thiazol-5-amin,
[17] N-tert-Butyl-6-(5-((2-fluorpyridin-4-yl)ethinyl)thiophen-2-yl)imidazo[2,1-b]thiazol-5-amin,
[18] N-tert-Butyl-6-(5-(thiophen-2-ylethinyl)thiophen-2-yl)imidazo[2,1-b]thiazol-5-amin,
[19] N-tert-Butyl-6-(5-(thiazol-2-ylethinyl)thiophen-2-yl)imidazo[2,1-b]thiazol-5-amin,
[20] 3-((5-(5-(tert-Butylamino)imidazo[2,1-b]thiazol-6-yl)thiophen-2-yl)ethinyl)phenol,
[21] 3-((5-(5-(tert-Butylamino)imidazo[2,1-b]thiazol-6-yl)thiophen-2-yl)ethinyl)benzonitril,
[22] N-Ethyl-6-(6-(phenylethinyl)pyridin-3-yl)imidazo[2,1-b]thiazol-5-amin,
[23] N-tert-Butyl-6-(5-((3-methylpyridin-2-yl)ethinyl)thiophen-2-yl)imidazo[2,1-b]thiazol-5-amin,
[24] N-(6-(5-(Pyridin-2-ylethinyl)thiophen-2-yl)imidazo[2,1-b]thiazol-5-yl)acetamid und
[25] N-(6-(5-(Pyridin-2-ylethinyl)thiophen-2-yl)imidazo[2,1-b]thiazol-5-yl)benzamid;

jeweils ggf. in Form entsprechender Salze, insbesondere der Hydrochloride, oder jeweils in Form entsprechender Solvate.

**14.** Arzneimittel enthaltend wenigstens eine Verbindung gemäß einem oder mehreren der Ansprüche 1 bis 13 und ggf. einen oder mehrere physiologisch verträgliche Hilfsstoffe.

**15.** Verwendung wenigstens einer Verbindung gemäß einem oder mehreren der Ansprüche 1 bis 13 zur Herstellung eines Arzneimittels zur Behandlung und/oder Prophylaxe von Schmerz, ausgewählt aus der Gruppe bestehend aus akutem Schmerz, chronischem Schmerz, neuropathischem Schmerz und visceralem Schmerz; Migräne; Depressionen; neurodegenerativen Erkrankungen, ausgewählt aus der Gruppe bestehend aus Multipler Sklerose, Morbus Alzheimer, Morbus Parkinson und Morbus Huntington; kognitiven Erkrankungen, kognitiven Mangelzuständen, des Aufmerksamkeit-Defizit-Syndroms (ADS); psychiatrische Störungen, ausgewählt aus der Gruppe bestehend aus Angstzuständen und Panikattacken; Epilepsie; Husten; Haminkontinenz; Diarrhöe; Pruritus; Schizophrenie; cerebralen Ischämien; Muskelspasmen; Krämpfen; Lungenkrankheiten, ausgewählt aus der Gruppe bestehend aus Asthma und Pseudokrupp; Regurgitation (Erbrechen); Antriebslosigkeit; Schläfrigkeit; Lustlosigkeit; Kehlkopfentzündung (Laryngitis); Störungen der Nahrungsaufnahme, ausgewählt aus der Gruppe bestehend aus Bulimie, Kachexie, Anorexie und Fettleibigkeit; Alkoholabhängigkeit; Medikamentenabhängigkeit; Drogenabhängigkeit, vorzugsweise Nikotin- und/oder Kokainabhängigkeit; Alkoholmißbrauch; Medikamentenmißbrauch; Drogenmißbrauch; Nikotin- und/oder Kokainmißbrauch; Entzugserscheinungen bei Alkohol-, Medikamenten- und/oder Drogen- Nikotin- und/oder Kokain-)abhängigkeit; Toleranzentwicklung gegenüber Medikamenten, gegenüber natürlichen oder synthetischen Opioiden; Magen-Ösaphagus-Reflux-Syndrom; gastroösophageale Refluxkrankheit; Reizdarmsyndrom; zur Diurese; zur Antinatriurese; zur Beeinflussung des kardiovaskulären Systems; zur Vigilanzsteigerung; zur Libidosteigerung; zur Modulation der Bewegungsaktivität oder zur Lokalanästhesie.

## Claims

**1.** Substituted imidazo[2,1-b]thiazole compounds of the general formula I,

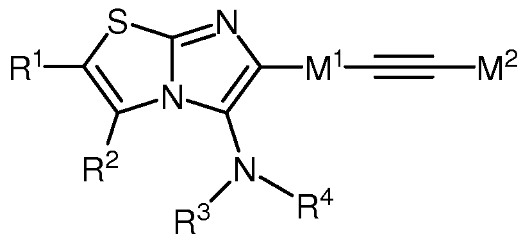

I,

in which

$R^1$ and $R^2$, independently of one another, respectively stand for a hydrogen residue; a halogen residue; $-NO_2$; -CN; $-NH_2$; $-NHR^5$; $-NR^6R^7$; $-NH-C(=O)-R^8$; $-C(=O)-R^9$,$-C(=O)-NH_2$; $-C(=O)-NHR^{10}$; $-C(=O)-NR^{11}R^{12}$; $-C(=O)-OR^{13}$; $-(CH_2)_m-C(=O)-OR^{14}$ with m = 1, 2, 3, 4 or 5; $-O-C(=O)-R^{15}$; $-(CH_2)_n-O-C(=O)-R^{16}$ with n = 1, 2, 3, 4 or 5; $-OR^{17}$; $-(CH_2)_o-O-R^{18}$ with o = 1, 2, 3; 4 or 5; $-SR^{19}$; $-(CH_2)_p-S(=O)_t-R^{20}$ with p = 1, 2, 3, 4 or 5 and t = 0, 1 or 2; $-NH-S(=O)_2-NR^{27}R^{28}$; $-S(=O)_2-NR^{29}R^{30}$; $-SF_5$; $-(CH_2)_u-O-S(=O)_2-R^{31}$ with u = 1, 2, 3, 4 or 5; $-(CH_2)_v-O-S(=O)_2-O-R^{32}$ with v = 1, 2, 3, 4 or 5; - $(CH_2)_w-O-P(=O)(OR^{33})(OR^{34})$ with w = 1, 2, 3, 4 or 5;
a linear or branched, saturated or unsaturated, unsubstituted or at least monosubstituted aliphatic residue;
a saturated or unsaturated, unsubstituted or at least monosubstituted cycloaliphatic residue optionally having at least one heteroatom as a ring member, which cycloaliphatic residue is bound via a linear or branched, unsubstituted or at least monosubstituted alkylene group and/or can be condensed with an unsubstituted or at least monosubstituted mono- or polycyclic ring system;
or an unsubstituted or at least monosubstituted aryl or heteroaryl residue, which can be bound via a linear or branched, unsubstituted or at least monosubstituted alkylene group and/or can be condensed with an unsubstituted or at least monosubstituted mono- or polycyclic ring system;
$R^3$ and $R^4$, independently of one another, respectively stand for a hydrogen residue;- $C(=O)-R^{21}$; $-(CH_2)_q-C(=O)-R^{22}$ with q = 1, 2, 3, 4 or 5; $-C(=O)-O-R^{23}$; $-(CH_2)_r-C(=O)-O-R^{24}$ with r = 1, 2, 3, 4 or 5; $-C(=O)-NHR^{25}$; $-(CH_2)_s-C(=O)-NHR^{26}$ with s = 1, 2, 3, 4 or 5; a linear or branched, saturated or unsaturated, unsubstituted or at least monosubstituted aliphatic residue;
a saturated or unsaturated, unsubstituted or at least monosubstituted cycloaliphatic residue optionally having at least one heteroatom as a ring member, which cycloaliphatic residue can be bound via a linear or branched, unsubstituted or at least monosubstituted alkylene group and/or can be condensed with an unsubstituted or at least monosubstituted mono- or polycyclic ring system; or an unsubstituted or at least monosubstituted aryl or heteroaryl residue, which can be bound via a linear or branched, unsubstituted or at least monosubstituted alkylene group and/or can be condensed with an unsubstituted or at least monosubstituted mono- or polycyclic ring system,
or $R^3$ and $R^4$ together with the nitrogen atom connecting them as a ring member form a saturated or unsaturated, unsubstituted or at least monosubstituted heterocycloaliphatic residue optionally having at least one further heteroatom as a ring member, which heterocycloaliphatic residue can be condensed with an unsubstituted or at least monosubstituted mono- or polycyclic ring system;
$R^5$, $R^6$, $R^7$, $R^8$, $R^{10}$, $R^{11}$, $R^{12}$, $R^{15}$ and $R^{16}$, respectively independently of one another, stand for a linear or branched, saturated or unsaturated, unsubstituted or at least monosubstituted aliphatic residue or an unsubstituted or at least monosubstituted aryl or heteroaryl residue, which can be bound via a linear or branched, unsubstituted or at least monosubstituted alkylene group and/or can be condensed with an unsubstituted or at least monosubstituted mono- or polycyclic ring system;
$R^9$, $R^{13}$, $R^{14}$, $R^{17}$, $R^{18}$, $R^{19}$, $R^{20}$, $R^{21}$, $R^{22}$, $R^{23}$, $R^{24}$, $R^{25}$, $R^{26}$, $R^{27}$, $R^{28}$, $R^{29}$, $R^{30}$, $R^{31}$, $R^{32}$, $R^{33}$ and $R^{34}$, respectively independently of one another, stand for a hydrogen residue; a linear or branched, saturated or unsaturated, unsubstituted or at least monosubstituted aliphatic residue or an unsubstituted or at least monosubstituted aryl or heteroaryl residue, which can be bound via a linear or branched, unsubstituted or at least monosubstituted alkylene group and/or can be condensed with an unsubstituted or at least monosubstituted mono- or polycyclic ring system;
$M^1$ stands for an aryl or heteroaryl residue, which can be substituted with at least one further substituent and/or can be condensed with an unsubstituted or at least monosubstituted mono- or polycyclic ring system;
and $M^2$ stands for an aryl or heteroaryl residue, which can be unsubstituted or at least monosubstituted and can be condensed with an unsubstituted or at least monosubstituted mono- or polycyclic ring system;
in each case in the form of one of the pure stereoisomers thereof, in particular enantiomers or diastereomers, the racemates thereof or in the form of a mixture of stereoisomers, in particular the enantiomers and/or diastereomers, in any desired mixing ratio, or in each case in the form of corresponding salts or in each case in the form of corresponding solvates.

**2.** Compounds according to claim 1, **characterised in that**
$R^1$ and $R^2$, independently of one another, respectively stand for a hydrogen residue; a halogen residue; $-NO_2$; -CN; $-NH_2$; $-NHR^5$; $-NR^6R^7$; $-NH-C(=O)-R^8$; $-C(=O)-R^9$,$-C(=O)-NH_2$; $-C(=O)-NHR^{10}$; $-C(=O)-NR^{11}R^{12}$; $-C(=O)-OR^{13}$; $-(CH_2)_m-C(=O)-OR^{14}$ with m = 1, 2, 3, 4 or 5; $-O-C(=O)-R^{15}$; $-(CH_2)_n-O-C(=O)-R^{16}$ with n = 1, 2, 3, 4 or 5; $-OR^{17}$; $-(CH_2)_o-O-R^{18}$ with o = 1, 2, 3; 4 or 5; $-SR^{19}$; $-(CH_2)_p-S(=O)_t-R^{20}$ with p = 1, 2, 3, 4 or 5 and t = 0, 1 or 2; $-NH-S(=O)_2-NR^{27}R^{28}$; $-S(=O)_2-NR^{29}R^{30}$; $-SF_5$; $-(CH_2)_u-O-S(=O)_2-R^{31}$ with u = 1, 2, 3, 4 or 5; $-(CH_2)_v-O-S(=O)_2-O-R^{32}$ with v = 1, 2, 3, 4 or 5; - $(CH_2)_w-O-P(=O)(OR^{33})(OR^{34})$ with w = 1, 2, 3, 4 or 5; a linear or branched, unsubstituted or at least monosubstituted $C_{1-10}$-alkyl residue, $C_{2-6}$-alkenyl residue or $C_{2-6}$-alkynyl residue;
a saturated or unsaturated, unsubstituted or at least monosubstituted cycloaliphatic $C_{3-8}$ residue optionally having

at least one heteroatom as a ring member, which residue can be bound via a linear or branched, unsubstituted or at least monosubstituted $C_{1-5}$-alkylene group;
or an unsubstituted or at least monosubstituted 5- or 6-membered aryl or heteroaryl residue, which can be bound via a linear or branched, unsubstituted or at least monosubstituted $C_{1-5}$-alkylene group and/or can be condensed with an unsubstituted or at least monosubstituted mono- or polycyclic ring system, wherein the rings of the ring system are in each case 5-, 6- or 7-membered;
$R^3$ and $R^4$, independently of one another, respectively stand for a hydrogen residue; -C(=O)-$R^{21}$; -$(CH_2)_q$-C(=O)-$R^{22}$ with q = 1, 2, 3, 4 or 5; -C(=O)-O-$R^{23}$; -$(CH_2)_r$-C(=O)-O-$R^{24}$ with r = 1, 2, 3, 4 or 5; -C(=O)-NH$R^{25}$; -$(CH_2)_s$-C(=O)-NH$R^{26}$ with s = 1, 2, 3, 4 or 5; a linear or branched, unsubstituted or at least monosubstituted $C_{1-10}$-alkyl residue, $C_{2-6}$-alkenyl residue or $C_{2-6}$-alkynyl residue;
a saturated or unsaturated, unsubstituted or at least monosubstituted cycloaliphatic $C_{3-8}$ residue optionally having at least one heteroatom as a ring member, which residue can be bound via a linear or branched, unsubstituted or at least monosubstituted $C_{1-5}$-alkylene group; or an unsubstituted or at least monosubstituted 5- or 6-membered aryl or heteroaryl residue, which can be bound via a linear or branched, unsubstituted or at least monosubstituted $C_{1-5}$-alkylene group and/or can be condensed with an unsubstituted or at least monosubstituted mono- or polycyclic ring system, wherein the rings of the ring system are in each case 5-, 6- or 7-membered,
or $R^3$ and $R^4$ together with the nitrogen atom connecting them as a ring member form a saturated or unsaturated, unsubstituted or at least monosubstituted heterocycloaliphatic $C_{4-10}$ residue optionally having at least one further heteroatom as a ring member, which residue can be condensed with an unsubstituted or at least monosubstituted mono- or polycyclic ring system, wherein the rings of the ring system are in each case 5-, 6- or 7-membered;
$R^5$, $R^6$, $R^7$, $R^8$, $R^{10}$, $R^{11}$, $R^{12}$, $R^{15}$ and $R^{16}$, respectively independently of one another, stand for a linear or branched, unsubstituted or at least monosubstituted $C_{1-10}$-alkyl residue, $C_{2-6}$-alkenyl residue or $C_{2-6}$-alkynyl residue; or an unsubstituted or at least monosubstituted 5- to 14-membered aryl or heteroaryl residue, which can be bound via a linear or branched, unsubstituted or at least monosubstituted $C_{1-5}$-alkylene group and/or can be condensed with an unsubstituted or at least monosubstituted mono- or polycyclic ring system, wherein the rings of the ring system are in each case 5-, 6- or 7-membered;
$R^9$, $R^{13}$, $R^{14}$, $R^{17}$, $R^{18}$, $R^{19}$, $R^{20}$, $R^{21}$, $R^{22}$, $R^{23}$, $R^{24}$, $R^{25}$, $R^{26}$, $R^{27}$, $R^{28}$, $R^{29}$, $R^{30}$, $R^{31}$, $R^{32}$, $R^{33}$ and $R^{34}$, respectively independently of one another, stand for a hydrogen residue; a linear or branched, unsubstituted or at least monosubstituted $C_{1-10}$-alkyl residue, $C_{2-6}$-alkenyl residue or $C_{2-6}$-alkynyl residue; or an unsubstituted or at least monosubstituted 5- to 14-membered aryl or heteroaryl residue, which can be bound via a linear or branched, unsubstituted or at least monosubstituted $C_{1-5}$-alkylene group and/or can be condensed with an unsubstituted or at least monosubstituted mono- or polycyclic ring system, wherein the rings of the ring system are in each case 5-, 6- or 7-membered;
$M^1$ stands for a 5- or 6-membered aryl or heteroaryl residue, which can be substituted with at least one further substituent and can be condensed with an unsubstituted or at least monosubstituted mono- or bicyclic ring system, wherein the rings of the ring system are in each case 5-, 6- or 7-membered, and
and $M^2$ stands for a 5- or 6-membered aryl or heteroaryl residue, which can be unsubstituted or at least monosubstituted and can be condensed with an unsubstituted or at least monosubstituted mono- or bicyclic ring system, wherein the rings of the ring system are in each case 5-, 6- or 7-membered;
wherein
the above-mentioned cycloaliphatic residues can optionally have 1, 2, 3, 4 or 5 heteroatom(s) as ring member(s) which respectively independently of one another can be selected from the group comprising nitrogen, oxygen and sulphur,
the above-mentioned heterocycloaliphatic residues can optionally have further 1, 2, 3, 4 or 5 heteroatom(s) as (the) ring member(s) which can be selected respectively independently of one another from the group comprising nitrogen, oxygen and sulphur,
the rings of the mono- or polycyclic ring system have in each case optionally 0, 1, 2 or 3 heteroatom(s) as (the) ring member(s) which are selected respectively independently from the group comprising oxygen, nitrogen and sulphur; and
the above-mentioned heteroaryl residues can optionally have 1, 2, 3, 4 or 5 heteroatom(s) as (the) ring member(s) which respectively independently of one another can be selected from the group comprising oxygen, sulphur and nitrogen.

3. Compounds according to claim 1 or 2, **characterised in that**
$R^1$ and $R^2$, independently of one another, respectively stand for a hydrogen residue; - F; -Cl; -Br; -I; -NO; -CN; -$NH_2$; -NH$R^5$; -N$R^6R^7$; -NH-C(=O)-$R^8$; -C(=O)-$R^9$, - C(=O)-$NH_2$; -C(=O)-NH$R^{10}$; -C(=O)-N$R^{11}R^{12}$; -C(=O)-O$R^{13}$; -$(CH_2)_m$-C(=O)-O$R^{14}$ with m = 1, 2 or 3; -O-C(=O)-$R^{15}$; -O$R^{17}$; -$(CH_2)_o$-O-$R^{18}$ with o = 1, 2 or 3; -S$(=O)_2$-$NH_2$; -$SF_5$; -$(CH_2)_u$-O-S$(=O)_2$-$R^{31}$ with u = 1, 2 or 3; -$(CH_2)_v$-O-S$(=O)_2$-O-$R^{32}$ with v = 1, 2 or 3; -$(CH_2)_w$-O-P(=O)(O$R^{33}$)(O$R^{34}$)

with w = 1, 2 or 3; a residue selected from the group comprising methyl, ethyl, n-propyl, isopropyl, n-butyl, isobutyl, 2-butyl, tert-butyl, n-pentyl, 2-pentyl, 3-pentyl, iso-pentyl, neo-pentyl, n-hexyl, n-heptyl, n-octyl, (2,4,4)-trimethyl-pent-2-yl, $-CF_3$, -CFH, $-CFH_2$, $-(CH_2)-(CF_3)$, $-(CH_2)-(CHF_2)$, $-(CH_2)-(CH_2F)$ and $-(CF_2)-(CF_3)$; a residue selected from the group comprising cyclopropyl, cyclobutyl, cyclopentyl, cyclohexyl, cycloheptyl and cyclooctyl, which in each case can be unsubstituted or substituted with optionally 1, 2, 3 or 4 substituents independently selected from the group comprising F, Cl, Br, I, -CN, - $NO_2$, methyl, ethyl, n-propyl, isopropyl, n-butyl, isobutyl, 2-butyl, tert-butyl, n-pentyl, neo-pentyl, -OH, oxo (=O), thioxo (=S), $-O-CH_3$, $-O-C_2H_5$, $-O-C_3H_7$, $-O-C(CH_3)_3$,$-CF_3$, $-CHF_2$, -CHF, $-O-CF_3$, $-O-CHF_2$ and $-O-CH-_2F$;

or a residue selected from the group comprising phenyl, benzyl, phenethyl, (3-phenyl)-prop-1-yl, furyl (furanyl), thienyl (thiophenyl), pyrazolyl, imidazolyl, thiazolyl, thiadiazolyl, pyridinyl, pyridazinyl, pyrimidinyl and pyrazinyl, which can in each case be unsubstituted or substituted with optionally 1, 2, 3, 4 or 5 substituents selected respectively independently from the group comprising F, Cl, Br, I, -CN, - $NO_2$, methyl, ethyl, n-propyl, isopropyl, n-butyl, isobutyl, 2-butyl, tert-butyl, n-pentyl, neo-pentyl, ethenyl, allyl, ethynyl, propynyl, -OH, -SH, $-SF_5$, $-NH_2$, -C(=O)-OH, $-S-CH_3$, $-S-C_2H_5$, $-O-CH_3$, $-O-C_2H_5$, $-O-C_3H_7$, $-O-C(CH_3)_3$, $-CF_3$, $-CHF_2$, $-CH_2F$, $-O-CF_3$,- $O-CHF_2$, $-O-CH_2F$, $-C(=O)-CF_3$, $-S-CF_3$, $-S-CHF_2$, $-S-CH_2F$, $-C(=O)-O-CH_3$, - $C(=O)-O-C_2H_5$, $-C(=O)-O-C(CH_3)_3$, cyclopropyl, cyclobutyl, cyclopentyl, cyclohexyl and phenyl;

$R^3$ and $R^4$, independently of one another, respectively stand for a hydrogen residue; $-C(=O)-R^{21}$; $-(CH_2)_q-C(=O)-R^{22}$ with q = 1, 2 or 3; -C(=O)-O-R $^{23}$; $-(CH_2)_r-C(=O)-OR^{24}$ with r = 1, 2 or 3; $-C(=O)-NHR^{25}$; $-(CH_2)_s-C(=O)-NHR^{26}$ with s = 1, 2 or 3; a residue selected from the group comprising methyl, ethyl, n-propyl, isopropyl, n-butyl, isobutyl, 2-butyl, tert-butyl, n-pentyl, 2-pentyl, 3-pentyl, iso-pentyl, neo-pentyl, n-hexyl, n-heptyl, n-octyl and (2,4,4)-trimethyl-pent-2-yl; a residue selected from the group comprising cyclopropyl, cyclobutyl, cyclopentyl, cyclohexyl, cycloheptyl and cyclooctyl, which in each case can be unsubstituted or substituted with optionally 1, 2, 3 or 4 substituents selected respectively independently from the group comprising F, Cl, Br, I, -CN, $-NO_2$, methyl, ethyl, n-propyl, isopropyl, n-butyl, isobutyl, 2-butyl, tert-butyl, n-pentyl, -OH, oxo (=O), thioxo (=S), $-O-CH_3$, $-O-C_2H_5$, $-O-C_3H_7$ and $-O-C(CH_3)_3$ and/or can be bound via a linear or branched $C_{1-3}$-alkylene group; or a residue selected from the group comprising phenyl, furyl (furanyl), thienyl (thiophenyl), pyrazolyl, imidazolyl, thiazolyl, thiadiazolyl, pyridinyl, pyridazinyl, pyrimidinyl and pyrazinyl, which in each case can be unsubstituted or substituted with optionally 1, 2, 3, 4 or 5 substituents selected respectively independently from the group comprising F, Cl, Br, I, -CN, $-NO_2$, methyl, ethyl, n-propyl, isopropyl, n-butyl, isobutyl, 2-butyl, tert-butyl, n-pentyl, neo-pentyl, ethenyl, allyl, -OH, -SH, $-SF_5$,$-NH_2$, -C(=O)-OH, $-S-CH_3$, $-S-C_2H_5$, $-O-CH_3$, $-O-C_2H_5$, $-O-C_3H_7$, $-O-C(CH_3)_3$, $-CF_3$,$-CHF_2$, $-CH_2F$, $-O-CF_3$, $-O-CHF_2$, $-O-CH_2F$, $-C(=O)-CF_3$, $-S-CF_3$, $-S-CHF_2$, $-S-CH_2F$,$--N(CH_3)_2$, $-N(C_2H_5)_2$, $-NH-CH_3$, $-NH-C_2H_5$, cyclopropyl, cyclobutyl and cyclopentyl and/or can be bound via a linear or branched $C_{1-3}$-alkylene group;

or $R^3$ and $R^4$ together with the nitrogen atom connecting them as a ring member form a residue selected from the group comprising imidazolidinyl, piperidinyl, piperazinyl, morpholinyl, pyrrolidinyl, thiomorpholinyl, azepanyl, diazepanyl and azocanyl, which in each case can be unsubstituted or substituted with optionally 1, 2, 3, 4 or 5 substituents selected respectively independently from the group comprising F, Cl, Br, I, -CN, methyl, ethyl, n-propyl, isopropyl, n-butyl, 2-butyl, isobutyl, tert-butyl, -OH, oxo, thioxo, $-O-CH_3$, $-O-C_2H_5$, $-O-C_3H_7$, $-NH_2$, $-N(CH_3)_2$, $-N(C_2H_5)_2$, $-NH-CH_3$, $-NH-C_2H_5$,- $NO_2$, $-CF_3$, $-O-CF_3$, $-S-CF_3$, -SH, $-SF_5$, $-S-CH_3$, $-S-C_2H_5$, $-S(=O)-CH_3$,$-S(=O)_2-CH_3$, $-S(=O)-C_2H_5$, $-S(=O)_2-C_2H_5$, $-C(=O)-CH_3$, $-C(=O)-C_2H_5$, $-C(=O)-N(CH_3)_2$, $-C(=O)-NH-CH_3$, $-C(=O)-NH_2$, $-C(=O)-O-CH_3$, $-C(=O)-O-C_2H_5$, $-C(=O)-O-C(CH_3)_3$ and phenyl;

$R^5$, $R^6$, $R^7$, $R^{10}$, $R^{11}$, $R^{12}$ and $R^{15}$, respectively independently of one another, stand for a residue selected from the group comprising methyl, ethyl, n-propyl, isopropyl, n-butyl, isobutyl, 2-butyl, tert-butyl, n-pentyl, 2-pentyl, 3-pentyl, iso-pentyl, neo-pentyl, n-hexyl, n-heptyl, n-octyl, (2,4,4)-trimethyl-pent-2-yl, $-CF_3$, -CFH, $-CFH_2$, $-(CH_2)-(CHF_2)$, $-(CH_2)-(CH_2F)$, $-(CF_2)-(CF_3)$, $-(CH_2)-(CH_2)-C(=O)-OH$, $-(CH_2)-(CH_2)-C(=O)-O-CH_3$ and $-(CH_2)-(CH_2)-C(=O)-O-C_2H_5$; or a residue selected from the group comprising phenyl, benzyl, phenethyl, furyl (furanyl), thienyl (thiophenyl), pyrazolyl, imidazolyl, thiazolyl, thiadiazolyl, pyridinyl, pyridazinyl, pyrimidinyl and pyrazinyl, which can in each case be unsubstituted or substituted with optionally 1, 2, 3, 4 or 5 substituents selected respectively independently from the group comprising F, Cl, Br, I, -CN, $-NO_2$, methyl, ethyl, n-propyl, isopropyl, n-butyl, isobutyl, 2-butyl, tert-butyl, n-pentyl, neo-pentyl, -OH, -SH, $-SF_5$, $-NH_2$, -C(=O)-OH, $-S-CH_3$, $-S-C_2H_5$, $-O-CH_3$, - $O-C_2H_5$, $-O-C_3H_7$, $-O-C(CH_3)_3$, $-CF_3$, $-CHF_2$, -CHF, $-O-CF_3$, $-O-CHF_2$, $-O-CH_2F$ and $-C(=O)-CF_3$;

$R^9$, $R^{13}$, $R^{14}$, $R^{17}$, $R^{18}$, $R^{21}$, $R^{22}$, $R^{23}$, $R^{24}$, $R^{25}$, $R^{26}$, $R^{31}$, $R^{32}$, $R^{33}$ and $R^{34}$, respectively independently of one another, stand for a hydrogen residue; a residue selected from the group comprising methyl, ethyl, n-propyl, isopropyl, n-butyl, isobutyl, 2-butyl, tert-butyl, n-pentyl, 2-pentyl, 3-pentyl, iso-pentyl, neo-pentyl, n-hexyl, n-heptyl, n-octyl, (2,4,4)-trimethyl-pent-2-yl, $-CF_3$, -CFH, $-CFH_2$, $-(CH_2)-(CF_3)$, $-(CH_2)-(CHF_2)$, $-(CH_2)-(CH_2F)$ and $-(CF_2)-(CF_3)$; or a residue selected from the group comprising phenyl, benzyl, phenethyl, furyl (furanyl), thienyl (thiophenyl), pyrazolyl, imidazolyl, thiazolyl, thiadiazolyl, pyridinyl, pyridazinyl, pyrimidinyl and pyrazinyl, which can in each case be unsubstituted or substituted with optionally 1, 2, 3, 4 or 5 substituents selected respectively independently from the group comprising F, Cl, Br, I, -CN, - $NO_2$, methyl, ethyl, n-propyl, isopropyl, n-butyl, isobutyl, 2-butyl, tert-butyl, n-pentyl,

neo-pentyl, -OH, -SH, $-SF_5$, $-NH_2$, -C(=O)-OH, $-S-CH_3$, $-S-C_2H_5$, $-O-CH_3$, $-O-C_2H_5$,$-O-C_3H_7$, $-O-C(CH_3)_3$, $-CF_3$, $-CHF_2$, -CHF, $-O-CF_3$, $-O-CHF_2$, $-O-CH_2F$ and $-C(=O)-CF_3$;

$M^1$ stands for a residue selected from the group comprising residues 1 to 9, 11, 21, 22 and 36 to 38,

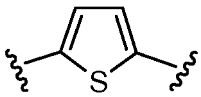
1

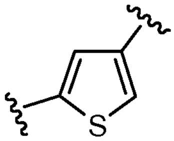
2

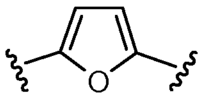
3

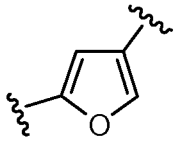
4

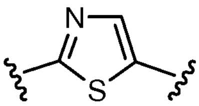
5

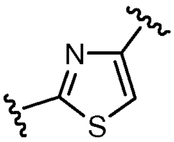
6

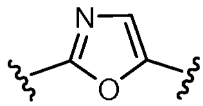
7

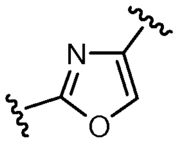
8

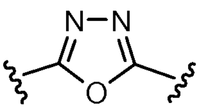
9

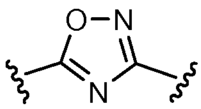
11

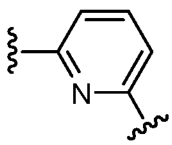
21

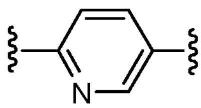
22

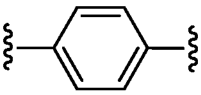
36

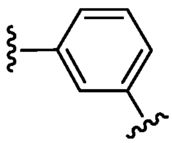
37

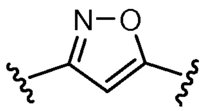
38

which in each case can be unsubstituted or substituted with optionally 1 or 2 substituents selected respectively independently from the group comprising F, Cl, Br, I, -CN, $-NO_2$, methyl, ethyl, n-propyl, isopropyl, n-butyl, isobutyl, 2-butyl, tert-butyl, n-pentyl, neo-pentyl, ethenyl, allyl, ethynyl, propynyl, -OH, -SH, $-SF_5$, $-NH_2$, -C(=O)-OH, $-S-CH_3$, $-S-C_2H_5$, $-S(=O)-CH_3$, $-S(=O)_2-CH_3$, $-S(=O)-C_2H_5$, $-S(=O)_2-C_2H_5$, $-O-CH_3$, $-O-C_2H_5$, $-O-C_3H_7$, $-O-C(CH_3)_3$, $-CF_3$, $-CHF_2$, $-CH_2F$, $-O-CF_3$, $-O-CHF_2$, $-O-CH_2F$, $-C(=O)-CF_3$, $-S-CF_3$, $-S-CHF_2$ and $-S-CH_2F$, and which in each case can be linked in any direction via the positions marked by a wavy line with the bicycle and the carbon atom of the triple bond;

and $M^2$ stands for a residue selected from the group comprising residues 1 to 36,

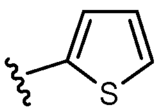
1

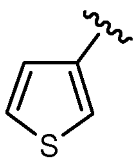
2

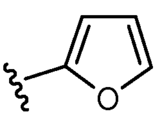
3

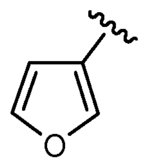
4

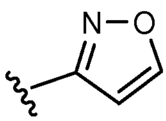
5

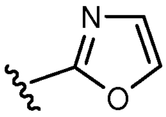 6
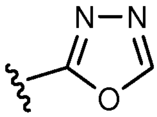 7
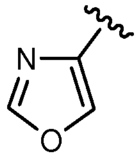 8
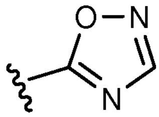 9
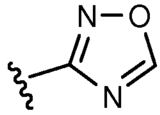 10

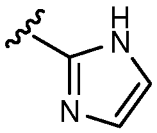 11
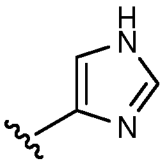 12
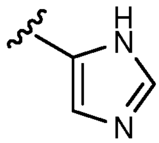 13
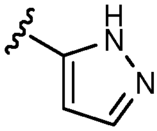 14
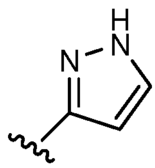 15

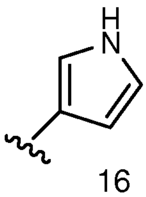 16
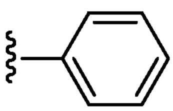 17
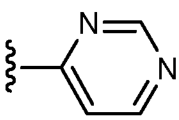 18
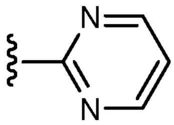 19
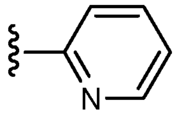 20

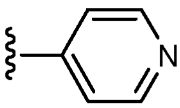 21
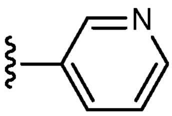 22
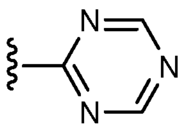 23
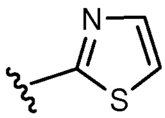 24
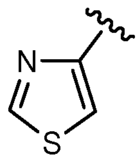 25

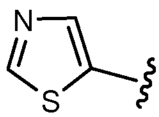 26
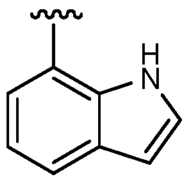 27
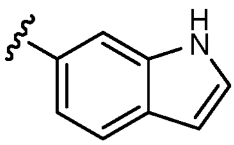 28
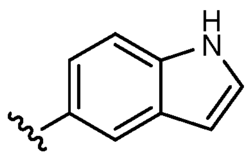 29

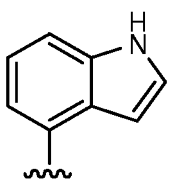 30
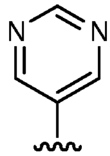 31
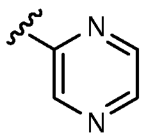 32
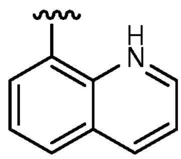 33

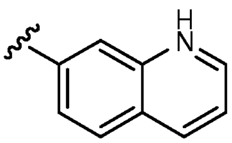

34

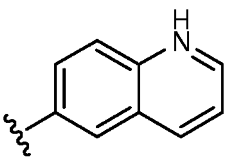

35

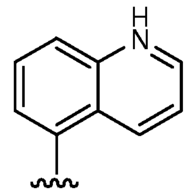

36

which in each case can be linked via the position marked by a wavy line with the carbon atom of the triple bond and can be unsubstituted or substituted with optionally 1, 2, 3, 4 or 5 substituents selected respectively independently from the group comprising F, Cl, Br, -CN, $-CF_3$, $-SF_5$, $-S-CH_3$, $-S-C_2H_5$, methyl, ethyl, n-propyl, iso-propyl, n-butyl, sec-butyl, iso-butyl, tert-butyl, ethenyl, propenyl, -OH, $-O-CH_3$, $-O-C_2H_5$, $-O-C_3H_7$, $-NH_2$, $-N(CH_3)_2$, $-NH-CH_3$, $-CH_2-NH_2$, $-N(C_2H_5)_2$, $-NO_2$, $-O-CF_3$, $-S-CF_3$, -SH, -C(=O)-H, $-C(=O)-O-CH_3$, $-C(=O)-O-C_2H_5$, phenyl, pyrrolyl, (1,3)-dioxolanyl, $-Si(phenyl)_2[C(CH_3)_3]$, $-C(=O)-CH_3$, $-C(=O)-C_2H_5$, $NH-S(=O)_2-CH_3$,$-NH-S(=O)_2-C_2H_5$, $-S(=O)_2-NH_2$, $-S(=O)_2-NH-CH_3$, $-CH_2-OH$, -C(=O)-OH, $-CH_2-O-CH_3$, $-C(=O)-NH_2$, $-C(=O)-NH-CH_3$, $-NH-C(=O)-CH_3$, $-NH-C(=NH)-NH_2$, $-NH-S(=O)_2-OH$ and $-S(=O)_2-N(CH_3)_2$;

in each case optionally in the form of one of the pure stereoisomers thereof, in particular enantiomers or diastereomers, the racemates thereof or in the form of a mixture of stereoisomers, in particular the enantiomers and/or diastereomers, in any desired mixing ratio, or in each case in the form of corresponding salts or in each case in the form of corresponding solvates.

**4.** Compounds according to one or more of claims 1 to 3, **characterised in that**

$R^1$ and $R^2$, independently of one another, respectively stand for a hydrogen residue; - F; -Cl; -Br; -I; $-NO_2$; -CN; $-NHR^5$; $-NR^6R^7$; $-C(=O)-R^9$, $-C(=O)-OR^{13}$; $-(CH_2)-O-C(=O)-R^{16}$; $-OR^{17}$; $-(CH_2)-O-S(=O)_2-R^{31}$; $-(CH_2)-O-S(=O)_2-O-R^{32}$; $-(CH_2)-O-P(=O)(OR^{33})(OR^{34})$; $-SF_5$; a residue selected from the group comprising methyl, ethyl, n-propyl, isopropyl, n-butyl, isobutyl, 2-butyl, tert-butyl, n-pentyl, $-CF_3$, $-CF_2H$,$-CFH_2$, $-(CH_2)-(CF_3)$, $-(CH_2)-(CHF_2)$, $-(CH_2)-(CH_2F)$ and $-(CF_2)-(CF_3)$; a residue selected from the group comprising cyclopropyl, cyclobutyl, cyclopentyl, cyclohexyl, cycloheptyl and cyclooctyl;

or a residue selected from the group comprising phenyl, benzyl, phenethyl and (3-phenyl)-prop-1-yl, which in each case can be unsubstituted or substituted with optionally 1, 2, 3, 4 or 5 substituents selected respectively independently from the group comprising F, Cl, Br, I, -CN, $-NO_2$, methyl, ethyl, n-propyl, isopropyl, n-butyl, isobutyl, 2-butyl, tert-butyl, $-O-CH_3$, $-O-C_2H_5$, $-O-C_3H_7$, $-O-C(CH_3)_3$, $-CF_3$, $-CHF_2$,$-CH_2F$, $-O-CF_3$, $-O-CHF_2$ and $-O-CH_2F$;

$R^3$ and $R^4$, independently of one another, respectively stand for a hydrogen residue; $-C(=O)-R^{21}$; $-(CH_2)_q-C(=O)-R^{22}$ with q = 1, 2 or 3; $-(CH_2)_r-C(=O)-O-R^{24}$ with r = 1, 2 or 3; a residue selected from the group comprising methyl, ethyl, n-propyl, isopropyl, n-butyl, isobutyl, 2-butyl, tert-butyl, n-pentyl, 2-pentyl, 3-pentyl, iso-pentyl, neo-pentyl, n-hexyl, n-heptyl, n-octyl and (2,4,4)-trimethyl-pent-2-yl; a residue selected from the group comprising cyclopropyl, cyclobutyl, cyclopentyl, cyclohexyl, cycloheptyl and cyclooctyl, which can be bound via a $-(CH_2)-$, $-(CH_2)_2-$, $-(CH(CH_3))-$or $-(CH_2)_3$ group;

or a residue selected from the group comprising phenyl, furyl (furanyl), thienyl (thiophenyl), pyridinyl, pyridazinyl, pyrimidinyl and pyrazinyl, which in each case can be unsubstituted or substituted with optionally 1, 2, 3, 4 or 5 substituents selected respectively independently from the group comprising F, Cl, Br, I, -CN, $-NO_2$, methyl, ethyl, n-propyl, isopropyl, n-butyl, isobutyl, 2-butyl, tert-butyl, $-O-CH_3$, $-O-C_2H_5$, $-O-C_3H_7$, $-O-C(CH_3)_3$, $-CF_3$, $-CHF_2$, -CHF, $-O-CF_3$, $-O-CHF_2$ and $-O-CH_2F$ and/or can be bound via a $-(CH_2)-$, $-(CH_2)_2-$, $-(CH(CH_3))-$ or $-(CH_2)_3$ group;

or $R^3$ and $R^4$ together with the nitrogen atom connecting them as a ring member form a residue selected from the group comprising piperidinyl, piperazinyl, morpholinyl and pyrrolidinyl, which in each case can be unsubstituted or substituted with optionally 1, 2, 3, 4 or 5 substituents selected respectively independently from the group comprising methyl, ethyl, n-propyl, isopropyl, n-butyl, 2-butyl, isobutyl, tert-butyl, $-S(=O)-CH_3$, $-S(=O)_2-CH_3$, $-S(=O)-C_2H_5$, $-S(=O)_2-C_2H_5$, $-C(=O)-CH_3$, $-C(=O)-C_2H_5$, $-C(=O)-N(CH_3)_2$, $-C(=O)-NH-CH_3$, $-C(=O)-NH_2$, $-C(=O)-O-CH_3$, $-C(=O)-O-C_2H_5$ and $-C(=O)-O-C(CH_3)_3$;

$R^5$, $R^6$, $R^7$ and $R^{16}$, respectively independently of one another, stand for a residue selected from the group comprising methyl, ethyl, n-propyl, isopropyl, n-butyl, isobutyl, 2-butyl, tert-butyl, n-pentyl, 2-pentyl, 3-pentyl, iso-pentyl, neo-pentyl, n-hexyl, n-heptyl, n-octyl, (2,4,4)-trimethyl-pent-2-yl, $-CF_3$, -CFH, $-CFH_2$, $-(CH_2)-(CHF_2)$, $-(CH_2)-(CH_2F)$, $-(CF_2)-(CF_3)$, $-(CH_2)-(CH_2)-C(=O)-OH$; $-(CH_2)-(CH_2)-C(=O)-O-CH_3$ and $-(CH_2)-(CH_2)-C(=O)-O-C_2H_5$; or a residue selected from the group comprising phenyl, benzyl and phenethyl, which in each case can be unsubstituted or substituted with optionally 1, 2, 3, 4 or 5 substituents selected respectively independently from the group comprising F, Cl, Br, I, -CN, $-NO_2$, methyl, ethyl, n-propyl, isopropyl, n-butyl, isobutyl, 2-butyl, tert-butyl, $-O-CH_3$ and $-O-C_2H_5$;

$R^9$, $R^{13}$, $R^{17}$, $R^{21}$, $R^{22}$, $R^{24}$, $R^{31}$, $R^{32}$, $R^{33}$ and $R^{34}$, respectively independently of one another, stand for a hydrogen residue; a residue selected from the group comprising methyl, ethyl, n-propyl, isopropyl, n-butyl, isobutyl, 2-butyl, tert-butyl, n-pentyl, 2-pentyl, 3-pentyl, iso-pentyl, neo-pentyl, n-hexyl, n-heptyl, n-octyl, (2,4,4)-trimethyl-pent-2-yl, $-CF_3$, -CFH, $-CFH_2$, $-(CH_2)-(CF_3)$, $-(CH_2)-(CHF_2)$, $-(CH_2)-(CH_2F)$ and - $(CF_2)-(CF_3)$; or a residue selected from the group comprising phenyl, benzyl and phenethyl, which in each case can be unsubstituted or substituted with optionally 1, 2, 3, 4 or 5 substituents selected respectively independently from the group comprising F, Cl, Br, I, -CN, $-NO_2$, methyl, ethyl, n-propyl, isopropyl, n-butyl, isobutyl, 2-butyl, tert-butyl,$-O-CH_3$ and $-O-C_2H_5$;

$M^1$ stands for a residue selected from the group comprising residues 1 to 6, 21, 22, 36 and 37,

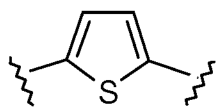
1

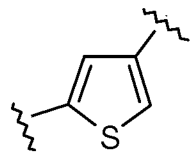
2

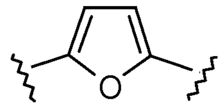
3

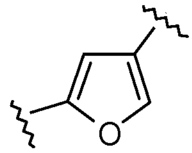
4

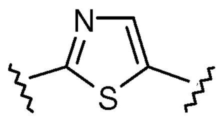
5

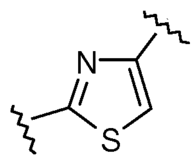
6

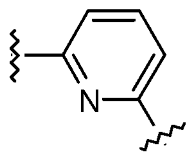
21

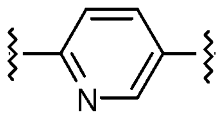
22

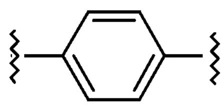
36

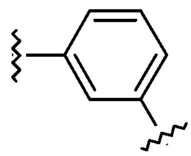
37

which in each case can be unsubstituted or substituted with optionally 1 or 2 substituents selected respectively independently from the group comprising F, Cl, Br, I, -CN, $-CH_2-CN$, $-NO_2$, methyl, ethyl, n-propyl, isopropyl, n-butyl, isobutyl, 2-butyl, tert-butyl, n-pentyl, neo-pentyl, $-SF_5$, $-O-CH_3$, $-O-C_2H_5$, $-O-C_3H_7$, $-O-C(CH_3)_3$, $-CF_3$,$-CHF_2$, -CHF, $-O-CF_3$, $-O-CHF_2$ and $-O-CH_2F$, and which in each case can be linked in any direction via the positions marked by a wavy line with the bicycle and the carbon atom of the triple bond;

and $M^2$ stands for a residue selected from the group comprising phenyl, 2-pyridinyl, 3-pyridinyl, 4-pyridinyl, 2-thiazolyl, 4-thiazolyl, 5-thiazolyl, 4-indolyl, 5-indolyl, 6-indolyl, 7-indolyl, 2-thiophenyl (2-thienyl), 3-thiophenyl (3-thienyl), 2-imidazolyl, 4-imidazolyl, 5-imidazolyl, 2-pyrimidinyl, 4-pyrimidinyl, 5-pyrimidinyl, 2-pyrazinyl, 5-quinolinyl, 6-quinolinyl, 7-quinolinyl and 8-quinolinyl, which in each case can be unsubstituted or substituted with optionally 1, 2, 3, 4 or 5 substituents selected respectively independently from the group comprising F, Cl, Br, -CN, $-CF_3$, $-SF_5$, $-S-CH_3$, $-S-C_2H_5$, methyl, ethyl, n-propyl, iso-propyl, n-butyl, sec-butyl, iso-butyl, tert-butyl, ethenyl, propenyl, -OH, $-O-CH_3$, $-O-C_2H_5$, $-O-C_3H_7$, -NH2, $-N(CH_3)_2$, $-NH-CH_3$,- $CH_2-NH_2$, $-N(C_2H_5)_2$, $-NO_2$, $-O-CF_3$, $-S-CF_3$, -SH, -C(=O)-H, $-C(=O)-O-CH_3$,$-C(=O)-O-C_2H_5$, phenyl, pyrrolyl, (1,3)-dioxolanyl, $-Si(phenyl)_2[C(CH_3)_3]$, $-C(=O)-CH_3$, $-C(=O)-C_2H_5$, $NH-S(=O)_2-CH_3$, $-NH-S(=O)_2-C_2H_5$, $-S(=O)_2-NH_2$, $-S(=O)_2-NH-CH_3$, $-CH_2-OH$, -C(=O)-OH, $-CH_2-O-CH_3$, $-C(=O)-NH_2$, $-C(=O)-NH-CH_3$, $-NH-C(=O)-CH_3$, $-NH-C(=NH)-NH_2$, $-NH-S(=O)_2-OH$ and $-S(=O)_2-N(CH_3)_2$;

in each case optionally in the form of one of the pure stereoisomers thereof, in particular enantiomers or diastereomers, the racemates thereof or in the form of a mixture of stereoisomers, in particular the enantiomers and/or diastereomers, in any desired mixing ratio, or in each case in the form of corresponding salts or in each case in the form of corresponding solvates.

**5.** Compounds of the general formula Ia according to claim 4,

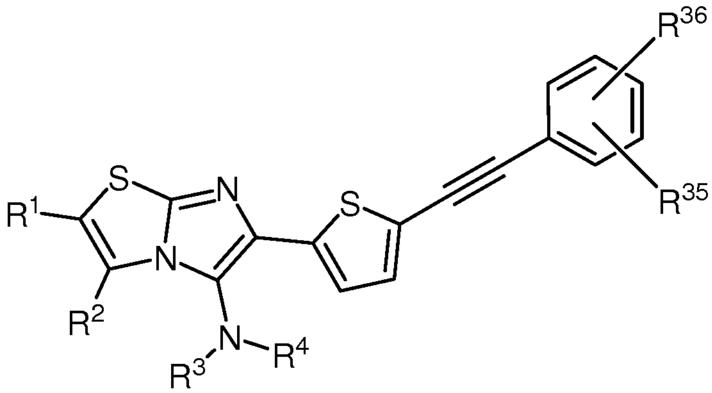

Ia,

in which

$R^1$, $R^2$, $R^3$ and $R^4$ have the meaning according to claim 4,
and $R^{35}$ and $R^{36}$, independently of one another, respectively stand for a residue selected from the group comprising H, F, Cl, Br, -CN, $-CF_3$, $-SF_5$, $-S-CH_3$, $-S-C_2H_5$, methyl, ethyl, n-propyl, iso-propyl, n-butyl, sec-butyl, iso-butyl, tert-butyl, ethenyl, propenyl, -OH, $-O-CH_3$, $-O-C_2H_5$, $-O-C_3H_7$, $-NH_2$, $-N(CH_3)_2$, $-NH-CH_3$, $-CH_2-NH_2$,$-N(C_2H_5)_2$, $-NO_2$, $-O-CF_3$, $-S-CF_3$, -SH, -C(=O)-H, $-C(=O)-O-CH_3$, $-C(=O)-O-C_2H_5$, phenyl, pyrrolyl, (1,3)-dioxolanyl, $-Si(phenyl)_2[C(CH_3)_3]$, $-C(=O)-CH_3$, $-C(=O)-C_2H_5$, $NH-S(=O)_2-CH_3$, $-NH-S(=O)_2-C_2H_5$, $-S(=O)_2-NH_2$, $-S(=O)_2-NH-CH_3$, $-CH_2-OH$,-C(=O)-OH, $-CH_2-O-CH_3$, $-C(=O)-NH_2$, $-C(=O)-NH-CH_3$, $-NH-C(=O)-CH_3$, $-NH-C(=NH)-NH_2$, $-NH-S(=O)_2-OH$ and $-S(=O)_2-N(CH_3)_2$;
in each case optionally in the form of corresponding salts, or in each case optionally in the form of corresponding solvates.

**6.** Compounds of the general formula Ib according to claim 4,

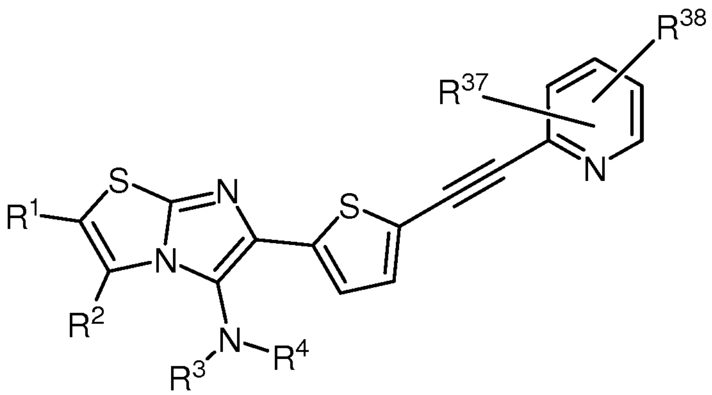

Ib,

in which

$R^1$, $R^2$, $R^3$ and $R^4$ have the meaning according to claim 4,
and $R^{37}$ and $R^{38}$, independently of one another, respectively stand for a residue selected from the group comprising H, F, Cl, Br, -CN, $-CF_3$, $-SF_5$, $-S-CH_3$, $-S-C_2H_5$, methyl, ethyl, n-propyl, iso-propyl, n-butyl, sec-butyl, iso-butyl, tert-butyl, ethenyl, propenyl, -OH, $-O-CH_3$, $-O-C_2H_5$, $-O-C_3H_7$, $-NH_2$, $-N(CH_3)_2$, $-NH-CH_3$, $-CH_2-NH_2$,$-N(C_2H_5)_2$, $-NO_2$, $-O-CF_3$, $-S-CF_3$, -SH, -C(=O)-H, $-C(=O)-O-CH_3$, $-C(=O)-O-C_2H_5$, phenyl, pyrrolyl, (1,3)-dioxolanyl, $-Si(phenyl)_2[C(CH_3)_3]$, $-C(=O)-CH_3$, $-C(=O)-C_2H_5$, $NH-S(=O)_2-CH_3$, $-NH-S(=O)_2-C_2H_5$, $-S(=O)_2-NH_2$, $-S(=O)_2-NH-CH_3$, $-CH_2-OH$,-C(=O)-OH, $-CH_2-O-CH_3$, $-C(=O)-NH_2$, $-C(=O)-NH-CH_3$, -NH-

$C(=O)-CH_3$, $-NH-C(=NH)-NH_2$, $-NH-S(=O)_2-OH$ and $-S(=O)_2-N(CH_3)_2$;
in each case optionally in the form of corresponding salts, or in each case optionally in the form of corresponding solvates.

**7.** Compounds of the general formula Ic according to claim 4,

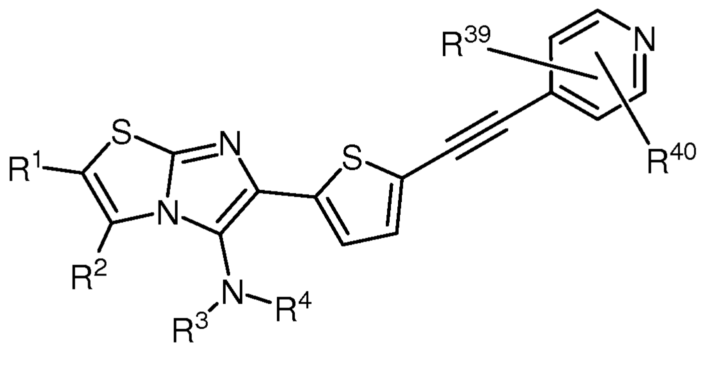

Ic,

in which

$R^1$, $R^2$, $R^3$ and $R^4$ have the meaning according to claim 4,
and $R^{39}$ and $R^{40}$, independently of one another, respectively stand for a residue selected from the group comprising H, F, Cl, Br, -CN, $-CF_3$, $-SF_5$, $-S-CH_3$, $-S-C_2H_5$, methyl, ethyl, n-propyl, iso-propyl, n-butyl, sec-butyl, iso-butyl, tert-butyl, ethenyl, propenyl, -OH, $-O-CH_3$, $-O-C_2H_5$, $-O-C_3H_7$, $-NH_2$, $-N(CH_3)_2$, $-NH-CH_3$, $-CH_2-NH_2$,$-N(C_2H_5)_2$, $-NO_2$, $-O-CF_3$, $-S-CF_3$, -SH, -C(=O)-H, $-C(=O)-O-CH_3$, $-C(=O)-O-C_2H_5$, phenyl, pyrrolyl, (1,3)-dioxolanyl, $-Si(phenyl)_2[C(CH_3)_3]$, $-C(=O)-CH_3$, $-C(=O)-C_2H_5$, $NH-S(=O)_2-CH_3$, $-NH-S(=O)_2-C_2H_5$, $-S(=O)_2-NH_2$, $-S(=O)_2-NH-CH_3$, $-CH_2-OH$,-C(=O)-OH, $-CH_2O-CH_3$, $-C(=O)-NH_2$, $-C(=O)-NH-CH_3$, $-NH-C(=O)-CH_3$, $-NH-C(=NH)-NH_2$, $-NH-S(=O)_2-OH$ and $-S(=O)_2-N(CH_3)_2$;
in each case optionally in the form of corresponding salts, or in each case optionally in the form of corresponding solvates.

**8.** Compounds of the general formula Id according to claim 4,

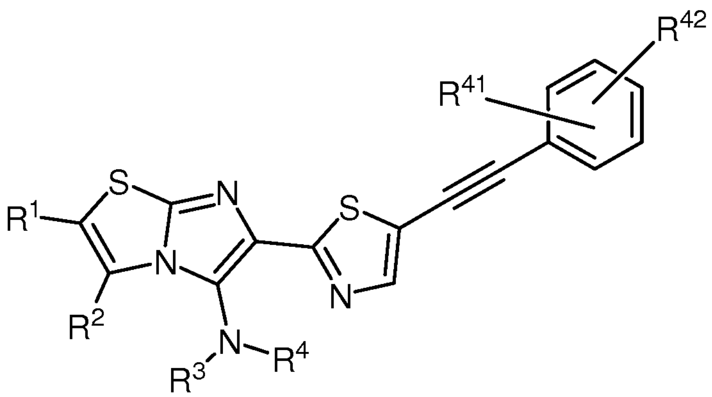

Id,

in which

$R^1$, $R^2$, $R^3$ and $R^4$ have the meaning according to claim 4,
and $R^{41}$ and $R^{42}$, independently of one another, respectively stand for a residue selected from the group comprising H, F, Cl, Br, -CN, $-CF_3$, $-SF_5$, $-S-CH_3$, $-S-C_2H_5$, methyl, ethyl, n-propyl, iso-propyl, n-butyl, sec-butyl, iso-butyl, tert-butyl, ethenyl, propenyl, -OH, $-O-CH_3$, $-O-C_2H_5$, $-O-C_3H_7$, $-NH_2$, $-N(CH_3)_2$, $-NH-CH_3$, $-CH_2-NH_2$,$-N(C_2H_5)_2$, $-NO_2$, $-O-CF_3$, $-S-CF_3$, -SH, -C(=O)-H, $-C(=O)-O-CH_3$, $-C(=O)-O-C_2H_5$, phenyl, pyrrolyl, (1,3)-dioxolanyl, $-Si(phenyl)_2[C(CH_3)_3]$, $-C(=O)-CH_3$, $-C(=O)-C_2H_5$, $NH-S(=O)_2-CH_3$, $-NH-S(=O)_2-C_2H_5$, $-S(=O)_2-NH_2$, $-S(=O)_2-NH-CH_3$, $-CH_2-OH$,-C(=O)-OH, $-CH_2-O-CH_3$, $-C(=O)-NH_2$, $-C(=O)-NH-CH_3$, $-NH-C(=O)-CH_3$, $-NH-C(=NH)-NH_2$, $-NH-S(=O)_2-OH$ and $-S(=O)_2-N(CH_3)_2$;
in each case optionally in the form of corresponding salts, or in each case optionally in the form of corresponding solvates.

**9.** Compounds of the general formula Ie according to claim 4,

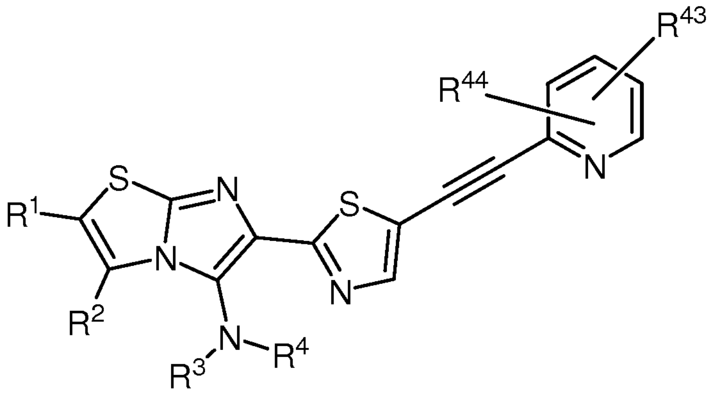

Ie,

in which

$R^1$, $R^2$, $R^3$ and $R^4$ have the meaning according to Claim 4,
and $R^{43}$ and $R^{44}$, independently of one another, respectively stand for a residue selected from the group comprising H, F, Cl, Br, -CN, $-CF_3$, $-SF_5$, $-S-CH_3$, $-S-C_2H_5$, methyl, ethyl, n-propyl, iso-propyl, n-butyl, sec-butyl, iso-butyl, tert-butyl, ethenyl, propenyl, -OH, $-O-CH_3$, $-O-C_2H_5$, $-O-C_3H_7$, $-NH_2$, $-N(CH_3)_2$, $-NH-CH_3$, $-CH_2-NH_2$,$-N(C_2H_5)_2$, $-NO_2$, $-O-CF_3$, $-S-CF_3$, -SH, -C(=O)-H, $-C(=O)-O-CH_3$, $-C(=O)-O-C_2H_5$, phenyl, pyrrolyl, (1,3)-dioxolanyl, $-Si(phenyl)_2[C(CH_3)_3]$, $-C(=O)-CH_3$, $-C(=O)-C_2H_5$, $NH-S(=O)_2-CH_3$, $-NH-S(=O)_2-C_2H_5$, $-S(=O)_2-NH_2$, $-S(=O)_2-NH-CH_3$, $-CH_2-OH$,-C(=O)-OH, $-CH_2-O-CH_3$, $-C(=O)-NH_2$, $-C(=O)-NH-CH_3$, $-NH-C(=O)-CH_3$, $-NH-C(=NH)-NH_2$, $-NH-S(=O)_2-OH$ and $-S(=O)_2-N(CH_3)_2$;
in each case optionally in the form of corresponding salts, or in each case optionally in the form of corresponding solvates.

**10.** Compounds of the general formula If according to claim 4,

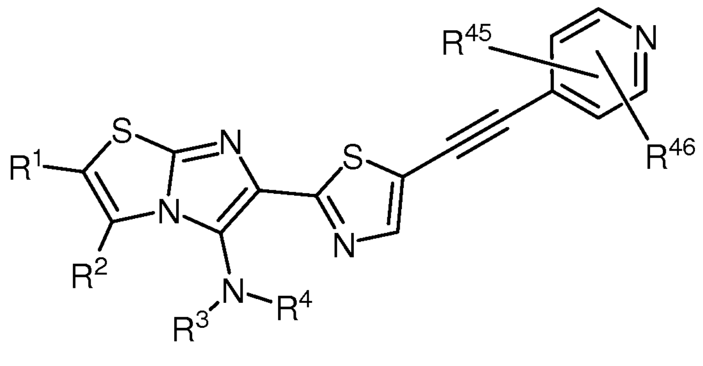

If,

in which

$R^1$, $R^2$, $R^3$ and $R^4$ have the meaning according to claim 4,
and $R^{45}$ and $R^{46}$, independently of one another, respectively stand for a residue selected from the group comprising H, F, Cl, Br, -CN, $-CF_3$, $-SF_5$, $-S-CH_3$, $-S-C_2H_5$, methyl, ethyl, n-propyl, iso-propyl, n-butyl, sec-butyl, iso-butyl, tert-butyl, ethenyl, propenyl, -OH, $-O-CH_3$, $-O-C_2H_5$, $-O-C_3H_7$, $-NH_2$, $-N(CH_3)_2$, $-NH-CH_3$, $-CH_2-NH_2$,$-N(C_2H_5)_2$, $-NO_2$, $-O-CF_3$, $-S-CF_3$, -SH, -C(=O)-H, $-C(=O)-O-CH_3$, $-C(=O)-O-C_2H_5$, phenyl, pyrrolyl, (1,3)-dioxolanyl, $-Si(phenyl)_2[C(CH_3)_3]$, $-C(=O)-CH_3$, $-C(=O)-C_2H_5$, $NH-S(=O)_2-CH_3$, $-NH-S(=O)_2-C_2H_5$, $-S(=O)_2-NH_2$, $-S(=O)_2-NH-CH_3$, $-CH_2-OH$,-C(=O)-OH, $-CH_2-O-CH_3$, $-C(=O)-NH_2$, $-C(=O)-NH-CH_3$, $-NH-C(=O)-CH_3$, $-NH-C(=NH)-NH_2$, $-NH-S(=O)_2-OH$ and $-S(=O)_2-N(CH_3)_2$;
in each case optionally in the form of corresponding salts, or in each case optionally in the form of corresponding solvates.

**11.** Compounds according to one or more of claims 1 to 10, **characterised in that**
$R^1$ stands for a hydrogen residue; -F; -Cl; -Br; -CN; $-O-CH_3$; $-C(=O)-R^9$, $-C(=O)-OR^{13}$; $-(CH_2)-O-C(=O)-R^{16}$; $-(CH_2)-O-S(=O)_2-R^{31}$; $-(CH_2)-O-S(=O)_2-O-R^{32}$; $-(CH_2)-O-P(=O)(OR^{33})(OR^{34})$; or a residue selected from the group comprising methyl, ethyl, n-propyl, isopropyl, n-butyl, isobutyl, 2-butyl, tert-butyl, n-pentyl, $-CF_3$, $-CF_2H$,$-CFH_2$, $-(CH_2)-(CF_3)$, $-(CH_2)-(CHF_2)$, $-(CH_2)-(CH_2F)$, and $-(CF_2)-(CF_3)$;
$R^2$ stands for a hydrogen residue; -F; -Cl; -Br; -CN; $-O-CH_3$; $-C(=O)-R^9$, $-C(=O)-OR^{13}$; $-(CH_2)-O-C(=O)-R^{16}$; $-(CH_2)-O-S(=O)_2-R^{31}$; $-(CH_2)-O-S(=O)_2-O-R^{32}$; $-(CH_2)-O-P(=O)(OR^{33})(OR^{34})$; or a residue selected from the group comprising methyl, ethyl, n-propyl, isopropyl, n-butyl, isobutyl, 2-butyl, tert-butyl, n-pentyl, $-CF_3$, $-CF_2H$,$-CFH_2$, $-(CH_2)-(CF_3)$, $-(CH_2)-(CHF_2)$, $-(CH_2)-(CH_2F)$, and $-(CF_2)-(CF_3)$;
$R^3$ stands for a hydrogen residue or a residue selected from the group comprising methyl, ethyl and isopropyl;
$R^4$ stands for a hydrogen residue; $-C(=O)-R^{21}$ or a residue selected from the group comprising methyl, ethyl, n-propyl, isopropyl, n-butyl, isobutyl, 2-butyl, tert-butyl, n-pentyl, 2-pentyl, 3-pentyl, iso-pentyl, neo-pentyl, n-hexyl, n-heptyl, n-octyl and (2,4,4)-trimethyl-pent-2-yl;
or $R^3$ and $R^4$ together with the nitrogen atom connecting them as a ring member form a residue selected from the group comprising pyrrolidinyl, piperidinyl and morpholinyl;
$R^{16}$ stands for a residue selected from the group comprising $-(CH_2)-(CH_2)-C(=O)-OH$; $-(CH_2)-(CH_2)-C(=O)-O-CH_3$ and $-(CH_2)-(CH_2)-C(=O)-O-C_2H_5$;
$R^9$, $R^{13}$, $R^{31}$, $R^{32}$, $R^{33}$ and $R^{34}$, respectively independently of one another, stand for a hydrogen residue; or a residue selected from the group comprising methyl, ethyl, n-propyl, isopropyl, n-butyl, isobutyl, 2-butyl, tert-butyl, n-pentyl, 2-pentyl, 3-pentyl, iso-pentyl, neo-pentyl, n-hexyl, n-heptyl, n-octyl, (2,4,4)-trimethyl-pent-2-yl, $-CF_3$,$-CF_2H$, $-CFH_2$, $-(CH_2)-(CF_3)$, $-(CH_2)-(CHF_2)$, $-(CH_2)-(CH_2F)$ and $-(CF_2)-(CF_3)$;
$R^{21}$ stands for a residue selected from the group comprising methyl, ethyl, n-propyl, isopropyl, n-butyl, isobutyl, 2-butyl, tert-butyl, n-pentyl, 2-pentyl, 3-pentyl, iso-pentyl, neo-pentyl, n-hexyl, n-heptyl, n-octyl, (2,4,4)-trimethyl-pent-2-yl, $-CF_3$,$-CF_2H$, $-CFH_2$, $-(CH_2)-(CF_3)$, $-(CH_2)-(CHF_2)$, $-(CH_2)-(CH_2F)$ and $-(CF_2)-(CF_3)$ or a phenyl residue, which in each case can be unsubstituted or substituted with optionally 1, 2, 3, 4 or 5 substituents selected respectively independently from the group comprising F, Cl, Br, I, -CN, $-NO_2$, methyl, ethyl, n-propyl, isopropyl, n-butyl, isobutyl, 2-butyl, tert-butyl,$-O-CH_3$ and $-O-C_2H_5$;
$M^1$ stands for a residue selected from the group comprising residues 1, 3, 5, 22, 36 and 37,

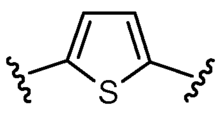
1

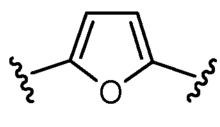
3

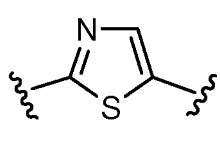
5

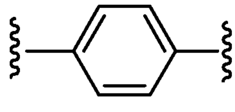
36

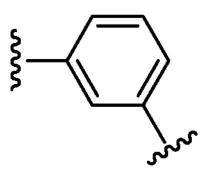
37

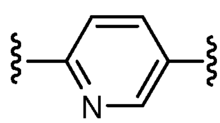
22

which in each case is unsubstituted, and which in each case can be linked in any direction via the positions marked by a wavy line with the bicycle and the carbon atom of the triple bond;
$M^2$ stands for a residue selected from the group comprising phenyl, 2-pyrimidinyl, 5-pyrimidinyl, 2-thiophenyl (2-thienyl), 3-thiophenyl (3-thienyl), 2-pyridinyl, 3-pyridinyl, 4-pyridinyl, 2-thiazolyl and 4-thiazolyl, which in each case can be unsubstituted or substituted with optionally 1, 2, 3, 4 or 5 substituents selected respectively independently from the group comprising F, Cl, Br, -CN, -O-$CH_3$, -OH,-$CF_3$, methyl, ethyl, n-propyl, iso-propyl, n-butyl, sec-butyl, iso-butyl and tert-butyl;
in each case optionally in the form of corresponding salts, or in each case optionally in the form of corresponding solvates.

**12.** Compounds according to one or more of claims 1 to 11, **characterised in that**
$R^1$ stands for a hydrogen residue; -F; -Cl; -Br; -CN; -C(=O)-$OR^{13}$; or a residue selected from the group comprising methyl, ethyl, n-propyl, isopropyl, n-butyl, isobutyl, 2-butyl, tert-butyl and n-pentyl;
$R^2$ stands for a hydrogen residue; -F; -Cl; -Br; -CN; -C(=O)-$OR^{13}$; or a residue selected from the group comprising methyl, ethyl, n-propyl, isopropyl, n-butyl, isobutyl, 2-butyl, tert-butyl and n-pentyl;
$R^3$ stands for a hydrogen residue;
$R^4$ stands for a hydrogen residue; -C(=O)-$R^{21}$ or a residue selected from the group comprising methyl, ethyl, n-propyl, isopropyl, n-butyl, isobutyl, 2-butyl, tert-butyl, n-pentyl and (2,4,4)-trimethyl-pent-2-yl;
$R^{13}$ stands for a residue selected from the group comprising methyl, ethyl, n-propyl, isopropyl, n-butyl, isobutyl, 2-butyl, tert-butyl, n-pentyl, 2-pentyl, 3-pentyl and (2,4,4)-trimethyl-pent-2-yl;
$R^{21}$ stands for a residue selected from the group comprising methyl, ethyl, n-propyl, isopropyl, n-butyl, isobutyl, 2-butyl, tert-butyl, n-pentyl and (2,4,4)-trimethyl-pent-2-yl or a phenyl residue which in each case is unsubstituted;
$M^1$ stands for a residue selected from the group comprising residues 1, 3, 5, 22 and 36,

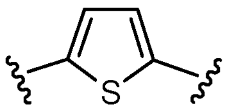
1

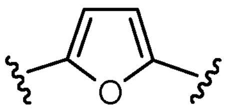
3

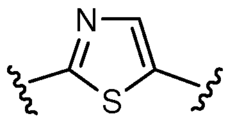
5

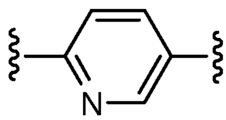
22

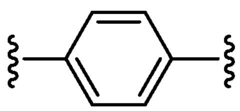
36

which in each case is unsubstituted, and which in each case can be linked in any direction via the positions marked by a wavy line with the bicycle and the carbon atom of the triple bond;
and $M^2$ stands for a residue selected from the group comprising phenyl, 2-pyrimidinyl, 5-pyrimidinyl, 2-thiophenyl (2-thienyl), 3-thiophenyl (3-thienyl), 2-pyridinyl, 3-pyridinyl, 4-pyridinyl, 2-thiazolyl and 4-thiazolyl, which in each case can be unsubstituted or substituted with optionally 1, 2, 3, 4 or 5 substituents selected respectively independently from the group comprising F, Cl, Br, -CN, -OH, -O-$CH_3$,-OH, -$CF_3$, methyl, ethyl, n-propyl, iso-propyl, n-butyl, sec-butyl, iso-butyl and tert-butyl;
in each case optionally in the form of corresponding salts, or in each case in the form of corresponding solvates.

**13.** Compounds according to one or more of claims 1 to 12 selected from the group comprising

[1] 6-(5-(phenylethynyl)thiophene-2-yl)-N-(2,4,4-trimethylpentane-2-yl)-imidazo[2,1-b]thiazole-5-amine,
[2] N-tert-butyl-6-(5-(pyridine-2-ylethynyl)thiophene-2-yl)-imidazo[2,1-b]thiazole-5-amine,
[3] N-tert-butyl-3-methyl-6-(5-(pyridine-2-ylethynyl)thiophene-2-yl)-imidazo[2,1-b]thiazole-5-amine,
[4] N-tert-butyl-2-methyl-6-(5-(pyridine-2-ylethynyl)thiophene-2-yl)-imidazo[2,1-b]thiazole-5-amine,
[5] N-tert-butyl-2,3-dimethyl-6-(5-(pyridine-2-ylethynyl)thiophene-2-yl)-imidazo[2,1-b]thiazole-5-amine,
[6] N-tert-butyl-2-chloro-6-(5-(pyridine-2-ylethynyl)thiophene-2-yl)-imidazo[2,1-b]thiazole-5-amine,
[7] N-tert-butyl-6-(5-(pyridine-4-ylethynyl)thiophene-2-yl)imidazo[2,1-b]thiazole-5-amine,

[8] 5-(tert-butylamino)-6-(5-(pyridine-2-ylethynyl)thiophene-2-yl)-imidazo[2,1-b]thiazole-2-carboxylic acid methylester,
[9] N-tert-butyl-6-(5-(pyridine-2-ylethynyl)thiazole-2-yl)imidazo[2,1-b]thiazole-5-amine;
[10] 6-(5-pyridine-2-ylethynyl)thiophene-2-yl)-N-(2,4,4-trimethylpentane-2-yl)imidazo[2,1-b]thiazole-5-amine,
[11] N-tert-butyl-2-methyl-6-(4-(pyridine-2-ylethynyl)phenyl)imidazo[2,1-b]thiazole-5-amine,
[12] N-tert-butyl-6-(5-(pyridine-2-ylethynyl)furan-2-yl)imidazo[2,1-b]thiazole-5-amine,
[13] N-tert-butyl-3-methyl-6-(5-(phenylethynyl)thiophene-2-yl)imidazo[2,1-b]thiazole-5-amine,
[14] 6-(5-(pyridine-2-ylethynyl)thiophene-2-yl)imidazo[2,1-b]thiazole-5-amine,
[15] N-tert-butyl-6-(5-(pyrimidine-2-ylethynyl)thiophene-2-yl)imidazo[2,1-b]thiazole-5-amine,
[16] N-tert-butyl-6-(5-((3-fluoropyridine-2-yl)ethynyl)thiophene-2-yl)imidazo[2,1-b]thiazole-5-amine,
[17] N-tert-butyl-6-(5-((2-fluoropyridine-4-yl)ethynyl)thiophene-2-yl)imidazo[2,1-b]thiazole-5-amine,
[18] N-tert-butyl-6-(5-(thiophene-2-ylethynyl)thiophene-2-yl)imidazo[2,1-b]thiazole-5-amine,
[19] N-tert-butyl-6-(5-(thiazole-2-ylethynyl)thiophene-2-yl)imidazo[2,1-b]thiazole-5-amine,
[20] 3-((5-(5-(tert-butylamino)imidazo[2,1-b]thiazole-6-yl)thiophene-2-yl)ethynyl)phenol,
[21] 3-((5-(5-(tert-butylamino)imidazo[2,1-b]thiazole-6-yl)thiophene-2-yl)ethynyl)benzonitrile,
[22] N-ethyl-6-(6-(phenylethynyl)pyridine-3-yl)imidazo[2,1-b]thiazole-5-amine,
[23] N-tert-butyl-6-(5-((3-methylpyridine-2-yl)ethynyl)thiophene-2-yl)imidazo[2,1-b]thiazole-5-amine,
[24] N-(6-(5-(pyridine-2-ylethynyl)thiophene-2-yl)imidazo[2,1-b]thiazole-5-yl)acetamide and
[25] N-(6-(5-(pyridine-2-ylethynyl)thiophene-2-yl)imidazo[2,1-b]thiazole-5-yl)benzamide;
in each case optionally in the form of corresponding salts, in particular the hydrochlorides, or in each case in the form of corresponding solvates.

**14.** Drug containing at least one compound according to one or more of claims 1 to 13 and optionally one or more physiologically acceptable auxiliary substances.

**15.** Use of at least one compound according to one or more of claims 1 to 13 for the production of a drug for the treatment and/or prevention of pain selected from the group comprising acute pain, chronic pain, neuropathic pain and visceral pain; migraine; depression; neurodegenerative diseases selected from the group comprising multiple sclerosis, Alzheimer's disease, Parkinson's disease and Huntington's disease; cognitive dysfunction, cognitive deficiency states, attention deficit disorder (ADD); psychiatric disorders selected from the group comprising anxiety states and panic attacks; epilepsy; coughing; urinary incontinence; diarrhoea; pruritus; schizophrenia; cerebral ischaemia; muscle spasms; cramps; lung disorders selected from the group comprising asthma and pseudocroup; regurgitation (vomiting); listlessness; drowsiness; weariness; laryngitis; eating disorders selected from the group comprising bulimia, cachexia, anorexia and obesity; alcohol dependency; dependency on medicines; drug dependency, preferably nicotine and/or cocaine dependency; alcohol abuse; abuse of medication; drug abuse; nicotine and/or cocaine abuse; withdrawal symptoms associated with dependency on alcohol, medications and/or drugs (nicotine and/or cocaine); development of tolerance to medications, to natural or synthetic opioids; gastro-oesophageal reflux syndrome; gastro-oesophageal reflux disease;
irritable bowel syndrome; for diuresis; for antinatriuresis; for influencing the cardiovascular system; for increasing vigilance; for increasing libido; for modulating locomotor activity or for local anaesthesia.

## Revendications

**1.** Composés d'imidazo[2,1-b]thiazole substitués de formule générale I

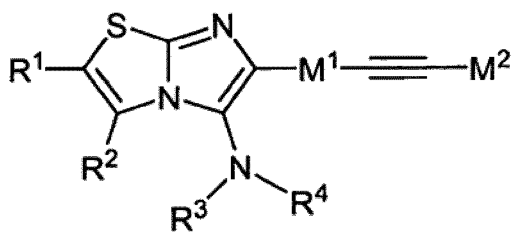

I,

dans laquelle

$R^1$ et $R^2$ représentent chacun indépendamment l'un de l'autre un radical hydrogène ; un radical halogène ;$-NO_2$ ; -CN ; $-NH_2$ ; $-NHR^5$ ; $-NR^6R^7$; $-NH-C(=O)-R^8$ ; $C(=O)-R^9$, $-C(=O)-NH_2$ ; $-C(=O)-NHR^{10}$ ; $-C(=O)-NR^{11}R^{12}$ ;$-C(=O)-OR^{13}$; $-(CH_2)_m-C(=O)-OR^{14}$ avec m = 1 , 2, 3, 4 ou 5 ; $-O-C(=O)-R^{15}$ ; $-(CH_2)_n-O-C(=O)-R^{16}$ avec n = 1, 2, 3, 4 ou 5 ; $-OR^{17}$ ; $-(CH_2)_o-O-R^{18}$ avec o = 1 , 2, 3, 4 ou 5 ; $-SR^{19}$ ; $-(CH_2)_p-S(=o)_t-R^{20}$ avec p = 1 , 2, 3, 4 ou 5 et t = 0, 1 ou 2 ; $-NH-S(=O)_2-NR^{27}R^{28}$ ; $-S(=O)_2-NR^{29}R^{30}$ ;$-SF_5$ ; $-(CH_2)_u-O-S(=O)_2-R^{31}$ avec u = 1 , 2, 3, 4 ou 5 ;$-(CH_2)_v-O-S(=O)_{2-O}-R^{32}$ avec v = 1, 2, 3, 4 ou 5 ; $-(CH_2)_w-O-P(=O)(OR^{33})(OR^{34})$ avec w = 1 , 2, 3, 4 ou 5 ;

un radical aliphatique linéaire ou ramifié, saturé ou insaturé, non substitué ou substitué au moins une fois ;

un radical cycloaliphatique saturé ou insaturé, non substitué ou substitué au moins une fois, comprenant éventuellement au moins un hétéroatome en tant qu'élément de cycle, qui peut être relié par un groupe alkylène linéaire ou ramifié, non substitué ou substitué au moins une fois, et/ou condensé avec un système cyclique mono- ou polycyclique non substitué ou substitué au moins une fois ;

ou un radical aryle ou hétéroaryle non substitué ou substitué au moins une fois, qui peut être relié par un groupe alkylène linéaire ou ramifié, non substitué ou substitué au moins une fois, et/ou condensé avec un système cyclique mono- ou polycyclique non substitué ou substitué au moins une fois ;

$R^3$ et $R^4$ représentent chacun indépendamment l'un de l'autre un radical hydrogène ; $-C(=O)-R^{21}$ ; $-(CH_2)_q-C(=O)-R^{22}$ avec q = 1 , 2, 3, 4 ou 5 ; $-C(=O)-O-R^{23}$;$-(CH_2)_r-C(=O)-O-R^{24}$ avec r = 1 , 2, 3, 4 ou 5 ; $-C(=O)-NHR^{25}$ ; $-(CH_2)S-C(=O)-NHR^{26}$ avec s = 1, 2, 3, 4 ou 5 ; un radical aliphatique linéaire ou ramifié, saturé ou insaturé, non substitué ou substitué au moins une fois ; un radical cycloaliphatique saturé ou insaturé, non substitué ou substitué au moins une fois, comprenant éventuellement au moins un hétéroatome en tant qu'élément de cycle, qui peut être relié par un groupe alkylène linéaire ou ramifié, non substitué ou substitué au moins une fois, et/ou condensé avec un système cyclique mono- ou polycyclique non substitué ou substitué au moins une fois ; ou un radical aryle ou hétéroaryle non substitué ou substitué au moins une fois, qui peut être relié par un groupe alkylène linéaire ou ramifié, non substitué ou substitué au moins une fois, et/ou condensé avec un système cyclique mono- ou polycyclique non substitué ou substitué au moins une fois ;

ou $R^3$ et $R^4$ forment ensemble avec l'atome d'azote qui les relie en tant qu'élément de cycle un radical hétérocycloaliphatique saturé ou insaturé, non substitué ou substitué au moins une fois, comprenant éventuellement au moins un hétéroatome supplémentaire en tant qu'élément de cycle, qui peut être condensé avec un système cyclique mono- ou polycyclique non substitué ou substitué au moins une fois ;

$R^5$, $R^6$, $R^7$, $R^8$, $R^{10}$, $R^{11}$, $R^{12}$, $R^{15}$ et $R^{16}$ représentent chacun indépendamment les uns des autres un radical aliphatique linéaire ou ramifié, saturé ou insaturé, non substitué ou substitué au moins une fois, ou un radical aryle ou hétéroaryle non substitué ou substitué au moins une fois, qui peut être relié par un groupe alkylène linéaire ou ramifié, non substitué ou substitué au moins une fois, et/ou condensé avec un système cyclique mono- ou polycyclique non substitué ou substitué au moins une fois ; $R^9$, $R^{13}$, $R^{14}$, $R^{17}$, $R^{18}$, $R^{19}$, $R^{20}$, $R^{21}$, $R^{22}$, $R^{23}$, $R^{24}$, $R^{25}$, $R^{26}$, $R^{27}$, $R^{28}$, $R^{29}$, $R^{30}$, $R^{31}$, $R^{32}$, $R^{33}$ et $R^{34}$ représentent chacun indépendamment les uns des autres un radical hydrogène ; un radical aliphatique linéaire ou ramifié, saturé ou insaturé, non substitué ou substitué au moins une fois, ou un radical aryle ou hétéroaryle non substitué ou substitué au moins une fois, qui peut être relié par un groupe alkylène linéaire ou ramifié, non substitué ou substitué au moins une fois, et/ou condensé avec un système cyclique mono- ou polycyclique non substitué ou substitué au moins une fois ;

$M^1$ représente un radical aryle ou hétéroaryle, qui peut être substitué avec au moins un substituant supplémentaire et/ou condensé avec un système cyclique mono- ou polycyclique non substitué ou substitué au moins une fois ;

et $M^2$ représente un radical aryle ou hétéroaryle, qui peut être non substitué ou substitué au moins une fois, et qui peut être condensé avec un système cyclique mono- ou polycyclique non substitué ou substitué au moins une fois ;

à chaque fois sous la forme d'un de leurs stéréoisomères purs, notamment énantiomères ou diastéréomères, de leurs racémats ou sous la forme d'un mélange de stéréoisomères, notamment les énantiomères et/ou diastéréomères, en un rapport de mélange quelconque, ou à chaque fois sous la forme des sels correspondants, ou à chaque fois sous la forme des solvates correspondants.

**2.** Composés selon la revendication 1, **caractérisés en ce que**
$R^1$ et $R^2$ représentent chacun indépendamment l'un de l'autre un radical hydrogène ; un radical halogène ;$-NO_2$ ; -CN ; $-NH_2$ ; $-NHR^5$ ; $-NR^6R^7$; $-NH-C(=O)-R^8$ ; $C(=O)-R^9$ ; $-C(=O)-NH_2$ ; $-C(=O)-NHR^{10}$ ; $-C(=O)-NR^{11}R^{12}$ ;$-C(=O)-OR^{13}$ ; $-(CH_2)_m-C(=O)-OR^{14}$ avec m = 1 , 2, 3, 4 ou 5 ; $-O-C(=O)-R^{15}$ ; $-(CH_2)_n-O-C(=O)-R^{16}$ avec n = 1 , 2, 3, 4 ou 5 ; $-OR^{17}$ ; $-(CH_2)_o-O-R^{18}$ avec o = 1, 2, 3, 4 ou 5 ; $-SR^{19}$ ; $-(CH_2)_p-S(=O)_t-R^{20}$ avec p = 1, 2, 3, 4 ou 5 et t = 0, 1 ou 2 ; $-NH-S(=O)_2-NR^{27}R^{22}$, $-S(=O)_2-NR^{29}R^{30}$ ;$-SF_5$ ; $-(CH_2)_u-O-S(=O)_2-R^{31}$ avec

u = 1, 2, 3, 4 ou 5 ;$-(CH_2)_v-O-S(=O)_2-O-R^{32}$ avec v = 1, 2, 3, 4 ou 5 ; $-(CH_2)_w-O-P(=O)(OR^{33})$ $(OR^{34})$ avec w = 1, 2, 3, 4 ou 5 ; un radical alkyle en $C_{1-10}$, un radical alcényle en $C_{2-6}$ ou un radical alcynyle en $C_{2-6}$, linéaire ou ramifié, non substitué ou substitué au moins une fois ;

un radical cycloaliphatique en $C_{3-8}$ saturé ou insaturé, non substitué ou substitué au moins une fois, comprenant éventuellement au moins un hétéroatome en tant qu'élément de cycle, qui peut être relié par un groupe alkylène en $C_{1-5}$ linéaire ou ramifié, non substitué ou substitué au moins une fois ;

ou un radical aryle ou hétéroaryle à 5 ou 6 éléments, non substitué ou substitué au moins une fois, qui peut être relié par un groupe alkylène en $C_{1-5}$ linéaire ou ramifié, non substitué ou substitué au moins une fois, et/ou condensé avec un système cyclique mono- ou polycyclique non substitué ou substitué au moins une fois, les cycles du système cyclique étant chacun à 5, 6 ou 7 éléments ;

$R^3$ et $R^4$ représentent chacun indépendamment l'un de l'autre un radical hydrogène ; $-C(=O)-R^{21}$ ; $-(CH_2)_q-C(=O)-R^{22}$ avec q = 1 , 2, 3, 4 ou 5 ; $-C(=O)-O-R^{23}$ ;$-(CH_2)_r-C(=O)-O-R^{24}$ avec r = 1 , 2, 3, 4 ou 5 ; $-C(=O)-NHR^{25}$ ; $-(CH_2)_s-C(=O)-NHR^{26}$ avec s = 1 , 2, 3, 4 ou 5 ; un radical alkyle en $C_{1-10}$, un radical alcényle en $C_{2-6}$ ou un radical alcynyle en $C_{2-6}$, linéaire ou ramifié, non substitué ou substitué au moins une fois ;

un radical cycloaliphatique en $C_{3-8}$ saturé ou insaturé, non substitué ou substitué au moins une fois, comprenant éventuellement au moins un hétéroatome en tant qu'élément de cycle, qui peut être relié par un groupe alkylène en $C_{1-5}$ linéaire ou ramifié, non substitué ou substitué au moins une fois ; ou un radical aryle ou hétéroaryle à 5 ou 6 éléments, non substitué ou substitué au moins une fois, qui peut être relié par un groupe alkylène en $C_{1-5}$ linéaire ou ramifié, non substitué ou substitué au moins une fois, et/ou condensé avec un système cyclique mono- ou polycyclique non substitué ou substitué au moins une fois, les cycles du système cyclique étant chacun à 5, 6 ou 7 éléments ;

ou $R^3$ et $R^4$ forment ensemble avec l'atome d'azote qui les relie en tant qu'élément de cycle un radical hétérocycloaliphatique en $C_{4-10}$ saturé ou insaturé, non substitué ou substitué au moins une fois, comprenant éventuellement au moins un hétéroatome supplémentaire en tant qu'élément de cycle, qui peut être condensé avec un système cyclique mono- ou polycyclique non substitué ou substitué au moins une fois, les cycles du système cyclique étant chacun à 5, 6 ou 7 éléments ;

$R^5$, $R^6$, $R^7$, $R^8$, $R^{10}$, $R^{11}$, $R^{12}$, $R^{15}$ et $R^{16}$ représentent chacun indépendamment les uns des autres un radical alkyle en $C_{1-10}$, un radical alcényle en $C_{2-6}$ ou un radical alcynyle en $C_{2-6}$, linéaire ou ramifié, non substitué ou substitué au moins une fois ; ou un radical aryle ou hétéroaryle de 5 à 14 éléments, non substitué ou substitué au moins une fois, qui peut être relié par un groupe alkylène en $C_{1-5}$ linéaire ou ramifié, non substitué ou substitué au moins une fois, et/ou condensé avec un système cyclique mono- ou polycyclique non substitué ou substitué au moins une fois, les cycles du système cyclique étant chacun à 5, 6 ou 7 éléments ;

$R^9$, $R^{13}$, $R^{14}$, $R^{17}$, $R^{18}$, $R^{19}$, $R^{20}$, $R^{21}$, $R^{22}$, $R^{23}$, $R^{24}$, $R^{25}$, $R^{26}$ $R^{27}$, $R^{28}$, $R^{29}$, $R^{30}$, $R^{31}$, $R^{32}$, $R^{33}$ et $R^{34}$ représentent chacun indépendamment les uns des autres un radical hydrogène ; un radical alkyle en $C_{1-10}$, un radical alcényle en $C_{2-6}$ ou un radical alcynyle en $C_{2-6}$, linéaire ou ramifié, non substitué ou substitué au moins une fois ; ou un radical aryle ou hétéroaryle de 5 à 14 éléments, non substitué ou substitué au moins une fois, qui peut être relié par un groupe alkylène en $C_{1-5}$ linéaire ou ramifié, non substitué ou substitué au moins une fois, et/ou condensé avec un système cyclique mono- ou polycyclique non substitué ou substitué au moins une fois, les cycles du système cyclique étant chacun à 5, 6 ou 7 éléments ;

$M^1$ représente un radical aryle ou hétéroaryle à 5 ou 6 éléments, qui peut être substitué avec au moins un substituant supplémentaire et condensé avec un système cyclique mono- ou polycyclique non substitué ou substitué au moins une fois, les cycles du système cyclique étant chacun à 5, 6 ou 7 éléments ;

et $M^2$ représente un radical aryle ou hétéroaryle à 5 ou 6 éléments, qui peut être non substitué ou substitué au moins une fois, et qui peut être condensé avec un système cyclique mono- ou polycyclique non substitué ou substitué au moins une fois, les cycles du système cyclique étant chacun à 5, 6 ou 7 éléments ;

les radicaux cycloaliphatiques mentionnés précédemment pouvant éventuellement comprendre 1, 2, 3, 4 ou 5 hétéroatomes en tant qu'éléments de cycle, qui peuvent chacun être choisis indépendamment les uns des autres dans le groupe constitué par l'azote, l'oxygène et le soufre,

les radicaux hétérocycloaliphatiques mentionnés précédemment pouvant éventuellement comprendre 1, 2, 3, 4 ou 5 hétéroatomes supplémentaires en tant qu'éléments de cycle, qui peuvent chacun être choisis indépendamment les uns des autres dans le groupe constitué par l'azote, l'oxygène et le soufre,

les cycles du système cyclique mono- ou polycyclique pouvant chacun éventuellement comprendre 0, 1, 2 ou 3 hétéroatomes en tant qu'éléments de cycle, qui sont choisis indépendamment les uns des autres dans le groupe constitué par l'azote, l'oxygène et le soufre,

et

les radicaux hétéroaryle mentionnés précédemment pouvant éventuellement comprendre 1, 2, 3, 4 ou 5 hétéroatomes en tant qu'éléments de cycle, qui peuvent chacun être choisis indépendamment les uns des autres dans le groupe constitué par l'azote, l'oxygène et le soufre.

**3.** Composés selon la revendication 1 ou 2, **caractérisés en ce que** $R^1$ et $R^2$ représentent chacun indépendamment l'un de l'autre un radical hydrogène ; -F ; -Cl ; -Br ; -I ;-$NO_2$ ; -CN ; -$NH_2$ ; -$NHR^5$ ; -$NR^6R^7$ ; -C(=O)-$R^9$; -C(=O)-$NH_2$ ; -C(=O)-$NHR^{10}$ ; -C(=O)-$NR^{11}R^{12}$ ; -C(=O)-$OR^{13}$ ;-$(CH_2)_m$-C(=O)-$OR^{14}$ avec m = 1, 2 ou 3 ; -O-C(=O)-$R^{15}$ ;-$OR^{17}$ ; -$(CH_2)_o$-O-$R^{18}$ avec o = 1, 2 ou 3 ; -$S(=O)_2$-$NH_2$ ;-$SF_5$ ; -$(CH_2)_u$-O-$S(=O)_2$-$R^{31}$ avec u = 1, 2 ou 3 ; -$(CH_2)_v$-O-$S(=O)_2$-O-$R^{32}$ avec v = 1, 2 ou 3 ; -$(CH_2)_w$-O-P(=O)($OR^{33}$)($OR^{34}$) avec w = 1, 2 ou 3 ; un radical choisi dans le groupe constitué par méthyle, éthyle, n-propyle, isopropyle, n-butyle, isobutyle, 2-butyle, tert-butyle, n-pentyle, 2-pentyle, 3-pentyle, iso-pentyle, néo-pentyle, n-hexyle, n-heptyle, n-octyle, (2, 4, 4)-triméthylpent-2-yle, -$CF_3$, -$CF_2H$, -$CFH_2$, - ($CH_2$)-($CF_3$), -($CH_2$)-($CHF_2$), -($CH_2$)-($CH_2F$) et - ($CF_2$)-($CF_3$); un radical choisi dans le groupe constitué par cyclopropyle, cyclobutyle, cyclopentyle, cyclohexyle, cycloheptyle et cyclooctyle, qui peut à chaque fois être non substitué ou substitué avec éventuellement 1, 2, 3 ou 4 substituants choisis indépendamment les uns des autres dans le groupe constitué par F, Cl, Br, I,-CN, -$NO_2$, méthyle, éthyle, n-propyle, isopropyle, n-butyle, isobutyle, 2-butyle, tert-butyle, n-pentyle, néo-pentyle, -OH, oxo (=O), thioxo (=S), -O-$CH_3$, -O-$C_2H_5$,- O-$C_3H_7$, -O-C$(CH_3)_3$, -$CF_3$, -$CHF_2$, -$CH_2F$, -O-$CF_3$, -O-$CHF_2$ et -O-$CH_2F$ ;

ou un radical choisi dans le groupe constitué par phényle, benzyle, phénéthyle, (3-phényl)-prop-1-yle, furyle (furanyle), thiényle (thiophényle), pyrazolyle, imidazolyle, thiazolyle, thiadiazolyle, pyridinyle, pyridazinyle, pyrimidinyle et pyrazinyle, qui peut à chaque fois être non substitué ou substitué avec éventuellement 1, 2, 3, 4 ou 5 substituants choisis indépendamment les uns des autres dans le groupe constitué par F, Cl, Br, I, -CN, -$NO_2$, méthyle, éthyle, n-propyle, isopropyle, n-butyle, isobutyle, 2-butyle, tert-butyle, n-pentyle, néo-pentyle, éthényle, allyle, éthynyle, propynyle, -OH, -SH, -$SF_5$, -$NH_2$, -C(=O)-OH, -S-$CH_3$, -S-$C_2H_5$, -O-$CH_3$, -O-$C_2H_5$, -O-$C_3H_7$, -O-C$(CH_3)_3$, -$CF_3$,-$CHF_2$, -$CH_2F$, -O-$CF_3$, -O-$CF_2$, -O-$CH_2F$, -C(=O)-$CF_3$, -S-$CF_3$,-S-$CHF_2$, -S-$CH_2F$, -C(=O)-O-$CH_3$, -C(=O)-O-$C_2H_5$, -C(=O)-O-C$(CH_3)_3$, cyclopropyle, cyclobutyle, cyclopentyle, cyclohexyle et phényle ;

$R^3$ et $R^4$ représentent chacun indépendamment l'un de l'autre un radical hydrogène ; -C(=O)-$R^{21}$ ; -$(CH_2)_q$-C(=O)-$R^{22}$ avec q = 1, 2 ou 3 ; -C(=O)-O-$R^{23}$ ; -$(CH_2)_r$-C(=O)-O-$R^{24}$ avec r = 1, 2 ou 3 ; -C(=O)-$NHR^{25}$ ; -$(CH_2)_s$-C(=O)-$NHR^{26}$ avec s = 1 , 2 ou 3 ; un radical choisi dans le groupe constitué par méthyle, éthyle, n-propyle, isopropyle, n-butyle, isobutyle, 2-butyle, tert-butyle, n-pentyle, 2-pentyle, 3-pentyle, iso-pentyle, néo-pentyle, n-hexyle, n-heptyle, n-octyle et (2,4,4)-triméthylpent-2-yle ; un radical choisi dans le groupe constitué par cyclopropyle, cyclobutyle, cyclopentyle, cyclohexyle, cycloheptyle et cyclooctyle, qui peut à chaque fois être non substitué ou substitué avec éventuellement 1, 2, 3 ou 4 substituants choisis indépendamment les uns des autres dans le groupe constitué par F, Cl, Br, I, -CN, -$NO_2$, méthyle, éthyle, n-propyle, isopropyle, n-butyle, isobutyle, 2-butyle, tert-butyle, n-pentyle, néo-pentyle, -OH, oxo (=O), thioxo (=S), -O-$CH_3$, -O-$C_2H_5$, -O-$C_3H_7$ et -O-C$(CH_3)_3$ et/ou relié par un groupe alkylène en $C_{1-3}$ linéaire ou ramifié ; ou un radical choisi dans le groupe constitué par phényle, furyle (furanyle), thiényle (thiophényle), pyrazolyle, imidazolyle, thiazolyle, thiadiazolyle, pyridinyle, pyridazinyle, pyrimidinyle et pyrazinyle, qui peut à chaque fois être non substitué ou substitué avec éventuellement 1, 2, 3, 4 ou 5 substituants choisis indépendamment les uns des autres dans le groupe constitué par F, Cl, Br, I, -CN, -$NO_2$, méthyle, éthyle, n-propyle, isopropyle, n-butyle, isobutyle, 2-butyle, tert-butyle, n-pentyle, néo-pentyle, éthényle, allyle,-OH, -SH, -$SF_5$, -$NH_2$, -C(=O)-OH, -S-$CH_3$, -S-$C_2H_5$, -O-$CH_3$, -O-$C_2H_5$, -O-$C_3H_7$, -O-C$(CH_3)_3$, -$CF_3$, -$CHF_2$, -$CH_2F$, -O=$CF_3$, -O-$CHF_2$, -O-$CH_2F$, -C(=O)-$CF_3$, -S-$CF_3$, -S-$CHF_2$, -S-$CH_2F$,-N$(CH_3)_2$, -N$(C_2H_5)_2$, -NH-$CH_3$, -NH-$C_2H_5$, cyclopropyle, cyclobutyle et cyclopentyle, et/ou être relié par un groupe alkylène en $C_{1-3}$ linéaire ou ramifié ;

ou $R^3$ et $R^4$ forment ensemble avec l'atome d'azote qui les relie en tant qu'élément de cycle un radical choisi dans le groupe constitué par imidazolidinyle, pipéridinyle, pipérazinyle, morpholinyle, pyrrolidinyle, thiomorpholinyle, azépanyle, diazépanyle et azocanyle, qui peut à chaque fois être non substitué ou substitué avec éventuellement 1, 2, 3, 4 ou 5 substituants choisis indépendamment les uns des autres dans le groupe constitué par F, Cl, Br, I, -CN, méthyle, éthyle, n-propyle, isopropyle, n-butyle, 2-butyle, isobutyle, tert-butyle, -OH, oxo, thioxo, -O-$CH_3$, -O-$C_2H_5$, -O-$C_3H_7$, -$NH_2$, -N$(CH_3)_2$, -N$(C_2H_5)_2$, -NH-$CH_3$, -NH-$C_2H_5$, -$NO_2$,-$CF_3$, -O-$CF_3$, -S-$CF_3$, -SH, -$SF_5$, -S-$CH_3$, -S-$C_2H_5$, -S(=O)-$CH_3$, -$S(=O)_2$-$CH_3$, -S(=O)-$C_2H_5$, -$S(=O)_2$-$C_2H_5$, -C(=O)-$CH_3$, -C(=O)-$C_2H_5$, -C(=O)-N$(CH_3)_2$, -C(=O)-NH-$CH_3$, -C(=O)-$NH_2$, -C(=O)-O-$CH_3$, -C(=O)-O-$C_2H_5$, -C(=O)-O-C$(CH_3)_3$ et phényle ;

$R^5$, $R^6$, $R^7$, $R^{10}$, $R^{11}$, $R^{12}$ et $R^{15}$ représentent chacun indépendamment les uns des autres un radical choisi dans le groupe constitué par méthyle, éthyle, n-propyle, isopropyle, n-butyle, isobutyle, 2-butyle, tert-butyle, n-pentyle, 2-pentyle, 3-pentyle, iso-pentyle, néo-pentyle, n-hexyle, n-heptyle, n-octyle, (2,4,4)-triméthylpent-2-yle, -$CF_3$, -$CF_2H$, -$CFH_2$, -($CH_2$)-($CHF_2$),-($CH_2$)-($CH_2F$), -($CF_2$)-($CF_3$), -($CH_2$)-($CH_2$)-C(=O)-OH, -($CH_2$)-($CH_2$)-C(=O)-O-$CH_3$ et -($CH_2$)-($CH_2$)-C(=O)-O-$C_2H_5$ ; ou un radical choisi dans le groupe constitué par phényle, benzyle, phénéthyle, furyle (furanyle), thiényle (thiophényle), pyrazolyle, imidazolyle, thiazolyle, thiadiazolyle, pyridinyle, pyridazinyle, pyrimidinyle et pyrazinyle, qui peut à chaque fois être non substitué ou substitué avec éventuellement 1, 2, 3, 4 ou 5 substituants choisis indépendamment les uns des autres dans le groupe constitué par F, Cl, Br, I, -CN, -$NO_2$, méthyle, éthyle, n-propyle, isopropyle, n-butyle, isobutyle, 2-butyle, tert-butyle, n-pentyle, néo-pentyle, -OH, -SH, -$SF_5$, -$NH_2$, -C(=O)-OH, -S-$CH_3$, -S-$C_2H_5$, -O-$CH_3$, -O-$C_2H_5$, -O-$C_3H_7$, -O-C$(CH_3)_3$, -$CF_3$, -$CHF_2$, -$CH_2F$, -O-$CF_3$, -O-$CHF_2$, -O-$CH_2F$ et -C(=O)-$CF_3$ ;

$R^9$, $R^{13}$, $R^{14}$, $R^{17}$, $R^{18}$, $R^{21}$, $R^{22}$, $R^{23}$, $R^{24}$, $R^{25}$, $R^{26}$, $R^{31}$, $R^{32}$, $R^{33}$ et $R^{34}$ représentent chacun indépendamment les uns des autres un radical hydrogène ; un radical choisi dans le groupe constitué par méthyle, éthyle, n-propyle, isopropyle, n-butyle, isobutyle, 2-butyle, tert-butyle, n-pentyle, 2-pentyle, 3-pentyle, iso-pentyle, néo-pentyle, n-hexyle, n-heptyle, n-octyle, (2,4,4)-triméthylpent-2-yle, $-CF_3$, $-CF_2H$, $-CFH_2$, $-(CH_2)-(CF_3)$, $(CH_2)-(CHF_2)$, $-(CH_2)-(CH_2F)$ et $-(CF_2)-(CF_3)$ ; ou un radical choisi dans le groupe constitué par phényle, benzyle, phénéthyle, furyle (furanyle), thiényle (thiophényle), pyrazolyle, imidazolyle, thiazolyle, thiadiazolyle, pyridinyle, pyridazinyle, pyrimidinyle et pyrazinyle, qui peut à chaque fois être non substitué ou substitué avec éventuellement 1, 2, 3, 4 ou 5 substituants choisis indépendamment les uns des autres dans le groupe constitué par F, Cl, Br, I, -CN, $-NO_2$, méthyle, éthyle, n-propyle, isopropyle, n-butyle, isobutyle, 2-butyle, tert-butyle, n-pentyle, néo-pentyle, -OH, -SH, $-SF_5$,$-NH_2$, -C(=O)-OH, $-S-CH_3$, $-S-C_2H_5$, $-O-CH_2$, $-O-C_2H_5$, $-O-C_3H_7$,$-O-C(CH_3)_3$,- $CF_3$, $-CHF_2$, $-CH_2F$, $-O-CF_3$, $-O-CHF_2$, $-O-CH_2F$ et-$C(=O)-CF_3$;

$M^1$ représente un radical choisi dans le groupe constitué par les radicaux 1 à 9, 11, 21, 22 et 36 à 38

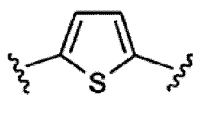
1

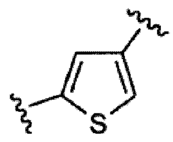
2

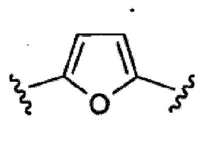
3

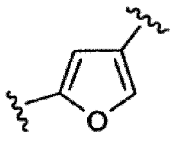
4

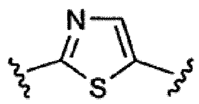
5

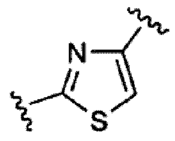
6

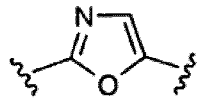
7

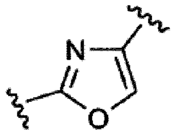
8

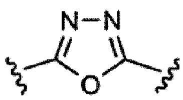
9

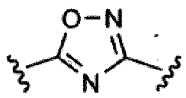
11

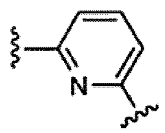
21

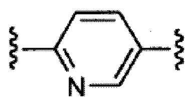
22

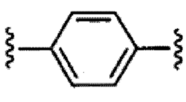
36

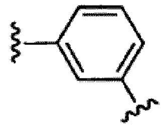
37

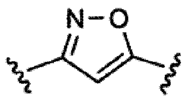
38

qui peut à chaque fois être non substitué ou substitué avec éventuellement 1 ou 2 substituants choisis indépendamment l'un de l'autre dans le groupe constitué par F, Cl, Br, I, -CN, $-NO_2$, méthyle, éthyle, n-propyle, isopropyle, n-butyle, isobutyle, 2-butyle, tert-butyle, n-pentyle, néo-pentyle, éthényle, allyle, éthynyle, propynyle, -OH, -SH, $-SF_5$, $-NH_2$, -C(=O)-OH, $-S-CH_3$, $-S-C_2H_5$, $-S(=O)-CH_3$, $-S(=O)_2-CH_3$, $-S(=O)-C_2H_5$, $-S(=O)_2-C_2H_5$, $-O-CH_3$, $-O-C_2H_5$, $-O-C_3H_7$, $-O-C(CH_3)_3$, $-CF_3$, $-CHF_2$, $-CH_2F$, $-O-CF_3$, $-O-CHF_2$, $-O-CH_2F$, $-C(=O)-CF_3$, $-S-CF_3$, $-S-CHF_2$ et $-S-CH_2F$, et qui peut à chaque fois être relié dans une direction quelconque aux positions **caractérisées par** une ligne ondulée avec le bicycle et l'atome de carbone de la triple liaison ;

et $M^2$ représente un radical choisi dans le groupe constitué par les radicaux 1 à 36

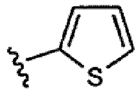
1

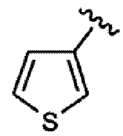
2

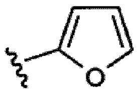
3

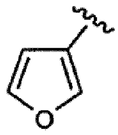
4

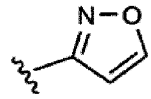
5

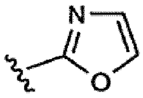
6

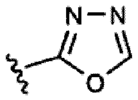
7

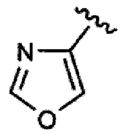
8

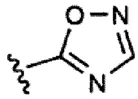
9

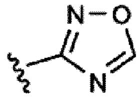
10

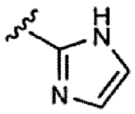
11

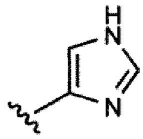
12

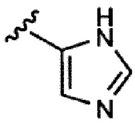
13

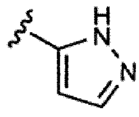
14

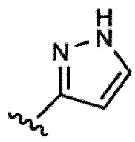
15

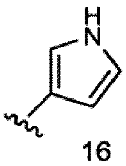
16

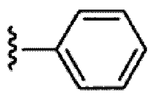
17

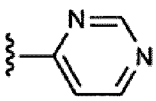
18

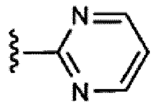
19

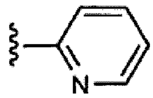
20

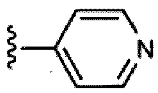
21

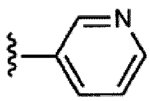
22

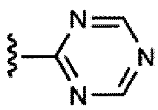
23

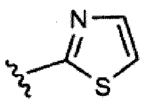
24

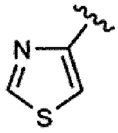
25

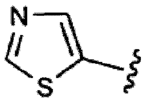
26

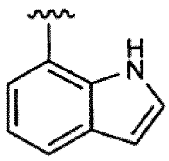
27

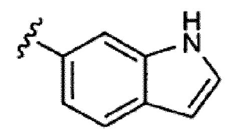
28

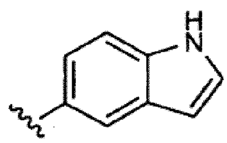
29

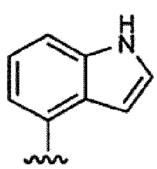
30

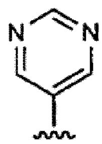
31

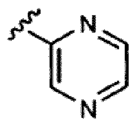
32

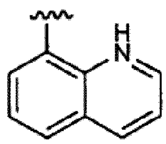
33

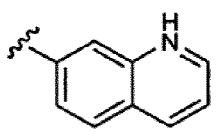

34

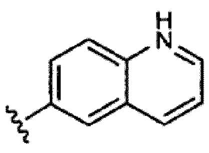

35

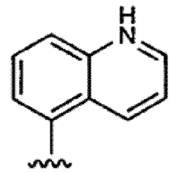

36

qui peut à chaque fois être relié à la position **caractérisée par** une ligne ondulée avec l'atome de carbone de la triple liaison et être non substitué ou substitué avec éventuellement 1, 2, 3, 4 ou 5 substituants choisis indépendamment les uns des autres dans le groupe constitué par F, Cl, Br, -CN,-$CF_3$, -$SF_5$, -S-$CH_3$, -S-$C_2H_5$, méthyle, éthyle, n-propyle, isopropyle, n-butyle, sec-butyle, iso-butyle, tert-butyle, éthényle, propényle, -OH, -O-$CH_3$, -O-$C_2H_5$, -O-$C_3H_7$, -$NH_2$, -N$(CH_3)_2$, -NH-$CH_3$, -$CH_2$-$NH_2$, -N$(C_2H_5)_2$, -$NO_2$, -O-$CF_3$,-S-$CF_3$, -SH, -C(=O)-H, -C(=O)-O-$CH_3$, -C(=O)-O-$C_2H_5$, phényle, pyrrolyle, (1,3)-dioxolanyle,-Si (phényl)$_2$ [C$(CH_3)_3$], -C (=O) -CH3, -C(=O)-$C_2H_5$, NH-S$(=O)_2$-$CH_3$, -NH-S(=O) $_2$-$C_2H_5$, -S(=O) $_2$-$NH_2$, -S(=O) $_2$-NH-$CH_3$, -$CH_2$-OH, -C(=O)-OH, -$CH_2$-O-$CH_3$, -C(=O)-$NH_2$, -C(=O)-NH-$CH_3$, -NH-C(=O)-$CH_3$, -NH-C(=NH)-$NH_2$, -NH-S$(=O)_2$-OH et -S$(=O)_2$-N$(CH_3)_2$ ;
à chaque fois éventuellement sous la forme d'un de leurs stéréoisomères purs, notamment énantiomères ou diastéréomères, de leurs racémats ou sous la forme d'un mélange de stéréoisomères, notamment les énantiomères et/ou diastéréomères, en un rapport de mélange quelconque, ou à chaque fois sous la forme des sels correspondants, ou à chaque fois sous la forme des solvates correspondants.

4. Composés selon une ou plusieurs des revendications 1 à 3, **caractérisés en ce que** $R^1$ et $R^2$ représentent chacun indépendamment l'un de l'autre un radical hydrogène ; -F ; -Cl ; -Br ; -I ;-$NO_2$ ; -CN ; -NH$R^5$ ; -N$R^6R^7$; -C(=O)-$R^9$, -C(=O)-O$R^{13}$ ; - ($CH_2$)-O-C(=O)-$R^{16}$ ; -O$R^{17}$; -($CH_2$)-O-S$(=O)_2$-$R^{31}$; -($CH_2$)-OS$(=O)_2$-O-$R^{32}$; -($CH_2$)-O-P(=O)(O$R^{33}$)(O$R^{34}$); -$SF_5$; un radical choisi dans le groupe constitué par méthyle, éthyle, n-propyle, isopropyle, n-butyle, isobutyle, 2-butyle, tert-butyle, n-pentyle, -$CF_3$, -$CF_2$H, -$CFH_2$, -($CH_2$)-($CF_3$), -($CH_2$)-($CHF_2$), -($CH_2$)-($CH_2$F) et -($CF_2$)-($CF_3$) ; un radical choisi dans le groupe constitué par cyclopropyle, cyclobutyle, cyclopentyle, cyclohexyle, cycloheptyle et cyclooctyle ;
ou un radical choisi dans le groupe constitué par phényle, benzyle, phénéthyle et (3-phényl)-prop-1-yle, qui peut à chaque fois être non substitué ou substitué avec éventuellement 1, 2, 3, 4 ou 5 substituants choisis indépendamment les uns des autres dans le groupe constitué par F, Cl, Br, I, -CN, -$NO_2$, méthyle, éthyle, n-propyle, isopropyle, n-butyle, isobutyle, 2-butyle, tert-butyle, -O-$CH_3$, -O-$C_2H_5$, -O-$C_3H_7$, -O-C$(CH_3)_3$, -$CF_3$,-$CHF_2$, -$CH_2$F, -O-$CF_3$, -O-$CHF_2$ et -O-$CH_2$F ;
$R^3$ et $R^4$ représentent chacun indépendamment l'un de l'autre un radical hydrogène ; -C(=O)-$R^{21}$ ; -$(CH_2)_q$-C(=O)-$R^{22}$ avec q = 1, 2 ou 3; -$(CH_2)_r$-C(=O)-O-$R^{24}$ avec r = 1, 2 ou 3 ; un radical choisi dans le groupe constitué par méthyle, éthyle, n-propyle, isopropyle, n-butyle, isobutyle, 2-butyle, tert-butyle, n-pentyle, 2-pentyle, 3-pentyle, iso-pentyle, néo-pentyle, n-hexyle, n-heptyle, n-octyle et (2,4,4)-triméthylpent-2-yle ; un radical choisi dans le groupe constitué par cyclopropyle, cyclobutyle, cyclopentyle, cyclohexyle, cycloheptyle et cyclooctyle, qui peut être relié par un groupe -($CH_2$)-, -$(CH_2)_2$-, -(CH($CH_3$))- ou -$(CH_2)_3$- ;
ou un radical choisi dans le groupe constitué par phényle, furyle (furanyle), thiényle (thiophényle), pyridinyle, pyridazinyle, pyrimidinyle et pyrazinyle, qui peut à chaque fois être non substitué ou substitué avec éventuellement 1, 2, 3, 4 ou 5 substituants choisis indépendamment les uns des autres dans le groupe constitué par F, Cl, Br, I, -CN, -$NO_2$, méthyle, éthyle, n-propyle, isopropyle, n-butyle, isobutyle, 2-butyle, tert-butyle, -O-$CH_3$, -O-$C_2H_5$, -O-$C_3H_7$, -O-C$(CH_3)_3$, -$CF_3$,-$CHF_2$, -$CH_2$F, -O-$CF_3$, -O-$CHF_2$ et -O-$CH_2$F et/ou relié par un groupe -($CH_2$)-, -$(CH_2)_2$-, -(CH($CH_3$))- ou -$(CH_2)_3$- ;
ou $R^3$ et $R^4$ forment ensemble avec l'atome d'azote qui les relie en tant qu'élément de cycle un radical choisi dans le groupe constitué par pipéridinyle, pipérazinyle, morpholinyle et pyrrolidinyle, qui peut à chaque fois être non substitué ou substitué avec éventuellement 1, 2, 3, 4 ou 5 substituants choisis indépendamment les uns des autres dans le groupe constitué par méthyle, éthyle, n-propyle, isopropyle, n-butyle, 2-butyle, isobutyle, tert-butyle, -S(=O)-$CH_3$, -S$(=O)_2$-$CH_3$, -S(=O)-$C_2H_5$,-S$(=O)_2$-$C_2H_5$, -C(=O)-$CH_3$, -C(=O)-$C_2H_5$, -C(=O)-N$(CH_3)_2$,-C(=O)-NH-$CH_3$, -C(=O)-$NH_2$, -C(=O)-O-$CH_3$, -C(=O)-O-$C_2H_5$ et-C(=O)-O-C$(CH_3)_3$ ;
$R^5$, $R^6$, $R^7$ et $R^{16}$ représentent chacun indépendamment les uns des autres un radical choisi dans le groupe constitué par méthyle, éthyle, n-propyle, isopropyle, n-butyle, isobutyle, 2-butyle, tert-butyle, n-pentyle, 2-pentyle, 3-pentyle, iso-pentyle, néo-pentyle, n-hexyle, n-heptyle, n-octyle, (2,4,4)-triméthylpent-2-yle, -$CF_3$, -$CF_2$H, -$CFH_2$, -($CH_2$)-($CHF_2$), -($CH_2$)-($CH_2$F), -($CF_2$)-($CF_3$),-($CH_2$)-($CH_2$)-C(=O)-OH, -($CH_2$)-($CH_2$)-C(=O)-O-$CH_3$ et -($CH_2$)-($CH_2$)-C(=O)-O-$C_2H_5$ ; ou un radical choisi dans le groupe constitué par phényle, benzyle et phénéthyle, qui

peut à chaque fois être non substitué ou substitué avec éventuellement 1, 2, 3, 4 ou 5 substituants choisis indépendamment dans le groupe constitué par F, Cl, Br, I, -CN, $-NO_2$, méthyle, éthyle, n-propyle, isopropyle, n-butyle, isobutyle, 2-butyle, tert-butyle, $-O-CH_3$ et $-O-C_2H_5$

$R^9$, $R^{13}$, $R^{17}$, $R^{21}$, $R^{22}$, $R^{24}$, $R^{31}$, $R^{32}$, $R^{33}$ et $R^{34}$ représentent chacun indépendamment les uns des autres un radical hydrogène ; un radical choisi dans le groupe constitué par méthyle, éthyle, n-propyle, isopropyle, n-butyle, isobutyle, 2-butyle, tert-butyle, n-pentyle, 2-pentyle, 3-pentyle, iso-pentyle, néo-pentyle, n-hexyle, n-heptyle, n-octyle, (2,4,4)-triméthylpent-2-yle, $-CF_3$,$-CF_2H$, $-CFH_2$, $-(CH_2)-(CF_3)$, $-(CH_2)-(CHF_2)$, $-(CH_2)-(CH_2F)$ et - $(CF_2)-(CF_3)$ ; ou un radical choisi dans le groupe constitué par phényle, benzyle et phénéthyle, qui peut à chaque fois être non substitué ou substitué avec éventuellement 1, 2, 3, 4 ou 5 substituants choisis indépendamment les uns des autres dans le groupe constitué par F, Cl, Br, I, -CN, $-NO_2$, méthyle, éthyle, n-propyle, isopropyle, n-butyle, isobutyle, 2-butyle, tert-butyle, $-O-CH_3$ et $-O-C_2H_5$ ;

$M^1$ représente un radical choisi dans le groupe constitué par les radicaux 1 à 6, 21, 22, 36 et 37

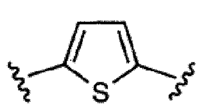
1

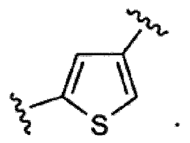
2

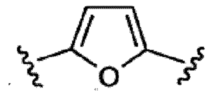
3

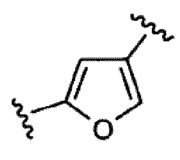
4

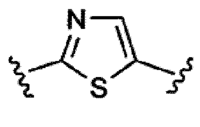
5

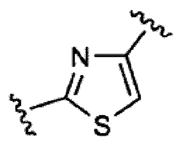
6

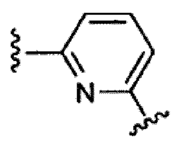
21

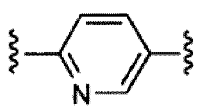
22

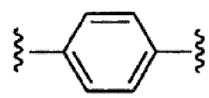
36

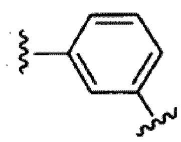
37

qui peut à chaque fois être non substitué ou substitué avec éventuellement 1 ou 2 substituants choisis indépendamment l'un de l'autre dans le groupe constitué par F, Cl, Br, I, -CN, $-CH_2-CN$, $-NO_2$, méthyle, éthyle, n-propyle, isopropyle, n-butyle, isobutyle, 2-butyle, tert-butyle, n-pentyle, néo-pentyle, $-SF_5$, $-O-CH_3$, $-O-C_2H_5$, $-O-C_3H_7$, $-O-C(CH_3)_3$, $-CF_3$, $-CHF_2$, $-CH_2F$, $-O-CF_3$, $-O-CHF_2$ et $-O-CH_2F$ , et qui peut à chaque fois être relié dans une direction quelconque aux positions **caractérisées par** une ligne ondulée avec le bicycle et l'atome de carbone de la triple liaison ;

et $M^2$ représente un radical choisi dans le groupe constitué par phényle, 2-pyridinyle, 3-pyridinyle, 4-pyridinyle, 2-thiazolyle, 4-thiazolyle, 5-thiazolyle, 4-indolyle, 5-indolyle, 6-indolyle, 7-indolyle, 2-thiophényle (2-thiényle), 3-thiophényle (3-thiényle), 2-imidazolyle, 4-imidazolyle, 5-imidazolyle, 2-pyrimidinyle, 4-pyrimidinyle, 5-pyrimidinyle, 2-pyrazinyle, 5-quinolinyle, 6-quinolinyle, 7-quinolinyle et 8-quinolinyle, qui peut à chaque fois être non substitué ou substitué avec éventuellement 1, 2, 3, 4 ou 5 substituants choisis indépendamment les uns des autres dans le groupe constitué par F, Cl, Br, -CN, $-CF_3$, $-SF_5$, $-S-CH_3$, $-S-C_2H_5$, méthyle, éthyle, n-propyle, iso-propyle, n-butyle, sec-butyle, iso-butyle, tert-butyle, éthényle, propényle, -OH, $-O-CH_3$, $-O-C_2H_5$, $-O-C_3H_7$, $-NH_2$, $-N(CH_3)_2$,$-NH-CH_3$, $-CH_2-NH_2$, $-N(C_2H_5)_2$, $-NO_2$, $-O-CF_3$, $-S-CF_3$, -SH,-C(=O)-H, $-C(=O)-O-CH_3$, $-C(=O)-O-C_2H_5$, phényle, pyrrolyle, (1,3)-dioxolanyle, $-Si(phényl)_2[C(CH_3)_3]$, $-C(=O)-CH_3$,$-C(=O)-C_2H_5$, $NH-S(=O)_2-CH_3$, $-NH-S(=O)_2-C_2H_5$, $-S(=O)_2-NH_2$,$-S(=O)_2-NH-CH_3$, $-CH_2-OH$, -C(=O)-OH, $-CH_2-O-CH_3$, $-C(=O)-NH_2$, $-C(=O)-NH-CH_3$, $-NH-C(=O)-CH_3$, $-NH-C(=NH)-NH_2$, $-NH-S(=O)_2-OH$ et $-S(=O)_2-N(CH_3)_2$ ;

à chaque fois éventuellement sous la forme d'un de leurs stéréoisomères purs, notamment énantiomères ou diastéréomères, de leurs racémats ou sous la forme d'un mélange de stéréoisomères, notamment les énantiomères et/ou diastéréomères, en un rapport de mélange quelconque, ou à chaque fois sous la forme des sels correspondants, ou à chaque fois sous la forme des solvates correspondants.

**5.** Composés de formule générale Ia selon la revendication 4

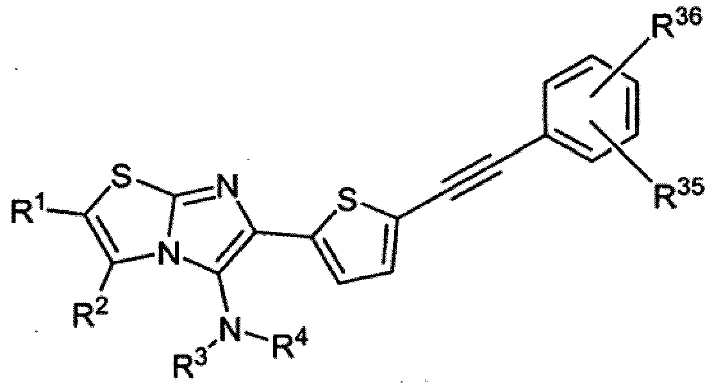

**Ia,**

dans laquelle
$R^1$, $R^2$, $R^3$ et $R^4$ ont la signification selon la revendication 4,
et $R^{35}$ et $R^{36}$ représentent chacun indépendamment l'un de l'autre un radical choisi dans le groupe constitué par H, F, Cl, Br, -CN, $-CF_3$, $-SF_5$, $-S-CH_3$, $-S-C_2H_5$, méthyle, éthyle, n-propyle, iso-propyle, n-butyle, sec-butyle, iso-butyle, tert-butyle, éthényle, propényle, -OH, $-O-CH_3$, $-O-C_2H_5$, $-O-C_3H_7$, $-NH_2$, $-N(CH_3)_2$, $-NH-CH_3$, $-CH_2-NH_2$,$-N(C_2H_5)_2$, $-NO_2$, $-O-CF_3$, $-S-CF_3$, -SH, -C(=O)-H, $-C(=O)-O-CH_3$, $-C(=O)-O-C_2H_5$, phényle, pyrrolyle, (1, 3)-dioxolanyle, $-Si(phényl)_2[C(CH_3)_3]$, $-C(=O)-CH_3$, $-C(=O)-C_2H_5$, $NH-S(=O)_2-CH_3$, $-NH-S(=O)_2-C_2H_5$, $-S(=O)_2-NH_2$, $-S(=O)_2-NH-CH_3$, $-CH_2-OH$, -C(=O)-OH, $-CH_2-O-CH_3$, $-C(=O)-NH_2$, $C(=O)-NH-CH_3$, $-NH-C(=O)-CH_3$, $-NH-C(=NH)-NH_2$, $-NH-S(=O)_2-OH$ et $-S(=O)_2-N(CH_3)_2$ ;
à chaque fois éventuellement sous la forme des sels correspondants ou à chaque fois éventuellement sous la forme des solvates correspondants.

**6.** Composés de formule générale Ib selon la revendication 4

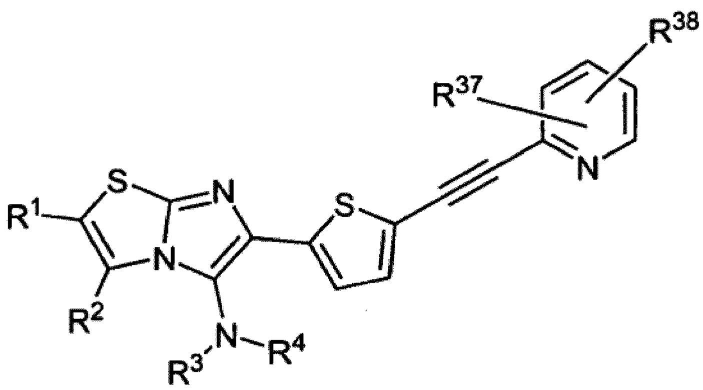

**Ib,**

dans laquelle

$R^1$, $R^2$, $R^3$ et $R^4$ ont la signification selon la revendication 4,
et $R^{37}$ et $R^{38}$ représentent chacun indépendamment l'un de l'autre un radical choisi dans le groupe constitué

par H, F, Cl, Br, -CN, $-CF_3$, $-SF_5$, $-S-CH_3$, $-S-C_2H_5$, méthyle, éthyle, n-propyle, iso-propyle, n-butyle, sec-butyle, iso-butyle, tert-butyle, éthényle, propényle, -OH, $-O-CH_3$, $-O-C_2H_5$, $-O-C_3H_7$, $-NH_2$, $-N(CH_3)_2$, $-NH-CH_3$, $-CH_2-NH_2$,$-N(C_2H_5)_2$, $-NO_2$, $-O-CF_3$, $-S-CF_3$, -SH, -C(=O)-H, $-C(=O)-O-CH_3$, $-C(=O)-O-C_2H_5$, phényle, pyrrolyle, (1, 3)-dioxolanyle, $-Si(phényl)_2[C(CH_3)_3]$, $-C(=O)-CH_3$, $-C(=O)-C_2H_5$, $NH-S(=O)_2-CH_3$, $-NH-S\ (=0)\ _2-C_2H_5$, $-S(=O)_2-NH_2$, $-S(=O)_2-NH-CH_3$, $-CH_2-OH$, -C(=O)-OH, $-CH_2-O-CH_3$, $-C(=O)-NH_2$, $C(=O)-NH-CH_3$, -NH-$C(=O)-CH_3$, $-NH-C(=NH)-NH_2$, $-NH-S(=O)_2-OH$ et $-S(=O)_2-N(CH_3)_2$;
à chaque fois éventuellement sous la forme des sels correspondants ou à chaque fois éventuellement sous la forme des solvates correspondants.

**7.** Composés de formule générale Ic selon la revendication 4

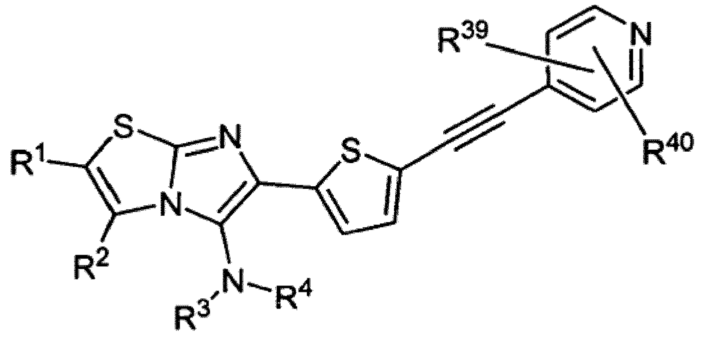

Ic,

dans laquelle

$R^1$, $R^2$, $R^3$ et $R^4$ ont la signification selon la revendication 4,
et $R^{39}$ et $R^{40}$ représentent chacun indépendamment l'un de l'autre un radical choisi dans le groupe constitué par H, F, Cl, Br, -CN, $-CF_3$, $-SF_5$, $-S-CH_3$, $-S-C_2H_5$, méthyle, éthyle, n-propyle, iso-propyle, n-butyle, sec-butyle, iso-butyle, tert-butyle, éthényle, propényle, -OH, $-O-CH_3$, $-O-C_2H_5$, $-O-C_3H_7$, $-NH_2$, $-N(CH_3)_2$, $-NH-CH_3$, $-CH_2-NH_2$,$-N(C_2H_5)_2$, $-NO_2$, $-O-CF_3$, $-S-CF_3$, -SH, -C(=O)-H, $-C(=O)-O-CH_3$, $-C(=O)-O-C_2H_5$, phényle, pyrrolyle, (1, 3)-dioxolanyle, $-Si(phényl)_2[C(CH_3)_3]$, $-C(=O)-CH_3$, $-C(=O)-C_2H_5$, $NH-S(=O)_2-CH_3$, $-NH-S(=O)_2-C_2H_5$, $-S(=O)_2-NH_2$, $-S(=O)_2-NH-CH_3$, $-CH_2-OH$, -C(=O)-OH, $-CH_2-O-CH_3$, $-C(=O)-NH_2$,$-C(=O)-NH-CH_3$, -NH-$C(=O)-CH_3$, $-NH-C(=NH)-NH_2$, $-NH-S(=O)_2-OH$ et $-S(=O)_2-N(CH_3)_2$;
à chaque fois éventuellement sous la forme des sels correspondants ou à chaque fois éventuellement sous la forme des solvates correspondants.

**8.** Composés de formule générale Id selon la revendication 4

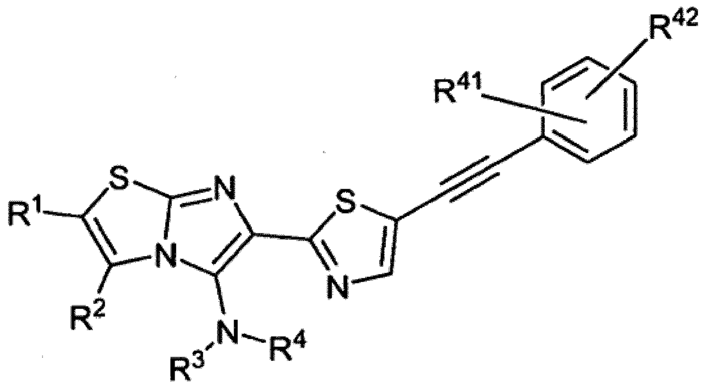

Id,

dans laquelle

$R^1$, $R^2$, $R^3$ et $R^4$ ont la signification selon la revendication 4,
et $R^{41}$ et $R^{42}$ représentent chacun indépendamment l'un de l'autre un radical choisi dans le groupe constitué par H, F, Cl, Br, -CN, $-CF_3$, $-SF_5$, $-S-CH_3$, $-S-C_2H_5$, méthyle, éthyle, n-propyle, iso-propyle, n-butyle, sec-butyle, iso-butyle, tert-butyle, éthényle, propényle, -OH, -0-CH3, $-O-C_2H_5$, $-O-C_3H_7$, $-NH_2$, $-N(CH_3)_2$, $-NH-CH_3$, $-CH_2-NH_2$,$-N(C_2H_5)_2$, $-NO_2$, $-O-CF_3$, $-S-CF_3$, -SH, -C(=O)-H, $-C(=O)-O-CH_3$, $-C(=O)-O-C_2H_5$, phényle, pyrrolyle, (1, 3)-dioxolanyle, $-Si(phényl)_2[C(CH_3)_3]$, $-C(=O)-CH_3$, $-C(=O)-C_2H_5$, $NH-S(=O)_2-CH_3$, $-NH-S(=0)\ _2-C_2H_5$, $-S(=O)_2-NH_2$, $-S(=O)_2-NH-CH_3$, $-CH_2-OH$, -C(=O)-OH, $-CH_2-O-CH_3$, $-C(=O)-NH_2$,$-C(=O)-NH-CH_3$, $-NH-C(=O)-CH_3$, $-NH-C(=NH)-NH_2$, $-NH-S(=O)_2-OH$ et $-S(=O)_2-N(CH_3)_2$ ;
à chaque fois éventuellement sous la forme des sels correspondants ou à chaque fois éventuellement sous la forme des solvates correspondants.

**9.** Composés de formule générale Ie selon la revendication 4

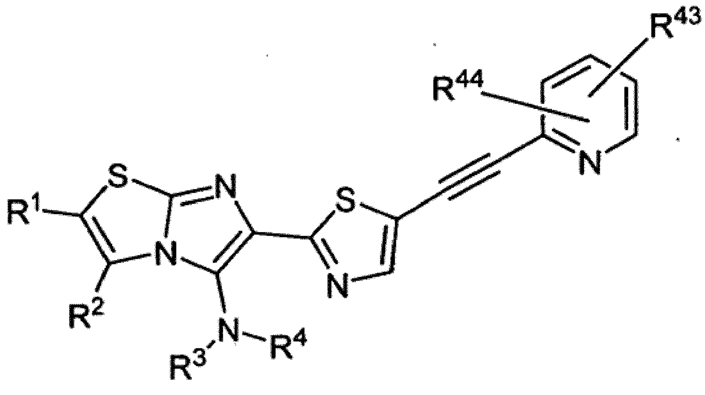

**Ie,**

dans laquelle

$R^1$, $R^2$, $R^3$ et $R^4$ ont la signification selon la revendication 4,
et $R^{43}$ et $R^{44}$ représentent chacun indépendamment l'un de l'autre un radical choisi dans le groupe constitué par H, F, Cl, Br, -CN, $-CF_3$, $-SF_5$, $-S-CH_3$, $-S-C_2H_5$, méthyle, éthyle, n-propyle, iso-propyle, n-butyle, sec-butyle, iso-butyle, tert-butyle, éthényle, propényle, -OH, -0-CH3, $-O-C_2H_5$, $-O-C_3H_7$, $-NH_2$, $-N(CH_3)_2$, $-NH-CH_3$, $-CH_2-NH_2$,$-N(C_2H_5)_2$, $-NO_2$, $-O-CF_3$, $-S-CF_3$, -SH, -C(=O)-H, $-C(=O)-O-CH_3$, $-C(=O)-O-C_2H_5$, phényle, pyrrolyle, (1, 3)-dioxolanyle, $-Si(phényl)_2[C(CH_3)_3]$, $-C(=O)-CH_3$, $-C(=O)-C_2H_5$, $NH-S(=O)_2-CH_3$, $-NH-S\ (=0)\ _2-C_2H_5$, $-S(=O)_2-NH_2$, $-S(=O)_2-NH-CH_3$, $-CH_2-OH$, -C(=O)-OH, $-CH_2-O-CH_3$, $-C(=O)-NH_2$,$-C(=O)-NH-CH_3$, $-NH-C(=O)-CH_3$, $-NH-C(=NH)-NH_2$, $-NH-S(=O)_2-OH$ et $-S(=O)_2-N(CH_3)_2$;
à chaque fois éventuellement sous la forme des sels correspondants ou à chaque fois éventuellement sous la forme des solvates correspondants.

**10.** Composés de formule générale If selon la revendication 4

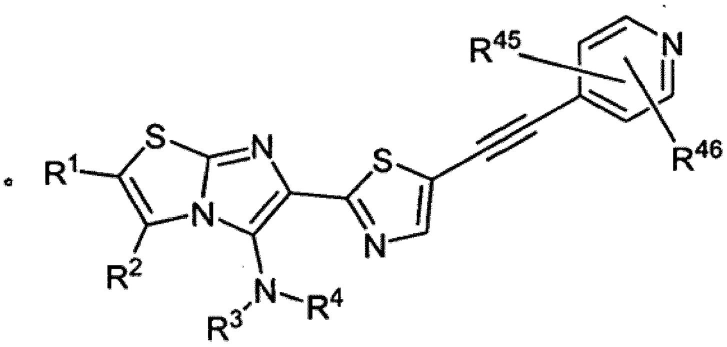

If,

dans laquelle

$R^1$, $R^2$, $R^3$ et $R^4$ ont la signification selon la revendication 4,
et $R^{45}$ et $R^{46}$ représentent chacun indépendamment l'un de l'autre un radical choisi dans le groupe constitué par H, F, Cl, Br, -CN, $-CF_3$, $-SF_5$, $-S-CH_3$, $-S-C_2H_5$, méthyle, éthyle, n-propyle, iso-propyle, n-butyle, sec-butyle, iso-butyle, tert-butyle, éthényle, propényle, -OH, -O-CH3, $-O-C_2H_5$, $-O-C_3H_7$, $-NH_2$, $-N(CH_3)_2$, $-NH-CH_3$, $-CH_2-NH_2$,$-N(C_2H_5)_2$, $-NO_2$, $-O-CF_3$, $-S-CF_3$, -SH, -C(=O)-H, $-C(=O)-O-CH_3$, $-C(=O)-O-C_2H_5$, phényle, pyrrolyle, (1,3)-dioxolanyle, $-Si(phényl)_2[C(CH_3)_3]$, $-C(=O)-CH_3$, $-C(=O)-C_2H_5$, $NH-S(=O)_2-CH_3$, $-NH-S(=O)_2-C_2H_5$, $-S(=O)_2-NH_2$, $-S(=O)_2-NH-CH_3$, $-CH_2-OH$, -C(=O)-OH, $-CH_2-O-CH_3$, $-C(=O)-NH_2$,$-C(=O)-NH-CH_3$, $-NH-C(=O)-CH_3$, $-NH-C(=NH)-NH_2$, $-NH-S(=O)_2-OH$ et $-S(=O)_2-N(CH_3)_2$ ;
à chaque fois éventuellement sous la forme des sels correspondants ou à chaque fois éventuellement sous la forme des solvates correspondants.

**11.** Composés selon une ou plusieurs des revendications 1 à 10, **caractérisés en ce que** $R^1$ représente un radical hydrogène ; -F ; -Cl ; -Br ;-CN ; $-O-CH_3$ ; $-C(=O)-R^9$, $-C(=O)-OR^{13}$ ; $-(CH_2)-O-C(=O)-R^{16}$ ; $-(CH_2)-O-S(=O)_2-R^{31}$; $-(CH_2)-O-S(=O)_2-O-R^{32}$; $-(CH_2)-O-P(=O)(OR^{33})(OR^{34})$ ; ou un radical choisi dans le groupe constitué par méthyle, éthyle, n-propyle, isopropyle, n-butyle, isobutyle, 2-butyle, tert-butyle, n-pentyle,$-CF_3$, $-CF_2H$, $-CFH_2$, $-(CH_2)-(CF_3)$, $-(CH_2)-(CHF_2)$, $-(CH_2)-(CH_2F)$ et $-(CF_2)-(CF_3)$ ;
$R^2$ représente un radical hydrogène ; -F ; -Cl ; -Br ;-CN ; $-O-CH_3$ ; $-C(=O)-R^9$, $-C(=O)-OR^{13}$ ; $-(CH_2)-O-C(=O)-R^{16}$ ; $-(CH_2)-O-S(=O)_2-R^{31}$; $-(CH_2)-O-S(=O)_2-O-R^{32}$; $-(CH_2)-O-P(=O)(OR^{33})(OR^{34})$; ou un radical choisi dans le groupe constitué par méthyle, éthyle, n-propyle, isopropyle, n-butyle, isobutyle, 2-butyle, tert-butyle, n-pentyle,$-CF_3$, $-CF_2H$, $-CFH_2$, $-(CH_2)-(CF_3)$, $-(CH_2)-(CHF_2)$, $-(CH_2)-(CH_2F)$ et $-(CF_2)-(CF_3)$ ;
$R^3$ représente un radical hydrogène ou un radical choisi dans le groupe constitué par méthyle, éthyle et isopropyle ;
$R^4$ représente un radical hydrogène ; $-C(=O)-R^{21}$ ou un radical choisi dans le groupe constitué par méthyle, éthyle, n-propyle, isopropyle, n-butyle, isobutyle, 2-butyle, tert-butyle, n-pentyle, 2-pentyle, 3-pentyle, iso-pentyle, néo-pentyle, n-hexyle, n-heptyle, n-octyle et (2,4,4)-triméthylpent-2-yle ;
ou $R^3$ et $R^4$ forment ensemble avec l'atome d'azote qui les relie en tant qu'élément de cycle un radical choisi dans le groupe constitué par pyrrolidinyle, pipéridinyle et morpholinyle ;
$R^{16}$ représente un radical choisi dans le groupe constitué par $-(CH_2)-(CH_2)-C(=O)-OH$ ; $-(CH_2)-(CH_2)-C(=O)-O-CH_3$ et $-(CH_2)-(CH_2)-C(=O)-O-C_2H_5$ ;
$R^9$, $R^{13}$, $R^{31}$, $R^{32}$, $R^{33}$ et $R^{34}$ représentent chacun indépendamment les uns des autres un radical hydrogène ; ou un radical choisi dans le groupe constitué par méthyle, éthyle, n-propyle, isopropyle, n-butyle, isobutyle, 2-butyle, tert-butyle, n-pentyle, 2-pentyle, 3-pentyle, iso-pentyle, néo-pentyle, n-hexyle, n-heptyle, n-octyle, (2,4,4)-triméthylpent-2-yle, $-CF_3$, $-CF_2H$, $-CFH_2$, $-(CH_2)-(CF_3)$, $-(CH_2)-(CHF_2)$, $-(CH_2)-(CH_2F)$ et $-(CF_2)-(CF_3)$ $R^{21}$ représente un radical choisi dans le groupe constitué par méthyle, éthyle, n-propyle, isopropyle, n-butyle, isobutyle, 2-butyle, tert-butyle, n-pentyle, 2-pentyle, 3-pentyle, iso-pentyle, néo-pentyle, n-hexyle, n-heptyle, n-octyle, (2,4,4)-triméthylpent-2-yle, $-CF_3$,$-CF_2H$, $-CFH_2$, $-(CH_2)-(CF_3)$, $-(CH_2)-(CHF_2)$, $-(CH_2)-(CH_2F)$ et - $(CF_2)-(CF_3)$ ou un radical phényle, qui peut à chaque fois être non substitué ou substitué avec éventuellement 1, 2, 3, 4 ou 5 substituants choisis indépendamment les uns des autres dans le groupe constitué par F, Cl, Br, I,-CN, $-NO_2$, méthyle, éthyle, n-propyle, isopropyle, n-butyle, isobutyle, 2-butyle, tert-butyle, $-O-CH_3$ et $-0-C_2H_5$;
$M^1$ représente un radical choisi dans le groupe constitué par les radicaux 1, 3, 5, 22, 36 et 37

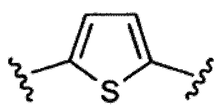
1
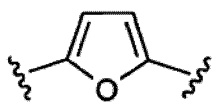
3
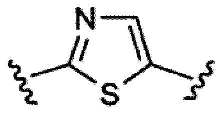
5

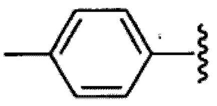
36
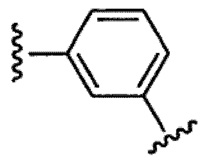
37
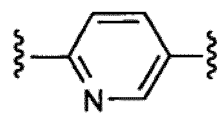
22

qui est à chaque fois non substitué, et qui peut à chaque fois être relié dans une direction quelconque aux positions **caractérisées par** une ligne ondulée avec le bicycle et l'atome de carbone de la triple liaison ;
$M^2$ représente un radical choisi dans le groupe constitué par phényle, 2-pyrimidinyle, 5-pyrimidinyle, 2-thiophényle (2-thiényle), 3-thiophényle (3-thiényle), 2-pyridinyle, 3-pyridinyle, 4-pyridinyle, 2-thiazolyle et 4-thiazolyle, qui peut à chaque fois être non substitué ou substitué avec éventuellement 1, 2, 3, 4 ou 5 substituants choisis indépendamment les uns des autres dans le groupe constitué par F, Cl, Br, -CN, -O-$CH_3$, -OH, -$CF_3$, méthyle, éthyle, n-propyle, iso-propyle, n-butyle, sec-butyle, iso-butyle et tert-butyle ;
à chaque fois éventuellement sous la forme des sels correspondants ou à chaque fois éventuellement sous la forme des solvates correspondants.

**12.** Composés selon une ou plusieurs des revendications 1 à 11, **caractérisés en ce que** $R^1$ représente un radical hydrogène ; -F ; -Cl ; -Br ;-CN ; -C(=O)-$OR^{13}$ ; ou un radical choisi dans le groupe constitué par méthyle, éthyle, n-propyle, isopropyle, n-butyle, isobutyle, 2-butyle, tert-butyle et n-pentyle ;
$R^2$ représente un radical hydrogène ; -F ; -Cl ; -Br ;-CN ; -C(=O)-$OR^{13}$ ; ou un radical choisi dans le groupe constitué par méthyle, éthyle, n-propyle, isopropyle, n-butyle, isobutyle, 2-butyle, tert-butyle et n-pentyle ;
$R^3$ représente un radical hydrogène ;
$R^4$ représente un radical hydrogène ; -C(=O)-$R^{21}$ ou un radical choisi dans le groupe constitué par méthyle, éthyle, n-propyle, isopropyle, n-butyle, isobutyle, 2-butyle, tert-butyle, n-pentyle et (2,4,4)-triméthylpent-2-yle ;
$R^{13}$ représente un radical choisi dans le groupe constitué par méthyle, éthyle, n-propyle, isopropyle, n-butyle, isobutyle, 2-butyle, tert-butyle, n-pentyle, 2-pentyle, 3-pentyle et (2,4,4)-triméthylpent-2-yle ; $R^{21}$ représente un radical choisi dans le groupe constitué par méthyle, éthyle, n-propyle, isopropyle, n-butyle, isobutyle, 2-butyle, tert-butyle, n-pentyle et (2,4,4)-triméthylpent-2-yle ou un radical phényle, qui est à chaque fois non substitué ;
$M^1$ représente un radical choisi dans le groupe constitué par les radicaux 1, 3, 5, 22 et 36

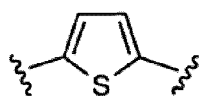
1
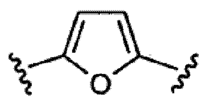
3
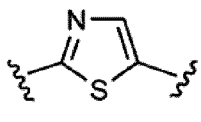
5
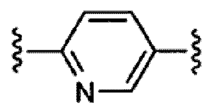
22

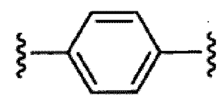
36

qui est à chaque fois non substitué et qui peut à chaque fois être relié dans une direction quelconque aux positions **caractérisées par** une ligne ondulée avec le bicycle et l'atome de carbone de la triple liaison ;

et $M^2$ représente un radical choisi dans le groupe constitué par phényle, 2-pyrimidinyle, 5-pyrimidinyle, 2-thiophényle (2-thiényle), 3-thiophényle (3-thiényle), 2-pyridinyle, 3-pyridinyle, 4-pyridinyle, 2-thiazolyle et 4-thiazolyle, qui peut à chaque fois être non substitué ou substitué avec éventuellement 1, 2, 3, 4 ou 5 substituants choisis indépendamment les uns des autres dans le groupe constitué par F, Cl, Br, -CN,-OH, $-CF_3$, méthyle, éthyle, n-propyle, iso-propyle, n-butyle, sec-butyle, iso-butyle et tert-butyle ;
à chaque fois éventuellement sous la forme des sels correspondants ou à chaque fois éventuellement sous la forme des solvates correspondants.

**13.** Composés selon une ou plusieurs des revendications 1 à 12, choisis dans le groupe constitué par

[1] 6-(5-(phényléthynyl)thiophén-2-yl)-N-(2,4,4-triméthylpentan-2-yl)-imidazo[2,1-b]thiazole-5-amine,
[2] N-tert-butyl-6-(5-(pyridin-2-yléthinyl)thiophén-2-yl)-imidazo[2,1-b]thiazole-5-amine,
[3] N-tert-butyl-3-méthyl-6-(5-(pyridin-2-yléthynyl)thiophén-2-yl)-imidazo[2,1-b]thiazole-5-amine,
[4] N-tert-butyl-2-méthyl-6-(5-(pyridin-2-yléthynyl)thiophén-2-yl)-imidazo[2,1-b]thiazole-5-amine,
[5] N-tert-butyl-2,3-diméthyl-6-(5-(pyridin-2-yléthynyl)thiophén-2-yl)-imidazo[2,1-b]thiazole-5-amine,
[6] N-tert-butyl-2-chloro-6-(5-(pyridin-2-yléthynyl)thiophén-2-yl)-imidazo[2,1-b]thiazole-5-amine,
[7] N-tert-butyl-6-(5-(pyridin-4-yléthynyl)thiophén-2-yl)imidazo[2,1-b]thiazole-5-amine,
[8] ester méthylique de l'acide 5-(tert-butylamino)-6-(5-(pyridin-2-yléthynyl)thiophén-2-yl)-imidazo[2,1-b]thiazole-2-carboxylique,
[9] N-tert-butyl-6-(5-(pyridin-2-yléthynyl)thiazol-2-yl)imidazo[2,1-b]thiazole-5-amine,
[10] 6-(5-pyridin-2-yléthynyl)thiophén-2-yl)-N-(2,4,4-triméthylpentan-2-yl)imidazo[2,1-b]thiazole-5-amine,
[11] N-tert-butyl-2-méthyl-6-(4-(pyridin-2-yléthynyl)phényl)imidazo[2,1-b]thiazole-5-amine,
[12] N-tert-butyl-6-(5-(pyridin-2-yléthynyl)furan-2-yl)imidazo[2,1-b]thiazole-5-amine,
[13] N-tert-butyl-3-méthyl-6-(5-(phényléthynyl)thiophén-2-yl)imidazo[2,1-b]thiazole-5-amine,
[14] 6-(5-(pyridin-2-yléthynyl)thiophén-2-yl)imidazo[2,1-b]thiazole-5-amine,
[15] N-tert-butyl-6-(5-(pyrimidin-2-yléthynyl)thiophén-2-yl)imidazo[2,1-b]thiazole-5-amine,
[16] N-tert-butyl-6-(5-((3-fluoropyridin-2-yl)éthynyl)thiophén-2-yl)imidazo[2,1-b]thiazole-5-amine,
[17] N-tert-butyl-6-(5-((2-fluoropyridin-4-yl)éthynyl)thiophén-2-yl)imidazo[2,1-b]thiazole-5-amine,
[18] N-tert-butyl-6-(5-(thiophén-2-yléthynyl)thiophén-2-yl)imidazo[2,1-b]thiazole-5-amine,
[19] N-tert-butyl-6-(5-(thiazol-2-yléthynyl)thiophén-2-yl)imidazo[2,1-b]thiazole-5-amine,
[20] 3-((5-(5-(tert-butylamino)imidazo[2,1-b]thiazol-6-yl)thiophén-2-yl)éthynyl)phénol,
[21] 3-((5-(5-(tert-butylamino)imidazo[2,1-b]thiazol-6-yl)thiophén-2-yl)éthynyl)benzonitrile,
[22] N-éthyl-6-(6-(phényléthynyl)pyridin-3-yl)imidazo[2,1-b]thiazole-5-amine,
[23] N-tert-butyl-6-(5-((3-méthylpyridin-2-yl)éthynyl)thiophén-2-yl)imidazo[2,1-b]thiazole-5-amine,
[24] N-(6-(5-(pyridin-2-yléthynyl)thiophén-2-yl)imidazo[2,1-b]thiazol-5-yl)acétamide et
[25] N-(6-(5-(pyridin-2-yléthynyl)thiophén-2-yl)imidazo[2,1-b]thiazole-5-yl)benzamide ;
à chaque fois éventuellement sous la forme des sels correspondants, notamment des chlorhydrates, ou à chaque fois sous la forme des solvates correspondants.

**14.** Médicament contenant au moins un composé selon une ou plusieurs des revendications 1 à 13 et éventuellement un ou plusieurs adjuvants physiologiquement compatibles.

**15.** Utilisation d'au moins un composé selon une ou plusieurs des revendications 1 à 13 pour la fabrication d'un médicament pour le traitement et/ou la prophylaxie de la douleur choisie dans le groupe constitué par la douleur aigue, la douleur chronique, la douleur neuropathique et la douleur viscérale ; de la migraine ; des dépressions ; des maladies neurodégénératives, choisies dans le groupe constitué par la sclérose en plaques, la maladie d'Alzheimer, la maladie de Parkinson et la maladie d'Huntington ; des maladies cognitives, des déficits cognitifs, du trouble déficitaire de l'attention (ADS) ; des troubles psychiatriques, choisis dans le groupe constitué par les états d'anxiété et les attaques de panique ; de l'épilepsie ; de la toux ; de l'incontinence urinaire ; de la diarrhée ; du prurit ; de la schizophrénie ; des ischémies cérébrales ; des spasmes musculaires ; des crampes ; des maladies pulmonaires, choisies dans le groupe constitué par l'asthme et la laryngite striduleuse ; de la régurgitation (vomissement) ; du manque d'énergie ; de la somnolence ; de l'atonie ; de l'inflammation du larynx (laryngite) ; des troubles de l'alimentation, choisis dans le groupe constitué par la boulimie, la cachexie, l'anorexie et l'obésité ; de la dépendance à l'alcool ; de la dépendance à des médicaments ; de la dépendance à des drogues, de préférence de la dépendance à la nicotine et/ou à la cocaïne ; de l'abus d'alcool ; de l'abus de médicaments ; de l'abus de drogues ; de l'abus de nicotine et/ou de cocaïne ; des phénomènes de sevrage en cas de dépendance à l'alcool, à des médicaments et/ou à des drogues (nicotine et/ou cocaïne) ; du développement d'une tolérance à des médicaments, à des opioïdes naturels ou synthétiques ; du syndrome du reflux gastro-oesophagien ; de la maladie du reflux gastro-oesophagien ;

du syndrome du côlon irritable ; pour la diurèse ; pour l'antinatriurèse ; pour la régulation du système cardiovasculaire ; pour l'augmentation de la vigilance ; pour l'augmentation de la libido ; pour la modulation de l'activité physique ou pour l'anesthésie locale.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 0127118 A


### In der Beschreibung aufgeführte Nicht-Patentliteratur


- Remington's Pharmaceutical Sciences. Mack Publishing Company, 1985
- **DUBUISSON, D. ; DENNIS, S.G.** *Pain,* 1977, vol. 4, 161-174
- **BENNETT ; XIE.** *Pain,* 1988, vol. 33, 87-107
- **ARMITAGE ; BERRY.** Stat. Methods in Medical Research. Blackwell Scientific Publications, 1987
- **HOGG, S.** A review of the validity and variability of the elevated plus-maze as an animal model of anxiety. *Pharmacol. Biochem. Behav,* 1996, vol. 54, 21-30
- The elevated plus-maze: pharmacology, methodology and ethology. **RODGERS, R.J. ; COLE, J.C.** Ethology and Psychopharmacology. Wiley & Sons, 1994, 9-44